# EUROPEAN PATENT SPECIFICATION

(11) **EP 2 994 535 B1**
(45) Date of publication and mention of the grant of the patent: **05.12.2018**
(21) Application number: 14725669.7
(22) Date of filing: 12.05.2014
(51) Int. Cl.: C12N 15/52, C12P 17/06, C12N 9/02, C12N 9/10

(54) **PRODUCTION OF MYXOPYRONIN AND OF ITS DERIVATIVES**
HERSTELLUNG VON MYXOPYRONIN UND DESSEN DERIVATEN
PRODUCTION DE MYXOPYRONINE ET DE SES DÉRIVÉS

(30) Priority: 10.05.2013 EP 13167392
(43) Date of publication of application: 16.03.2016
(73) Proprietor: Helmholtz-Zentrum für Infektionsforschung GmbH, 38124 Braunschweig (DE)
(72) Inventor: SUCIPTO, Hilda, 66123 Saarbrücken (DE); SAHNER, Jan Henning, 66119 Saarbrücken (DE); WENZEL, Silke, 66123 Saarbrücken (DE); GROH, Matthias, 66440 Blieskastel (DE); HARTMANN, Rolf, 66123 Saarbrücken (DE); MÜLLER, Rolf, 66123 Saarbrücken (DE)
(74) Representative: Taruttis, Stefan Georg
(86) International application number: PCT/EP2014/059677
(87) International publication number: WO 2014/181000

(56) References cited:
- ÖZLEM EROL ET AL: "Biosynthesis of the Myxobacterial Antibiotic Corallopyronin A", CHEMBIOCHEM, vol. 11, no. 9, 14 June 2010 (2010-06-14), pages 1253-1265, XP055134018, ISSN: 1439-4227, DOI: 10.1002/cbic.201000085
- KOHL W ET AL: "DIE BIOSYNTHESE DES ANTIBIOTIKUMS MYXOPYRONIN A AUS MYXOCOCCUS FULVUS STAMM MX F50", LIEBIGS ANNALEN DER CHEMIE, VERLAG CHEMIE GMBH. WEINHEIM, DE, 1 January 1985 (1985-01-01), pages 1080-1093, XP002920663, ISSN: 0170-2041
- LIRA ET AL: "Syntheses of novel myxopyronin B analogs as potential inhibitors of bacterial RNA polymerase", BIOORGANIC & MEDICINAL CHEMISTRY LETTERS, PERGAMON, AMSTERDAM, NL, vol. 17, no. 24, 17 October 2007 (2007-10-17), pages 6797-6800, XP022339575, ISSN: 0960-894X, DOI: 10.1016/J.BMCL.2007.10.017
- DOUNDOULAKIS T ET AL: "Myxopyronin B analogs as inhibitors of RNA polymerase, synthesis and biological evaluation", BIOORGANIC & MEDICINAL CHEMISTRY LETTERS, PERGAMON, AMSTERDAM, NL, vol. 14, no. 22, 15 November 2004 (2004-11-15), pages 5667-5672, XP004598614, ISSN: 0960-894X, DOI: 10.1016/J.BMCL.2004.08.045
- HILDA SUCIPTO ET AL: "Exploring Chemical Diversity of [alpha]-Pyrone Antibiotics: Molecular Basis of Myxopyronin Biosynthesis", CHEMBIOCHEM, vol. 14, no. 13, 26 August 2013 (2013-08-26), pages 1581-1589, XP055133925, ISSN: 1439-4227, DOI: 10.1002/cbic.201300289

### SEQUENCE LISTING

<110> Helmholtz-Zentrum fÃ¼r Infektionsforschung GmbH
   UniversitÃ¤t des Saarlandes
<120> Production of Myxopyronin and of its derivatives
<130> G1030PCT
<150> EP13167392
   <151> 2013-05-10
<160> 285
<170> BiSSAP 1.2
<210> 1
   <211> 798
   <212> PRT
   <213> Myxococcus fulvus
<220>
   <221> CHAIN
   <222> 1..798
   <223> MxnA
<400> 1
   Met Gln Arg Gly Pro Ala Met Thr Phe Thr Val Val Phe Pro Gly Gln
   1               5                   10                  15
   Gly Ala Gln Ser Arg Gly Met Gly Ala Ala Leu Phe Asp Lys Tyr Pro
               20                  25                  30
   Arg Leu Ile Arg Asp Ala Asp Asp Ile Leu Gly Tyr Ser Ile Arg Ser
           35                  40                  45
   Leu Cys Leu Glu Asp Pro Asp Lys Arg Leu Glu Asp Thr Ala Tyr Thr
       50                  55                  60
   Gln Pro Ala Leu Tyr Val Val Asn Ala Leu Ser Tyr Leu Gln Lys Gln
   65                  70                  75                  80
   Glu Leu Gly Leu Ala Pro Asn Tyr Ala Ala Gly His Ser Leu Gly Glu
                   85                  90                  95
   Tyr Asn Ala Leu Phe Ala Ala Gly Val Phe Asp Phe His Thr Gly Leu
               100                 105                 110
   Arg Leu Val Gln Lys Arg Gly Gln Leu Met Gly Arg Ala Arg Gly Gly
           115                 120                 125
   Gly Met Ala Ala Val Ile Gly Leu Ser Ala Glu Ala Val Gln Gln Val
       130                 135                 140
   Leu Ala Arg Glu Gly Leu Ser Gly Val Asp Ile Ala Asn Phe Asn Ala
   145                 150                 155                 160
   Pro Thr Gln Leu Val Ile Ser Gly Leu Lys Glu Asp Val Ala Arg Ala
                   165                 170                 175
   Glu Ala Pro Leu Lys Glu Ala Gly Ala Arg His Phe Val Leu Leu Arg
               180                 185                 190
   Val Ser Ala Ala Phe His Ser Arg Tyr Met Val Glu Ala Glu Lys Glu
           195                 200                 205
   Phe Thr Ser Phe Leu Asp Ala Phe Arg Phe Ala Ser Pro Arg Phe Pro
       210                 215                 220
   Val Phe Ser Asn Val His Ala Leu Pro Tyr Glu Ala Asp Arg Ile His
   225                 230                 235                 240
   Glu Thr Leu Ala Ala Gln Ile Ser Arg Pro Val Arg Trp Ala Glu Ile
                   245                 250                 255
   Val Arg Arg Leu Leu Ser Leu Asp Gly Ala Glu Phe Glu Glu Val Gly
               260                 265                 270
   Pro Gly Asn Val Leu Ala Gly Leu Leu Arg Lys Ile Arg Ala Glu Ser
           275                 280                 285
   Glu Pro Leu Pro Trp Arg Pro Ser Ser Glu Pro Gln Pro Val Ala Lys
       290                 295                 300
   Pro Ser Pro Val Ala Thr Asn Pro Ser Ala Arg Val Glu Pro Pro Val
   305                 310                 315                 320
   Val Ser Leu Glu Leu Ser Arg Pro Lys Pro Val Pro Ala Gly Arg Pro
                   325                 330                 335
   Pro Arg Leu Thr Ala Glu Ser Leu Gly Ser Ala Asp Phe Leu Arg Asp
               340                 345                 350
   Phe Gly Leu Arg Cys Ala Cys Val Ser Gly Ala Met Tyr Arg Gly Ile
           355                 360                 365
   Ala Ser Glu Ala Leu Val Ile Ala Met Gly Arg Ala Gly Phe Met Gly
       370                 375                 380
   Ile Phe Gly Ser Ala Gly Leu Ser Pro Asp Arg Val Asp Lys Ala Ile
   385                 390                 395                 400
   Gln Glu Leu Gln Val Ala Leu Gly Thr Gln Val Pro Phe Gly Met Asn
                   405                 410                 415
   Leu Leu His Asp Ile Asn His Pro Glu Thr Glu Glu Thr Met Val Asp
               420                 425                 430
   Leu Phe Leu Lys Arg Gly Val Arg Ile Val Glu Ala Ser Ala Phe Met
           435                 440                 445
   Gln Val Thr Pro Ala Leu Val Arg Tyr Arg Ala Arg Gly Leu Arg Arg
       450                 455                 460
   Glu Pro Asp Gly Ser Ile Ser Ile His His Arg Val Val Gly Lys Val
   465                 470                 475                 480
   Ser Arg Pro Glu Val Ala Gln Ala Phe Leu Ser Pro Ala Pro Glu Arg
                   485                 490                 495
   Leu Leu Glu Gln Leu Val Ala Ala Lys Lys Leu Thr Ala Glu Glu Ala
               500                 505                 510
   Arg Leu Ala Arg Ala Val Pro Met Val Asp Ala Leu Thr Val Glu Ala
           515                 520                 525
   Asp Ser Gly Gly His Thr Asp Gln Gly Val Ala Phe Ala Leu Leu Pro
       530                 535                 540
   Ala Met Met Ala Leu Arg Asp Arg Leu Ala Asp Lys His Pro Gly Ala
   545                 550                 555                 560
   Arg Arg Ile His Leu Gly Ala Ala Gly Gly Ile Gly Thr Pro Gln Ala
                   565                 570                 575
   Ala Ala Ala Ala Phe Met Met Gly Ala Asp Tyr Ile Val Thr Gly Ser
               580                 585                 590
   Ile Asn Gln Cys Thr Val Glu Ser Gly Ala Ser Ala Glu Val Lys Ala
           595                 600                 605
   Leu Leu Gln Glu Ala Gly Val Gln Asp Thr Thr Met Ala Pro Ser Gly
       610                 615                 620
   Asp Met Phe Glu Leu Gly Ala Arg Val Gln Val Leu Arg Arg Gly Leu
   625                 630                 635                 640
   Phe Phe Pro Ala Arg Ala Asn Lys Leu Tyr Glu Leu Tyr Arg Arg Tyr
                   645                 650                 655
   Asp Ala Leu Glu Gln Ile Asp Glu Lys Thr Arg Val Gln Leu Glu Thr
               660                 665                 670
   Lys Tyr Phe Lys Arg Ser Ile Ala Gln Val Tyr Glu Glu Thr Lys Ser
           675                 680                 685
   Tyr Phe Arg Val Arg Ala Pro Glu Glu Ile Glu Lys Ala Glu Ala Asn
       690                 695                 700
   Pro Lys His Lys Met Ala Leu Val Phe Arg Trp Tyr Phe Ala Tyr Ser
   705                 710                 715                 720
   Thr Arg Leu Ala Leu Ala Gly Asp Ala Glu Gly Arg Val Asp Tyr Gln
                   725                 730                 735
   Val His Ser Gly Pro Ala Met Gly Ser Phe Asn Ala Trp Val Lys Gly
               740                 745                 750
   Thr Glu Leu Glu Pro Trp Gln Asn Arg His Val Ala Arg Val Thr Ser
           755                 760                 765
   Lys Leu Met Ser Asp Thr Ala Met Phe Leu Gly Glu Arg Phe Thr Ala
       770                 775                 780
   Leu Gly Leu Tyr Gly Pro Gly Thr Gln Gln Ala Leu Ala Gly
   785                 790                 795
<210> 2
   <211> 335
   <212> PRT
   <213> Myxococcus fulvus
<220>
   <221> CHAIN
   <222> 1..335
   <223> MxnB
<400> 2
   Met Asn Asn Ser Gly Ser Phe Ser Leu Pro Phe Lys Leu Ala Gly Leu
   1               5                   10                  15
   Gly Arg Tyr Val Pro Glu Asp Ile Val Leu Ser Ser Glu Leu Glu Lys
               20                  25                  30
   Lys Tyr Asp Leu Pro Gln Gly Trp Cys Leu Glu Lys Gln Gly Ile Arg
           35                  40                  45
   Glu Arg Arg Trp Val Lys Gly Glu Thr Ala Ala Phe Met Gly Ala Glu
       50                  55                  60
   Ala Ala Lys Glu Ala Val Arg Asp Ala Gly Leu Gln Leu Ser Asp Ile
   65                  70                  75                  80
   Asp Leu Ile Ile Ser Ala Ser Gly Ser Pro Gln Gln Ala Val Pro Asp
                   85                  90                  95
   Gly Gly Pro Leu Val Gln Arg Glu Leu Gly Leu Gly Arg Ser Gly Thr
               100                 105                 110
   Pro Ala Ile Thr Val Asn Ala Ser Cys Leu Ser Phe Phe Val Ala Leu
           115                 120                 125
   Glu Val Ala Ser Asn Tyr Leu Asn Met Arg Arg Tyr Arg Arg Ile Leu
       130                 135                 140
   Val Val Ser Ala Asp Ile Ala Ser Val Ser Leu Asp Phe Arg Lys Pro
   145                 150                 155                 160
   Glu Asn Phe Thr Leu Phe Gly Asp Ala Ala Ala Ala Ala Val Val Thr
                   165                 170                 175
   Leu Pro Glu Pro Gly Glu Lys Ser Cys Ile His Ala Ser His Leu Arg
               180                 185                 190
   Thr Tyr Gly Phe Gly Ala Glu Phe Ser Glu Val Arg Gly Gly Gly Ser
           195                 200                 205
   Arg Lys Pro Pro Asn Ser Lys Asp Thr Arg Pro Glu Asp Asn Tyr Leu
       210                 215                 220
   His Met Asn Gly Ala Glu Leu Leu Lys Ile Gly Phe Glu Tyr Leu Pro
   225                 230                 235                 240
   Lys Phe Thr Glu Ser Leu Trp Lys Gln Cys Pro Asp Ile Thr Val Arg
                   245                 250                 255
   Asp Val Lys Tyr Ile Ile Pro His Gln Pro Ser Arg Val Val Leu Asp
               260                 265                 270
   Tyr Leu Ser Leu Ser Tyr Pro Glu Glu Lys Leu Ile Arg Ile Ile Glu
           275                 280                 285
   Arg Phe Gly Asn Cys Ile Gly Ala Ser Met Pro Met Ala Leu Tyr Glu
       290                 295                 300
   Ala Val Lys Leu Arg Gly Leu Gln Arg Gly Asp Lys Ala Val Leu Thr
   305                 310                 315                 320
   Gly Thr Gly Ser Gly Val Ser Phe Val Gly Met Val Phe Thr Tyr
                   325                 330                 335
<210> 3
   <211> 83
   <212> PRT
   <213> Myxococcus fulvus
<220>
   <221> CHAIN
   <222> 1..82
   <223> MxnC
<400> 3
   Met Arg Arg Glu Glu Ile Phe Ala Ile Val Lys Gly His Ala Ala Ser
   1               5                   10                  15
   Val Leu Ser Ile Ser Pro Glu Thr Val Pro Trp Glu Gly Lys Leu Val
               20                  25                  30
   Glu Leu Gly Ala Asn Ser Ile Asp Arg Leu Glu Ile Val Thr Met Thr
           35                  40                  45
   Met Glu Thr Leu Gly Leu Lys Phe Ser Leu Val Glu Leu Ser Glu Val
       50                  55                  60
   Asp Asn Ile Lys Ala Leu Val Asp Phe Phe His Glu Lys Thr Gln Arg
   65                  70                  75                  80
   Gly Ala Ala
<210> 4
   <211> 423
   <212> PRT
   <213> Myxococcus fulvus
<220>
   <221> CHAIN
   <222> 1..423
   <223> MxnD
<400> 4
   Val Ser Pro Thr Ala Ile Thr Gly Met Gly Ile Val Cys Ser Leu Gly
   1               5                   10                  15
   Gln Gly Val Asp Ala Leu Thr Glu Gly Leu Arg Ala Gly Arg Ser Gly
               20                  25                  30
   Ile Gly Phe Arg Ser Arg Ile Ala Ser Ser Leu Ala Gly Gly Ala Arg
           35                  40                  45
   Ile Gly Ala Glu Ile Arg Gly Phe Ser Phe Glu Ala Ala Leu Glu Gln
       50                  55                  60
   Cys Lys Val Arg Val Glu Pro Pro Arg Ala Arg Ala Leu Lys Leu Gly
   65                  70                  75                  80
   Arg Arg Ala Pro Phe Thr Val Gln Thr Ala Leu Val Ala Thr Leu Glu
                   85                  90                  95
   Ala Trp Ala His Ala Gly Leu Glu Thr Ala Ser Val Ala Pro Glu Arg
               100                 105                 110
   Val Gly Leu Val Val Ala Gly Ser Asn Leu Thr Gln Glu Tyr Val Phe
           115                 120                 125
   Arg Met His Glu Ala Tyr Gly Arg Asp Pro Thr Phe Ile Asn Pro Arg
       130                 135                 140
   Phe Ala Leu Gln Phe Leu Asp Thr Phe His Leu Gly Val Leu Ser Glu
   145                 150                 155                 160
   Val Leu Gly Ala Gln Gly Glu Gly Cys Thr Val Gly Gly Ala Ser Ala
                   165                 170                 175
   Ser Gly Asn Val Ala Leu Ala Arg Ala His Gln Leu Leu Ala Leu Gly
               180                 185                 190
   Leu Cys Asp Val Cys Val Val Ala Ala Pro Pro Ala Glu Leu Ser Ile
           195                 200                 205
   Leu Glu Leu Gln Ala Leu Ala Asn Thr Ser Ala Leu Cys Lys Asp Phe
       210                 215                 220
   Val Glu Glu Pro Ser Arg Thr Cys Arg Pro Phe Asp Arg Arg His Asn
   225                 230                 235                 240
   Gly Phe Val Pro Gly Glu Ala Ala Ala Cys Val Val Leu Glu Arg Glu
                   245                 250                 255
   Glu Phe Ala Arg Gln Arg Gly Ala Val Ile Gln Gly Arg Leu Leu Gly
               260                 265                 270
   Ala Ala Val Gln Leu Asp Gly Asn Ala Ser Ser Asp Pro Ser Ala Ala
           275                 280                 285
   Gly Glu Ala Arg Ala Ile Arg Gly Ala Leu Arg Gln Gly Gly Leu Glu
       290                 295                 300
   Pro Arg Ala Ile Asp Tyr Ile Asn Ala His Gly Ser Ala Ser Pro Leu
   305                 310                 315                 320
   Gly Asp Arg Thr Glu Val Glu Ala Ile Lys Ser Val Trp Gly Glu Glu
                   325                 330                 335
   Val Ala Lys Val Asp Ile Asn Ser Thr Lys Gly Leu Val Gly His Cys
               340                 345                 350
   Leu Ser Ala Ala Gly Leu Val Glu Val Ile Ala Thr Val Ser Gln Met
           355                 360                 365
   Arg His Gly Phe Val His Pro Asn Ala Asn Leu Glu Glu Pro Ile Asp
       370                 375                 380
   Gly Gln Cys Leu Phe Val Gly Gly Ser Ala Arg Arg Arg Glu Leu Arg
   385                 390                 395                 400
   His Ala Val Ser Asn Ser Phe Gly Phe Gly Gly Ile Asn Ser Cys Val
                   405                 410                 415
   Val Val Gly Glu Ala Arg Thr
               420
<210> 5
   <211> 410
   <212> PRT
   <213> Myxococcus fulvus
<220>
   <221> CHAIN
   <222> 1..410
   <223> MxnE
<400> 5
   Met Arg Val Gly Ile Glu Arg Met Asn Val Tyr Gly Gly Ala Ala Ala
   1               5                   10                  15
   Ile Gly Val Arg Asp Leu Phe Val Ala Arg Gly Leu Asn Leu Gln Arg
               20                  25                  30
   Phe Asp Asn Leu Met Met Arg Glu Lys Ser Val Gly Leu Pro Trp Glu
           35                  40                  45
   Asp Pro Val Thr His Ala Val Asn Ala Ala Arg Pro Leu Ile Asp Arg
       50                  55                  60
   Leu Ser Glu Ala Glu Lys Ala Arg Ile Glu Met Val Val Thr Ala Ser
   65                  70                  75                  80
   Glu Ser Gly Ile Asp Phe Gly Lys Ser Ile Ser Thr Tyr Val His His
                   85                  90                  95
   His Leu Gln Leu Ser Arg Arg Cys Lys Leu Phe Glu Val Lys Gln Ala
               100                 105                 110
   Cys Tyr Gly Gly Thr Ala Ala Leu Gly Ile Ala Val Asn His Val Ile
           115                 120                 125
   Ala His Ala Ala Tyr Asp Pro Lys Val Leu Val Ile Ala Thr Asp Val
       130                 135                 140
   Ala Arg Ser Ala Asp Lys Leu His Tyr Ala Glu Pro Ser Gln Gly Ser
   145                 150                 155                 160
   Gly Ala Val Ala Phe Leu Val Gly Thr Ser Pro Glu Val Leu Glu Ile
                   165                 170                 175
   Asp Val Gly Ala Thr Gly Phe His Gly Tyr Glu Val Met Asp Thr Cys
               180                 185                 190
   Arg Pro Ala Tyr Asp Ile Glu Thr Gly Asn Pro Asp Leu Ser Leu Phe
           195                 200                 205
   Ser Tyr Leu Asp Cys Leu Glu Arg Cys Phe Glu Ala Tyr Ala Asp Arg
       210                 215                 220
   Val Glu Gly Ala Asp Val Val Lys Thr Phe Glu Ala Leu Ala Phe His
   225                 230                 235                 240
   Thr Pro Phe Gly Gly Met Val Lys Gly Ala His Arg Lys Leu Leu Arg
                   245                 250                 255
   Lys Phe Ala Gly Leu Asp Ser Gly Ala Ile Glu Arg Asp Phe Ala Ala
               260                 265                 270
   Arg Val Gly Pro Ser Leu Glu Tyr Cys Val Arg Val Gly Asn Leu Tyr
           275                 280                 285
   Ser Ala Ser Leu Tyr Leu Ala Leu Ser Ser Leu Val Asp Asn Val Arg
       290                 295                 300
   Ser Ala Leu Pro Met Arg Val Gly Leu Phe Ser Tyr Gly Ser Gly Cys
   305                 310                 315                 320
   Ser Ser Glu Phe Phe Ser Gly Leu Leu Gly Pro Ala Ser Arg Arg Val
                   325                 330                 335
   Met Ala Glu Met Asn Ile Gly Ala Arg Leu Asp Arg Arg Cys Arg Leu
               340                 345                 350
   Ser Met Glu Glu Tyr Asp Ala Leu Leu Asp Ser Ala Gly Ser Phe Pro
           355                 360                 365
   Phe Gly Ala Lys Asp Leu Glu Val Gly Val Ser Arg Phe Gly Arg Ile
       370                 375                 380
   Tyr Glu Glu Gly Phe Ala Gly Glu Lys Arg Leu Val Leu Arg Arg Ile
   385                 390                 395                 400
   Arg Asp Tyr His Arg Glu Tyr Glu Trp Ser
                   405                 410
<210> 6
   <211> 253
   <212> PRT
   <213> Myxococcus fulvus
<220>
   <221> CHAIN
   <222> 1..253
   <223> MxnF
<400> 6
   Met Ser Thr Pro Val Ile Leu Arg Gln Arg Asp Glu Leu Ser Leu Arg
   1               5                   10                  15
   Leu Thr Phe Asn Arg Val Glu Ala Arg Asn Ser Ile Asn Gln Ala Leu
               20                  25                  30
   Leu Val Ala Leu Asn Glu Ala Leu Asp Glu Ala Glu His Ser Lys Glu
           35                  40                  45
   Thr Lys Val Val Val Leu Ser Ala His Gly Asp Thr Phe Ser Ala Gly
       50                  55                  60
   Met Asp Phe Thr Glu Leu Met Glu Met Ser Ala Ile Ser Ala Leu Tyr
   65                  70                  75                  80
   Phe Arg Thr Leu Gln Arg Leu Ala Thr Phe Pro Lys Leu Val Val Ser
                   85                  90                  95
   Leu Val Asp Gly Lys Val Leu Ala Gly Gly Val Gly Leu Val Ala Ala
               100                 105                 110
   Ser Asp Leu Val Leu Ala Thr Pro Ala Ala Thr Phe Ser Leu Pro Glu
           115                 120                 125
   Ile Leu Trp Gly Leu Leu Pro Ala Met Val Leu Pro Phe Leu Ala Arg
       130                 135                 140
   Arg Val Gly Met His Glu Ala Tyr Thr Leu Ala Leu Thr Ala Arg Gln
   145                 150                 155                 160
   Leu Thr Ala Glu Gln Ala Leu Ala Leu Arg Leu Val Asp Glu Leu Gly
                   165                 170                 175
   Glu Asp Ala Glu Arg Ala Leu His Arg Leu Leu Leu Arg Phe Lys Arg
               180                 185                 190
   Ile Pro Ala Glu Gly Ile His Gln Leu Lys Arg Tyr Val Asn Glu Ala
           195                 200                 205
   Phe Pro Ile Asp Glu Arg Val Glu Arg Leu Ala Val Arg Thr Thr Ser
       210                 215                 220
   Gly Leu Ala Gln Asp Pro Glu Val Arg Arg Arg Ile His Asp Phe Leu
   225                 230                 235                 240
   Thr His Gln Arg Leu Pro Trp Glu Gly Glu Val Gly Arg
                   245                 250
<210> 7
   <211> 254
   <212> PRT
   <213> Myxococcus fulvus
<220>
   <221> CHAIN
   <222> 1..254
   <223> MxnG
<400> 7
   Met Asn Pro Ser Val Thr Leu Thr Arg Arg Gly Ala Ile Gly Val Val
   1               5                   10                  15
   Ala Leu Glu Asp Arg Glu Ser Arg Asn Thr Phe Ser Pro Ala Phe Val
               20                  25                  30
   Gln Asp Leu Lys Arg Thr Phe Asp Thr Ile Ala Arg Met Pro Glu Ile
           35                  40                  45
   His Val Val Val Val His Gly Phe Asp Asn Tyr Phe Cys Cys Gly Gly
       50                  55                  60
   Thr Lys Glu Glu Leu Ile Ala Leu Val Glu Gly Val Arg Ser Gly Asp
   65                  70                  75                  80
   Ala Gln Gly Ala Ser Leu Asp Gly Leu Leu Phe Tyr Asp Leu Phe Leu
                   85                  90                  95
   Arg Cys Glu Val Pro Val Ile Ala Ala Met Gln Gly His Ala Ile Gly
               100                 105                 110
   Gly Gly Leu Ala Leu Gly Leu Phe Ala Asp Ile Val Ile Leu Ala Glu
           115                 120                 125
   Glu Ser Ile Tyr Ser Ala Val Phe Met Lys Tyr Gly Phe Thr Pro Gly
       130                 135                 140
   Met Gly Ser Thr Tyr Ile Leu Pro Lys Arg Met Gly Glu Val Leu Gly
   145                 150                 155                 160
   Ala Glu Met Leu Phe Thr Ala Arg Asn Tyr Tyr Gly Leu Glu Leu Lys
                   165                 170                 175
   Ala Arg Gly Ala Gln Val Gln Val Glu Lys Lys Ala Glu Val Val Lys
               180                 185                 190
   Val Ala Leu Arg Ile Ala Ala Glu Leu Ala Glu Lys Pro Arg Leu Ser
           195                 200                 205
   Leu Ser Leu Leu Lys Ala His Leu Thr Gln Thr Thr Arg Gln Glu Leu
       210                 215                 220
   Gln Arg Val Ile Ala Gly Glu Arg Ala Met His Arg Gln Ser Phe Ala
   225                 230                 235                 240
   Gln Pro Glu Val Arg Glu Lys Ile Glu Lys Leu Phe Gly Thr
                   245                 250
<210> 8
   <211> 286
   <212> PRT
   <213> Myxococcus fulvus
<220>
   <221> CHAIN
   <222> 1..286
   <223> MxnH
<400> 8
   Met Ser Thr Ala Tyr Glu Leu Val Gly Arg Thr Ala Phe Val Ile Ala
   1               5                   10                  15
   Asn Trp Arg Ala Glu Glu Thr Glu Ala Ala Ser Pro Leu Phe Ser Asp
               20                  25                  30
   His Val Ala Asn Ala Phe Leu Asn Asp Glu Ala Thr Gln Ala Ser Asn
           35                  40                  45
   Ala Ile Ala Ser Ala Ser Pro Ser Thr Arg Tyr Leu Val Arg Tyr Arg
       50                  55                  60
   Thr Arg Tyr Phe Asp Gln Ile Phe Leu Glu Lys Met Glu Ala Gly Val
   65                  70                  75                  80
   Arg Gln Phe Leu Ile Leu Gly Ser Gly Leu Asp Thr Arg Pro Ile Arg
                   85                  90                  95
   Leu Gly Thr Glu Gly Val Arg Phe Phe Glu Val Glu Gln Gly His Val
               100                 105                 110
   Leu Glu Phe Lys Arg Lys Gln Leu Glu Met His Gly Tyr Ala Pro Ala
           115                 120                 125
   Ser Thr Phe Val Pro Ala Glu Tyr Thr Ser Val Asp Phe Leu Glu Leu
       130                 135                 140
   Leu Ala Arg Gln Gly Phe Asp Phe Glu Ala Gln Thr Phe Ile Leu Trp
   145                 150                 155                 160
   Glu Gly Asn Val Phe Tyr Leu Glu Tyr Glu Ser Ala Val Lys Val Leu
                   165                 170                 175
   Thr Ser Leu Arg Glu Arg Leu Lys Arg Phe Glu Ile Thr Phe Asp Tyr
               180                 185                 190
   Leu Ser Lys Lys Leu Ile Thr Lys Ser Thr Gly Tyr Arg Lys Ser Glu
           195                 200                 205
   Ala Leu Leu Asp Gly Phe Arg Gln Leu Gly Ala Pro Trp Asn Thr Gly
       210                 215                 220
   Phe Asp Asp Ile Arg Glu Leu Ala Arg Thr Val Asp Leu Glu Val Ala
   225                 230                 235                 240
   Gly Asn Phe Leu Ile Ala Asp Tyr Ala Asn Glu Ala Gly Leu Glu Met
                   245                 250                 255
   Lys Val Asp Arg Ser Leu Phe Asp Asp Tyr Ser Ile Ala Thr Phe Ala
               260                 265                 270
   Asn Ala Ala Gly Arg Leu Gly Pro Val Ser Arg Gly His Ser
           275                 280                 285
<210> 9
   <211> 3906
   <212> PRT
   <213> Myxococcus fulvus
<220>
   <223> mxnI
<220>
   <221> CHAIN
   <222> 1..3906
   <223> MxnI
<220>
   <221> CHAIN
   <222> 1..530
   <223> MxnI: LM
<220>
   <221> CHAIN
   <222> 1..466
   <223> MxnI: LM
<220>
   <221> CHAIN
   <222> 1..1781
   <223> MxnI: 1E
<220>
   <221> CHAIN
   <222> 1..1759
   <223> MxnI: 1E
<220>
   <221> CHAIN
   <222> 694..885
   <223> MxnI-KS-lE
<220>
   <221> CHAIN
   <222> 1945..2135
   <223> MxnI-KS-2E
<220>
   <221> CHAIN
   <222> 2903..3050
   <223> MxnI-KR-2E
<220>
   <221> CHAIN
   <222> 424..433
   <223> MxnI-CP-L
<220>
   <221> CHAIN
   <222> 1720..1726
   <223> MxnI-CP-lE
<220>
   <221> CHAIN
   <222> 3192..3198
   <223> MxnI-CP-2E
<220>
   <221> CHAIN
   <222> 366..372
   <223> MxnJ-CP-3E
<220>
   <221> CHAIN
   <222> 533..3717
   <223> part of MxnI: 1E-6E
<220>
   <221> CHAIN
   <222> 467..3717
   <223> part of MxnI: 1E-6E
<220>
   <221> CHAIN
   <222> 1786..3717
   <223> part of MxnI: 2E-6E
<220>
   <221> CHAIN
   <222> 1760..3717
   <223> part of MxnI: 2E-6E
<220>
   <221> CHAIN
   <222> 2402..2414
   <223> MxnI-DH-2E
<220>
   <221> CHAIN
   <222> 53..65
   <223> MxnJ-DH-3E
<220>
   <221> CHAIN
   <222> 1091..1103
   <223> MxnJ-DH-4E
<400> 9
   Met Ser Thr Pro Gly Met Ser Glu Val Arg Ala Gly Glu Val Val Leu
   1               5                   10                  15
   Val Leu Ala Arg Arg Asp Asp Leu His Ala Lys Leu Val Glu Gln Ala
               20                  25                  30
   Lys Arg Gly Gly Pro Leu Ala Ser Ala Thr Phe Val Leu Val Lys Pro
           35                  40                  45
   Gly Arg Leu Phe Arg Arg Leu Gly Glu Arg Leu Phe Gln Ile Ala Pro
       50                  55                  60
   Asp Ser Ala Ala His Phe Gly Arg Leu Leu Ser Glu Val Glu Ala Ala
   65                  70                  75                  80
   His Gly Arg Val His Thr Leu Leu His Leu Trp Asn Tyr Glu Ala Trp
                   85                  90                  95
   Asn Val Asp Tyr Val Gly Arg Arg Asn Leu Lys Ser Leu Thr Asp Ser
               100                 105                 110
   Leu Ala Gly Asn Trp Ser Phe Ala Leu Gly Ala Val Glu Lys Leu Arg
           115                 120                 125
   Gly Ala Leu Arg Asp Pro Gly Gly Arg Asp Pro Val Arg Met Leu Tyr
       130                 135                 140
   Val His His Gly Asp Gly Leu Glu Leu Gln Pro His Asn Val Ile Val
   145                 150                 155                 160
   Gly Ala Asp Thr Glu Ala Gly Pro Glu Leu Val Leu Arg Gly Leu Gln
                   165                 170                 175
   Val Glu Lys Arg Leu Pro Asp Leu Asp Glu Ala Ser Arg Leu Leu Leu
               180                 185                 190
   Ala Ala Leu Asp Ser Val Val Arg Gly Glu Pro Val Val Gly Arg Pro
           195                 200                 205
   Leu Gln Ala Ala Leu Pro Glu Ala Glu Arg Ile Ser Cys Arg Arg Gly
       210                 215                 220
   Gly Val Tyr Leu Val Val Asp Pro Ser Gly Gly Ala Ser Lys Glu Trp
   225                 230                 235                 240
   Val Glu Gln Leu Thr Ala Arg His Gly Cys Gln Val Met Leu Val Gln
                   245                 250                 255
   Ala Gly Met Leu Glu Asp Phe Glu Thr Leu Arg Ser Phe Val Arg Ala
               260                 265                 270
   Lys Val Lys Asn Asp Phe Gly Arg Leu Asp Gly Val Ile His Phe Leu
           275                 280                 285
   Asp Ala Ala Gln Glu Ile Ser Arg Ser Leu Asp Glu Val Leu Ala Leu
       290                 295                 300
   Asp Asp Ile Thr Ala Glu Leu Pro Leu Asp Phe Phe Thr Leu Ser Val
   305                 310                 315                 320
   Pro Arg Ala Ser Ala Gly Met Thr Arg Glu Leu Ala Arg Ala Phe Ser
                   325                 330                 335
   Gln Leu Arg Ala Ser Leu Thr Gly Ala Gly Arg Arg Ala Gly Glu Ser
               340                 345                 350
   Leu Phe Val Ala Gly Ala Pro Arg Val Glu Val Arg Glu Ala Thr Asp
           355                 360                 365
   Ala Gln Ala Ser Ala Gly Ala Thr Arg Val Asp Phe Glu Ser Glu Glu
   370 375 380
   Thr Arg Arg Glu Val Leu Arg Leu Leu Thr Ala Glu Val Leu Gly Ile
   385 390 395 400
   Val Ala Glu Thr Leu Ser Val Pro Val Ala Ser Ile Ser Leu Asp Leu
   405 410 415
   Ser Met Asp Leu Asp Ser Ala Gly Gly Ala His Phe Asp Ser Ile Ser
   420 425 430
   Leu Thr Arg Leu Ala Asp Arg Ile Gly Thr Lys Leu Gly Phe Phe Leu
   435 440 445
   Thr Pro Val Leu Phe Phe Lys Tyr Lys Lys Leu Arg Ala Phe Ile Thr
   450 455 460
   His Leu His Glu Ala Arg Tyr Pro Ala Leu Ala Asp His Tyr Ala Leu
   465 470 475 480
   Arg Leu Val Thr Arg Arg Ala Pro Val Glu Ala Leu Ala Pro Gln Val
   485 490 495
   Val Glu Ser Ser Pro Ala Ala Val Ala Ser Pro Ala Ala Thr Ser Glu
   500 505 510
   Lys Gly Trp Gly Trp Leu Glu Val Ser Pro Ser Gln Glu Ser Ala Asp
   515 520 525
   Gly Pro Met Asp Val Ala Ile Ile Gly Val Ser Gly Arg Phe Pro Gly
   530 535 540
   Ala Gln Asp Val Phe Gly Phe Trp Lys Thr Leu Thr Ser Gly Arg Asp
   545 550 555 560
   Thr Val Thr Glu Ile Pro Ala Glu Arg Trp Asp Trp Arg Ala Trp Phe
   565 570 575
   Gly Glu Pro Ser Arg Glu Ala Asn Lys Thr His Ile Lys Trp Gly Ser
   580 585 590
   Phe Leu Asp Gly Ile Asp Gln Phe Asp Pro Ala Phe Phe Gly Ile Ser
   595 600 605
   Glu Arg Glu Ala Val Ile Met Asp Pro Ser Gln Arg Leu Phe Leu Glu
   610 615 620
   Thr Thr Trp Arg Leu Leu Glu Asp Ala Gly Tyr Arg Ala Ser Glu Leu
   625 630 635 640
   Ala Gly Ser Arg Thr Gly Val Phe Ile Gly Ala Gly Leu Pro Glu Tyr
   645 650 655
   Asp Glu Leu Tyr Arg Ala Ser Gly Asn Ala Ile Glu Glu Gln Ser Ser
   660 665 670
   Thr Gly Arg Ser Asn Ser Ile Leu Ala Asn Arg Val Ser Phe Phe Phe
   675 680 685
   Asp Phe Gln Gly Pro Ser Glu Ala Val Asp Ala Ala Cys Ala Ser Ser
   690 695 700
   Leu Val Ala Leu His Arg Ala Leu Gln Ser Leu Arg Ser Glu Glu Cys
   705 710 715 720
   Ser Met Ala Ile Ala Gly Gly Val His Leu Ile Leu Ser Pro Ala Met
   725 730 735
   His Val Ser Phe Gly Arg Gly Gly Val Leu Ala Ala Asp Gly Lys Cys
   740 745 750
   Arg Ala Phe Asp Lys Ala Ala Ala Gly Tyr Thr Arg Ala Glu Gly Ile
   755 760 765
   Gly Ala Val Leu Leu Lys Pro Leu Arg Lys Ala Arg Glu Asp Gly Asp
   770 775 780
   Asn Ile Tyr Ala Val Val Arg Ala Ser Ala Val Ser His Gly Gly Arg
   785 790 795 800
   Ser Asn Ser Met Thr Ser Pro Asn Pro Asn Ala Gln Glu Ala Leu Leu
   805 810 815
   Leu Asp Ile Tyr Gly Arg Gln Lys Val Asp Pro Arg Ser Ile Gln Tyr
   820 825 830
   Val Glu Ala His Gly Thr Gly Thr Ser Phe Gly Asp Ser Ile Glu Phe
   835 840 845
   Thr Ser Leu Ala Ser Ala Phe Glu Arg Ala Ala Ala Ala Arg Gly Pro
   850 855 860
   Val Lys Trp Thr Pro Gln Ser Cys Gly Val Gly Ser Leu Lys Ser Asn
   865                 870                 875                 880
   Ile Gly His Ala Glu Glu Ala Ala Gly Ile Ala Gly Val Leu Lys Val
                   885                 890                 895
   Ile Leu Gly Met Arg Tyr Gly Arg Leu Pro Gly Asn Leu His Phe Ser
               900                 905                 910
   Ala Leu Asn Pro Tyr Ile His Leu Glu Gly Ser Pro Phe Tyr Val Val
           915                 920                 925
   Asp Arg Ala Arg Glu Trp Pro Arg Pro Gly Gly Gly Val Arg Arg Gly
       930                 935                 940
   Ala Val Ser Ala Phe Gly Phe Gly Gly Ala Leu Ala His Val Val Leu
   945                 950                 955                 960
   Glu Glu Val Ala Ser Arg Gly Glu Ala Gly Thr Glu Ala Gln Gly Arg
                   965                 970                 975
   Pro Tyr Leu Val Pro Leu Ser Ala Ser Ser Asp Gly Val Leu Arg Ala
               980                 985                 990
   Gln Ala Glu Met Leu Arg Ser Phe Leu Glu Asp Glu Leu Ser Ala Gly
           995                 1000                1005
   Leu Ala Leu Arg Asp Leu Ala Phe Thr Leu Gln Val Gly Arg Glu Pro
       1010                1015                1020
   Met Ser His Arg Leu Ala Leu Val Ala Ala Ser Arg Ala Glu Val Val
   1025                1030                1035                1040
   Ser Ala Leu Ser Ala Phe Leu Ser Gly Ala Pro Arg Ala Arg Gly Val
                   1045                1050                1055
   Phe Thr Gly Gln Val Ser Lys Arg Ser Glu Val Gly Thr Arg Gly Ile
               1060                1065                1070
   Glu Glu Thr Leu Thr Ala Gly His Val Glu Glu Leu Gly Gln Ala Trp
           1075                1080                1085
   Val Ala Gly His Glu Leu Asp Trp Arg Arg Leu Tyr Thr Arg Ser Thr
       1090                1095                1100
   Pro Arg Arg Val Ser Leu Pro Thr Tyr Pro Phe Glu Lys Arg Arg Tyr
   1105                1110                1115                1120
   Trp Phe Thr Glu Arg Arg Pro Ser Gly Gly Gly Gly Gly Gly Gly Gly
                   1125                1130                1135
   Leu Ala Pro Glu Thr Arg Lys Lys Ala Pro Val Gly Trp Ile Glu Thr
               1140                1145                1150
   Val Leu Asn Tyr Pro Arg Glu Lys Thr Phe Ala Glu Leu Phe Thr Glu
           1155                1160                1165
   Arg Ala Arg Glu Ala Gly Glu Arg Val Ala Ala Leu Cys Ala Gly Arg
       1170                1175                1180
   Ser Ile Thr Tyr Gly Glu Leu Asp Ala Arg Ser Ser Leu Val Ala Ala
   1185                1190                1195                1200
   Ser Leu Asn His Leu Gly Val Thr Arg Gly Thr Pro Val Gly Ile Phe
                   1205                1210                1215
   Phe Asp Arg Ser Leu Glu Met Leu Val Ser Leu Leu Gly Val Leu Lys
               1220                1225                1230
   Ser Gly Gly Thr Tyr Val Pro Leu Asp Pro Thr Phe Pro Asp Glu Arg
           1235                1240                1245
   Leu Arg Tyr Ile Ala Gln Cys Ser Gly Leu Gly Phe Leu Leu Thr Asn
       1250                1255                1260
   Arg Leu Ala Ser Gly Ser Pro Pro Trp Phe Ser Gly Arg Val Ile Pro
   1265                1270                1275                1280
   Val Gly Glu Ser Phe Phe Glu Glu Ala Arg Ala Arg Phe Gly Gly Thr
                   1285                1290                1295
   Trp Gln Gln Ala Ala His Ala Arg Ala Thr Pro Glu Asp Val Ala Tyr
               1300                1305                1310
   Ile Leu Phe Thr Ser Gly Ser Thr Gly Glu Pro Lys Gly Ile Gln Val
           1315                1320                1325
   His Gln Arg Gly Leu Thr His Phe Leu Cys Ala Met Glu Arg Leu Leu
       1330                1335                1340
   Gly Met Thr Ala Asp Ala His Leu Leu Ala Val Thr Thr Ile Cys Phe
   1345                1350                1355                1360
   Asp Ile Ala Gly Leu Glu Leu Phe Leu Pro Leu Leu Cys Gly Gly Arg
                   1365                1370                1375
   Val Glu Ile Leu Gln Glu Glu Ser Cys Lys Asp Gly Leu Arg Leu Lys
   1380 1385 1390
   Asp Ala Leu Asp Arg Gly Ala Ala Thr Phe Met Gln Ala Thr Pro Ala
   1395 1400 1405
   Thr Trp Gln Met Leu Val Ser Ser Gly Trp Arg Gly Lys Pro Ser Leu
   1410 1415 1420
   Thr Ala Leu Cys Gly Gly Glu Ala Leu Ser Arg Glu Leu Ala Asp Ala
   1425 1430 1435 1440
   Leu Leu Gly Arg Val Ala Gly Leu Trp Asn Leu Tyr Gly Pro Thr Glu
   1445 1450 1455
   Thr Thr Ile Trp Ser Thr Leu Trp Lys Val Val Pro Gly Glu Glu Ile
   1460 1465 1470
   Ser Ile Gly Gly Pro Ile Gly Asn Thr Lys Leu Tyr Ile Leu Asp Glu
   1475 1480 1485
   His Asn Ala Pro Val Pro Val Gly Gln Thr Gly Glu Leu Cys Ile Gly
   1490 1495 1500
   Gly Asp Gly Val Ala Lys Gly Tyr Leu His Lys Pro Glu Leu Thr Ala
   1505 1510 1515 1520
   Gln Arg Phe Ile Gln Asn Pro Phe Ile Ala Gly Gly Asp Glu Arg Ile
   1525 1530 1535
   Tyr Lys Thr Gly Asp Leu Ala His Val Arg Ser Asp Gly Ala Leu Met
   1540 1545 1550
   Tyr Ala Gly Arg Ile Asp Asn Gln Val Lys Leu Arg Gly Tyr Arg Ile
   1555 1560 1565
   Glu Leu Glu Glu Ile Glu Lys Val Leu Gln Ala Val Ala Ser Ile Thr
   1570 1575 1580
   Lys Ala Val Val Val Val Arg Ala Asp Arg Leu Gly Gln Gln Gly Leu
   1585 1590 1595 1600
   His Ala Phe Cys Leu Ser Glu Gly Glu Ala Ala Leu Ser Val Ala Glu
   1605 1610 1615
   Leu Gln Ala Arg Leu Gly Gln Arg Leu Pro Glu Tyr Met Val Pro Thr
   1620 1625 1630
   Thr Phe Tyr Arg Met Arg Ser Phe Pro Met Thr Leu Asn Ala Lys Val
   1635 1640 1645
   Asp Arg Lys Ala Leu Ala Gly Leu Glu Leu Asp Glu Leu Leu Arg Leu
   1650 1655 1660
   His Gly Ala Gly Pro Glu Arg Thr Glu Ala Arg Ala Ala Thr Val Ser
   1665 1670 1675 1680
   Pro Gly Asp Gly Ile Thr Ala Val Ser Ser Val Glu Thr Val Leu Arg
   1685 1690 1695
   Glu Ala Ala Ala Val Val Leu Gly Ile Pro Ser Glu Arg Leu Glu Pro
   1700 1705 1710
   Ala Ala Thr Phe Ala Gln Ser Gly Phe Thr Ser Val Thr Leu Thr Ala
   1715 1720 1725
   Leu Arg Ala Arg Ile Leu Glu Arg Leu Ser Ile Gln Val Asp Pro Pro
   1730 1735 1740
   Val Leu Phe Lys Tyr Pro Ser Leu Arg Ala Leu Gly Ala His Leu Thr
   1745 1750 1755 1760
   Glu Arg Leu Gly Ser Arg Ser Glu Ser Pro Leu Val Thr Ala Pro Val
   1765 1770 1775
   Arg Glu Glu Gly Pro Glu Pro Ile Ala Val Ile Gly Met Ala Ser Arg
   1780 1785 1790
   Phe Pro Gly Ser Glu Asp Pro Glu Gly Phe Phe Glu Gly Leu Leu Ser
   1795 1800 1805
   Gly Lys Asp Ser Ile Gly Glu Ile Pro Lys Asp Arg Trp Asp Phe Arg
   1810 1815 1820
   Ala Tyr Tyr Gly Asn Pro Lys Thr Glu Lys Asn Lys Ser Asp Ala Ile
   1825 1830 1835 1840
   Trp Gly Gly Phe Ile Ser Asp Val Asp Lys Phe Asp Ala Ala Phe Phe
   1845 1850 1855
   Asn Ile Ser Pro Arg Glu Ala Arg Thr Met Asp Pro Arg Gln Arg Ile
   1860 1865 1870
   Ser Leu Glu Thr Val Tyr Lys Thr Phe Glu Asp Ala Gly Tyr Lys Pro
               1875                1880                1885
   Ala Asp Phe Ser Gly Thr Arg Thr Gly Val Phe Leu Gly Ala Ile Ser
       1890                1895                1900
   Ser Asp Tyr Trp Asp Leu Gln Arg Glu Arg Gly Val Asp Leu Glu Gly
   1905                1910                1915                1920
   Tyr Ser Leu Ser Gly Phe Ala Gly Ser Ile Val Pro Asn Arg Val Ser
                   1925                1930                1935
   Phe Phe Phe Asn Leu Leu Gly Pro Ser Glu Ala Val Asp Thr Ala Cys
               1940                1945                1950
   Ser Ser Ser Leu Val Ala Ile His Arg Ala Leu Ser Ala Leu Arg Asn
           1955                1960                1965
   Lys Glu Cys Asp Ala Ala Ile Ala Gly Gly Val Asn Leu Ile Leu Ser
       1970                1975                1980
   Pro Asn Leu Tyr Leu Ala Leu Ser Gln Asn Gly Met Leu Ser Pro Asp
   1985                1990                1995                2000
   Gly Arg Cys Lys Ala Phe Asp Gln Ser Ala Asn Gly Tyr Val Arg Gly
                   2005                2010                2015
   Glu Gly Val Gly Thr Leu Leu Leu Lys Arg Leu Ser Asp Ala Arg Arg
               2020                2025                2030
   Asp Gly Asp Ser Ile Tyr Ala Val Ile Arg Gly Ser Ala Val Asn His
           2035                2040                2045
   Gly Gly Arg Ala Asn Ser Leu Thr Ala Pro Asn Pro Asp Ala Gln Thr
       2050                2055                2060
   Gln Leu Leu Glu Asp Ala Tyr Ala Gln Ala Gln Val Asp Leu Arg Thr
   2065                2070                2075                2080
   Val Ser Phe Ile Glu Thr His Gly Thr Gly Thr Lys Leu Gly Asp Ser
                   2085                2090                2095
   Ile Glu Val Gln Ser Leu Thr Ser Ala Phe Glu Ala Ala Ser Gln Gly
               2100                2105                2110
   Lys Gly Pro Leu Arg Pro Gly Ala Tyr Cys Ala Leu Gly Ala Val Lys
           2115                2120                2125
   Thr Asn Ile Gly His Leu Glu Ala Ala Ala Gly Ile Ala Gly Val Ile
       2130                2135                2140
   Lys Ala Ile Ala Ser Leu Lys Ala Arg Arg Ile Pro Gly Asn Leu His
   2145                2150                2155                2160
   Phe His Thr Leu Asn Lys Leu Ile Asp Leu Ala Gly Ser Pro Leu Phe
                   2165                2170                2175
   Val Ala Ser Glu Ala Arg Asp Trp Thr Glu Ile Ala Pro Gly Val Pro
               2180                2185                2190
   Arg Arg Ala Gly Val Ser Ser Phe Gly Phe Gly Gly Ala Ser Ala His
           2195                2200                2205
   Ile Val Leu Glu Glu Cys Pro Glu Glu Ala Arg Gln Asp Glu Ala Gln
       2210                2215                2220
   Pro Gly Pro Val Val Ile Ala Leu Ser Ala Lys Ser Ala Gly Gln Leu
   2225                2230                2235                2240
   Arg Thr Gln Ala Glu Ala Leu Leu Met His Leu Glu Arg Met Glu Lys
                   2245                2250                2255
   Gly Ser Ser Ser Phe Leu Arg Lys Ala Arg Leu Gly Asp Val Ala Tyr
               2260                2265                2270
   Thr Leu Gln Val Gly Arg Ala Glu Met Glu Ser Arg Leu Ala Phe Val
           2275                2280                2285
   Ala Ser Thr Gly Asp Glu Val Lys Arg Gly Leu Arg Ser Phe Leu Glu
       2290                2295                2300
   Gly Gln Gly Pro Leu Gly Gln Ala Ala Ala Gly Gly Pro His Glu Arg
   2305                2310                2315                2320
   Leu Ala Arg Leu Trp Ala Ser Gly Gly Glu Val Asp Trp Ser Val Leu
                   2325                2330                2335
   His Arg Gly Pro Arg Pro Arg Arg Val Pro Leu Pro Thr Tyr Pro Phe
               2340                2345                2350
   Glu Lys Gln Arg His Trp Val Gly Glu Gly Thr Gly Gly Arg Thr Gly
           2355                2360                2365
   Ser Ala Ser Val Gln Gly Leu Gly Ala Leu Val Asp Arg Asn Glu Ser
       2370                2375                2380
   Thr Leu Asp Glu Val Val Phe Arg Lys Arg Leu Leu Pro Glu Ala Ser
   2385 2390 2395 2400
   Tyr Leu Arg Asp His Val Ile Gly Gly Lys Lys Leu Leu Pro Ala Val
   2405 2410 2415
   Val Ser Leu Glu Met Val Arg Met Ala Gly Glu Leu Ala Gly Gly Ser
   2420 2425 2430
   Gly Gln Val Ala Arg Met Lys Asn Leu Val Trp Leu Lys Pro Leu Val
   2435 2440 2445
   Val Asp Glu Ser Phe Arg Asp Ala Ala Val Arg Phe Glu Arg Lys Asp
   2450 2455 2460
   Gly Ala Val Gly Phe Lys Val Val Thr Asp Gly Ala Glu Val His Met
   2465 2470 2475 2480
   Gln Gly Gly Leu Glu Tyr Gly Leu Ala Gly Asp Thr Ser Pro Ala Arg
   2485 2490 2495
   Val Asp Val Glu Ala Leu Lys Arg Arg Cys Pro Thr Val His Asp Arg
   2500 2505 2510
   Ser Arg Phe Tyr Gly Pro Phe Ala Gly Leu Gly Tyr Gly Tyr Gly Ala
   2515 2520 2525
   Ser Leu Lys Pro Val Gln Lys Val Tyr Arg Asn Glu Arg Glu Ala Leu
   2530 2535 2540
   Val Glu Leu Gln Leu Pro Asp Gly Ala Glu Gly Gly Asp Asp Phe Val
   2545 2550 2555 2560
   Leu His Pro Ser Met Met Glu Gly Met Leu Gln Ala Leu Ala Gly Leu
   2565 2570 2575
   Leu Phe Asn Pro Glu Ser Glu Ser Asn Thr Ala Phe Leu Pro Phe Ser
   2580 2585 2590
   Met Lys Glu Leu Glu Ile Phe Gly Arg Arg Ile Pro Ala Ser Cys Phe
   2595 2600 2605
   Val His Val Ser Pro Arg Asp Glu His Ala Arg Pro Ser Leu Gln Ser
   2610 2615 2620
   Arg Phe Asp Leu Ala Leu Leu Ser Pro Ser Gly Glu Val Leu Val Arg
   2625 2630 2635 2640
   Val Gln Asp Tyr Ser Val Arg Pro Val Lys Lys Asp Asp Ala Val Arg
   2645 2650 2655
   Asp Ala Pro Pro Ser Leu Tyr Arg Ser Val Phe Val Arg Ser Glu Leu
   2660 2665 2670
   Ala Ser Gly Ala Thr Pro Ser Arg Glu Lys Gly Leu Gly Pro Val Leu
   2675 2680 2685
   Val Leu Ala Arg Asp Ala Arg Phe Val Glu Arg Leu Arg Glu Arg Leu
   2690 2695 2700
   Thr Thr Gly Pro Gly Glu Arg Pro Gln Leu Val Trp Ala Arg Pro Ala
   2705 2710 2715 2720
   Asp Arg Phe Ala Glu Arg Asp Gly Gly Leu Ala Phe Asp Phe Asp Ser
   2725 2730 2735
   Arg Arg Pro Glu Glu Leu Ala Arg Leu Leu Asp Thr Leu Arg Thr Arg
   2740 2745 2750
   Gly Leu Ala Pro Ser Val Val Ile His Gly Cys Ser Thr Ala Ala Phe
   2755 2760 2765
   Ser Tyr Gly Arg Asp Glu Leu Gln Ala Gln Leu Gln Asp Gly Ala Leu
   2770 2775 2780
   Ser Val Phe His Leu Ser Lys Ala Leu Leu Arg Glu Arg Asn Glu Arg
   2785 2790 2795 2800
   Pro Val Ser Phe Leu Tyr Val Tyr Pro Ala Thr Glu Leu Arg Pro Gln
   2805 2810 2815
   Pro Leu Ser Ala Ala Leu Thr Gly Leu Ala Arg Ser Val Arg Leu Glu
   2820 2825 2830
   Thr Glu Gly Leu Lys Leu Lys Val Val Gln Val Glu Ser Pro Glu Glu
   2835 2840 2845
   Met Ala Asp Ala Ala Ala Ser Glu Leu Ala Pro Asp Ala Asp Gly Asp
   2850 2855 2860
   Ser Phe Val Val Tyr Arg Gly Thr Ser Arg His Val Glu Thr Leu Glu
   2865 2870 2875 2880
   Pro Leu Cys Phe Asp Glu Ala Gly Ala Ala Gln Pro Arg Phe Lys Asp
   2885 2890 2895
   Gly Gly Ala Tyr Leu Ile Thr Gly Gly Ala Gly Ala Leu Gly Leu His
   2900 2905 2910
   Leu Ala Arg His Leu Thr Gln Arg Val Arg Ala Arg Val Ala Leu Cys
   2915 2920 2925
   Gly Arg Ala Arg Leu Ser Glu Ala Gln Gln Arg Glu Val Asp Gly Leu
   2930 2935 2940
   Arg Ala Leu Gly Ala Glu Val Asp Tyr Phe Val Thr Asp Val Thr Asp
   2945 2950 2955 2960
   Phe Ser Asp Val Glu Met Leu Ala Asp Thr Leu Arg Ala Arg Tyr Gly
   2965 2970 2975
   Gly Val Gln Gly Ile Ile His Ala Ala Gly Gln Arg Gln Asp Gln Leu
   2980 2985 2990
   Leu Ala Arg Lys Asp Ala Ala Gln Phe Ala Ala Val Ile Ala Pro Lys
   2995 3000 3005
   Val Phe Gly Thr Ile Asn Leu Asp Arg Ala Phe Ala Ala Glu Arg Leu
   3010 3015 3020
   Asp Phe Phe Val Val Phe Ser Ser Leu Val Ala Leu Thr Gly Asn Val
   3025 3030 3035 3040
   Gly Gln Thr Asp Tyr Ala Phe Ala Asn Ala Phe Leu Lys Gly Phe Thr
   3045 3050 3055
   Ala Phe Arg Glu Gly Leu Val Leu Lys Gly Met Arg His Gly Thr Asn
   3060 3065 3070
   His Ala Ile Ser Trp Pro Tyr Trp Lys Glu Gly Gly Met Lys Val Asp
   3075 3080 3085
   Ala Ala Thr Glu Gln Phe Ile Arg Ser Phe Phe Gly Thr Gln Pro Leu
   3090 3095 3100
   Glu Thr Ala Ala Gly Leu Arg Ala Phe Glu Glu Ile Val Gly Arg Gly
   3105 3110 3115 3120
   Ile Pro Glu Ala Leu Val Leu Ser Pro Gly Ala Val Leu Pro Ala Arg
   3125 3130 3135
   Phe Leu Lys Asp Ser Ala Val Ala Thr Gln Ala Met Val Ala Pro Val
   3140 3145 3150
   Arg His Glu Pro Ala Pro Ala Gln Arg Ala Ile Arg Glu Glu Val Arg
   3155 3160 3165
   Arg Ile Ala Ala Gly Leu Leu Gln Met Ala Pro Thr Ala Leu Asp Glu
   3170 3175 3180
   His Lys Asp Leu Ala Glu Tyr Gly Tyr Asp Ser Ile Ser Phe Thr Glu
   3185 3190 3195 3200
   Leu Ala Thr Ala Leu Asn Ala Ala Phe Gly Ala Glu Leu Thr Pro Ala
   3205 3210 3215
   Val Phe Phe Glu Leu Thr Pro Pro Thr Leu Asn Ala Leu Val Ala Tyr
   3220 3225 3230
   Leu Glu Ala Arg His Gly Ser Ser Pro Arg Ala Ser Ser Glu Ala Ala
   3235 3240 3245
   Ser Pro Ala Arg Thr Gln Ala Pro Leu Pro Leu Ala Thr Val Ala Ala
   3250 3255 3260
   Pro Ala Ser Gln Ala Ser Ser Asp Arg Ser Ala Arg Glu Asp Glu Leu
   3265 3270 3275 3280
   Ile Ala Ile Ile Gly Met Ser Gly Lys Met Pro Arg Ser Pro Ser Leu
   3285 3290 3295
   Glu Gln Phe Trp Lys Asn Leu Glu Glu Gly Arg Gln Leu Ala Arg Val
   3300 3305 3310
   Val Pro Gly Asp Arg Trp Asp Trp Arg Glu Phe His Gly Asp Ser Lys
   3315 3320 3325
   Gln Gly Arg Asn Lys Ser Val Ser Lys Trp Gly Gly Phe Leu Asp Glu
   3330 3335 3340
   Val Asp Lys Phe Asp Ala Pro Phe Phe Gly Ile Pro Ala Glu Glu Ala
   3345 3350 3355 3360
   Arg Leu Met Asp Pro Gln Gln Arg Leu Phe Leu Glu Thr Val Trp Gly
   3365 3370 3375
   Ala Ile Glu Asn Ala Gly Tyr Asp Pro Ser Ser Leu Ser Gly Ser Gln
   3380 3385 3390
   Thr Gly Leu Tyr Val Gly Val Thr Asn Arg Asp Tyr Thr Asp Val Val
           3395                3400                3405
   Glu Ser Ala Ser Glu Gly Val Arg Ser Ala Ala Thr Phe Gly Asn Ala
       3410                3415                3420
   His Ala Phe Leu Ala Asn Arg Val Ser Phe Leu Leu Asn Leu His Gly
   3425                3430                3435                3440
   Pro Ser Glu Pro Val Asp Thr Leu Cys Ser Ser Ser Leu Val Ala Ile
                   3445                3450                3455
   His Lys Ala Val Glu Asp Leu Arg Arg Gly Leu Cys Glu Leu Ala Ile
               3460                3465                3470
   Val Gly Gly Val Ser Val Ile Ala Ser Pro Arg Met Asp Ile Thr Phe
           3475                3480                3485
   Ser Glu Ala Gly Met Leu Ser Pro Ser Gly Ala Cys Arg Ser Phe Ser
       3490                3495                3500
   Gln Gly Ala Asp Gly Phe Ile Arg Gly Glu Gly Ser Gly Ala Leu Leu
   3505                3510                3515                3520
   Leu Lys Pro Leu Arg Ala Ala Glu Arg Asp Gly Asp Ser Ile Leu Gly
                   3525                3530                3535
   Val Ile Arg Ala Ala Gly Val Asn His Gly Gly Arg Ala Asn Thr Leu
               3540                3545                3550
   Thr Ala Pro Asn Gly Asn Ala Gln Val Gln Leu Leu Gln Gln Val Tyr
           3555                3560                3565
   Glu Arg Ala Asp Ile Asp Pro Arg Thr Val Ser Leu Ile Glu Ala His
       3570                3575                3580
   Gly Thr Gly Thr Glu Ile Gly Asp Ala Val Glu Leu Asn Ser Leu Lys
   3585                3590                3595                3600
   Arg Ala Phe Gln Glu Leu Ala Arg Ala Arg Gly Gly Ala Leu Gly Met
                   3605                3610                3615
   Arg Pro Val Cys Ala Val Ser Cys Val Lys Thr Asn Val Gly His Leu
               3620                3625                3630
   Glu Ser Ala Ala Gly Ile Ala Gly Val Leu Lys Val Leu Leu Ser Leu
           3635                3640                3645
   Arg His Lys Thr Leu Ala Pro Leu Ala Asn Phe His Gln Val Asn Pro
       3650                3655                3660
   Tyr Leu Asp Leu Gln Asp Ser His Phe Tyr Leu Val Thr Arg Pro Glu
   3665                3670                3675                3680
   Pro Trp Lys Glu Pro Leu Asp Ala Ala Gly Asn Arg Leu Pro Arg Arg
                   3685                3690                3695
   Ala Gly Val Ser Ser Phe Gly Ala Gly Gly Ser Asn Ala His Val Val
               3700                3705                3710
   Ile Glu Glu Tyr Leu Pro Pro Thr Arg Val Ala Glu Glu Ala Ala Val
           3715                3720                3725
   Gly Ala Gln Leu Ile Pro Leu Ser Ala Arg Asp Glu Gly Ala Leu Arg
       3730                3735                3740
   Arg Tyr Ala Ala Ala Leu Arg Glu His Val Ser Glu Leu Leu Ala His
   3745                3750                3755                3760
   Glu Ser Pro Glu Arg Ala Leu Glu Asp Leu Ala Tyr Thr Phe Gln Thr
                   3765                3770                3775
   Gly Arg Lys Ser Gln Gly Gln Arg Leu Val Ile Met Ala Gly Asp Leu
               3780                3785                3790
   Arg Gln Leu Glu Ala Ser Leu Ala Asn Phe Cys Ala Gly Ala Lys Asp
           3795                3800                3805
   Glu Arg Thr Tyr Glu Gly Val Val Gly Glu Ser Pro Pro Thr Ala Glu
       3810                3815                3820
   Gly Gly Leu Pro Ala Leu Ala Gln Arg Trp Val Arg Gly Gly Glu Val
   3825                3830                3835                3840
   Asp Trp Gln Ser Leu His Ala Gly Arg Thr Pro Ser Arg Ile Pro Ala
                   3845                3850                3855
   Pro Thr Tyr Pro Phe Glu Arg Ile Arg Tyr Trp Phe Glu Gly Asp Ala
               3860                3865                3870
   Pro Gln Ala Gly Arg Lys His Ser Ser Asn Gly Thr Ser Glu Gly Ser
           3875                3880                3885
   Arg Glu Arg Ile Ala Val Gln Ser Ala Gly Glu His Thr Thr Glu Ala
               3890                3895                3900
   Arg Ser
   3905
<210> 10
   <211> 3862
   <212> PRT
   <213> Myxococcus fulvus
<220>
   <223> MxnJ
<220>
   <221> CHAIN
   <222> 1..3862
   <223> MxnJ
<220>
   <221> CHAIN
   <222> 25..3849
   <223> part of MxnI: 1E-6E MxnI-MxnJ: 4E to 6E
<220>
   <221> CHAIN
   <222> 25..3849
   <223> part of MxnI: 2E-6E
<220>
   <221> CHAIN
   <222> 1..405
   <223> MxnJ: 3E
<220>
   <221> CHAIN
   <222> 451..2276
   <223> MxnJ: 4E
<220>
   <221> CHAIN
   <222> 2325..3849
   <223> MxnJ: 5E to 6E
<220>
   <221> CHAIN
   <222> 2277..3849
   <223> MxnJ: 5E to 6E
<220>
   <221> CHAIN
   <222> 2325..3006
   <223> MxnJ: 5E
<220>
   <221> CHAIN
   <222> 3007..3849
   <223> MxnJ: 6E
<220>
   <221> CHAIN
   <222> 3053..3849
   <223> MxnJ: 6E
<220>
   <221> CHAIN
   <222> 1..450
   <223> Mxn: 3E
<220>
   <221> CHAIN
   <222> 1..3052
   <223> MxnJ: 1E to 5E
<220>
   <221> CHAIN
   <222> 1..3006
   <223> MxnJ: 1E to 5E
<220>
   <221> CHAIN
   <222> 2238..2244
   <223> MxnJ-CP-4E
<220>
   <221> CHAIN
   <222> 2967..2973
   <223> MxnJ-CP-5E
<220>
   <221> CHAIN
   <222> 3810..3816
   <223> MxnJ-CP-6E
<220>
   <221> CHAIN
   <222> 3566..3572
   <223> MxnJ-CP*-6E
<220>
   <221> CHAIN
   <222> 1982..1987
   <223> MxnJ-MT-4E
<400> 10
   Met Thr Glu Glu Leu Asn Lys Leu Ala Pro Ser Ala His Ala Ser Glu
   1               5                   10                  15
   Ser Gln Val Pro Ser Arg Arg Lys His Gly Leu Leu Thr Arg Lys Ala
               20                  25                  30
   Leu Arg Leu Thr Gly Thr Arg Pro His Glu Gly Phe Glu Leu Gly Tyr
           35                  40                  45
   Glu Asp Ala Ala Leu Arg Asp His Thr Val Phe Gly Gln Arg Val Leu
       50                  55                  60
   Leu Gly Val Thr Tyr Ala Ser Leu Ala Leu Glu Val Gly Arg Trp His
   65                  70                  75                  80
   Phe Val Asp Arg Pro Pro Val Gly Ile Lys Asn Leu Leu Phe Leu Gln
                   85                  90                  95
   Pro Leu Val Leu Gly Gln Gly Glu Ser Ala Arg Leu Met Val Ser Leu
               100                 105                 110
   Arg Gly Ser Ser Phe Glu Val Ala Tyr His Lys Ala Pro Asp Ala Pro
           115                 120                 125
   Gly Thr Pro Cys Ala Thr Gly Glu Leu Leu Phe Asp Met Glu Gly Gly
       130                 135                 140
   Arg Glu Arg Ser Gln Gly Val Leu Asp Val Ala Arg Phe Leu Arg Glu
   145                 150                 155                 160
   Ala Glu Arg Thr Thr Glu Gly Pro Arg Val Tyr Glu Glu Met Arg Lys
                   165                 170                 175
   Val Ala Val Asp Tyr Gly Ala Ser Leu Phe Thr Ala Arg Arg Thr Phe
               180                 185                 190
   His Arg Ala Gly Glu Val Leu Gly Glu Leu Val Val Ser Asp Glu Ala
           195                 200                 205
   Ala Ala Gly Phe Glu Phe Leu Leu Pro Pro Ser Leu Leu Asp Gly Ala
       210                 215                 220
   Ile Val Ala Gly Ala Phe Glu Gln Leu Val Ala Arg Gly Lys Pro Cys
   225                 230                 235                 240
   Ile Pro Met Phe Val Glu Arg Leu Val Ile Arg Gln Ala Pro Gly Ser
                   245                 250                 255
   Arg Cys Tyr Cys His Cys Ala Val Arg Lys Ala Asn Asp Glu Val Leu
               260                 265                 270
   Val Val Asp Thr His Leu Leu Asp Glu Ala Gly Gln Pro Leu Val Glu
           275                 280                 285
   Met Thr Gly Val Thr Leu Lys Asn Val Pro Val Pro Gly Asn Pro Phe
       290                 295                 300
   Val Pro Gly Ala Trp Thr Pro Ser Pro Val Leu Pro Pro Leu Pro Pro
   305                 310                 315                 320
   Ser Phe Gln Ala Pro Ala Phe Ala Ser Asp Glu Asp Leu Ala Arg Gln
                   325                 330                 335
   Leu Glu Leu Arg Leu Arg Lys Arg Ile Ala Ala Leu Leu Asp Val Pro
               340                 345                 350
   Glu Ser Arg Ile Glu Pro Thr Arg Asn Phe Met Glu Thr Gly Leu Thr
           355                 360                 365
   Ser Leu Ser Leu Val Gly Phe Met Arg Ala Leu Gly Asp Glu Leu Gly
       370                 375                 380
   Val Ser Leu Tyr Pro Thr Leu Ala Phe Glu Phe Gln Ser Pro Arg Ser
   385                 390                 395                 400
   Leu Ala Leu His Leu Ala Gln Glu His Ala Ser Leu Leu Thr Arg Pro
                   405                 410                 415
   Arg Glu Glu Pro Ala Val Val Glu Arg Gln Arg Pro Leu Pro Val Lys
               420                 425                 430
   Val Met Glu Pro Val Thr Arg Pro Ala Ser Val Pro Asp Thr Gly Ala
           435                 440                 445
   Arg Ala Glu Pro Val Ala Ile Val Gly Leu Ala Gly Val Phe Pro Gln
       450                 455                 460
   Ser Lys Asp Leu Glu Glu Phe Trp Ala His Leu Ala Arg Gly Ala Asp
   465                 470                 475                 480
   Leu Val Thr Pro Val Pro Glu Asp Arg Pro Tyr Leu Lys Ile Asp Gly
                   485                 490                 495
   Arg Gly Ser Trp Gln Ala Ser Phe Ile Ser Asp Val Asp Lys Phe Asp
               500                 505                 510
   Ala Ala Phe Phe Gly Ile Ser Pro Arg Glu Ala Ala Leu Met Asp Pro
           515                 520                 525
   Lys Gln Arg Leu Leu Leu Glu Leu Val Trp Arg Ala Ile Glu Asp Ser
       530                 535                 540
   Gly His Arg Ala Ser Glu Leu Arg Gly Ser Arg Thr Gly Leu Phe Val
   545                 550                 555                 560
   Gly Val Gly Ala Leu Glu Tyr Gly Ala Phe Leu Gly Thr Ala Ser Glu
                   565                 570                 575
   Ala Ile Glu Ala Tyr Ser Ala Thr Gly Ile Ala Gly Thr Met Leu Pro
               580                 585                 590
   Asn Arg Val Ser Tyr Leu Met Asp Trp His Gly Pro Ser Glu Ala Ile
           595                 600                 605
   Asp Thr Ala Cys Ser Ser Ser Leu Val Ala Ile His Arg Ala Ile Glu
       610                 615                 620
   Ser Ile Gln Asn Gly Asp Cys Asp Gln Ala Leu Val Gly Gly Ile Asn
   625                 630                 635                 640
   Leu Ile Leu Ser Pro Phe Leu Thr Ile Ala Phe Ser Lys Ala Gly Met
                   645                 650                 655
   Leu Ser Gly Asp Gly Arg Cys Lys Thr Phe Asp Arg Ser Ala Asp Gly
               660                 665                 670
   Tyr Val Arg Gly Glu Gly Ala Gly Ile Leu Val Leu Lys Pro Leu Ser
           675                 680                 685
   Lys Ala Val Ala Asp Gly Asp Gln Ile His Ala Val Ile Arg Gly Ser
       690                 695                 700
   Ala Ile Asn His Gly Gly His Ala Asn Ser Leu Thr Ala Pro Asn Pro
   705                 710                 715                 720
   Lys Ala Gln Glu Gln Leu Ile Thr Gln Ala Tyr Arg Lys Ala Arg Val
                   725                 730                 735
   Asp Pro Arg Thr Val Ser Tyr Ile Glu Ala His Gly Thr Gly Thr Lys
               740                 745                 750
   Leu Gly Asp Pro Val Glu Thr Arg Ala Leu Val Asn Ala Phe Lys Ala
           755                 760                 765
   Leu Glu Gln Gly Ser Gly Gln Ala Ala Gln Pro His Arg Cys Gly Leu
       770                 775                 780
   Gly Ser Val Lys Thr Asn Ile Gly His Leu Glu Ala Ala Ala Gly Ile
   785                 790                 795                 800
   Ala Gly Val Val Lys Val Leu Leu Ser Phe Arg His Gln Lys Leu Pro
                   805                 810                 815
   Gly Thr Val His Phe Lys Ala Leu Asn Glu Phe Ile Ala Leu Glu Gln
               820                 825                 830
   Ser Pro Phe Gln Val Val Ala Glu Thr Thr Asp Trp Pro Arg Leu Ala
           835                 840                 845
   Pro Gly Ile Pro Arg Arg Ala Gly Val Ser Ser Phe Gly Phe Gly Gly
       850                 855                 860
   Val Asn Ala His Val Val Leu Glu Glu Tyr Glu Asp Ile Ala Ser Asp
   865                 870                 875                 880
   Asp Val Ser Asn Val Glu Gln Gly Pro Leu Ala Tyr Val Leu Ser Ala
                   885                 890                 895
   Lys Thr Arg Ala Ala Leu Glu Arg Gln Ala Ala Glu Leu Leu Arg Trp
               900                 905                 910
   Leu Ala Arg Arg Ser Pro Glu Gly Asp Arg Ile Arg Glu Gly Gly Trp
           915                 920                 925
   Leu Gln Ser Leu Ser His Thr Leu His Val Gly Arg Glu Val Met Pro
       930                 935                 940
   His Arg Leu Ala Val Leu Ala Ser Thr Ala Ala Glu Leu Arg Ala Lys
   945                 950                 955                 960
   Leu Gln Ala Phe Val Thr Ala Thr Pro Thr Glu Gly Leu Tyr Val Gly
                   965                 970                 975
   Ala Pro Arg Glu Glu Asp Ser Thr Val Leu Glu Ala Leu Glu Gly Ala
               980                 985                 990
   Asp Gly Gln Asp Tyr Val Arg Gly Leu Leu Arg Lys Ala Arg Phe Asp
           995                 1000                1005
   Lys Val Ala Arg Leu Trp Ala Ala Gly Leu Glu Val Asp Trp Gly Phe
       1010                1015                1020
   Leu Tyr Glu Gly Thr Thr Pro Arg Arg Leu Ser Leu Pro Thr Tyr Pro
   1025                1030                1035                1040
   Phe Glu Arg Glu Arg His Trp Asn Ala Ala Leu Leu Arg Gly Gly Ser
                   1045                1050                1055
   Ala Arg Glu Gln Val Leu His Pro Leu Val Asp Gln Val Val Pro Thr
               1060                1065                1070
   Leu Glu Gly Asn Thr Leu Arg Tyr Glu Lys Thr Leu Thr Arg Asp Glu
           1075                1080                1085
   Pro Phe Leu Arg Asp His Val Val Gly Ser Arg Gly Val Leu Pro Gly
       1090                1095                1100
   Ala Cys Tyr Leu Glu Met Ala Trp Arg Ser Phe Leu Leu Pro Glu His
   1105                1110                1115                1120
   Gln Gly Arg Arg Phe Ser Leu Ser Arg Val Val Leu Lys Gln Leu Leu
                   1125                1130                1135
   Ala Val Glu Glu Arg Cys Arg Val Ser Val Glu Leu Thr Arg Glu Gln
               1140                1145                1150
   Gly Thr Thr Ser Phe Val Ile Thr Ser His Arg Pro Glu Gly Pro Val
           1155                1160                1165
   Gln His Ala Thr Gly Glu Tyr Gln Phe Glu Ala Glu Glu Ala Ala Pro
       1170                1175                1180
   Gln Thr Leu Asp Leu Ala Ser Val Ala Glu Arg Cys Thr Gln Glu Val
   1185 1190 1195 1200
   Glu Gly Arg Glu Val Tyr Ala Arg Leu Glu Arg Ala Gly Leu Arg Tyr
   1205 1210 1215
   Gly Pro Phe Phe Arg Ser Leu Gln Arg Leu Lys Thr Asn Gly Arg Glu
   1220 1225 1230
   Ser Val Gly Trp Leu Glu Leu Ser Pro Glu Ala Thr Glu Gly Phe Ser
   1235 1240 1245
   Asp Tyr Thr Leu His Pro Ala Leu Leu Asp Gly Ala Leu His Val Val
   1250 1255 1260
   Leu Gly Gln Val Ser Val Ala Ala Gly Ala Gly Arg Ala Lys Leu Leu
   1265 1270 1275 1280
   Leu Pro Phe Val Val Asp Ala Val Glu Val Leu Gly Pro Val Ala Pro
   1285 1290 1295
   Ser Met Ala Val Tyr Val Lys Ser Glu Asp Gly Leu Ser Phe Asp Ile
   1300 1305 1310
   Ala Leu Val Gly Ala Asp Gly Arg Val Ala Ile Lys Val Tyr Gly Leu
   1315 1320 1325
   Glu Ala Arg Glu Met Ala Glu Pro His Val Pro Met Leu Tyr Ala Pro
   1330 1335 1340
   Tyr Phe Glu Ala Val Arg Val Gln Ala Val Glu Glu Arg Pro Val Glu
   1345 1350 1355 1360
   Lys Arg Gly Ala Leu Ile Val Tyr Leu Asp Gly Ala Ala Ala Phe Ala
   1365 1370 1375
   Gln Ala Trp Ser Glu Glu Leu Glu Glu Arg Tyr Ala Glu Thr Lys Ser
   1380 1385 1390
   Leu Ser Leu Arg Asp Val Glu Ala Gly Gly Leu Glu Pro Leu Leu Glu
   1395 1400 1405
   Gly Gly Phe Pro Leu Asp Ser Val Phe Phe Leu Ala Pro Gly Met Gly
   1410 1415 1420
   Arg Asp Ser Phe Glu Ala Leu Gln Ala Glu Asp Ser Arg Ala Leu Tyr
   1425 1430 1435 1440
   Cys Leu Tyr Glu Val Ala Arg Met Leu Asp Ala Arg Gly Val Leu Glu
   1445 1450 1455
   Arg Pro Leu Asp Phe Ser Val Phe Thr Trp Gly Ala Leu Lys Thr Arg
   1460 1465 1470
   Ala Asp Glu Ser Val Gln Leu Ser Gly Ala Ala Leu Gly Gly Phe Leu
   1475 1480 1485
   Arg Ser Ile Ala Gly Glu Tyr Pro Gln Cys Arg Phe Arg Val Val Asp
   1490 1495 1500
   Phe Gly Gly Glu Arg Gln Thr Asp Val Leu Arg Ser Glu Val Leu Lys
   1505 1510 1515 1520
   Arg Tyr Leu Phe Thr Glu Ala Met Glu Gly Pro Ala Glu Val Ala Leu
   1525 1530 1535
   Arg Asp Gly Ala Leu Phe Gln Arg Arg Leu Gly Val Leu Glu Ala His
   1540 1545 1550
   Gly Pro Glu Gln Pro Val Phe Arg Gln Gly Gly Ser Tyr Val Ile Val
   1555 1560 1565
   Gly Gly Thr Gly Gly Ile Gly Met Ala Leu Ala Glu His Leu Leu Arg
   1570 1575 1580
   Gly Phe Gly Ala Arg Val Ala Leu Leu Gly Arg Ser Asn Pro Gly Glu
   1585 1590 1595 1600
   Glu Gln Glu Arg Lys Phe His Ala Leu Arg Ala Leu Gly Gly Glu Val
   1605 1610 1615
   Leu Arg Val Gln Ala Asp Val Thr Asp Ile Val Ala Leu Arg Arg Ala
   1620 1625 1630
   Leu Ala Gln Val Lys Glu Arg Phe Gly Ala Ile His Gly Val Phe His
   1635 1640 1645
   Ser Ala Leu Val Leu Gln Asp Ala Ser Leu Lys Lys Leu Ser Arg Ser
   1650 1655 1660
   Ser Leu Gln Arg Val Leu Ala Pro Lys Val Ala Gly Ser Leu Val Leu
   1665 1670 1675 1680
   Ala Glu Val Leu Ala Ala Glu Gln Leu Asp Phe Leu Met Phe Phe Ser
   1685 1690 1695
   Ser Ala Val Gly Ile Ala Gly Gly Ala Gly Gln Ser Asn Tyr Ala Ala
   1700 1705 1710
   Ala Ser Arg Phe Gln Asp Ala Leu Ala Leu Ser Leu Ser Asp Ala Leu
   1715 1720 1725
   Pro Phe Pro Val Lys Val Ile Asp Trp Gly Phe Trp Gly Thr Val Gly
   1730 1735 1740
   Ala Val Ala Thr Pro Gly Tyr Asn Leu Leu Leu Arg Glu Glu Gly Phe
   1745 1750 1755 1760
   Gly Ser Ile Val Pro Glu Glu Gly Phe Arg Ala Ile Glu Gln Val Leu
   1765 1770 1775
   Gly Leu Pro Leu Thr Gln Val Val Ala Ile Lys Gly Asp Glu Arg Val
   1780 1785 1790
   Leu Gly Arg Ile Ser Ala Ser Thr Arg Thr Ser Val Ser Val Thr Arg
   1795 1800 1805
   Glu Thr His Ala Ser Thr Leu Ala Gln Val His Gln Gln Leu Gly Thr
   1810 1815 1820
   Phe Val Thr Thr Leu Ser Glu Glu Pro Ala Arg Arg Val Val Ala Gly
   1825 1830 1835 1840
   Phe Glu Leu Phe Glu Glu Tyr Ala Arg Arg Gly Leu Arg Arg Arg Leu
   1845 1850 1855
   Glu Gly Val Val Val Arg Glu Glu Glu Ile Leu Pro Glu Gln Ala Arg
   1860 1865 1870
   Leu Tyr Arg Glu Cys Gln Ala Val Arg Ala Lys Ala Pro Ala Gly Glu
   1875 1880 1885
   Arg Ser Leu Glu Glu Leu Arg Ala Ala Val Leu Glu Arg Leu Pro Asp
   1890 1895 1900
   Leu Thr Pro Tyr Val Glu Leu Leu Asp Arg Cys Val Gln Ser Leu Pro
   1905 1910 1915 1920
   Glu Phe Trp Thr Gly Arg His Ser Ala Met Asp Val Met Phe Pro Asn
   1925 1930 1935
   Gly Ser Thr Glu Leu Val Glu Lys Val Tyr Ala Ser Ser Leu His Ser
   1940 1945 1950
   Asp Leu Phe Asn Thr Leu Thr Ala Glu Ala Val Arg Ala Trp Val Glu
   1955 1960 1965
   Val Arg Leu Arg Gln Ala Pro Lys Glu Pro Leu Leu Ile Val Glu Val
   1970 1975 1980
   Gly Ala Gly Thr Gly Gly Thr Thr Arg Phe Val Leu Asp Ala Leu Arg
   1985 1990 1995 2000
   Pro Tyr Ala Glu Arg Val Arg Tyr Val Phe Ser Asp Leu Ser Val His
   2005 2010 2015
   Phe Leu Asn Lys Ala Arg Glu Arg Phe Gln His Ser Pro Val Pro Val
   2020 2025 2030
   Glu Tyr Arg Ile Leu Asp Leu Glu Arg Gly Ala Asp Met Lys Asp Val
   2035 2040 2045
   Asp Leu Val Leu Gly Thr Asn Val Leu His Ala Thr Ala Glu Leu Ala
   2050 2055 2060
   Ser Thr Leu Arg Arg Ile Lys Ala Met Leu Arg Arg Asn Gly Leu Leu
   2065 2070 2075 2080
   Met Leu Asn Glu Thr Ile Ala Ala Lys Asp Phe Ala Thr Leu Thr Phe
   2085 2090 2095
   Gly Thr Met Lys Gly Trp Trp Leu Phe Arg Asp Ala Glu Asn Arg Ile
   2100 2105 2110
   Pro Gly Ser Pro Val Val Asn Gln Glu Thr Trp Glu Arg Leu Leu Ala
   2115 2120 2125
   Gln Gly Gly Phe Arg Gly Thr Lys Ala Leu Gly Leu Pro Gly Val Pro
   2130 2135 2140
   Pro Ala His Val Trp Gln Ser Val Ile Leu Ala Glu Ser Asp Gly Val
   2145 2150 2155 2160
   Ala Val Arg Pro Arg Glu Glu Gly Val Ala Ala Leu Ala Ser Val His
   2165 2170 2175
   Pro Thr Pro Ala Val Arg Val Pro Arg Ala Pro Pro Ala Ser Val Ala
   2180 2185 2190
   Thr Ser Ala Pro Ala Arg Ser Pro Val Ala Ser Pro Ala Val Gly Gly
   2195 2200 2205
   Thr Ser Ala Tyr Val Lys Arg Val Phe Ser Glu Val Leu Lys Gln Asn
   2210 2215 2220
   Pro Ala Ala Ile Ala Glu Gly Ala Thr Phe Asp Glu Tyr Gly Val Asp
   2225 2230 2235 2240
   Ser Leu Ile Gly Leu Glu Ile Arg Arg Arg Leu Glu Gln Asp Leu Gly
   2245 2250 2255
   Lys Leu Pro Ser Thr Leu Leu Phe Glu Gln Leu Ser Val Arg Ala Leu
   2260 2265 2270
   Ala Glu Tyr Leu Glu Lys Asn Lys Gly Val Glu Leu Ala Arg Val Thr
   2275 2280 2285
   Gly Ser Ala Ser Pro Ala Ala Pro Arg Gln Glu Thr Leu Ser Phe Glu
   2290 2295 2300
   Arg Lys Pro Thr Arg Gly Ser Ala Ser Glu Glu Arg Pro Arg Gln Gly
   2305 2310 2315 2320
   Thr Glu Leu Glu Ile Ala Ile Thr Gly Leu Ser Gly Arg Tyr Pro Gly
   2325 2330 2335
   Ala Glu Thr Leu Asp Ala Leu Leu Glu Asn Leu Arg Ala Gly Arg Ser
   2340 2345 2350
   Gly Phe Ser Pro Ile Pro Ala Glu Arg Trp Gly Thr Gly Glu Gly Ser
   2355 2360 2365
   Val Arg Trp Gly Ala Phe Leu Gln Asp Val Asp Lys Phe Asp Pro Leu
   2370 2375 2380
   Phe Phe Asn Ile Ala Pro Ala Glu Ala Glu Glu Met Asp Pro Gln Glu
   2385 2390 2395 2400
   Arg Leu Met Leu Glu Leu Ser Leu His Ala Leu Glu Asp Ala Gly Tyr
   2405 2410 2415
   Pro Arg Gly Val Leu Leu Lys Gln Ala Pro Lys Ala Gly Val Phe Ile
   2420 2425 2430
   Gly Ala Met Asn Pro Asp Tyr Glu Trp Val Gly Ala Arg Ala Ser Ala
   2435 2440 2445
   Ala Gly Leu Ala Asn Arg Ala Ser Ser Arg Phe Trp Ser Ile Ala Asn
   2450 2455 2460
   Arg Ile Ser Tyr Trp Phe Asn Leu Arg Gly Pro Ser Phe Ala Val Asp
   2465 2470 2475 2480
   Ser Ala Cys Ser Ala Ser Leu Thr Ala Ile His Leu Ala Val Gln Ser
   2485 2490 2495
   Leu Ala Arg Gly Glu Cys Glu Leu Ala Ile Ala Gly Gly Ile Asn Leu
   2500 2505 2510
   Ile Leu His Pro Asp His Leu Ala Arg Leu Ala His Ala Gly Leu Leu
   2515 2520 2525
   Thr Lys Gly Glu Gln Thr Arg Ser Phe Gly Ala Gln Ala Asp Gly Phe
   2530 2535 2540
   Val Asp Gly Glu Gly Ala Gly Leu Val Val Leu Lys Pro Leu Ala Arg
   2545 2550 2555 2560
   Ala Arg Ala Asp Gly Asp Val Ile Tyr Gly Val Ile Lys Gly Ser Ser
   2565 2570 2575
   Ile Asn Ala Gly Gly Lys Thr Ser Gly Tyr Leu Thr Pro Asn Pro Arg
   2580 2585 2590
   Ala Gln Ala Asp Ala Ile Asp Glu Ala Leu Gln Arg Ala Gln Val Ser
   2595 2600 2605
   Pro Arg Thr Ile Ser Tyr Val Glu Cys Ala Ala Ala Gly Ser Ser Leu
   2610 2615 2620
   Gly Asp Ala Ile Glu Val Ser Gly Leu Lys Glu Ala Phe Ala Arg His
   2625 2630 2635 2640
   Thr Gln Glu Arg Gly Phe Cys Thr Val Gly Ser Ile Lys Ala Asn Val
   2645 2650 2655
   Gly His Leu Glu Ser Ala Ser Gly Ile Ala Ala Leu Thr Lys Val Leu
   2660 2665 2670
   Leu Gln Met Lys Thr Arg Gln Leu Phe Pro Ser Pro His Ala Asp Ala
   2675 2680 2685
   Ser Ser Pro Glu Leu Asp Leu Ser Asp Ser Pro Phe Arg Leu Gln Gly
   2690 2695 2700
   Thr Leu Ala Pro Trp Leu Pro Ile Pro Ser Arg Glu Ser Gly Ala Gln
   2705 2710 2715 2720
   Asp Ser Leu Leu Arg Ala Gly Val Ser Ala Leu Gly Ala Gly Gly Ala
   2725 2730 2735
   Asn Ala His Leu Ile Leu Glu Glu His Arg Asp Ser Arg Pro Arg Ser
   2740 2745 2750
   Ala Thr Ser Ser Pro Arg Leu Val Leu Val Pro Leu Ser Ala Lys Thr
   2755 2760 2765
   Glu Glu Arg Leu Gln Ala Ser Ala Arg Ala Leu Ala Asp His Leu Thr
   2770 2775 2780
   Arg His Arg Glu Thr Ile Phe Asp Ser Asp Val Val Ser Ile Ala Tyr
   2785 2790 2795 2800
   Thr Leu Gln Thr Ser Arg Glu Ala Leu Glu Gln Arg Val Ala Phe Val
   2805 2810 2815
   Val Ala Ser Thr Gly Glu Leu Arg Glu Ala Ile Asp Gly Phe Leu Ser
   2820 2825 2830
   Gly Arg Thr Glu Ala Arg Gln Arg Leu Arg Ala Gly Arg Ala Gly Ser
   2835 2840 2845
   Ser Gly Glu Pro Leu Ala Gln Gly Arg Gly Leu Ala Gln Thr Arg Glu
   2850 2855 2860
   Glu Leu Leu Gln Trp Gly Glu Gln Trp Val Ala Gly Ala Gln Val Glu
   2865 2870 2875 2880
   Trp Arg Ser Leu Tyr Ala Asp Gly Thr Pro Thr Arg Met Thr Leu Pro
   2885 2890 2895
   Gly Tyr Pro Phe Ala Arg Arg Arg Cys Trp Ile Ser Glu Arg Thr Pro
   2900 2905 2910
   Glu Ser Ser Val Asp Thr Val Thr Lys Pro Ala Gly Gly Gly Ser Val
   2915 2920 2925
   Gln Asp Ala Asn Ala Leu Val Ser Val Val Ser Glu Asp Leu Arg Asp
   2930 2935 2940
   Ile Val Cys Gln Val Leu Lys Val Arg Pro Asp Glu Val Ser Thr Asp
   2945 2950 2955 2960
   Asp Asp Met Ser Gln Tyr Gly Leu Asp Ser Ile Arg Val Val Gln Met
   2965 2970 2975
   Val Gly Glu Leu Asn Thr Arg Leu Gly Leu Asp Ala Thr Pro Pro Leu
   2980 2985 2990
   Phe Tyr Gly His Val Ser Ile Gln Gly Phe Ala Arg Thr Val Cys Gln
   2995 3000 3005
   Lys Phe Gly Glu Lys Leu Thr Ala His Tyr Gln Gly Arg Val Ser Ala
   3010 3015 3020
   Lys Pro Pro Glu Asp Val Gln Val Pro Val Ser Ala Pro Val Arg Ala
   3025 3030 3035 3040
   Val Glu Arg Ala Gln Ala Pro Thr Ala Thr Ala Ser Glu Pro Val Ala
   3045 3050 3055
   Ile Ile Gly Val Ser Gly Val Phe Pro Gln Ser Glu Ser Pro Arg Ser
   3060 3065 3070
   Phe Trp Glu Asn Leu Leu Arg Gly Gln Asp Leu Ile Ser Glu Val Pro
   3075 3080 3085
   Ala Ser Arg Trp Asn Ala Glu Leu Leu Ala Lys Glu Ser Pro Ala Leu
   3090 3095 3100
   Arg Trp Gly Gly Phe Met Pro Gly Val Asp Leu Phe Asp Pro Glu Phe
   3105 3110 3115 3120
   Phe Gly Leu Ser Pro Gln Glu Ala Arg Leu Met Asp Pro Gln His Arg
   3125 3130 3135
   Leu Phe Ile Glu Ala Val Trp Lys Ala Phe Glu Asp Ala Gly His His
   3140 3145 3150
   Pro Leu Ser His Trp Gly Gln Arg Val Gly Val Phe Val Gly Val Ala
   3155 3160 3165
   Asn His Asp Tyr Thr Asn Leu Leu Leu Glu Arg Arg Ala Arg Ala His
   3170 3175 3180
   Met Ala Thr Gly Asn Ala His Ser Met Leu Ala Asn Arg Ile Ser Tyr
   3185 3190 3195 3200
   Leu Leu Asn Phe Thr Gly Pro Ser Glu Pro Val Asp Thr Ala Cys Ser
   3205 3210 3215
   Ser Ser Leu Ile Ala Val Gln Lys Ala Val Glu Ala Leu Gln Arg Gly
   3220 3225 3230
   Asp Cys Thr Val Ala Val Ala Gly Gly Val Asn Thr Leu Leu Ser Pro
   3235 3240 3245
   Asn Leu Tyr Leu Ala Phe Ser Asp Ala Gly Met Leu Ser Pro Ser Gly
   3250 3255 3260
   Arg Cys Arg Thr Phe Asp Ser Arg Ala Asp Gly Tyr Val Arg Gly Glu
   3265 3270 3275 3280
   Gly Val Gly Ala Ile Leu Leu Lys Pro Leu Arg Gln Ala Val Glu Asp
   3285 3290 3295
   Gly Asp Pro Ile Tyr Cys Val Ile Lys Gly Ala Ala Ala Asn His Gly
   3300 3305 3310
   Gly His Thr Ala Ser Ile Thr Ala Pro Asn Pro Leu Ala Gln Arg Asp
   3315 3320 3325
   Val Ile Ile Ala Ala His Glu Lys Ala Gly Val Asp Pro Glu Thr Leu
   3330 3335 3340
   Gly Tyr Ile Glu Ala His Gly Thr Gly Thr Ala Leu Gly Asp Pro Ile
   3345 3350 3355 3360
   Glu Ile Asn Gly Leu Lys Asp Val Phe Ala Glu Phe Ala Arg Arg Lys
   3365 3370 3375
   Gly Arg Ala Val Ser Thr Arg Lys Leu Cys Gly Ile Gly Ala Val Lys
   3380 3385 3390
   Thr Asn Ile Gly His Leu Glu Thr Ala Ala Gly Ile Ala Gly Leu Val
   3395 3400 3405
   Lys Ala Met Phe Ala Leu Lys His Gln Val Leu Pro Lys Asn Ala His
   3410 3415 3420
   Phe Glu Lys Leu Asn Pro Tyr Ile Gln Leu Asp Asp Ser Pro Leu Tyr
   3425 3430 3435 3440
   Ile Leu Asp Glu Thr Lys Pro Trp Gln Pro Ile Leu Gly Glu Ala Gly
   3445 3450 3455
   Lys Pro Leu Pro Arg Arg Ala Ala Val Ser Ser Phe Gly Phe Gly Gly
   3460 3465 3470
   Val Asn Ala His Val Val Leu Glu Glu Tyr Val Ala Glu Glu His Ala
   3475 3480 3485
   Ala Pro Glu Gln Glu Thr Gly Pro Phe Val Val Pro Val Ser Ala Gln
   3490 3495 3500
   Ala Leu Pro Val Leu Lys Asp Tyr Val Arg Ala Leu Ala Glu His Leu
   3505 3510 3515 3520
   Arg Gly Ser Thr Gly Val Val Glu Thr Glu Ser Arg Ser Ala Arg Gly
   3525 3530 3535
   Leu Arg Gly Gln Leu Arg Glu Leu Ala Ala Arg Ile Leu Gly Val Asp
   3540 3545 3550
   Ala Arg Thr Val Asp Glu Asp Glu Pro Leu Val Glu Leu Gly Phe Asp
   3555 3560 3565
   Gln Leu Ala Leu Ser Arg Leu Val Glu Glu Ala Asn Ile Arg Leu Gly
   3570 3575 3580
   Leu Gln Gln Gln Val Lys Ser Leu Ala Gly Ala Gln Asn Leu Gly Ser
   3585 3590 3595 3600
   Val Phe Asp Ala Leu Cys Ser Ala Gly Ala Pro Thr Ala Ser Ala Ala
   3605 3610 3615
   Ser Gly Pro Val Pro Ile Ser Leu Arg Ser Leu Ala Tyr Thr Leu Gln
   3620 3625 3630
   Val Gly Arg Met Ala Met Thr Glu Arg Val Ala Phe Leu Val Glu Ser
   3635 3640 3645
   Pro Arg Glu Leu Leu Ala His Leu Glu Ala Phe Leu Arg Asp Glu Pro
   3650 3655 3660
   Gly His Ala Asp Ser Leu Tyr Val Gly Ser Ala Pro Ala Arg Gly Arg
   3665 3670 3675 3680
   Phe Gln Gly Leu Arg Thr Gly Gly Lys Asp Gly Gly Val Glu Ala Leu
   3685 3690 3695
   Arg Ala Asp Ala Gly Ser Leu Arg Gly Leu Cys Gln Arg Trp Val Ala
               3700                3705                3710
   Gly Glu Asp Val Asp Trp Arg Gly Leu Tyr Val Ser Gly Pro Thr Pro
           3715                3720                3725
   Arg Lys Leu Ser Leu Pro Thr Tyr Pro Phe Ala Arg Lys Arg Tyr Trp
       3730                3735                3740
   Phe Asp Glu Val Pro Phe Glu Ser Ser Pro Glu Ala Glu Ala Leu Gly
   3745                3750                3755                3760
   Arg Ala Ser Pro Arg Gly Gln Glu Pro Gln Lys Ser Gly Gly Ala Pro
                   3765                3770                3775
   Glu Pro Gly Arg Glu Val Val Leu Ile Arg Ala Ala Leu Gly Ser Ile
               3780                3785                3790
   Leu Gly Thr Asp Pro Lys Ala Ile Ala Glu Asp Val Glu Phe Lys Asp
           3795                3800                3805
   Leu Gly Leu Asp Ser Ile Met Thr Ile Lys Leu Val Lys Gln Leu Asn
       3810                3815                3820
   Gln Glu Phe Glu Leu Ser Leu Asn Pro Met Gln Val Ile Glu Asn Gln
   3825                3830                3835                3840
   Thr Leu Glu Lys Leu Ser Gln Leu Leu Val Glu Leu Arg Pro Ser Gly
                   3845                3850                3855
   Ser Ala Gly Ala Lys Pro
               3860
<210> 11
   <211> 6045
   <212> PRT
   <213> Myxococcus fulvus
<220>
   <223> MxnK
<220>
   <221> CHAIN
   <222> 1..2531
   <223> MxnK: 1W-2W
<220>
   <221> CHAIN
   <222> 1..2467
   <223> MxnK: 1W-2W
<220>
   <221> CHAIN
   <222> 2532..6023
   <223> MxnK: 3W-5W
<220>
   <221> CHAIN
   <222> 2468..6023
   <223> MxnK: 3W-5W
<220>
   <221> CHAIN
   <222> 1..3348
   <223> MxnK: 1W-3W
<220>
   <221> CHAIN
   <222> 1..3272
   <223> MxnK: 1W-3W
<220>
   <221> CHAIN
   <222> 3349..6023
   <223> MxnK: 4W-5W
<220>
   <221> CHAIN
   <222> 3273..6023
   <223> MxnK: 4W-5W
<220>
   <221> CHAIN
   <222> 1..5203
   <223> MxnK: 1W-4W
<220>
   <221> CHAIN
   <222> 1..5251
   <223> MxnK: 1W-4W
<220>
   <221> CHAIN
   <222> 5252..6023
   <223> MxnK: 5W
<220>
   <221> CHAIN
   <222> 5204..6023
   <223> MxnK: 5W
<220>
   <221> CHAIN
   <222> 1683..1689
   <223> MxnK-CP-1W
<220>
   <221> CHAIN
   <222> 2429..2435
   <223> MxnK-CP-2W
<220>
   <221> CHAIN
   <222> 3234..3240
   <223> MxnK-CP-3W
<220>
   <221> CHAIN
   <222> 5165..5171
   <223> MxnK-CP-4W
<220>
   <221> CHAIN
   <222> 5894..5900
   <223> MxnK-CP*-5W
<220>
   <221> CHAIN
   <222> 5984..5990
   <223> MxnK-CP-5W
<220>
   <221> CHAIN
   <222> 955..967
   <223> MxnK-DH-1W
<220>
   <221> CHAIN
   <222> 3980..3992
   <223> MxnK-DH-4W
<220>
   <221> CHAIN
   <222> 4914..4919
   <223> MxnK-MT-4W
<400> 11
   Met Ser Ala His Glu Lys Pro Gly Ser Ser Arg Glu Ala Val Ala Ser
   1               5                   10                  15
   Pro Glu Val Ala Ile Ile Gly Met Ala Cys Arg Phe Pro Leu Ala Ser
               20                  25                  30
   Thr Tyr Gln Glu Tyr Trp Asn Asn Leu Ile Ser Gly Arg Asp Cys Ile
           35                  40                  45
   Arg Glu Ile Pro Ser Ser Arg Trp Asp Thr Arg Arg Tyr Tyr Ser Pro
       50                  55                  60
   Asp Ile Asp Gln Pro Asp Thr Ser Val Ser Arg Trp Cys Gly Leu Val
   65                  70                  75                  80
   Asp Asp Val Glu Arg Phe Asp His Lys Phe Phe Glu Ile Ser Pro Arg
                   85                  90                  95
   Glu Ala Lys Asn Met Asp Pro Gln Gln Arg Leu Leu Leu Glu Glu Thr
               100                 105                 110
   Leu His Cys Ile Glu Asp Ser Gly Val Pro Leu Ala Glu Leu Arg Ala
           115                 120                 125
   Arg Lys Thr Ser Val Phe Ala Gly Val Met Ala Thr Asp Tyr His Gln
       130                 135                 140
   Glu Ala Val His Thr Ser Val His Pro Asp Ser Phe Ala Gly Thr Gly
   145                 150                 155                 160
   Asn Tyr Val Cys Ile Leu Ala Asn Arg Val Ser Tyr Ala Phe Gly Phe
                   165                 170                 175
   Gln Gly Lys Ser Phe Thr Ile Asp Ala Ala Cys Ala Ser Ser Leu Val
               180                 185                 190
   Ala Leu His Glu Ala Arg Cys Ser Leu Gln Arg Gly Glu Ser Asp Tyr
           195                 200                 205
   Ala Ile Val Ala Gly Val Ser Leu Asn Leu His Pro Trp Lys Tyr Ile
       210                 215                 220
   Thr Trp Ser Lys Ala Arg Met Leu Ser Pro Asp Gly Arg Cys Lys Thr
   225                 230                 235                 240
   Phe Asp Lys Asp Ala Asn Gly Tyr Val Pro Gly Asp Gly Ile Gly Val
                   245                 250                 255
   Leu Leu Leu Gln Arg Arg Asp Glu Gly Met Arg Asp Arg Asn Arg Ile
               260                 265                 270
   Tyr Gly Val Val Lys Gly Ser Ala Val Asn His Gly Gly His Thr Ala
           275                 280                 285
   Ser Met Thr Ala Pro Arg Ile Asp Ala Gln Arg Asp Val Ile Leu Ser
       290                 295                 300
   Ala Ile Glu Ser Ala Gly Ile Asn Ala Arg Thr Ile Gly Tyr Val Glu
   305                 310                 315                 320
   Ala His Gly Thr Gly Thr Ser Leu Gly Asp Pro Ile Glu Val Glu Ala
                   325                 330                 335
   Leu Thr Arg Ala Phe Arg Cys Ser Thr Asp Asp Thr Gln Phe Ala Lys
               340                 345                 350
   Leu Gly Ser Cys Lys Thr Asn Ile Gly His Leu Glu Ala Ala Ala Gly
           355                 360                 365
   Val Ala Gly Val Ile Lys Val Leu Leu Met Met Arg His Gly Gln Val
       370                 375                 380
   Pro Lys Thr Leu Asn Val Thr Thr Leu Asn Pro Ile Ile Asp Phe Pro
   385                 390                 395                 400
   Arg Thr Pro Phe Ser Val Ala Leu Glu Asn Ser Glu Trp Pro Arg Ala
                   405                 410                 415
   Glu Ser Ser Leu Pro Arg Arg Ala Gly Val Ser Ser Phe Gly Ile Gly
               420                 425                 430
   Gly Ala Asn Ser His Val Val Leu Glu Glu His Val Thr Asp Glu Val
           435                 440                 445
   Thr Asn Pro Arg Thr Ala Arg Ser Ser Gly Leu Ala Gly Arg Lys His
       450                 455                 460
   Leu Phe Leu Leu Ser Ala Lys Ser Pro Gly Ser Phe Thr Arg Arg Val
   465                 470                 475                 480
   Ala Arg Trp Arg Glu Trp Leu Arg Gly Thr Asp Ser Ala Arg Glu Asp
                   485                 490                 495
   Leu Lys Asp Val Cys Glu Thr Thr Ala Val Ser Ser Gly His Tyr Pro
               500                 505                 510
   Leu Arg Ser Gly Ala Leu Val Gly Thr Phe Glu Glu Leu Gly Glu Phe
           515                 520                 525
   Leu Asp Gly Ala Gly Ala Lys Ala Val Ser Pro Arg Ala Pro Pro Ser
       530                 535                 540
   Thr Leu Val Leu Arg His His Thr Phe Thr Gly Phe Arg Ser Val Arg
   545                 550                 555                 560
   Ser Leu Tyr Glu Gly Asn Ala Leu Phe Arg Gly Arg Val Asp Ala Leu
                   565                 570                 575
   Leu Arg Glu Cys Lys Val Pro Gly Leu Arg Gly Gly Leu Glu Thr Ala
               580                 585                 590
   Phe Arg Ala Ala Ser Trp Pro Arg Lys Ala Arg Pro Leu Leu Ser Cys
           595                 600                 605
   Leu Val Gly His Ala Phe Trp Ala Ser Leu Gly Glu Leu Gly Phe Ser
       610                 615                 620
   Ala Arg Glu Leu His Gly Glu Lys His Gly Phe Ile Val Ala Leu Ser
   625                 630                 635                 640
   Leu Ser Gly Met Val Glu Pro Ala Glu Ala Leu Ala Leu Ala Ser Gly
                   645                 650                 655
   Gly Val Thr Ala Val Gly Arg Val Arg Ala Pro Asp Ala Ser Leu Phe
               660                 665                 670
   Asp Pro Val Gly Arg Glu Leu Ile Pro Ser Leu Ala Ile Arg Arg Glu
           675                 680                 685
   Tyr Leu Glu Phe Leu Cys Gly Glu Leu Gln Leu Ser Glu Ala Glu Leu
       690                 695                 700
   Glu Ala Ser Val Gly Arg Ala Arg Leu Leu Phe His Gly Gln Arg Thr
   705                 710                 715                 720
   Phe Gln Arg Phe Leu Glu Glu Trp Asn Glu Ala Leu Ala Pro Leu Arg
                   725                 730                 735
   Leu Thr Met Glu Arg Leu Leu His Asp Glu Arg Arg Thr Ser Gly Ala
               740                 745                 750
   Glu Ala Ala Arg Ala Arg Leu Leu Leu Phe Val Ala Val Glu Ser Ala
           755                 760                 765
   Phe Arg Lys Leu Ser Thr Lys Trp Ser Phe Glu Pro Arg Pro Pro Ala
       770                 775                 780
   Arg Asp Glu Arg Leu Thr Glu Leu Val Ala Leu Val Ala Asp Gly Val
   785                 790                 795                 800
   Leu Ser Arg Thr Ala Thr Leu Glu Leu Leu Leu Ser Asp Thr Pro Glu
                   805                 810                 815
   Phe Glu Lys Leu Ala Arg Ala Ala Gln Ser Ala Ala Ser Thr Arg Ser
               820                 825                 830
   Gln Gly Arg Tyr Pro Leu Leu Glu Arg Phe Ala Arg Leu Pro Glu Gln
           835                 840                 845
   Glu Gly Trp Ile Gln Ala Ala Val Gly Ala Ser Leu Gly Asp Gly Pro
       850                 855                 860
   Ala Pro Gly Pro Leu Val Phe Glu Trp Ala Pro Asp Gly Ala Glu Glu
   865                 870                 875                 880
   Gly Glu Glu Leu Gln Ala Leu Phe Asp Arg Thr Val Leu Glu Leu Trp
                   885                 890                 895
   Glu Arg Gly Ala Pro Ile Gln Trp Glu Arg Leu His Pro Val Gly Ser
               900                 905                 910
   Tyr Arg Lys Ala Ser Leu Pro Ala Tyr Pro Phe Glu Gly Glu Ala Phe
           915                 920                 925
   Trp Ile Arg Glu Arg Thr Phe Gln Glu Ala Pro Val Thr Ser Gln Ala
       930                 935                 940
   Arg Leu Pro Leu Ser Pro Ser Asp Pro Ile Ile Arg Asp His Val Val
   945                 950                 955                 960
   Gly Gly Asn Arg Leu Ile Pro Gly Ala Leu Met Met His Leu Ala Leu
                   965                 970                 975
   Arg Ala Val Arg Ser Ala Gly Arg Gly Asp Gly Ala Ser Leu Arg Asn
               980                 985                 990
   Val Val Phe His Thr Pro Gly Ile Val Ser Ser Ala Val Glu Leu Arg
           995                 1000                1005
   Ala Glu Leu Glu Gly Asp Val Ala Arg Val Phe Val Glu Gly Leu Pro
       1010                1015                1020
   Leu Ser Gln Ala Glu Val Ala Arg Glu Gly Asp Val Gly Pro Gly Ala
   1025                1030                1035                1040
   Gly Ala Pro Ser Gly Met Asp Ala Leu Leu Glu Pro His Asp Val Gly
                   1045                1050                1055
   Ala Leu Tyr Glu Gly Leu Ala Ala Ser Gly Phe Gln Tyr Gly Glu Ser
               1060                1065                1070
   Leu Arg Leu Leu Arg Arg Phe Glu Ser Ser Asp Ala Thr Cys Phe Ala
           1075                1080                1085
   Glu Leu Ser Thr Glu Pro Leu Arg Glu Gln Gly Glu Gly Gln Val Pro
       1090                1095                1100
   His Val Leu Asp Thr Val Leu Gln Ala Gly Leu Leu Ala Gly Tyr Val
   1105                1110                1115                1120
   Ser Gly His Leu Ser Phe Gly Arg Asp Arg Phe Val Pro Ser Phe Val
                   1125                1130                1135
   Gly Ala Leu Trp Leu Ser Gly Thr Leu Pro Pro Val Ile Arg Leu Gln
               1140                1145                1150
   Val Val Arg Arg Pro Ser Gly Lys Ser Glu His Arg Phe Ser Val Asp
           1155                1160                1165
   Val Thr Gly Gln Asp Leu Ser Gly Arg Arg Val Leu Glu Leu Arg Glu
       1170                1175                1180
   Val Val Tyr Gln Arg Ala Asp Gly Ala Arg Thr Glu Arg Ser Pro Leu
   1185                1190                1195                1200
   Asp Trp Glu Met Lys Val Pro Ser Trp Lys Glu Ser Pro Ala Ser Met
                   1205                1210                1215
   Ala Ser Ala Gln Asp Val Arg Ala Val Val Val Asp Ser Arg Gly Glu
               1220                1225                1230
   His Ala Gly Ser Phe Ile Glu Arg Ala Arg Arg Val His Gly Arg Val
           1235                1240                1245
   Gln Val Val Ser Ala Gly Gly Gly Asp Phe Thr Ala Val Phe Glu Thr
       1250                1255                1260
   Leu Thr Lys Ser Pro Gly Arg Val Thr Leu Tyr Val Leu Asp Ala Leu
   1265                1270                1275                1280
   Gly Ala Asp Glu Leu Asp Glu Ala Glu Arg Ala Leu Gln Ser Leu Phe
                   1285                1290                1295
   Leu Leu Val Lys Ala His Leu Arg Leu Ala Ser Asp His Glu Phe Arg
               1300                1305                1310
   Val Leu Val Pro Val Arg Lys Ala Phe Val Ala Thr Asp Ser Asp Tyr
           1315                1320                1325
   Gly Glu Gly Phe Ala Ala Ala Ala Leu Leu Gly Leu Ala Lys Thr Ala
       1330                1335                1340
   Met Leu Glu Ser Pro Arg Leu Gln Val Ser Leu Val Asp Val Asp Ala
   1345                1350                1355                1360
   Leu Asp Ala Val Gly Met Glu Ala Leu Leu Arg Glu Gly Met Val Glu
                   1365                1370                1375
   Gly Arg Ala Glu Asp Leu Val Val Tyr Arg Gln Gly Arg Arg Leu Val
               1380                1385                1390
   Arg Glu Leu Arg Ala Leu Gln Ala Gly Gln Ser Ser Thr Ala Ser Leu
           1395                1400                1405
   Phe Thr Gly Gly Lys Asn Tyr Leu Leu Val Gly Gly Gly Gly Gly Leu
       1410                1415                1420
   Gly Arg Ala Leu Ile Asp His Leu Ala Ala Arg Val Ser Cys Arg Ile
   1425 1430 1435 1440
   Phe Val Met Gly Arg Ser Ala Arg Ser Ser Pro Glu Ser Thr Glu Gly
   1445 1450 1455
   Arg Gln Cys Glu Val His Ser Leu Arg Cys Asp Val Thr Asp Arg Arg
   1460 1465 1470
   Gln Val Glu Gln Ala Ile Gln Gln Leu Thr Ala Asp Phe Gly Pro Leu
   1475 1480 1485
   His Gly Val Leu His Leu Gly Gly Val Leu Ser Asp Lys Leu Leu Leu
   1490 1495 1500
   Thr Lys Ser Trp Glu Glu Phe Gln Arg Val Leu Ala Pro Lys Leu Ala
   1505 1510 1515 1520
   Gly Thr Leu Phe Leu His Arg Ala Thr Gln Asp Gln Pro Leu Asp Phe
   1525 1530 1535
   Phe Ile Gly Phe Ser Ser Ile Val Ser Leu Leu Gly Asn Val Gly Gln
   1540 1545 1550
   Ala Asp Tyr Ala Ala Gly Asn Ser Phe Leu Asp Ala Phe Met Ala Phe
   1555 1560 1565
   Arg Ala Arg Ser Gly Ala Pro Gly Arg Ser Leu Val Val Asn Trp Pro
   1570 1575 1580
   Leu Trp Glu Gly Val Gly Met Ala Ser Arg Leu Thr Ala Asp Arg Ser
   1585 1590 1595 1600
   Val Glu Val Arg His Leu Arg Pro Ile Arg Val Glu Ala Gly Met Arg
   1605 1610 1615
   Ala Phe Glu Ala Val Leu Ala Gln Gly Asp Ser Val Gln Val Leu Val
   1620 1625 1630
   Gln Gly Gly Ala Arg Pro Asp Ala Lys Lys Pro Leu Pro Ala Val Val
   1635 1640 1645
   Ala Gln Val Arg Asn Val Glu Gly Tyr Leu Arg Glu Leu Val Ala Lys
   1650 1655 1660
   Arg Leu Gly Leu Pro Gly Pro Gly Phe Asp Glu Thr Glu Ser Phe Phe
   1665 1670 1675 1680
   Thr Met Gly Leu Asp Ser Leu Ile Leu Gln Glu Leu Met Ala Glu Leu
   1685 1690 1695
   Asp Arg Glu Phe Ser Gly Leu Pro Pro Thr Leu Val Phe Glu His Pro
   1700 1705 1710
   Asn Leu Lys Lys Leu Val Ala Tyr Phe Glu Lys Gln Gly Leu Ala Ser
   1715 1720 1725
   Pro Val Ala Pro Ser Glu Ala Ala Gly Pro Glu Pro Arg Val Thr Pro
   1730 1735 1740
   Glu Glu Ser Ile Pro Pro Glu Pro Glu Arg Pro Ala Pro Val Arg Gly
   1745 1750 1755 1760
   Gly Tyr Asp Ile Ala Ile Ile Gly Met Ser Gly Arg Phe Pro Gln Ala
   1765 1770 1775
   Arg Thr Val Ser Asp Phe Trp Arg Asn Leu Val Glu Gly Arg Asp Cys
   1780 1785 1790
   Ile Thr Asp Ile Pro Thr Asp Arg Trp Asp Ala Arg Ala Ile Gln Gly
   1795 1800 1805
   Ser Arg Thr Asp Ile Gly Ala Arg Ser Tyr Gly Arg Trp Gly Gly Phe
   1810 1815 1820
   Leu Ala Glu Val Asp Arg Phe Asp Pro Leu Phe Phe Asn Ile Ser Pro
   1825 1830 1835 1840
   Lys Glu Ala Glu Glu Met Asp Pro Gln Gln Arg Leu Phe Leu Glu Cys
   1845 1850 1855
   Val Trp Glu Thr Leu Glu Asp Ala Gly Tyr Ala Pro Arg Arg Arg Tyr
   1860 1865 1870
   Gln His Lys Ser Val Gly Leu Phe Val Gly Thr Met Trp Asn Glu Tyr
   1875 1880 1885
   Ser Leu Leu Ala His Glu Arg Gly Phe Leu Gln Gly Asp Tyr Lys Gly
   1890 1895 1900
   Pro Gly Ser Leu Tyr Trp Ala Ile Ala Asn Arg Thr Ser Phe Phe Met
   1905 1910 1915 1920
   Asp Phe Lys Gly Pro Ser Leu Ala Val Asp Thr Ala Cys Ser Ser Ser
   1925 1930 1935
   Leu Ala Ala Ile His Leu Ala Cys Gln Ser Leu Leu Ser Gly Asp Cys
   1940 1945 1950
   Asp Met Ala Val Ala Gly Gly Ile Asn Leu Ser Ile His Pro Gly Lys
   1955 1960 1965
   Tyr Val Tyr Leu Ser Gln Ala Lys Phe Leu Ser Ser Asp Gly Arg Cys
   1970 1975 1980
   Arg Ser Phe Gly Glu Gly Gly Asp Gly Tyr Val Pro Gly Glu Gly Val
   1985 1990 1995 2000
   Gly Thr Val Leu Leu Lys Pro Leu Asp Ala Ala Leu Ala Asp Gly Asp
   2005 2010 2015
   Gln Ile His Ala Val Ile Arg Gly Ser Ala Ser Asn His Gly Gly Lys
   2020 2025 2030
   Ala Ala Gly Phe Thr Val Pro Asn Pro Glu Gln Gln Ala Arg Leu Ile
   2035 2040 2045
   Glu Lys Ser Met Glu Arg Ala Ala Val Arg Pro Glu Asp Ile Asn Tyr
   2050 2055 2060
   Val Glu Cys Gln Ala Thr Gly Thr Ser Leu Gly Asp Pro Leu Glu Ile
   2065 2070 2075 2080
   Arg Gly Leu Gln Leu Ala Phe Gly Gln Gln Arg Gly Gln Ala Arg Ala
   2085 2090 2095
   Cys Pro Ile Gly Ser Val Lys Pro Asn Ile Gly His Leu Glu Ala Ala
   2100 2105 2110
   Ser Gly Ile Ala Gly Val Ile Lys Val Ala Leu Ala Met Arg His Gln
   2115 2120 2125
   Val Ile Pro Arg Thr Leu Gln Glu Asp Ala Pro Asn Pro Lys Leu Glu
   2130 2135 2140
   Leu Gly Gln Thr Pro Phe Tyr Val Val Lys Gln Asn Gln His Trp Ser
   2145 2150 2155 2160
   Val Glu Pro Gly His Ala Arg Ile Ala Ala Ile Ser Ser Phe Gly Ala
   2165 2170 2175
   Gly Gly Ser Asn Ala His Leu Ile Leu Glu Glu Ser Pro His Arg Arg
   2180 2185 2190
   Ser Leu Pro Ala Ala Ala His Val Ser His Ala Glu Pro Asp Val Phe
   2195 2200 2205
   Val Val Ser Ala Arg Asp Ser Gln Arg Leu Ser Glu Gln Val Gly Arg
   2210 2215 2220
   Leu His Ala Phe Leu Ala Thr Asp Glu Ala Lys Gln Leu Glu Leu Gly
   2225 2230 2235 2240
   Asp Ile Ala His Thr Leu Arg Val Gly Arg Asn Ala Phe Thr Glu Arg
   2245 2250 2255
   Leu Ala Val Val Ala Ser Gly Leu Val Glu Leu Arg Glu Ala Leu Ala
   2260 2265 2270
   Ser Ser Ser Gly Ala Gly Thr Arg Gly Asp Ala Arg Tyr Gly Glu Leu
   2275 2280 2285
   Ser Leu Leu Ala Ala Ala Trp Gln Arg Gly Glu Ser Val Asp Trp Ala
   2290 2295 2300
   Pro Leu Ala Ser Arg Arg Pro Arg Arg Ile Val Ser Leu Pro Thr Tyr
   2305 2310 2315 2320
   Pro Phe Arg Arg Asp Arg Tyr Trp Leu Asp Gly Ala Arg Pro Ser Ala
   2325 2330 2335
   Gly Pro Pro Asn Arg Pro Pro Ser Asp Gly Glu Leu Glu Ala Glu Gly
   2340 2345 2350
   Pro Leu Val Gly Ala Leu Arg Thr Lys Arg Ala Gln Ala His Glu Gly
   2355 2360 2365
   Leu Glu Ala Phe Glu Val Ala Leu Asn Ser Arg Glu Gln Gln Gly Met
   2370 2375 2380
   Ala Ile Thr Gly Asn Lys Glu Glu Asn Asp Ser Leu Leu Asp Lys Val
   2385 2390 2395 2400
   Gln Ala Glu Thr Met Asp Ile Met Ser Arg Leu Leu Lys Val Asp Arg
   2405 2410 2415
   Arg Asp Ile His Leu Thr Glu Asp Leu Arg Glu Tyr Gly Phe Asp Ser
   2420 2425 2430
   Ile Thr Phe Thr Gln Phe Ala Asn Glu Leu Asn Glu His Phe Gly Leu
           2435                2440                2445
   Asp Leu Thr Pro Ala Leu Phe Phe Glu Tyr Ala Thr Ile Arg Asp Phe
       2450                2455                2460
   Ser Ala Phe Leu Leu Glu Asn His Gly Glu Val Ile Arg Lys Arg Glu
   2465                2470                2475                2480
   Arg Ala Met Pro Leu Ala Glu Pro Lys Arg Glu Pro Ala Pro Ser Arg
                   2485                2490                2495
   Met Ser Thr Ser Gly Ala Val Glu Ala Ser Ala Pro Ala Gln Ser Pro
               2500                2505                2510
   Ser Ala Ser Gln Ala Glu Val Arg Gly Pro Val Ser Ala Ala Gly Arg
           2515                2520                2525
   Pro Gly Asp Glu Gly Ile Ala Val Ile Gly Met Ala Gly Glu Met Pro
       2530                2535                2540
   Gln Ser Gly Asp Leu His Glu Leu Trp Gln His Leu Glu Ala Gly Arg
   2545                2550                2555                2560
   Glu Leu Val Gln Glu Val Pro Ala Ser Arg Trp Asp Ala Ser Arg Ser
                   2565                2570                2575
   Arg Val Arg Trp Gly Gly Phe Leu Lys Gln Val Asp Gly Phe Asp Ala
               2580                2585                2590
   Ala Phe Phe Gly Val Thr Pro Arg Glu Ala Glu Leu Met Asp Pro Gln
           2595                2600                2605
   Gln Arg Leu Phe Leu Glu Thr Val Trp Arg Thr Leu Glu Asp Ala Gly
       2610                2615                2620
   Tyr Arg Pro Ser Ser Leu Ser Gly Ser Arg Thr Gly Leu Phe Val Gly
   2625                2630                2635                2640
   Val Val Thr Ser Asp Tyr Gly Ala Leu Leu Lys Glu Asn Gly Leu Glu
                   2645                2650                2655
   Gly Glu Ala Leu Ala Pro Thr Gly Asn Phe His Ser Leu Val Ala Asn
               2660                2665                2670
   Arg Ile Ser Tyr His Phe Asn Phe His Gly Pro Ser Glu Pro Cys Asp
           2675                2680                2685
   Thr Ala Cys Ser Ser Ser Leu Ile Ala Ile His Arg Ala Cys Glu Ala
       2690                2695                2700
   Ile Arg Glu Gly Ser Cys Glu Gln Ala Ile Ala Gly Gly Val Asn Leu
   2705                2710                2715                2720
   Leu Leu Ser Pro Thr Leu Phe Val Ala Phe Asp Ser Ala Gly Met Leu
                   2725                2730                2735
   Ser Pro Ser Gly Lys Leu Arg Ala Phe Asp Glu Gly Ala Asn Gly Tyr
               2740                2745                2750
   Val Arg Gly Glu Gly Val Ala Ala Val Leu Leu Lys Pro Leu Asn Lys
           2755                2760                2765
   Ala Ile Ala Asp Gly Asp His Ile Tyr Gly Val Ile Lys Gly Ser Ala
       2770                2775                2780
   Thr Asn His Gly Gly Arg Ala His Ser Leu Thr Ala Pro Asn Pro Asn
   2785                2790                2795                2800
   Ala Gln Ala Asp Leu Leu Met Thr Val Tyr Arg Lys Ala Gly Ile Ala
                   2805                2810                2815
   Pro Asp Arg Val Ser Leu Ile Glu Ala His Gly Ser Gly Thr Ala Leu
               2820                2825                2830
   Gly Asp Pro Ile Glu Ile Asn Gly Ile Lys Lys Ala Phe Glu Thr Leu
           2835                2840                2845
   Tyr Gly Glu Trp Lys Leu Pro Tyr Gly Gly Pro Ala Arg Cys Ala Ile
       2850                2855                2860
   Gly Ser Leu Lys Gly Asn Ile Gly His Leu Glu Ala Ala Ala Gly Ile
   2865                2870                2875                2880
   Ala Gly Val Ala Lys Val Leu Leu Ala Leu Ser His Lys Arg Ile Pro
                   2885                2890                2895
   Pro Asn Ala Thr Leu Thr Lys Leu Asn Pro Gln Ile Arg Leu Asp Asp
               2900                2905                2910
   Thr Pro Phe His Val Val Lys Gln Leu Gln Pro Trp Glu Ala Arg Thr
           2915                2920                2925
   Gly Ala Asp Gly Glu Thr Leu Arg Val Ala Gly Val Ser Ser Phe Gly
       2930                2935                2940
   Phe Gly Gly Ala Asn Ala His Val Ala Ile Glu Glu Tyr Val Gly Ser
   2945 2950 2955 2960
   Pro Glu Ala Arg Thr Glu Ala Val Gly Pro His Val Val Val Leu Ser
   2965 2970 2975
   Ala Arg Thr Arg Ser Ala Leu Thr Glu Tyr Ala Arg Arg Leu Leu Arg
   2980 2985 2990
   Asp Ser Glu Ser Gly Arg Leu Ser Gly Val Pro Leu Ala Asp Leu Ala
   2995 3000 3005
   Phe Thr Ser Gln Val Gly Arg Glu Pro Met Val Glu Arg Leu Ala Leu
   3010 3015 3020
   Leu Val Ser Asp Leu Pro Glu Leu Glu Ser Lys Leu Arg Ala Phe Ala
   3025 3030 3035 3040
   Glu Thr Gly Met Thr Gly Val Gly Val Phe Gln Gly Arg Ala Glu Arg
   3045 3050 3055
   Gln Gly Ala Arg Pro Gly Gly Met Ala Ser His Asp Ser Trp Val Ala
   3060 3065 3070
   Glu Arg Val Gln Asp Gly Arg Trp Ala Glu Leu Cys Glu Gly Trp Val
   3075 3080 3085
   Glu Gly Arg Asp Leu Glu Trp Ser Ala Leu His Arg Gly Gln Pro Arg
   3090 3095 3100
   Arg Arg Val Ser Phe Ser Ser Tyr Pro Phe Glu Arg Lys Arg Phe Trp
   3105 3110 3115 3120
   Cys Asp Ser Lys Leu Phe Ser Arg Pro Thr Ala Ala Ala Val Thr Val
   3125 3130 3135
   Ala Asp Thr Val Gly Thr Val Glu Leu Glu Arg Lys Asp Lys Pro Val
   3140 3145 3150
   Met Lys Leu Lys Leu Lys Glu Pro Gln Ala Thr Glu Ser Gln Val Arg
   3155 3160 3165
   Glu Pro Gln Ala Thr Val Pro Ala Lys Ser Pro Glu Pro Arg Ser Arg
   3170 3175 3180
   Glu Pro Glu His Val Ser Gln Pro Ser Ser Pro Pro Ala Pro Gly Val
   3185 3190 3195 3200
   Ser Gln Gly Glu Leu Arg Arg Lys Leu Lys Gly Leu Leu Ala Ala Thr
   3205 3210 3215
   Leu His Val Pro Gln Glu Glu Leu Ser Glu Thr Arg Pro Phe Ala Glu
   3220 3225 3230
   Leu Gly Leu Asp Ser Ile Leu Ala Leu Glu Phe Val Lys Cys Ile Asn
   3235 3240 3245
   Glu Glu Leu Gly Ala Ser Leu Ala Ala Val Gln Ile Phe Asp His Gly
   3250 3255 3260
   Asp Ile Thr Arg Leu Ala Lys Ala Leu Arg Asp Gln Ala Pro Arg Ala
   3265 3270 3275 3280
   Val Glu Pro Ala Pro Glu Pro Val Arg Glu Pro Val Ser Lys Val Val
   3285 3290 3295
   Arg Glu Leu Ala Pro Val Pro Val Pro Val Val Ala Pro Val Ala Leu
   3300 3305 3310
   Leu Asp Glu Lys Leu Pro Gly Asp Glu Leu Leu Glu Leu Trp Arg Ser
   3315 3320 3325
   Thr Pro Ala Pro Val Ser Val Pro Gln Pro Arg Arg Ala Arg Ala Val
   3330 3335 3340
   Glu Val Ala Ser Gln Asp Ile Ala Val Ile Gly Met Ala Ala Lys Leu
   3345 3350 3355 3360
   Pro Gly Ala Arg Asn Val Asp Glu Tyr Trp Arg Asn Leu Lys Ala Gly
   3365 3370 3375
   Val Ser Ser Ile Arg Glu Val Pro Ser Asp Arg Trp Asp Ala Ser Arg
   3380 3385 3390
   Ala Gly His Arg Cys Lys Trp Gly Gly Phe Leu Asp Glu Val Asp Ala
   3395 3400 3405
   Phe Asp Pro Leu Phe Phe Asn Ile Ser Pro Ser Glu Ala Ala Leu Met
   3410 3415 3420
   Asp Pro Gln Gln Arg Leu Phe Leu Glu Thr Ser Trp Thr Ala Leu Glu
   3425 3430 3435 3440
   Asp Ala Gly Tyr Ser Ala Glu Arg Leu Asp Gly Glu Arg Cys Gly Val
                   3445                3450                3455
   Tyr Val Gly Val Leu Asn Asn Asp Tyr Tyr Glu Leu Leu Cys Gly Asp
               3460                3465                3470
   Ala Arg Ala Ala Arg Gln Ala Gln Ser Met Thr Gly Asn Ala Asn Ser
           3475                3480                3485
   Ile Leu Ala Ala Arg Ile Ala Tyr Phe Leu Asn Leu Lys Gly Pro Ala
       3490                3495                3500
   Met Gln Val Asp Thr Ala Cys Ser Ser Ser Leu Val Ala Val His Leu
   3505                3510                3515                3520
   Ala Arg Gln Ser Leu Val Asn Asp Glu Ala Asp Met Met Leu Val Gly
                   3525                3530                3535
   Gly Val Thr Leu Tyr Leu Ala Glu Thr Ser Tyr Ala Arg Met Ser Glu
               3540                3545                3550
   Ala Gly Met Leu Ser Pro Thr Gly Gln Cys His Thr Phe Asp Gln Arg
           3555                3560                3565
   Ala Asp Gly Phe Val Pro Gly Glu Gly Val Gly Ala Val Val Leu Lys
       3570                3575                3580
   Arg Leu Asp Lys Ala Leu Lys Asp Gly Asp Arg Ile Tyr Gly Val Ile
   3585                3590                3595                3600
   Lys Gly Thr Ala Thr Asn Gln Asp Gly Lys Thr Asn Gly Ile Thr Ala
                   3605                3610                3615
   Pro Ser Ala Gln Ser Gln Thr Ala Leu Gln Val Ala Leu Tyr Arg Arg
               3620                3625                3630
   Phe Gly Ile Asp Pro Glu Thr Leu Thr Tyr Val Glu Ala His Gly Thr
           3635                3640                3645
   Gly Thr Lys Leu Gly Asp Pro Ile Glu Val Lys Ala Leu Thr Asp Ala
       3650                3655                3660
   Phe Arg Ala Phe Thr Pro Arg Thr Arg Phe Cys Ala Leu Gly Ser Val
   3665                3670                3675                3680
   Lys Thr Asn Ile Gly His Thr Ser Ala Ala Ala Gly Val Ala Gly Leu
                   3685                3690                3695
   Ile Lys Val Leu Leu Ala Leu Arg His Arg Gln Leu Pro Pro Ser Leu
               3700                3705                3710
   Asn Phe Ala Arg Glu Asn Gly Leu Ile Gly Phe Glu Ala Thr Pro Phe
           3715                3720                3725
   Arg Val Val Thr Glu Leu Thr Asp Trp Val Pro Ala Pro Gly Met Pro
       3730                3735                3740
   Arg Arg Ala Ala Ile Ser Ser Phe Gly Phe Ser Gly Thr Asn Ala His
   3745                3750                3755                3760
   Leu Val Val Glu Glu Tyr Pro Ser Val Pro Pro Ala Arg Ser Ser Val
                   3765                3770                3775
   His Val Arg Pro Gln Val Val Val Leu Ser Ala Arg Asp Thr Glu Arg
               3780                3785                3790
   Leu Arg Ala Gln Ala Arg Glu Leu Ala Asp Phe Ile Ala Ala Arg Gly
           3795                3800                3805
   Glu Leu Leu Ser Leu Asp Ala Val Ala Trp Thr Leu Gln Ser Gly Arg
       3810                3815                3820
   Thr Ala Met Glu Glu Arg Leu Ala Phe Val Ala Ser Ser Leu Pro Glu
   3825                3830                3835                3840
   Val Arg Glu Arg Leu Leu Gly Phe Leu Glu Gly Ala Gly Asp Ala Gly
                   3845                3850                3855
   Leu His His Arg Arg Leu Thr Glu Gly Ser Ser Glu Ala Asp Trp Leu
               3860                3865                3870
   Leu Glu Gly His Glu Gly Glu Gln Tyr Met Lys Ala Leu Leu Ser Gly
           3875                3880                3885
   Gly Ala Leu Glu Lys Val Ala Lys Leu Trp Thr Met Gly Val Arg Leu
       3890                3895                3900
   Asp Trp Ser Arg Leu His Asp Gln Ser Ser Arg Arg Pro Glu Ile Val
   3905                3910                3915                3920
   Ser Leu Pro Thr Tyr Pro Phe Ala Arg Glu Arg Cys Trp Phe Arg Gly
                   3925                3930                3935
   Ser Glu Gly Ile Gly Ala Ala Ala Ala Gly Met Gly Ala Gly Ser Ser
               3940                3945                3950
   Leu Leu Gln Val Asp Leu Ser Arg Gly Leu Glu Glu Gly Gly Val Val
   3955 3960 3965
   Phe Lys Ala Arg Val Leu Ala Gln Asp Val Leu Val Ser Gln His Gln
   3970 3975 3980
   Val His Gly Arg Pro Val Phe Pro Gly Thr Gly Trp Leu Glu Leu Ala
   3985 3990 3995 4000
   Thr Glu Gly Ala Arg Leu Leu Gly Gly Gly Arg Pro Leu Arg Leu Val
   4005 4010 4015
   Asn Val Ala Phe Leu Gln Pro Leu Phe Ala Ser Glu Asp Arg Asp Leu
   4020 4025 4030
   Glu Val Leu Ala Thr Lys Gln Gly Gly Thr Tyr Arg Leu Val Leu Gln
   4035 4040 4045
   Ser Arg Asp Ser Asp Gly Ala Lys Leu Thr His Ala Ser Gly Asp Leu
   4050 4055 4060
   Leu Pro Asp Glu Glu Gly Gly Pro Val Ser Arg Leu Val Leu Ser Glu
   4065 4070 4075 4080
   Leu Arg Ala Arg Cys Gln Gln Pro Val Ala Val Asp Ala Leu His Ala
   4085 4090 4095
   Arg Ala Gln Ala Leu Gly Leu Phe Tyr Gly Pro Arg Phe Gln Gly Leu
   4100 4105 4110
   Lys Ala Cys Trp Ala Gly Gln Glu Glu Ala Leu Val Asp Leu Glu Leu
   4115 4120 4125
   Pro Pro Glu Thr Ala Asn Glu Leu Ala Arg Arg Pro Phe His Pro Ile
   4130 4135 4140
   Leu Leu Asp Cys Val Leu Arg Ser Ile Leu Val His Gly Leu Gly Gly
   4145 4150 4155 4160
   Met Ala Gly Ala Arg Thr Ser Ala Leu Val Pro Phe Thr Ile Gly Ala
   4165 4170 4175
   Val Arg Leu Leu Lys Pro Leu Pro Ser Lys Gly Val Ala Tyr Ala Arg
   4180 4185 4190
   Arg Gln Gly Thr Gly Ala Ala Ser Tyr Gln Val Val Leu Leu Asp Asp
   4195 4200 4205
   Ala Gly Asp Thr Arg Ala Val Phe Asp Glu Val Val Pro Arg Pro Leu
   4210 4215 4220
   Ala Asp Pro Leu Glu Gly Phe Leu Phe Ala Pro Arg Trp Leu Glu Arg
   4225 4230 4235 4240
   Pro Arg Thr Asp Pro Ser Gly Asp Ala Ser Ala Met Ser Ala Arg Thr
   4245 4250 4255
   Gly Val Val Leu Tyr Arg Pro His Asp Ala Glu Glu His Ala Leu Ala
   4260 4265 4270
   Glu Glu Leu Val Ala Glu His Ala Arg Arg Gly Glu Asp Val Arg Ser
   4275 4280 4285
   Val Pro Leu Arg Asp Asp Gln Val Pro Gly Glu Leu Trp Glu Gly His
   4290 4295 4300
   Arg Ser Ala Ser Pro Gly Val Ile Tyr Phe Leu Ser Ala Gly Gly Gly
   4305 4310 4315 4320
   Ala Gln Arg Ser Arg Ser Gln Gly Pro Ala Arg Leu His Asp Val Arg
   4325 4330 4335
   Ala Ala Gln Glu Gln Gln Leu Gln Met Leu Phe Arg Leu Val Lys Ser
   4340 4345 4350
   Leu Val Ser His Gly Phe Ala Ala Arg Ser Leu Glu Leu Lys Val Val
   4355 4360 4365
   Thr Arg Asp Ala His Arg Ala Leu Gly Asp Glu Lys Val Arg Pro Met
   4370 4375 4380
   Ala Ser Ala Val Ser Gly Phe Val Arg Ser Met Ser Arg Glu Phe Leu
   4385 4390 4395 4400
   Arg Trp Asn Val Val Arg Val Asp Val Gly Gly Gln Glu Pro Ala Ser
   4405 4410 4415
   Pro Thr Met Ala Arg Ala Ile Ile Asp Glu Gln Ala Arg Ala His Glu
   4420 4425 4430
   Ala Glu Val Ala Leu Arg Glu Gly Arg Arg Tyr Glu Gln Val Leu Glu
   4435 4440 4445
   Arg Leu Val Leu Pro Ser Ala Pro Gly Ala Thr Pro Phe Lys Gln Arg
       4450                4455                4460
   Gly Val Tyr Phe Ile Val Gly Gly Leu Gly Gly Leu Gly Phe Glu Leu
   4465                4470                4475                4480
   Ser Lys Ser Leu Ala Glu Thr Val His Ala Arg Leu Val Leu Val Gly
                   4485                4490                4495
   Arg Arg Pro Ala Asp Ala Arg Met Gln Glu Gln Leu Arg Glu Leu Glu
               4500                4505                4510
   Gly Leu Gly Ala Glu Val Leu Tyr Gln Val Ala Asp Val Ser Asp Leu
           4515                4520                4525
   Ala Arg Met Gln Asp Ala Tyr Gly Gln Ala Arg Ala Arg Phe Gly Ser
       4530                4535                4540
   Val Asp Gly Val Val His Ser Ala Ile Val Leu Lys Asp Ala Ile Leu
   4545                4550                4555                4560
   Ala Asn Met Thr Glu Pro Val Leu Glu Glu Val Leu Gly Pro Lys Val
                   4565                4570                4575
   Thr Gly Thr Val Ala Leu Phe Glu Val Phe Arg Gly Val Pro Leu Asp
               4580                4585                4590
   Phe Met Leu Phe Phe Ser Ser Ala Glu Ser Phe Ile Cys Glu Ala Gly
           4595                4600                4605
   Gln Ser Asn Tyr Ala Ala Ala Ser Arg Phe Glu Asp Ser Gln Ala Ser
       4610                4615                4620
   Leu Ala Arg Gly Asp Thr Gly Phe Pro Val Val Val Ile Asn Trp Gly
   4625                4630                4635                4640
   Tyr Trp Gly Ser Val Gly Ile Val Ala Thr Glu Arg Tyr Arg Arg Glu
                   4645                4650                4655
   Leu Ala Lys Arg Gly Val Gln Ser Ile Glu Pro Ala Glu Gly Leu Glu
               4660                4665                4670
   Ile Ile Arg Arg Val Leu Ala Gln Arg Glu Arg Leu Gly Gln Val Val
           4675                4680                4685
   Val Leu Lys Gly Asp Val Ser Leu Leu Gly Thr Leu His Leu His Ala
       4690                4695                4700
   Gly Gly Arg Gln Glu Leu Leu Pro Ala Asp Arg Ala Ala Ile Pro Leu
   4705                4710                4715                4720
   Asp Pro Leu Pro Ala Val Ser Ala Asp Leu Arg Lys Leu Arg Asp Leu
                   4725                4730                4735
   Glu Gly Ala Phe Glu Arg Ile Asp Val Leu Ser Gln Asp Leu Leu Leu
               4740                4745                4750
   Gly Ala Phe Gln Arg Met Gly Val Phe Gln Ser Ala Gly Glu Arg Ala
           4755                4760                4765
   Thr Leu Ser Glu Leu Ser Ala Arg Leu Glu Val Val Asp Thr His Arg
       4770                4775                4780
   Lys Leu Phe Gln Ala Leu Leu Gly Ile Leu Glu Arg Gly Arg Ile Leu
   4785                4790                4795                4800
   Glu Arg Glu Gly Arg Ser Phe Ile Thr Thr Pro Arg Val Glu Ala Ala
                   4805                4810                4815
   Arg Ala Gly Ala Ser Arg Gly Ala Ser Glu Gln Ala Arg Leu Val Gln
               4820                4825                4830
   Glu Phe Pro Glu Leu Glu Pro His Val Arg Leu Leu Arg Thr Cys Val
           4835                4840                4845
   Gln Ser Tyr Pro Glu Val Leu Ser Gly Arg Ile Asp Ser Thr Glu Val
       4850                4855                4860
   Met Phe Pro Gly Gly Ser Thr Ser Leu Val Glu Gly Val Tyr Arg Gly
   4865                4870                4875                4880
   Ser Ala Leu Ser Asp Tyr Phe Asn His Leu Ser Ala Glu Ala Val Arg
                   4885                4890                4895
   Thr Gln Val Glu Arg Arg Leu Ala Leu Asp Ala Ser Ala Thr Val Thr
               4900                4905                4910
   Val Leu Glu Val Gly Ala Gly Thr Gly Gly Thr Ser Glu Phe Val Leu
           4915                4920                4925
   Ser Arg Leu Lys Gly Leu Gly Gly Arg Val Arg Tyr Leu Tyr Thr Asp
       4930                4935                4940
   Ile Ser Gln Ala Phe Val Arg His Gly Ala Thr Arg Tyr Gly Ala Gln
   4945                4950                4955                4960
   His Pro Phe Ala Glu Phe Val Thr Phe Asp Val Glu Thr Pro Val Asp
   4965 4970 4975
   Gln Gln Ala Val Pro Ala Gly Ser Val Asp Ile Val Leu Gly Ala Asn
   4980 4985 4990
   Val Leu His Ala Thr Arg Asp Ile Arg Arg Thr Val Ala Asn Ile Lys
   4995 5000 5005
   Arg Leu Leu Lys Ala His Gly Leu Leu Leu Val Asn Asp Thr Thr Ala
   5010 5015 5020
   Val Gln Gly Tyr Val Thr Met Thr Phe Gly Leu Met Lys Gly Trp Trp
   5025 5030 5035 5040
   Arg Phe Asp Asp Pro Ala Ala Arg Leu Glu Gly Ala Pro Leu Leu Asp
   5045 5050 5055
   Ala Gly Gln Trp Leu Ser Val Leu Gln Gly Gly Gly Phe Glu Pro Val
   5060 5065 5070
   Arg Ala Leu Gly Ser Pro Val Ala Gly Pro Glu Val Ser Arg Gln Cys
   5075 5080 5085
   Leu Leu Leu Ala Val Ser Asn Gly Leu Thr Gln Val Asp Gly Asp Val
   5090 5095 5100
   Val Ala Pro Trp Thr Pro Pro Arg Leu Glu Ser Pro Asn Thr Pro Pro
   5105 5110 5115 5120
   Arg Thr Gln Phe Pro Val Ala Ala Pro Gly Ala Leu Gln Ala Arg Val
   5125 5130 5135
   Leu Asp Tyr Val Arg Thr Val Phe Ser Glu Val Leu Gly Ile Ala Lys
   5140 5145 5150
   Glu Arg Leu Pro Glu Asp Ala Pro Leu Glu Arg Phe Gly Val Asp Ser
   5155 5160 5165
   Leu Val Asn Leu Arg Ile Val Arg Arg Phe Glu Asp Asp Leu Gly Gln
   5170 5175 5180
   Gln Pro Ser Thr Leu Leu Phe Glu Asn Met Thr Leu Leu Lys Leu Thr
   5185 5190 5195 5200
   Glu His Phe Val Thr Ala His Pro Glu Ala Leu Gly Arg Leu Phe Gly
   5205 5210 5215
   Gly Val Glu Arg Pro Ala Glu Pro Glu Ala Pro Leu Ala Pro Pro Pro
   5220 5225 5230
   Leu Glu Ser Ala Pro Arg Ser Gln Pro Arg Ala Ser Ser Leu Leu Glu
   5235 5240 5245
   Ser Ala Glu Gly Asp Val Ala Ile Ile Gly Cys Ser Gly Arg Tyr Pro
   5250 5255 5260
   Gln Ala Glu Asn Val Glu Ala Phe Trp Glu Asn Leu Arg Glu Gly Arg
   5265 5270 5275 5280
   Asp Cys Ile Gly Pro Val Pro Ser Glu Arg Trp Asn Pro Glu Gly Gln
   5285 5290 5295
   Gly Ser Asp Val Tyr Ser Arg Ser Gly Gly Phe Ile Thr Asp Ala Asp
   5300 5305 5310
   Lys Phe Asp Tyr Gly Phe Phe Asn Ile Val Pro Glu Asp Ala Asn Ala
   5315 5320 5325
   Leu Asp Pro Gln Ala Arg Ile Phe Leu Glu Thr Ala Trp His Ala Leu
   5330 5335 5340
   Glu Asp Gly Gly Gln Gly Pro Arg Ala Leu Asp Ala Ile Glu Arg Arg
   5345 5350 5355 5360
   Val Gly Ile Phe Val Gly Ala Met Asn His Asp Tyr Glu Leu Phe Ser
   5365 5370 5375
   Gly Glu Val His Gly Lys Gly Gly Phe Thr Arg Ala Gln Ser Ala Pro
   5380 5385 5390
   Trp Ala Ile Ser Asn Arg Val Ser Tyr His Leu Asp Leu His Gly Pro
   5395 5400 5405
   Ser Leu Met Val Asp Thr Ala Cys Ser Ser Ser Leu Thr Ala Leu His
   5410 5415 5420
   Leu Ala Val Glu Ser Leu Arg Arg Gly Glu Cys Arg Leu Ala Ile Ala
   5425 5430 5435 5440
   Gly Gly Val Asn Leu Ile Leu His Pro Leu His Tyr Lys Arg Leu Cys
   5445 5450 5455
   Gly Val Asn Met Leu Ser His Gly Asp Arg Asn Lys Thr Phe Gly Glu
   5460 5465 5470
   Gly Ala Asp Gly Phe Val Asp Gly Glu Gly Val Gly Ala Val Leu Leu
   5475 5480 5485
   Lys Pro Leu Arg Glu Ala Ile Arg Asp Gln Asp Phe Ile Tyr Ala Val
   5490 5495 5500
   Ile Lys Ala Thr Ala Val Asn Ser Gly Gly Lys Thr Ser Gly Phe Thr
   5505 5510 5515 5520
   Val Pro Asn Leu Asn Ala Gln Ala Glu Val Val Ala Thr Ala Leu Gln
   5525 5530 5535
   Arg Ser Gly Ile Pro Ala His Thr Leu Ser Tyr Val Glu Ala His Gly
   5540 5545 5550
   Thr Gly Thr Ala Leu Gly Asp Pro Ile Glu Val Ala Ala Leu Thr Lys
   5555 5560 5565
   Ala Phe Arg Thr Gln Thr Ser Asp Val Gly Phe Cys Ala Leu Gly Ser
   5570 5575 5580
   Val Lys Ser Asn Ile Gly His Leu Glu Ser Ala Ala Gly Ile Ala Gly
   5585 5590 5595 5600
   Leu Thr Lys Val Ile Leu Gln Leu Lys Glu Arg Thr Leu Val Pro Thr
   5605 5610 5615
   Leu His Ala Asp Val Leu Asn Pro Lys Ile Ala Phe Asp Ser Ser Pro
   5620 5625 5630
   Phe Arg Val Gln Gln Arg Leu Ala Pro Trp Val Leu Pro Ser Asn Ala
   5635 5640 5645
   Gly Pro Ser Ala Lys Arg Arg Ala Gly Leu Ser Ser Phe Gly Ala Gly
   5650 5655 5660
   Gly Ala Asn Ala His Val Ile Leu Glu Glu Tyr Val Gly Asp Ala Thr
   5665 5670 5675 5680
   Ala Arg Glu Gly Arg Ala Glu Val His Leu Pro Glu Gly Leu Phe Val
   5685 5690 5695
   Leu Ser Ala Lys Arg Pro Glu Ala Leu Lys Ala Ser Ala Ala Arg Met
   5700 5705 5710
   Arg Ala Phe Leu Arg Arg Arg Gly Gly Arg Leu Ser Leu Ala Glu Val
   5715 5720 5725
   Leu Tyr Thr Leu Gln Met Gly Arg Arg Glu Met Ser Glu Arg Leu Ala
   5730 5735 5740
   Val Val Val Thr Ser Met Asp Ala Leu Ile Glu Lys Leu Glu Ala Phe
   5745 5750 5755 5760
   Cys Glu Gly Arg Ala Ala Pro Gly Val Phe Glu Gly Thr Ala Ser Asp
   5765 5770 5775
   Gly Lys Arg Glu Gln Glu Ser Thr Arg Gly Arg Glu Leu Arg Glu Val
   5780 5785 5790
   Ala Arg Leu Trp Val Glu Gly Val Thr Phe Asp Trp Asp Ser Leu Tyr
   5795 5800 5805
   Pro Thr Arg Pro His Arg Val Pro Leu Pro Thr Tyr Ala Phe Glu Arg
   5810 5815 5820
   Arg Arg Cys Trp Phe Thr Asp Ser Ala Thr Gly Glu Glu Ala Arg Pro
   5825 5830 5835 5840
   Gln Val Gln Ala Val Ala Ala Pro Val Thr Val Pro Ser Val Met Arg
   5845 5850 5855
   Thr Thr Val Glu Pro Pro Ser Thr Ile Gln Gly Gln Ile Leu Gln Ile
   5860 5865 5870
   Val Ala Thr Val Met Lys Arg Gln Leu Ser Gly Phe Asp Ala Leu Lys
   5875 5880 5885
   Ser Leu Gln Asp Gln Gly Val Asp His Leu Asp Met Met Thr Ile Ile
   5890 5895 5900
   Leu Arg Leu Lys Ala Val Phe Gly Pro Ala Phe Glu Pro Met Thr Val
   5905 5910 5915 5920
   Val Ser Gly Lys Ser Val Ala Glu Ile Ala Ser Thr Ile Arg Ala Phe
   5925 5930 5935
   Glu Ser Pro Arg Glu Glu Ala Pro Lys Ala Val Ala Pro Asp Ala Arg
   5940 5945 5950
   Leu Gln Val Ala Pro Arg Val Arg Met Ala Ala Ala Arg Ile Leu Lys
   5955 5960 5965
   Val Asp Glu Ser Glu Val Asp Met His Val Asp Ile Ser Glu Tyr Gly
   5970 5975 5980
   Leu Asp Ser Ile Asn Val Met Phe Met Val Ala Lys Leu Arg Glu Asp
   5985 5990 5995 6000
   Phe Gly Val Gln Leu Glu Pro Gly Asp Val Val Ala His Arg Thr Leu
   6005 6010 6015
   Ala Ser Leu Ala Asp Tyr Val Ala Arg Leu Gln Ala Pro Ala Pro Ala
   6020 6025 6030
   Ala Val Gly Asp Gly Ala Ala Glu Asn Glu Trp Cys Ala
   6035 6040 6045
<210> 12
   <211> 582
   <212> PRT
   <213> Myxococcus fulvus
<220>
   <221> CHAIN
   <222> 1..582
   <223> MxnL
<400> 12
   Met Thr Lys Ala Leu Ser Thr Ser Ser Ser Arg Pro Gln Gln Leu Leu
   1               5                   10                  15
   Thr Arg Ile Leu Glu Glu Pro Glu Leu Val Ser Ile Val Gln Ser Leu
               20                  25                  30
   Glu Ala Pro Val Leu Ser Lys Leu Ile Asp His Ile Gly Leu Glu Asp
           35                  40                  45
   Ser Gly Glu Leu Val Ala Leu Ala Thr Thr Glu Gln Leu Lys Gln Ile
       50                  55                  60
   Phe Asp Glu Asp Leu Trp Lys Ser Gln Lys Pro Gly Lys Asp Ala Thr
   65                  70                  75                  80
   Phe Asp Ala Glu Arg Phe Gly Leu Trp Leu Gln Val Met Met Glu Ala
                   85                  90                  95
   Gly Val Glu Phe Ala Ala Gln Lys Leu Thr Glu Leu Asp Glu Asp Leu
               100                 105                 110
   Val Thr Leu Ala Leu Cys Lys His Val Leu Val Ile Asn Ile Asp Gln
           115                 120                 125
   Leu Ala Glu Arg Met Ser Gly Asn Ser Arg Ser Asp Asp Asp Asp Leu
       130                 135                 140
   Thr Asp Lys Ala Leu Glu Ser Cys Leu Tyr Glu Glu Phe Ala Glu Tyr
   145                 150                 155                 160
   Gln Val Ile Ala Lys Asp His Gln Ser Trp Asp Ala Ile Leu Ala Val
                   165                 170                 175
   Leu Ile Glu Leu Asp Lys Asn Ser His Asp Phe Met Thr Arg Met Leu
               180                 185                 190
   Asp Arg Cys Cys Tyr Ala Ser Thr Glu Tyr Ile Glu Asp Asn Gly Gly
           195                 200                 205
   Leu Tyr Asn Val Leu Ser Ser Glu Asp Met Leu Glu Ala Asp Val Ala
       210                 215                 220
   Ala Glu Arg Glu Asp Arg Arg Glu Gln Glu Gly Tyr Val Ala Pro Ser
   225                 230                 235                 240
   Ser Ala Thr Ser Phe Leu Asn Leu Ala Arg Val Thr Pro Thr Glu Gln
                   245                 250                 255
   Leu Ala Ala Ser Gln Ala Arg Asp His Ile Thr Gln Ser Tyr Phe Arg
               260                 265                 270
   Ala Val Glu Ala Pro Arg Arg Ala Gly Pro Lys Lys Asp Ala Gln Ala
           275                 280                 285
   Leu Pro Arg Gly Ala Pro Gly Gln Ser Ala Gln Ser Glu Lys Val Val
       290                 295                 300
   Leu Phe Leu Gln Gln Leu Arg Glu Ala Glu Val Leu Glu Glu Pro Arg
   305                 310                 315                 320
   Gln Asn Pro Leu Leu Ala Ala Gly Glu Gln Gln Val Val Arg Ala Glu
                   325                 330                 335
   Pro Leu Ser Lys His Ala Ile Ala Ala Leu Arg Glu Arg Asn Leu Thr
               340                 345                 350
   Ala Tyr Leu Glu Arg Leu Lys Glu Leu Ser Tyr Leu Ala Asn Val Leu
           355                 360                 365
   Met Ser Gly Cys Ser Phe Gly Gly Arg Val Phe Arg Pro Leu Glu Ala
       370                 375                 380
   Ala Asp Ala Ala Val Ala Val Cys Asn Leu Gly Leu Glu Arg Phe Leu
   385                 390                 395                 400
   Gln Glu Asp Ala Gly Ala Ala Val Ser Glu Met Thr Val Glu Gln Ala
                   405                 410                 415
   Ala Glu Leu Leu Glu Arg Gln Asp Leu Val Lys Leu Phe Gln Val Gly
               420                 425                 430
   Trp Ser Ile Leu His Gln Asp Val Leu Leu Phe Thr Ala Arg Ser Leu
           435                 440                 445
   Gln Asp Leu Leu Thr Arg Met Ala Ser Gly Leu Arg Asp Ala Gln Lys
       450                 455                 460
   Val Lys Glu Leu Thr Arg Met Ala Ala Thr Leu Lys Ala Asp Ile Asn
   465                 470                 475                 480
   Ala Gly Lys Pro Trp Gln Ser Arg Gly Arg Leu Asp Gly Leu Gly Leu
                   485                 490                 495
   Ile Leu Asp Arg Ser Thr Leu Leu Ser Leu Lys Ala Leu Leu Ser Glu
               500                 505                 510
   Tyr Pro Phe Leu Pro Arg Ala Ile Ala Asp Gly Ala Val Asp Thr Lys
           515                 520                 525
   Lys Arg Glu Ala Arg Phe Ile Phe Thr Ala Lys His Ile Gln Ala Val
       530                 535                 540
   Gln Asp Tyr Leu Lys Gly Leu Gly Lys Asp Pro Ala Ser Pro Ser Arg
   545                 550                 555                 560
   Gly Arg Pro Ala Ala Pro Ala Pro Ala Lys Glu Ser Pro Pro Pro Glu
                   565                 570                 575
   Lys Asp Gly Arg Arg Arg
               580
<210> 13
   <211> 326
   <212> PRT
   <213> Myxococcus fulvus
<220>
   <221> CHAIN
   <222> 1..326
   <223> MxnM
<400> 13
   Met Ser Ala Glu Thr Val Phe Leu Cys Gly Gly Gln Gly Ser Gln Phe
   1               5                   10                  15
   Phe Gly Met Gly Gln Pro Leu His Ala Glu His Pro Val Phe Arg Ala
               20                  25                  30
   Glu Leu Arg Ala Leu Asp Asp Val Ala Arg Glu Leu Leu Gly Arg Ser
           35                  40                  45
   Val Val Asp Ala Val Tyr Gly Ala Gln Arg Arg Val Thr Glu Pro Leu
       50                  55                  60
   Asp Asp Leu Leu Leu Ser His Pro Ala Ile Leu Met Val Glu Val Ala
   65                  70                  75                  80
   Leu Ala Arg Thr Leu Ala Ala Gln Gly Ile Leu Pro Ser Phe Val Val
                   85                  90                  95
   Gly Ala Ser Leu Gly Glu Leu Ala Ala Leu Val Ile Ala Gly Ala Val
               100                 105                 110
   Ser Gly Glu Asp Ala Leu Arg Ala Cys Ile Thr Gln Ala Arg Ser Leu
           115                 120                 125
   Leu Ala Arg Cys Glu Glu Gly Gly Met Val Ala Ile Leu Glu Asp Val
       130                 135                 140
   Ser Ala Phe His Arg Glu Ala Trp Leu His Glu Gly Met Trp Leu Ala
   145                 150                 155                 160
   Ala Val Asn Phe Ser Arg His Phe Val Val Ser Gly Pro Ser Trp Arg
                   165                 170                 175
   Cys Glu Glu Ile Asp Arg Arg Cys Lys Glu Lys Gly Ile Leu Cys Gln
               180                 185                 190
   Arg Leu Pro Val Arg Arg Ala Phe His Ser Pro Met Ile Asp Ala Ala
           195                 200                 205
   Glu Gly Asp Ile Leu Arg Ala Met Glu Ala Val Pro Leu Gly Arg Leu
       210                 215                 220
   Gln Val Pro Val Tyr Ser Ala Thr Gln Ala Gly Trp Ile Gly Glu Val
   225                 230                 235                 240
   Ser Pro Arg Tyr Phe Trp Gln Val Leu Arg Glu Pro Ile Arg Phe Leu
                   245                 250                 255
   Glu Ala Leu Gly Thr Val Glu Gln Leu Arg Pro Gly Thr Val Val Asp
               260                 265                 270
   Leu Ser Pro Gly Gly Thr Leu Gly Thr Phe Leu Asn His His Gly Ala
           275                 280                 285
   Gly Arg Glu Gly Lys Arg Leu Ala Cys Phe Pro Ile Met Thr Pro Phe
       290                 295                 300
   Arg Thr Asp Leu Ala Ala Leu Ala Lys Leu Asp Ala His Gln Arg Gly
   305                 310                 315                 320
   Arg Glu Ala Ser Ala Val
                   325
<210> 14
   <211> 53391
   <212> DNA
   <213> Myxococcus fulvus
<220>
   <221> source
   <222> 1..53391
   <223> /mol_type="unassigned DNA" /organism="Myxococcus fulvus"
<220>
   <221> gene
   <222> 1..53391
   <223> /gene="Myxopyronin gene cluster"
<220>
   <221> gene
   <222> 281..2677
   <223> /gene="mxnA"
<220>
   <221> gene
   <222> 2709..3716
   <223> /gene="mxnB"
<220>
   <221> gene
   <222> 3736..3987
   <223> /gene="mxnC"
<220>
   <221> gene
   <222> 4002..5273
   <223> /gene="mxnD"
<220>
   <221> gene
   <222> 5276..6508
   <223> /gene="mxnE"
<220>
   <221> gene
   <222> 6510..7271
   <223> /gene="mxnF"
<220>
   <221> gene
   <222> 7268..8032
   <223> /gene="mxnG"
<220>
   <221> gene
   <222> 8067..8927
   <223> /gene="mxnH"
<220>
   <221> gene
   <222> 8924..20644
   <223> /gene="mxnI"
<220>
   <221> gene
   <222> 20641..32229
   <223> /gene="mxnJ"
<220>
   <221> gene
   <222> 32226..50363
   <223> /gene="mxnK"
<220>
   <221> gene
   <222> 50461..52209
   <223> /gene="mxnL"
<220>
   <221> gene
   <222> 52206..53186
   <223> /gene="mxnM"
<400> 14
<210> 15
   <211> 763
   <212> PRT
   <213> Corallococcus coralloides
<220>
   <221> CHAIN
   <222> 1..763
   <223> CorA
<400> 15
   Met Gly Ala Ala Leu Phe Asp Gln Tyr Pro Arg Gln Val Gln Gln Ala
   1               5                   10                  15
   Asp Glu Ile Leu Gly Tyr Ser Ile Arg Ser Leu Cys Leu Glu Asp Pro
               20                  25                  30
   Asp Arg Arg Leu Glu Glu Thr Ser Tyr Thr Gln Pro Ala Leu Tyr Val
           35                  40                  45
   Val Ser Ala Leu Ser Tyr Leu Lys Ser Gln Glu Ala Gly Val Thr Pro
       50                  55                  60
   Ser Tyr Ala Ala Gly His Ser Leu Gly Glu Tyr Asn Ala Leu Phe Ala
   65                  70                  75                  80
   Ala Gly Val Phe Asp Phe Gln Thr Gly Leu Arg Leu Val Gln Lys Arg
                   85                  90                  95
   Gly Gln Leu Met Gly Arg Ala Lys Gly Gly Gly Met Ala Ala Val Ile
               100                 105                 110
   Gly Leu Gly Glu Glu Ala Ile Gln Gln Thr Leu Ala Ala Glu Ser Leu
           115                 120                 125
   Ser Gly Val Ser Ile Ala Asn Phe Asn Thr Pro Ser Gln Leu Val Ile
       130                 135                 140
   Ser Gly Pro Arg Asp Glu Val Ala Arg Ala Glu Ala Pro Leu Leu Ala
   145                 150                 155                 160
   Lys Gly Ala Arg His Phe Val Met Leu Arg Val Ser Ala Ala Phe His
                   165                 170                 175
   Ser Arg Tyr Met Ile Glu Ala Glu Gln Glu Phe Ser Ala Phe Leu Ser
               180                 185                 190
   Gly Phe Arg Leu Ala Pro Pro Arg Phe Pro Val Phe Ser Asn Val His
           195                 200                 205
   Ala Leu Pro Tyr Glu Pro Glu Arg Leu His Glu Thr Leu Ala Ala Gln
       210                 215                 220
   Ile Ser Arg Pro Val Arg Trp Ala Glu Ile Met Arg Arg Leu Leu Ser
   225                 230                 235                 240
   Leu Glu Asp Val Arg Phe Glu Glu Val Gly Pro Gly Asn Val Leu Thr
                   245                 250                 255
   Gly Met Leu Arg Lys Ile Arg Ala Glu Ser Ser Pro Leu Pro Phe Gln
               260                 265                 270
   Thr Pro Ala Lys Arg Ala Pro Glu Pro Ala Ser Glu Pro Ala Arg Ala
           275                 280                 285
   Glu Arg Pro Glu Pro Val Arg Pro Ala Pro Pro Val Ala Pro Ala Gly
       290                 295                 300
   Ser Ile Arg Pro Leu Ala Pro Glu Arg Leu Gly Ser Ala Glu Phe Arg
   305                 310                 315                 320
   Arg Asp Phe Gly Leu Arg Tyr Ala Cys Val Ser Gly Ala Met Tyr Arg
                   325                 330                 335
   Gly Ile Ala Ser Thr Ala Leu Val Ile Ala Met Gly Arg Ala Gly Cys
               340                 345                 350
   Met Gly Phe Phe Gly Ser Ala Gly Leu Ser Leu Glu Arg Val Glu Gln
           355                 360                 365
   Ala Ile Gln Glu Leu Gln Ala Ala Leu Gly Thr Gln Thr Pro Phe Gly
       370                 375                 380
   Met Asn Leu Leu His Asp Leu Asn Arg Pro Glu Thr Glu Asp Ala Leu
   385                 390                 395                 400
   Val Asp Leu Phe Leu Gln Arg Gly Ile Arg Phe Ile Glu Ala Ser Ala
                   405                 410                 415
   Phe Met Gln Met Thr Pro Ala Leu Val Arg Tyr Arg Ala Thr Gly Leu
               420                 425                 430
   Arg Arg Glu Ala Asp Gly Thr Ile Thr Arg Leu His Arg Val Val Gly
           435                 440                 445
   Lys Val Ser Arg Pro Glu Val Ala Gln Ser Phe Leu Ser Pro Ala Pro
       450                 455                 460
   Glu Arg Leu Leu Glu Gln Leu Val Ala Ala Arg Lys Leu Thr Pro Glu
   465                 470                 475                 480
   Gln Ala Gln Leu Ala Arg Ala Val Pro Met Ala Asp Ala Leu Thr Val
                   485                 490                 495
   Glu Ala Asp Ser Gly Gly His Thr Asp Gln Gly Val Ala Phe Ala Leu
               500                 505                 510
   Leu Pro Ala Met Met Ser Leu Arg Asp Arg Leu Ala Arg Ala Leu Pro
           515                 520                 525
   Gly Gly Pro Arg Val His Leu Gly Ala Ala Gly Gly Ile Gly Thr Pro
       530                 535                 540
   Gln Ala Ala Ala Ala Ala Phe Met Met Gly Ala Asp Tyr Ile Val Thr
   545                 550                 555                 560
   Gly Ser Ile Asn Gln Cys Thr Val Glu Ser Gly Ala Ser Asp Ala Met
                   565                 570                 575
   Lys Ala Leu Leu Gln Glu Ala Gly Val Gln Asp Thr Thr Thr Ala Pro
               580                 585                 590
   Ala Gly Asp Met Phe Glu Leu Gly Ala Arg Val Gln Val Leu Arg Arg
           595                 600                 605
   Gly Leu Phe Phe Pro Ala Arg Ala Asn Lys Leu Tyr Glu Leu Tyr Arg
       610                 615                 620
   Gln His Glu Ser Leu Asp Ser Leu Asp Ala Lys Thr Arg Asn Gln Leu
   625                 630                 635                 640
   Glu Thr Lys Tyr Phe Lys Arg Thr Leu Glu Gln Val Phe Glu Glu Cys
                   645                 650                 655
   Gln Ala Tyr Phe Arg Thr Arg Ala Pro Glu Glu Leu Glu Lys Ala Arg
               660                 665                 670
   Ala Asn Pro Lys His Lys Met Ala Leu Val Phe Arg Trp Tyr Phe Ala
           675                 680                 685
   Tyr Ser Thr Arg Leu Ala Leu Ser Gly Asp Pro Glu Gly Arg Val Asp
       690                 695                 700
   Tyr Gln Ile His Cys Gly Pro Ala Leu Gly Ala Phe Asn Ala Trp Val
   705                 710                 715                 720
   Lys Gly Thr Asp Leu Glu Pro Trp Gln Asn Arg Gly Val Ala Arg Ile
                   725                 730                 735
   Thr Thr Lys Leu Leu Cys Asp Thr Ala Leu Phe Leu Glu Glu Arg Ile
               740                 745                 750
   Ala Val Leu Gly Met Ser Ala Thr Ser Gly Val
           755                 760
<210> 16
   <211> 335
   <212> PRT
   <213> Corallococcus coralloides
<220>
   <221> CHAIN
   <222> 1..335
   <223> CorB
<400> 16
   Met Asn Gln Gly Gly Val Phe Pro Leu Pro Phe Lys Ile Ala Gly Leu
   1               5                   10                  15
   Gly Arg Tyr Val Pro Ala Asp Val Val Leu Ser Ser Asp Leu Glu Lys
               20                  25                  30
   Lys Tyr Asp Leu Pro Pro Gly Trp Cys Val Glu Lys Gln Gly Ile Arg
           35                  40                  45
   Glu Arg Arg Trp Val Lys Asp Glu Thr Ala Ser Phe Met Gly Ala Glu
       50                  55                  60
   Ala Ala Lys Glu Ala Val Arg Asp Ala Gly Leu Lys Leu Glu Asp Ile
   65                  70                  75                  80
   Asp Leu Ile Ile Asn Ala Ser Gly Ser Pro Glu Gln Ala Val Pro Asp
                   85                  90                  95
   Gly Gly Pro Leu Val Gln Arg Glu Leu Gly Leu Gly Arg Ser Gly Val
               100                 105                 110
   Pro Ser Ile Thr Val Asn Ala Ser Cys Leu Ser Phe Phe Val Ala Leu
           115                 120                 125
   Asp Val Ala Ala Asn Tyr Leu Asn Met Arg Arg Tyr Lys Arg Ile Leu
       130                 135                 140
   Ile Val Ser Ser Asp Ile Ser Ser Val Ala Leu Asp Phe Arg Lys Pro
   145                 150                 155                 160
   Glu Asn Phe Thr Leu Phe Gly Asp Ala Ala Ala Ala Ala Val Val Thr
                   165                 170                 175
   Leu Pro Glu Pro Gly Glu Lys Ser Cys Ile His Ala Ser Gln Val Arg
               180                 185                 190
   Thr Tyr Gly Tyr Gly Ala Glu Phe Ser Met Val Pro Gly Gly Gly Ser
           195                 200                 205
   Arg Arg His Pro Asn Gly Lys Asn Thr Thr Pro Glu Asp Asn Tyr Leu
       210                 215                 220
   His Met Asn Gly Ala Glu Leu Leu Lys Ile Gly Phe Glu Tyr Leu Pro
   225                 230                 235                 240
   Arg Phe Asn Glu Ala Leu Trp Lys Gln Cys Pro Asp Ile Thr Ile Lys
                   245                 250                 255
   Asp Cys Arg Tyr Val Ile Pro His Gln Pro Ser Arg Val Val Leu Asp
               260                 265                 270
   Tyr Leu Ser Leu Thr Tyr Pro Asp Asp Lys Leu Val Arg Ile Ile Asp
           275                 280                 285
   Arg Phe Ala Asn Cys Ile Gly Ala Ser Met Pro Met Ala Leu Tyr Glu
       290                 295                 300
   Ala Val Lys Val Gly Gly Leu Arg Arg Gly Glu Arg Gly Val Leu Thr
   305                 310                 315                 320
   Gly Thr Gly Ser Gly Val Ser Phe Val Gly Met Val Phe Thr Tyr
                   325                 330                 335
<210> 17
   <211> 83
   <212> PRT
   <213> Corallococcus coralloides
<220>
   <221> CHAIN
   <222> 1..83
   <223> CorC
<400> 17
   Met Gly Pro Asp Ala Ile Phe Ser Ile Val Arg Gly His Ala Ala Thr
   1               5                   10                  15
   Val Leu Ser Ile Ser Pro Asp Ser Val Pro Phe Asp Gly Arg Leu Val
               20                  25                  30
   Asp Leu Gly Ala Asn Ser Ile Asp Arg Leu Glu Ile Val Thr Met Thr
           35                  40                  45
   Met Glu Ser Leu Gly Leu Lys Phe Ser Leu Val Glu Leu Ser His Val
       50                  55                  60
   Asp Asn Ile Arg Asp Leu Val Gly Phe Phe His Ala Lys Ala Gln Arg
   65                  70                  75                  80
   Gly Ala Ala
<210> 18
   <211> 423
   <212> PRT
   <213> Corallococcus coralloides
<220>
   <221> CHAIN
   <222> 1..423
   <223> CorD
<400> 18
   Met Thr Ala Thr Ala Ile Thr Gly Met Gly Val Val Cys Ala Leu Gly
   1               5                   10                  15
   Gln Gly Val Asp Ala Leu Leu Asp Gly Leu Arg Ala Gly Arg Ser Gly
               20                  25                  30
   Ile Ala Leu Arg Glu Arg Thr Ser Thr Pro Leu Leu Glu Gly Cys Arg
           35                  40                  45
   Ile Gly Ala Glu Ile Arg Gly Phe Ala Leu Asp Val Ala Leu Glu Arg
       50                  55                  60
   Ala Pro Gly Leu Pro Glu Ala Arg Arg Ala Arg Ala Leu Lys Leu Gly
   65                  70                  75                  80
   Arg Arg Ala Pro Phe Thr Val Gln Thr Ser Ile Val Ala Ala Leu Glu
                   85                  90                  95
   Ala Trp Gly Ala Ala Gly Leu Glu Gly Ser Gly Val Asp Pro Glu Arg
               100                 105                 110
   Val Gly Val Leu Val Ala Gly Asn Asn Leu Thr Gln Gln Tyr Val Phe
           115                 120                 125
   Gln Thr His Gly Glu Tyr Gly Ser Asp Pro Thr Phe Ile Asn Pro Arg
       130                 135                 140
   Tyr Ala Leu Gln Phe Leu Asp Thr Phe His Leu Gly Val Val Ser Glu
   145                 150                 155                 160
   Leu Leu Glu Ala Arg Gly Glu Gly Cys Ser Val Gly Gly Ala Ser Ala
                   165                 170                 175
   Ser Gly Asn Val Ala Leu Ala Arg Ala His Gln Leu Leu Ala Leu Asp
               180                 185                 190
   Leu Cys Asp Val Cys Val Val Val Ala Pro Pro Ala Asp Leu Ser Val
           195                 200                 205
   Leu Glu Leu Gln Ala Leu Ala Asn Ala Gly Ala Leu Tyr Lys Asp Phe
       210                 215                 220
   Thr Asp Ala Pro Ala Arg Ala Cys Arg Pro Phe Asp Arg Arg His Asn
   225                 230                 235                 240
   Gly Phe Val Pro Gly Glu Ala Ala Ala Cys Val Val Leu Glu Arg Glu
                   245                 250                 255
   Ala Phe Ala Arg Ala Arg Gly Ala Pro Val Gln Gly Arg Leu Leu Gly
               260                 265                 270
   Val Ala Val Gln Leu Asp Gly Asn Ala Ser Ser Asp Pro Ser Val Asp
           275                 280                 285
   Gly Glu Ala Arg Thr Met Arg Arg Ala Leu Glu Gln Ala Gly Leu Gly
       290                 295                 300
   Ala Gly Asp Val Asp Tyr Val Asn Ala His Gly Ser Ala Ser Pro Leu
   305                 310                 315                 320
   Gly Asp Arg Thr Glu Val Glu Ala Leu Arg Arg Val Trp Gly Ala Arg
                   325                 330                 335
   Val Gly Glu Val Glu Val Asn Ser Thr Lys Gly Trp Thr Gly His Cys
               340                 345                 350
   Leu Gly Ala Ala Gly Leu Val Glu Ala Ile Ala Thr Val Gly Gln Leu
           355                 360                 365
   Arg His Gly Phe Val His Pro Asn Ala Asn Leu Glu Ala Pro Ile Asp
       370                 375                 380
   Gly Glu Cys Arg Phe Ala Gly Ala Ser Ala Thr Gln Arg Pro Leu Arg
   385                 390                 395                 400
   His Ala Leu Ser Asn Ser Phe Gly Phe Gly Gly Ile Asn Ala Cys Val
                   405                 410                 415
   Val Leu Gly Gly Ala Arg Thr
               420
<210> 19
   <211> 410
   <212> PRT
   <213> Corallococcus coralloides
<220>
   <221> CHAIN
   <222> 1..410
   <223> CorE
<400> 19
   Met Gln Val Gly Ile Glu Arg Met Asn Val Tyr Gly Gly Ala Ala Val
   1               5                   10                  15
   Ile Gly Val Arg Asp Ile Phe His Ala Arg Gly Leu Asn Leu Asp Arg
               20                  25                  30
   Phe Asp Asn Leu Leu Met Arg Glu Lys Ala Val Gly Leu Pro Trp Glu
           35                  40                  45
   Asp Pro Val Thr His Ala Val Asn Ala Ala Lys Ala Leu Val Asp Gly
       50                  55                  60
   Leu Thr Glu Ala Gln Lys Ala Arg Ile Glu Met Val Val Thr Ala Thr
   65                  70                  75                  80
   Glu Ser Gly Ile Asp Phe Gly Lys Ser Leu Ser Thr Tyr Val His His
                   85                  90                  95
   His Leu Gly Leu Ser Arg Arg Cys Lys Leu Phe Glu Val Lys Gln Ala
               100                 105                 110
   Cys Tyr Gly Gly Thr Ala Ala Leu Gly Ile Ala Ala Asn His Val Leu
           115                 120                 125
   Ala His Ala Ala Tyr Asp Pro Lys Val Leu Val Ile Ala Thr Asp Val
       130                 135                 140
   Ala Arg Ser Ala Asp Thr Leu Ser Tyr Ala Glu Pro Ser Gln Gly Cys
   145                 150                 155                 160
   Gly Ala Val Ala Phe Leu Val Gly Thr Ser Pro Asp Val Leu Glu Leu
                   165                 170                 175
   Asp Val Gly Ala Thr Gly Phe His Gly Tyr Glu Val Met Asp Thr Cys
               180                 185                 190
   Arg Pro Ser Tyr Glu Val Glu Thr Gly Asn Pro Asp Val Ser Leu Phe
           195                 200                 205
   Ser Tyr Leu Asp Cys Met Glu Arg Cys Phe Glu Ala Tyr Ser Glu Arg
       210                 215                 220
   Val Glu Gly Ala Asp Val Met Thr Thr Phe Gly Ala Leu Ala Phe His
   225                 230                 235                 240
   Thr Pro Phe Gly Gly Met Val Lys Gly Ala His Arg Lys Leu Val Arg
                   245                 250                 255
   Lys Phe Gly Lys Leu Asp Ser Ala Ala Ile Glu Arg Asp Phe Ala Ala
               260                 265                 270
   Arg Val Ala Pro Ser Leu Glu Tyr Cys Met Arg Val Gly Asn Leu Tyr
           275                 280                 285
   Ser Ala Ser Leu Tyr Met Ala Leu Ala Ser Leu Ile Asp His Leu Gly
       290                 295                 300
   Ala Arg Ala Glu Thr Arg Val Gly Leu Phe Ser Tyr Gly Ser Gly Cys
   305                 310                 315                 320
   Ser Ser Glu Phe Phe Ser Gly Leu Val Gly Pro Gln Ser Thr Arg Ala
                   325                 330                 335
   Leu Ala Gly Met Glu Met Gly Arg Arg Leu Asp Gln Arg Tyr Arg Leu
               340                 345                 350
   Ser Met Gln Glu Tyr Asp Ala Leu Leu Asp Asp Leu Arg Ala Phe Pro
           355                 360                 365
   Phe Gly Ala Lys Asp Leu Glu Val Gly Leu Ser Arg Phe Gly Arg Ile
       370                 375                 380
   His Glu Gln Ala Phe Ala Gly Gln Arg Arg Leu Val Leu Arg Arg Ile
   385                 390                 395                 400
   Lys Asp Tyr His Arg Glu Tyr Glu Trp Ser
                   405                 410
<210> 20
   <211> 261
   <212> PRT
   <213> Corallococcus coralloides
<220>
   <221> CHAIN
   <222> 1..261
   <223> CorF
<400> 20
   Met Arg Pro Pro Ala Ile Leu Arg Glu Ser Asp Ala Leu Ser Val Arg
   1               5                   10                  15
   Leu Thr Phe Asn Arg Thr Asp Ala Arg Asn Ser Ile Asn Leu Glu Leu
               20                  25                  30
   Leu Thr Ala Leu Asn Ala Ala Leu Asp Glu Ala Glu Arg Ser Ala Ser
           35                  40                  45
   Thr Lys Val Val Val Leu Arg Ala Gln Gly Asp Thr Phe Ser Ser Gly
       50                  55                  60
   Met Asp Phe Thr Glu Leu Leu Ala Ser Gly Glu Pro Gly Ala Ser Gly
   65                  70                  75                  80
   Met Asp Ala Ser Cys Ala Leu Tyr Phe Arg Thr Leu Arg Arg Leu Ser
                   85                  90                  95
   Gly Phe Pro Lys Leu Val Val Ser Leu Val Glu Gly Lys Val Met Ala
               100                 105                 110
   Gly Gly Val Gly Ile Val Ala Ala Ser Asp Leu Val Leu Ala Ser Arg
           115                 120                 125
   Ala Ala Ser Phe Ser Leu Pro Glu Ile Leu Trp Gly Ile Leu Pro Ala
       130                 135                 140
   Met Val Leu Pro Phe Leu Ala Arg Arg Val Gly Met His His Ala Tyr
   145                 150                 155                 160
   Thr Leu Ala Leu Thr Ala Gln Gln Leu Gly Ala Glu Arg Ala Leu Glu
                   165                 170                 175
   Leu Gly Leu Val Asp Glu Leu Gly Asp Asp Ala Glu Arg Ala Leu Gln
               180                 185                 190
   Arg Leu Leu Leu Arg Phe Lys Arg Ile Pro Ala Glu Gly Ile His Lys
           195                 200                 205
   Leu Lys Arg Tyr Ala Asn Asp Val Phe Pro Ile Asp Glu Arg Val Glu
       210                 215                 220
   Ser Leu Ala Val Gln Thr Thr Thr Gly Leu Ser Leu Asp Pro Ala Val
   225                 230                 235                 240
   Arg Gln Arg Ile His Ala Phe Leu Thr His Gln Ser Leu Pro Trp Glu
                   245                 250                 255
   Gln Glu Gly Pro Arg
               260
<210> 21
   <211> 254
   <212> PRT
   <213> Corallococcus coralloides
<220>
   <221> CHAIN
   <222> 1..254
   <223> CorG
<400> 21
   Met Ser Ser Thr Val Thr Leu Thr Arg Gln Gly Thr Val Gly Val Val
   1               5                   10                  15
   Ala Met Glu Asp Arg Gln Ser Lys Asn Thr Phe Ser Pro Ala Phe Val
               20                  25                  30
   Arg Asp Leu Lys Gln Thr Phe Asp Asp Val Ala Arg Thr Pro Asp Leu
           35                  40                  45
   Arg Val Val Val Val His Gly Phe Asp Asn Tyr Phe Cys Cys Gly Gly
       50                  55                  60
   Thr Lys Glu Glu Leu Val Gly Leu Val Glu Gly Val Gln Ser Gln Asp
   65                  70                  75                  80
   Ala Arg Gly Val Ser Leu Asp Gly Leu Leu Phe Tyr Asp Leu Phe Leu
                   85                  90                  95
   Arg Cys Asp Val Pro Val Ile Ala Ala Met Gln Gly His Ala Ile Gly
               100                 105                 110
   Gly Gly Leu Ala Leu Gly Leu Phe Ala Asp Phe Cys Ile Leu Ala Glu
           115                 120                 125
   Glu Cys Ile Tyr Ser Ala Val Phe Met Lys Tyr Gly Phe Thr Pro Gly
       130                 135                 140
   Met Gly Ser Thr Tyr Ile Leu Pro Arg Arg Leu Gly Ala Thr Leu Gly
   145                 150                 155                 160
   Ala Glu Met Leu Phe Thr Ala Arg Asn Tyr Tyr Gly Leu Glu Leu Lys
                   165                 170                 175
   Thr Arg Gly Ala Gln Val Gln Val Glu Lys Lys Ala Asp Val Val Lys
               180                 185                 190
   Ala Ala Leu Arg Met Ala Ser Asp Leu Ala Glu Lys Pro Leu Leu Ser
           195                 200                 205
   Leu Lys Leu Leu Lys Ala His Leu Asn Gln Thr Thr Arg Gln Glu Leu
       210                 215                 220
   Gln Arg Val Ile Glu Gly Glu Arg Ala Met His Arg Gln Thr Phe Ala
   225                 230                 235                 240
   Gln Pro Asp Val Arg Glu Lys Ile Glu Arg Leu Phe Gly Thr
                   245                 250
<210> 22
   <211> 286
   <212> PRT
   <213> Corallococcus coralloides
<220>
   <221> CHAIN
   <222> 1..286
   <223> CorH
<400> 22
   Met Ser Thr Ala Tyr Glu Met Val Gly Arg Thr Ala Phe Val Ile Ala
   1               5                   10                  15
   Asn Trp Arg Ala Glu Glu Thr Glu Ala Ala Ser Pro Leu Phe Ser Asp
               20                  25                  30
   His Val Ala Asn Ala Phe Leu Asn Ala Glu Ala Thr Glu Ala Ser Asn
           35                  40                  45
   Ala Ile Ala Ala Ala Ser Pro Ser Thr Arg Tyr Leu Val Arg Tyr Arg
       50                  55                  60
   Thr Arg Tyr Phe Asp Gln Thr Phe Leu Asp Lys Ile Ala Ala Gly Val
   65                  70                  75                  80
   Arg Gln Phe Leu Ile Leu Gly Ser Gly Leu Asp Thr Arg Pro Ile Arg
                   85                  90                  95
   Leu Ala Ala Asp Gly Val Arg Tyr Phe Glu Val Glu Gln Gly His Val
               100                 105                 110
   Leu Glu Phe Lys Arg Lys Gln Leu Glu Leu His Gly Tyr Ala Gln Ala
           115                 120                 125
   Ala Thr Phe Val Pro Ala Glu Tyr Thr Ser Val Asp Phe Leu Ala Leu
       130                 135                 140
   Leu Glu Ala Gln Gly Phe Asp Phe Asp Ala Gln Thr Phe Ile Leu Trp
   145                 150                 155                 160
   Glu Gly Asn Val Phe Tyr Leu Glu Tyr Gln Ser Ala Val Asn Thr Leu
                   165                 170                 175
   Ile Ala Leu Arg Asp Arg Leu Arg Arg Phe Glu Ile Thr Phe Asp Tyr
               180                 185                 190
   Leu Ser Lys Lys Leu Ile Thr Lys Ala Thr Gly Tyr Arg Lys Ser Glu
           195                 200                 205
   Ala Leu Leu Asp Gly Phe Arg Ser Leu Gly Ala Pro Trp Asn Thr Gly
       210                 215                 220
   Phe Asp Asp Ile Thr Glu Leu Thr Arg Thr Val Gly Leu Asp Val Ala
   225                 230                 235                 240
   Gly Asn Phe Leu Ile Ala Asp Tyr Ala Asn Asp Ala Gly Leu Glu Met
                   245                 250                 255
   Lys Val Asp Arg Thr Leu Phe Asp Asp Tyr Ser Ile Ala Thr Phe Ala
               260                 265                 270
   Asn Ala Ala Gly Arg Leu Asp Thr Val Ala Gly Gly Arg Ser
           275                 280                 285
<210> 23
   <211> 3883
   <212> PRT
   <213> Corallococcus coralloides
<220>
   <221> CHAIN
   <222> 1..3883
   <223> CorI
<220>
   <221> CHAIN
   <222> 416..425
   <223> CorI-CP-L
<220>
   <221> CHAIN
   <222> 1706..1712
   <223> CorI-CP-1E
<220>
   <221> CHAIN
   <222> 3166..3172
   <223> CorI-CP-2E
<220>
   <221> CHAIN
   <222> 2380..2392
   <223> CorI-DH-2E
<400> 23
   Met Ser Thr Gln Gly Thr Arg Asp Val Val Leu Val Leu Ala Arg Arg
   1               5                   10                  15
   Asp Asp Leu His Ala Arg Leu Thr Ala Arg Ala Lys Gln Val Glu Ala
               20                  25                  30
   Leu Ala Gly Ala Thr Phe Val Leu Val Lys Pro Gly Arg Val Phe Arg
           35                  40                  45
   Arg Leu Gly Glu Arg Leu Phe Gln Val Asp Pro Asp Asp Ala Ala His
       50                  55                  60
   Phe Gln Arg Leu Leu Pro Glu Val Glu Ala Ala His Gly Arg Val Arg
   65                  70                  75                  80
   Ala Ile Leu His Leu Trp Asn Tyr Glu Ala Trp Asn Val Asp Tyr Ala
                   85                  90                  95
   Gly Arg Arg Asn Leu Lys Ser Leu Thr Asp Ser Ile Ala Gly Asn Trp
               100                 105                 110
   Lys Phe Ala Leu Gly Ala Val Glu Lys Leu Arg Gly Ala Leu Ser Gly
           115                 120                 125
   Arg Gly Pro Thr Pro Leu Val Tyr Val His His Gly Glu Gly Leu Glu
       130                 135                 140
   Ala Gln Pro His Asn Ala Ile Val Gly Ala Gly Pro Ala Glu Gly Ala
   145                 150                 155                 160
   Pro Leu Val Thr His Pro Leu His Val Glu Arg Arg Leu Pro Asp Leu
                   165                 170                 175
   Asp Glu Ala Ser Arg Arg Leu Thr Ser Ala Leu Glu Ala Val Val Arg
               180                 185                 190
   Gly Gln Ser Leu Ser Gly Ser Ala Ala Ala Ala Ala Pro Ser Ala Ala
           195                 200                 205
   Glu Arg Leu Ala Cys Arg Lys Gly Gly Val Tyr Leu Val Val Glu Pro
       210                 215                 220
   Ser Gly Ala Ala Ser Arg Pro Leu Val Glu Glu Leu Thr Gly Ala His
   225                 230                 235                 240
   Gly Cys Arg Val Met Ala Val His Ala Asp Ala Leu Glu Asp Phe Glu
                   245                 250                 255
   Ala Leu Arg Ser Ala Val Arg Lys Arg Ile Gln Asp Asp Phe Gly Arg
               260                 265                 270
   Leu Asp Gly Val Ile Gln Phe Leu Glu Pro Ser Gly Asn Ile Ala Arg
           275                 280                 285
   Thr Leu Glu Gln Val Leu Ala Leu Asp Asp Ile Thr Ser Glu Leu Pro
       290                 295                 300
   Leu Asp Leu Phe Ala Leu Val Ala Pro Arg Pro Asp Ala Gly Met Ala
   305                 310                 315                 320
   Trp Ala Leu Ala Arg Ser Phe Asn Gly Leu Arg Ala Glu Leu Thr Arg
                   325                 330                 335
   Ala Gly Arg Arg Leu Gly Thr Ser Leu Phe Val Glu Gly Pro Pro Arg
               340                 345                 350
   Leu Glu Val Arg Glu Ala Asp Asp Ala Gln Ala Ser Ala Gly Ala Thr
           355                 360                 365
   Arg Ile Asp Phe Asn Ser Glu Glu Thr Arg Ala Glu Val Leu Arg Leu
       370                 375                 380
   Leu Thr Ala Asp Val Val Gly Ile Val Ala Asp Met Leu Gly Lys Pro
   385                 390                 395                 400
   Ala Ala Ser Ile Asp Leu Gly Met Ser Met Asp Leu Asp Ser Ala Ser
                   405                 410                 415
   Gly Ala His Phe Asp Ser Ile Ser Leu Thr Arg Leu Ala Asp Arg Ile
               420                 425                 430
   Gly Thr Lys Leu Gly Leu Ser Leu Thr Pro Ile Leu Phe Phe Lys Tyr
           435                 440                 445
   Lys Lys Leu Arg Ala Phe Ile Ala His Leu His Glu Ala Arg Tyr Pro
       450                 455                 460
   Ala Leu Ser Glu Arg Tyr Ala Leu Arg Leu Val Thr Arg Arg Ala Ala
   465                 470                 475                 480
   Glu Ala Pro Ala Pro Val Pro Val Ala Pro Ala Pro Ser Gln Ala Pro
                   485                 490                 495
   Val Ala Ala Pro Ala Glu Gly Pro Ala Lys Gly Trp Gly Trp Leu Glu
               500                 505                 510
   Thr Ser Gly Ala Pro Ala Ala Pro Ala Ala Glu Ala Arg Asp Val Ala
           515                 520                 525
   Ile Ile Gly Val Ser Gly Arg Phe Pro Gly Gly Gln Asp Val Phe Asp
       530                 535                 540
   Leu Trp Lys Thr Leu Thr Ser Gly Lys Asp Pro Val Thr Glu Val Pro
   545                 550                 555                 560
   Ala Glu Arg Trp Asp Trp Arg Ala Trp Phe Gly Glu Pro His Gly Ala
                   565                 570                 575
   Pro Asn Thr Thr His Val Lys Trp Gly Ser Phe Leu Asp Gly Ile Asp
               580                 585                 590
   Gln Phe Asp Pro Ala Phe Phe Gly Val Ser Glu Lys Glu Ala Val Ile
           595                 600                 605
   Met Asp Pro Ser His Arg Leu Leu Leu Glu Thr Thr Trp Arg Leu Leu
       610                 615                 620
   Glu Asp Ala Gly Tyr Arg Ala Ser Asp Val Ala Gly Ser Arg Thr Gly
   625                 630                 635                 640
   Val Phe Ile Gly Ala Gly Leu Pro Glu Tyr Asp Glu Leu Tyr Arg Ala
                   645                 650                 655
   Ser Gly Asn Ala Ile Glu Glu Gln Ser Ser Thr Gly Arg Ser Asn Ser
               660                 665                 670
   Ile Leu Ala Asn Arg Val Ser Phe Phe Phe Asp Leu Gln Gly Pro Ser
           675                 680                 685
   Glu Ala Val Asp Ala Ala Cys Ala Ser Ser Leu Val Ala Leu His Arg
       690                 695                 700
   Ala Leu Gln Ser Leu Arg Asn Glu Glu Cys Thr Met Ala Ile Ala Gly
   705                 710                 715                 720
   Gly Val His Leu Ile Leu Thr Pro Ala Met His Val Ser Phe Gly Arg
                   725                 730                 735
   Gly Gly Val Leu Ala Ala Asp Gly Lys Cys Arg Thr Phe Asp Lys Asp
               740                 745                 750
   Ala Ala Gly Tyr Thr Arg Ala Glu Gly Ile Gly Ala Val Leu Leu Lys
           755                 760                 765
   Pro Leu Arg Gln Ala Leu Ala Asp Gly Asp Asn Val Tyr Ala Val Ile
       770                 775                 780
   Arg Ala Ser Ala Val Ser His Gly Gly Arg Ser Asn Ser Met Thr Ser
   785                 790                 795                 800
   Pro Asn Pro Asn Ala Gln Glu Ala Leu Leu Leu Asp Val Tyr Gly Arg
                   805                 810                 815
   Gln Gln Val Asp Pro Ser Ser Ile Gln Tyr Val Glu Ala His Gly Thr
               820                 825                 830
   Gly Thr Pro Phe Gly Asp Ser Ile Glu Val Ala Ser Leu Gly Ala Ala
           835                 840                 845
   Phe Glu Arg Ala Ala Ala Ala Arg Gly Gln Pro Ala Arg Trp Thr Pro
       850                 855                 860
   Arg Ser Cys Gly Ile Gly Ser Leu Lys Ser Asn Ile Gly His Ala Glu
   865                 870                 875                 880
   Glu Ala Ala Gly Ile Ala Gly Val Leu Lys Val Val Leu Gly Met Arg
                   885                 890                 895
   His Gly Val Leu Pro Gly Asn Leu His Phe Asn Ala Leu Asn Pro Tyr
               900                 905                 910
   Ile His Leu Asp Gly Ser Pro Phe Tyr Ile Val Asp Arg Thr Arg Asp
           915                 920                 925
   Trp Pro Arg Pro Gln Ala Gly Pro Arg Arg Gly Ala Val Ser Ala Phe
       930                 935                 940
   Gly Phe Gly Gly Ala Leu Ala His Val Val Leu Glu Glu Val Val Ala
   945                 950                 955                 960
   Arg Pro Glu Ser Thr Asp Ala Gly Pro Lys Gly Arg Ala Tyr Ala Val
                   965                 970                 975
   Pro Leu Ser Ala Ala Thr Pro Ala Ala Leu Arg Ala Gln Ala Glu Ala
               980                 985                 990
   Leu Arg Ala Phe Leu Ala Glu Pro Pro Gly Ala Ser Leu Ala Leu Arg
           995                 1000                1005
   Asp Val Ala Phe Thr Leu Gln Val Gly Arg Glu Pro Leu Arg His Arg
       1010                1015                1020
   Leu Ala Val Val Ala His Asp Thr Ala Glu Leu Ala Ala Leu Leu Ala
   1025                1030                1035                1040
   Ala Phe Leu Arg Gly Glu Gln Pro Ser Arg Lys Leu Phe Thr Gly Gln
                   1045                1050                1055
   Ala Ala Asp Ala Ser Gln Ala Gly Gly Ala Ala Met Gly Glu Gly Asp
               1060                1065                1070
   Ala Glu Arg Ile Ala Glu Ser Trp Val Thr Gly Ala Ser Val Asp Trp
           1075                1080                1085
   Arg Arg Leu Ala Thr Ala Ala Ser Ala Arg Arg Val Ser Leu Pro Thr
   1090 1095 1100
   Tyr Arg Phe Asp Lys Arg Arg Tyr Trp Phe Thr Asp Lys Arg Pro Ser
   1105 1110 1115 1120
   Pro Gly Gly Gly Gly Gly Gly Pro Gly Gly Gly Lys Pro Val Val Pro
   1125 1130 1135
   Glu Gly Thr Gly Ala Val Leu Asp Tyr Pro Arg Glu Lys Thr Phe Ala
   1140 1145 1150
   Glu Leu Phe Ala Val Arg Ala Arg Glu Gln Gly Asp Arg Val Ala Ala
   1155 1160 1165
   Leu Cys Ala Gly Arg Ser Ile Ser Tyr Arg Gln Leu Asp Glu Gln Ser
   1170 1175 1180
   Ser Leu Val Ala Ala Ser Leu Gln Ser Leu Gly Val Thr Arg Gly Thr
   1185 1190 1195 1200
   Pro Val Gly Ile Phe Phe Asp Arg Ser Leu Glu Met Leu Ile Ser Leu
   1205 1210 1215
   Leu Gly Val Leu Lys Ser Gly Gly Thr Tyr Val Pro Leu Asp Pro Thr
   1220 1225 1230
   Phe Pro Asp Glu Arg Leu Ala Tyr Ile Ala Arg Thr Ala Glu Leu Lys
   1235 1240 1245
   Leu Gln Leu Thr Asn Arg Gly Ala His Gly Ala Pro Arg Trp Phe Ser
   1250 1255 1260
   Gly Arg Ala Val Glu Val Asp Gly Ala Phe Phe Asp Ala Ala Arg Ala
   1265 1270 1275 1280
   Arg Phe Pro Asp Ala Gly Ser Trp Met Ala Ala Arg Ala Thr Pro Glu
   1285 1290 1295
   Asp Ile Ala Tyr Val Leu Phe Thr Ser Gly Ser Thr Gly Glu Pro Lys
   1300 1305 1310
   Gly Ile Gln Val Leu His Arg Gly Leu Ala His Phe Leu Cys Ala Met
   1315 1320 1325
   Arg Gln His Leu Gly Met Thr Ser Arg Asp His Leu Leu Ala Val Thr
   1330 1335 1340
   Thr Leu Cys Phe Asp Ile Ala Gly Leu Glu Leu Phe Leu Pro Leu Val
   1345 1350 1355 1360
   Cys Gly Gly Arg Val Glu Ile Leu Thr Glu Asp Val Thr Arg Asp Gly
   1365 1370 1375
   Leu Arg Leu Lys Asp Ala Leu Asp Arg Gly Thr Ala Thr Phe Met Gln
   1380 1385 1390
   Ala Thr Pro Ala Thr Trp Gln Met Leu Leu Ser Ala Gly Trp Gln Gly
   1395 1400 1405
   Ala Arg Gly Leu Thr Ala Leu Cys Gly Gly Glu Ala Leu Gly Arg Glu
   1410 1415 1420
   Leu Ala Asp Ala Leu Leu Glu Arg Val Arg Gly Leu Trp Asn Leu Tyr
   1425 1430 1435 1440
   Gly Pro Thr Glu Thr Thr Ile Trp Ser Thr Leu Trp Lys Val Val Pro
   1445 1450 1455
   Gly Glu Ala Ile Ser Ile Gly Gly Pro Ile Gly Thr Thr Thr Leu His
   1460 1465 1470
   Val Leu Asp Glu Gln Leu Ala Pro Val Pro Pro Gly Thr Thr Gly Glu
   1475 1480 1485
   Leu Cys Ile Gly Gly Asp Gly Val Ala Lys Gly Tyr Leu His Gln Pro
   1490 1495 1500
   Glu Leu Thr Ala Arg Arg Phe Ile Arg Asp Pro Phe Ser Lys Asp Pro
   1505 1510 1515 1520
   Ser Ala Arg Leu Tyr Arg Thr Gly Asp Gln Ala Tyr Leu Arg Pro Asp
   1525 1530 1535
   Gly Met Leu Met Tyr Ala Gly Arg Ala Asp Asn Gln Val Lys Leu Arg
   1540 1545 1550
   Gly Tyr Arg Ile Glu Leu Glu Glu Ile Glu Lys Val Leu Gln Ala Ala
   1555 1560 1565
   Ala Gly Thr Pro Arg Ala Val Val Val Leu Arg Ala Asp Arg Leu Gly
   1570 1575 1580
   Gln Gln Ala Leu His Ala Phe Cys Leu Thr Asp Gly Ala Ala Pro Leu
   1585                1590                1595                1600
   Asp Val Glu Ala Leu Gln Ala Arg Leu Arg Gln Arg Leu Pro Glu Tyr
                   1605                1610                1615
   Met Val Pro Ser Ala Phe Phe Gly Met Arg Ser Phe Pro Met Thr Leu
               1620                1625                1630
   Ser Ala Lys Val Asp Arg Lys Ala Leu Ala Ala Leu Glu Leu Asp Ala
           1635                1640                1645
   Leu Leu Arg Asp His Gly Ala Gly Pro Val Pro Ser Ala Ser Ala Pro
       1650                1655                1660
   Val Arg Val Pro Pro Ala Ala Thr Ala Pro Gly Asp Leu Glu Gly Val
   1665                1670                1675                1680
   Leu Arg Glu Val Ala Ala Ser Val Leu Gly Val Gly Pro Glu Gln Leu
                   1685                1690                1695
   Gln Pro Thr Ala Thr Phe Ala Gln Ala Gly Phe Asn Ser Val Thr Leu
               1700                1705                1710
   Thr Ala Leu Arg Ala Arg Leu Leu Glu Arg Leu Ser Val Gln Val Asp
           1715                1720                1725
   Pro Pro Ala Leu Phe Lys Tyr Pro Ser Val Arg Ala Leu Ala Ala His
       1730                1735                1740
   Leu Ala Glu Arg Gly Ala Ser Pro Ala Ala Val Ala Arg Glu Glu Gly
   1745                1750                1755                1760
   Pro Glu Pro Ile Ala Ile Ile Gly Met Ala Ser Arg Phe Pro Gly Ser
                   1765                1770                1775
   Glu Asp Pro Glu Gly Phe Phe Asp Gly Leu Leu Ala Gly Arg Asp Ser
               1780                1785                1790
   Val Arg Glu Val Pro Leu Glu Arg Trp Asp Ser Arg Ala Tyr Tyr Gly
           1795                1800                1805
   Asn Pro Lys Thr Gln Lys Asn Lys Ser Asp Ala Phe Phe Gly Gly Phe
       1810                1815                1820
   Ile Ser Asp Val Asp Arg Phe Asp Ala Ala Phe Phe Asn Ile Ser Pro
   1825                1830                1835                1840
   Arg Glu Ala Arg Val Met Asp Pro Arg Gln Arg Leu Ser Leu Glu Thr
                   1845                1850                1855
   Val Tyr Lys Thr Phe Glu Asp Ala Gly Tyr Ala Pro Ser Ala Phe Ala
               1860                1865                1870
   Gly Thr Arg Thr Gly Val Phe Leu Gly Ala Ile Asn Ser Asp Tyr Trp
           1875                1880                1885
   Asp Val Gln Arg Glu His Gly Val Asp Leu Glu Gly Tyr Ser Leu Ser
       1890                1895                1900
   Gly Phe Ala Gly Ser Leu Val Pro Asn Arg Val Ser Phe Phe Phe Asp
   1905                1910                1915                1920
   Leu Leu Gly Pro Ser Glu Ala Val Asp Thr Ala Cys Ser Ser Ser Leu
                   1925                1930                1935
   Val Ala Ile His Arg Ala Val Gly Ala Leu Arg Gly Gly Glu Cys Asp
               1940                1945                1950
   Ala Ala Ile Ala Gly Gly Val Asn Leu Ile Leu Ser Pro Asn Leu Tyr
           1955                1960                1965
   Leu Ala Leu Ser Gln Asn Gly Met Leu Ser Pro Asp Gly Arg Cys Lys
       1970                1975                1980
   Ala Phe Asp Gln Ala Ala Asn Gly Tyr Val Arg Gly Glu Gly Ile Gly
   1985                1990                1995                2000
   Thr Leu Leu Leu Lys Arg Leu Ser Asp Ala Arg Arg Asp Gly Asp Thr
                   2005                2010                2015
   Ile His Ala Val Ile Arg Gly Ser Ala Val Asn His Gly Gly Arg Ala
               2020                2025                2030
   Asn Ser Leu Thr Ala Pro Asn Pro Asp Ala Gln Thr Gln Leu Leu Ala
           2035                2040                2045
   Asp Ala Tyr Ala Gln Ala Lys Val Asp Leu Arg Thr Val Ser Phe Ile
       2050                2055                2060
   Glu Thr His Gly Thr Gly Thr Lys Leu Gly Asp Ser Ile Glu Val Gln
   2065                2070                2075                2080
   Ser Leu Val Ser Ala Phe Glu Glu Ala Gly Lys Gly Lys Pro Pro Leu
                   2085                2090                2095
   Gln Pro Pro Ala Ser Cys Val Leu Gly Ala Val Lys Thr Asn Ile Gly
   2100 2105 2110
   His Leu Glu Ala Ala Ala Gly Ile Ala Gly Val Ile Lys Ala Val Met
   2115 2120 2125
   Ala Leu Arg Thr Arg Lys Leu Pro Gly Asn Leu His Phe His Ser Leu
   2130 2135 2140
   Asn Pro Leu Ile Asp Leu Ser Gly Thr Pro Leu Ser Val Ala Thr Ser
   2145 2150 2155 2160
   Thr Arg Asp Trp Pro Ala Pro Ala His Gly Gln Pro Arg Arg Ala Gly
   2165 2170 2175
   Val Ser Ser Phe Gly Phe Gly Gly Ala Ser Gly His Val Val Leu Glu
   2180 2185 2190
   Glu Tyr Pro Val Glu Arg Ala Gly Thr Gln Glu Ala Ala Gly Pro Phe
   2195 2200 2205
   Val Phe Pro Leu Ser Ala Lys Thr Pro Glu Arg Leu Arg Gln Arg Ala
   2210 2215 2220
   Gln Glu Leu Leu Ala His Leu Arg Arg His Ala Gly Gln Gly Met Arg
   2225 2230 2235 2240
   Pro Ala Asp Val Ala Tyr Thr Leu Gln Val Gly Arg Ala Ala Met Glu
   2245 2250 2255
   Ser Arg Ala Ala Phe Val Ala Ser Asp Val Ala Gly Leu Glu Gln Gln
   2260 2265 2270
   Leu Ser Ala Phe Leu Asp Gly Leu Gln Ala Ala Pro Pro Phe Pro Asp
   2275 2280 2285
   Gly Glu Ala Ala Arg Val Ala Leu Arg Trp Ala Ser Gly Glu Gly Val
   2290 2295 2300
   Asp Trp Ser Ala Leu His Val Gly Thr Gln Pro Arg Arg Val Pro Leu
   2305 2310 2315 2320
   Pro Thr Tyr Pro Phe Glu Lys Gln Arg His Trp Val Thr Glu Gly Thr
   2325 2330 2335
   Gly Pro Arg Ser Leu Pro Leu Thr Gly Glu Ala Ser Ala Pro Ala Ala
   2340 2345 2350
   Pro Gly Ala Leu Val Asp Ala Asn Glu Ser Thr Leu Asp Gly Val Ala
   2355 2360 2365
   Phe Arg Lys Arg Leu Gln Pro Glu Ala Arg Tyr Leu Arg Asp His Glu
   2370 2375 2380
   Ile Gly Gly Lys Arg Ile Leu Pro Ala Val Val Ser Leu Glu Met Ala
   2385 2390 2395 2400
   Arg Met Ala Gly Glu Leu Ala Ser Gly Gly Ala Pro Val Thr Arg Leu
   2405 2410 2415
   Arg Asn Leu Val Trp Leu Lys Pro Leu Val Val Asp Gly Ala Pro Arg
   2420 2425 2430
   Asp Ala Trp Val Arg Phe Glu Arg Arg Asp Gly Ala Val Glu Phe Lys
   2435 2440 2445
   Leu Val Thr Ala Asp Gly Asp Val His Val Gln Gly Gly Val Glu Ala
   2450 2455 2460
   Gly Pro Val Glu Gly Ala Ala Pro Val Arg Val Asp Val Asp Ala Ile
   2465 2470 2475 2480
   Arg Arg Arg Cys Ala Ala Ala Asp Glu Arg Ala Arg Phe Tyr Gln Pro
   2485 2490 2495
   Phe Glu Gly Leu Gly Tyr Ala Tyr Gly Ala Ser Leu Lys Pro Val Glu
   2500 2505 2510
   Arg Val His Arg Gly Ala His Glu Ala Leu Val Glu Leu Gln Leu Pro
   2515 2520 2525
   Glu Gly Ala Glu Ala Gly Asp Ala Tyr Val Leu His Pro Ser Leu Met
   2530 2535 2540
   Glu Gly Met Leu Gln Ser Val Ala Gly Leu Leu Phe Thr Pro Gly Ser
   2545 2550 2555 2560
   Ser Ser Asn Val Ala Phe Leu Pro Phe Ser Met Lys Ser Val Glu Leu
   2565 2570 2575
   Phe Gly Arg Ala Leu Pro Ala Ala Cys Cys Val His Val Ser Val Ala
   2580 2585 2590
   Glu Gly Gln Gly Gln Pro Ser Leu Gln Ser Arg Phe Asp Leu Thr Leu
           2595                2600                2605
   Met Ser Pro Gly Gly Glu Val Leu Val Arg Val Arg Asp Tyr Ser Val
       2610                2615                2620
   Arg Pro Val Lys Arg Asp Glu Ala Ala Lys Pro Glu Ala Ala Thr Leu
   2625                2630                2635                2640
   Tyr Arg Thr Val Leu Glu Arg Ala Asp Val Thr Pro Arg Gly Val Ser
                   2645                2650                2655
   Gly Pro Val Leu Val Val Ser Arg Asp Ala Gly Phe Val Glu Gln Leu
               2660                2665                2670
   Arg Ser Leu Leu Asp Gly Ala Gly Ala Arg Pro Ser Leu Val Trp Ala
           2675                2680                2685
   Arg Pro Ala Glu Arg Phe Gln Ala Arg Asp Asp Gly Trp Thr Phe Asp
       2690                2695                2700
   Phe Asp Ala Arg Arg Pro Glu Glu Pro Gly Arg Val Leu Glu Ala Leu
   2705                2710                2715                2720
   Arg Ala Arg Lys Leu Ser Pro Ala Ala Val Val His Ala Gly Pro Thr
                   2725                2730                2735
   Ala Ala Phe Thr Tyr Asp Ala Asp Ala Leu Lys Ala Gln Leu Gln Asp
               2740                2745                2750
   Gly Ala Leu Arg Val Phe His Leu Cys Gln Ala Leu Leu Arg Asp Gly
           2755                2760                2765
   Leu Glu Arg Pro Leu Pro Phe Leu Ser Val Phe Pro Ala Ser Glu Ala
       2770                2775                2780
   Arg Pro His Pro Leu Ser Ala Ala Leu Lys Gly Phe Ala Arg Ser Val
   2785                2790                2795                2800
   Arg Leu Glu Ser Asp Leu Leu Asn Leu Thr Val Val Gln Ala Glu Arg
                   2805                2810                2815
   Pro Glu Ser Met Ala Asp Ala Ala Phe Ala Glu Leu Ser Ala Gly Thr
               2820                2825                2830
   Thr Ala Glu Pro Phe Val Val His Arg Gly Gly Ala Arg Phe Val Glu
           2835                2840                2845
   Val Leu Ala Pro Met Ser Ser Gly Glu Ala Glu Pro Ala Arg Pro Val
       2850                2855                2860
   Leu Pro Asn Gly Gly Thr Cys Leu Ile Thr Gly Gly Ala Gly Ala Leu
   2865                2870                2875                2880
   Gly Leu His Leu Ala Arg His Leu Ala Arg Arg Pro Gly Val Arg Val
                   2885                2890                2895
   Ala Leu Cys Gly Arg Ser Ala Leu Thr Glu Ala Thr Arg Ala Arg Leu
               2900                2905                2910
   Glu Ser Leu Arg Ala Gln Gly Ala Asp Val Glu Tyr Phe Val Ala Asp
           2915                2920                2925
   Val Thr Glu Pro Gly Asp Val Glu Leu Leu Val Asp Thr Leu Arg Ala
       2930                2935                2940
   Arg Tyr Gly Gly Leu Gln Gly Val Ile His Ala Ala Gly Glu Arg Arg
   2945                2950                2955                2960
   Asp Ala Arg Leu Val Lys Lys Thr Ala Ala Glu Phe Glu Ala Val Leu
                   2965                2970                2975
   Ala Pro Lys Val Thr Gly Thr Leu Leu Leu Asp Arg Ala Leu Ala Ala
               2980                2985                2990
   Glu Lys Leu Glu Phe Phe Ala Val Phe Ser Ser Leu Val Ala Arg Thr
           2995                3000                3005
   Gly Asn Val Gly Gln Thr Asp Tyr Ala Phe Ala Asn Ala Phe Leu Glu
       3010                3015                3020
   Asp Phe Arg Ala Phe Arg Asp Gly Leu Cys Gln Arg Gly Leu Arg His
   3025                3030                3035                3040
   Gly Thr Ser His Ala Ile Ala Trp Pro Tyr Trp Arg Glu Gly Gly Met
                   3045                3050                3055
   Lys Val Asp Ala Ala Thr Glu Gln Phe Ile Arg Ser Phe Phe Gly Thr
               3060                3065                3070
   Glu Pro Leu Asp Thr Ala Thr Gly Leu Asp Ala Phe Asp Ala Val Leu
           3075                3080                3085
   Gly Arg Arg Leu Pro Glu Ala Leu Val Leu Gly Arg Gly Ala Leu Pro
       3090                3095                3100
   Ala Arg Phe Leu Lys Ala Ala Ala Glu Pro Ser Leu Pro Glu Arg Ser
   3105 3110 3115 3120
   Glu Pro Val Pro Ala Val Ala Pro Pro Arg Pro Ala Glu Leu Pro Gly
   3125 3130 3135
   Leu Leu Asp Glu Val Arg His Ile Val Ala Gly Leu Leu Gln Leu Glu
   3140 3145 3150
   Pro Ala Ser Leu Asp Pro Arg Lys Asp Leu Ala Glu Tyr Gly Tyr Asp
   3155 3160 3165
   Ser Ile Ser Phe Thr Glu Leu Ala Thr Ala Leu Asn Thr Ala Phe Gly
   3170 3175 3180
   Ser Glu Leu Thr Pro Ala Val Phe Phe Glu Leu Thr Pro Pro Ser Leu
   3185 3190 3195 3200
   Asp Ala Leu Val Ala His Leu Arg Glu Arg Tyr Gly Ala Lys Leu Ala
   3205 3210 3215
   Ala Arg Ala Thr Pro Ser Ala Pro Ala Thr Ala Gln Pro Ala Pro Val
   3220 3225 3230
   Ile Ala Val Gln Glu Ala Pro Ala Val Ala Ala Thr Ser Ser Gly Val
   3235 3240 3245
   Asp Ala Asp Glu Pro Ile Ala Ile Ile Gly Met Ser Gly Arg Met Pro
   3250 3255 3260
   Arg Ser Ala Ser Leu Glu Ala Phe Trp Lys Asn Leu Glu Glu Gly Arg
   3265 3270 3275 3280
   Ser Leu Val Ser Val Val Pro Ala Asp Arg Trp Asp Trp Arg Ala Tyr
   3285 3290 3295
   His Gly Asp Pro Lys Ala Pro Gly Arg Asn Lys Ser Val Ser Lys Trp
   3300 3305 3310
   Gly Gly Phe Leu Glu Glu Val Asp Arg Phe Asp Ala Pro Phe Phe Gly
   3315 3320 3325
   Ile Pro Ala Glu Asp Ala Arg Leu Met Asp Pro Gln Gln Arg Leu Phe
   3330 3335 3340
   Leu Glu Thr Val Trp Ser Ala Ile Glu Asp Ser Gly Arg Asp Pro Ala
   3345 3350 3355 3360
   Ser Leu Ser Gly Thr Arg Thr Gly Leu Tyr Val Gly Val Thr Asn Arg
   3365 3370 3375
   Asp Tyr Thr Asp Val Val Glu Ser Ala Val Asp Gly Val Arg Ser Ala
   3380 3385 3390
   Ala Thr Phe Gly Asn Ala His Ala Phe Leu Ala Asn Arg Val Ser Tyr
   3395 3400 3405
   Leu Leu Asn Leu His Gly Pro Ser Glu Pro Val Asp Thr Leu Cys Ser
   3410 3415 3420
   Ser Ser Leu Val Ala Ile His Lys Ala Val Glu Asp Leu Arg Arg Gly
   3425 3430 3435 3440
   Leu Cys Ser Leu Ala Leu Val Gly Gly Val Ser Val Ile Ala Ser Pro
   3445 3450 3455
   Arg Met Asp Ile Thr Phe Ser Glu Ala Gly Met Leu Ser Pro Thr Gly
   3460 3465 3470
   Ala Cys Arg Ser Phe Ser Arg Gly Ala Asp Gly Phe Val Arg Gly Glu
   3475 3480 3485
   Gly Ser Gly Ala Leu Val Leu Lys Pro Leu Arg Ala Ala Glu Ala Asp
   3490 3495 3500
   Gly Asp Arg Ile Leu Gly Val Ile Arg Ala Thr Gly Val Asn His Gly
   3505 3510 3515 3520
   Gly Arg Ala Asn Ser Leu Thr Ala Pro Asn Gly Asn Ala Gln Val Gln
   3525 3530 3535
   Leu Leu Arg Gln Val Tyr Glu Arg Ala Gly Val Asp Pro Arg Thr Val
   3540 3545 3550
   Ser Leu Ile Glu Ala His Gly Thr Gly Thr Glu Ile Gly Asp Ala Val
   3555 3560 3565
   Glu Phe Asn Ser Leu Lys Arg Ala Phe Gln Glu Met Thr Arg Glu Trp
   3570 3575 3580
   Gly Gly Glu Ala Pro Ala Arg Pro Tyr Cys Ala Val Thr Cys Val Lys
   3585 3590 3595 3600
   Thr Asn Val Gly His Leu Glu Ser Ala Ala Gly Ile Ala Gly Val Leu
                   3605                3610                3615
   Lys Val Leu Leu Ala Leu Arg His Gln Thr Leu Pro Pro Leu Ala Asn
               3620                3625                3630
   Leu Glu Glu Val Asn Pro Tyr Leu Asp Leu Gln Gly Ser Pro Phe Phe
           3635                3640                3645
   Leu Val Ala Arg Ala Thr Pro Trp Ala Ala Arg Thr Asp Ala Arg Gly
       3650                3655                3660
   His Ala Leu Pro Arg Arg Ala Gly Val Ser Ser Phe Gly Ala Gly Gly
   3665                3670                3675                3680
   Ala Asn Ala His Val Val Ile Glu Glu Tyr Val Pro Lys Ala Arg Ala
                   3685                3690                3695
   Glu Ala Ala Ala Pro Gly Pro His Val Val Pro Leu Ser Ala Arg Asp
               3700                3705                3710
   Ala Ser Ala Leu Arg Arg Tyr Ala Ala Ala Leu Arg Glu His Val Ala
           3715                3720                3725
   Ala Leu Arg Ser Gly Gly Ala Ser Ser Gln Ala Leu Ser Arg Leu Ala
       3730                3735                3740
   Tyr Thr Leu Gln Thr Gly Arg Ser Val His Gly Arg Arg Leu Ala Ile
   3745                3750                3755                3760
   Val Ala Ser Asp Leu Ala Glu Leu Glu Ser Ser Leu Ala Arg Tyr Val
                   3765                3770                3775
   Glu Gly Ala Ala Asp Glu Arg Thr His Glu Arg Ser Ala Thr Asp Arg
               3780                3785                3790
   Glu Ser Ser Pro Val Arg Gly Thr Glu Pro Arg Ala Leu Ala Ala Gly
           3795                3800                3805
   Trp Val Arg Gly Glu Ala Val Asp Trp Arg Ala Leu Tyr Gly Asp Ala
       3810                3815                3820
   Thr Pro Ala Arg Val Ala Ala Pro Thr Tyr Pro Phe Glu Arg Leu Arg
   3825                3830                3835                3840
   Tyr Trp Phe Asp Ala Glu Thr Pro Pro Ala Ala Glu Arg Lys His Arg
                   3845                3850                3855
   Asp Asp Thr Pro Leu Thr Asn Gly His Trp Glu His Ala Ala Ala Pro
               3860                3865                3870
   Arg Ala Glu Gly Leu Lys Ser Gly Arg Gly Ala
           3875                3880
<210> 24
   <211> 3817
   <212> PRT
   <213> Corallococcus coralloides
<220>
   <221> CHAIN
   <222> 1..3817
   <223> CorJ
<220>
   <221> CHAIN
   <222> 350..356
   <223> CorJ-CP-3E
<220>
   <221> CHAIN
   <222> 2206..2212
   <223> CorJ-CP-4E
<220>
   <221> CHAIN
   <222> 2935..2941
   <223> CorJ-CP-5E
<220>
   <221> CHAIN
   <222> 3530..3536
   <223> CorJ-CP*-6E
<220>
   <221> CHAIN
   <222> 3765..3771
   <223> CorJ-CP-6E
<220>
   <221> CHAIN
   <222> 44..56
   <223> CorJ-DH-3E
<220>
   <221> CHAIN
   <222> 1073..1085
   <223> CorJ-DH-4E
<220>
   <221> CHAIN
   <222> 1962..1967
   <223> CorJ-MT-4E
<400> 24
   Met Thr Val Glu Ser Asp Lys Val Thr Thr Val Ser Ser Ser Pro Gly
   1               5                   10                  15
   Gly Leu Leu Thr Arg Arg Ala Leu Arg Val Val Gly Ala Arg Arg Glu
               20                  25                  30
   Asp Gly Phe Thr Leu Thr His Asp Asp Pro Ala Leu Arg Asp His Thr
           35                  40                  45
   Val Leu Gly Gln Arg Val Leu Leu Gly Val Thr Tyr Ala Ser Trp Val
       50                  55                  60
   Leu Glu Ala Gly Arg Arg His Phe Gln Asp Arg Pro Pro Val Gly Leu
   65                  70                  75                  80
   Arg Asp Leu Leu Phe His Gln Pro Leu Val Leu Gly Pro Gly Glu Ala
                   85                  90                  95
   Ala Arg Val Thr Val Ser Val Arg Asp Ala Ser Phe Glu Val Ser Phe
               100                 105                 110
   Gln Leu Gly Ala Asp Ala Pro Pro Val Arg Cys Ala Thr Gly Thr Phe
           115                 120                 125
   Leu Phe Asp Ala Gly Ala Gly Pro Thr Pro Ala Thr Leu Asp Val Ala
       130                 135                 140
   Arg Phe Gln Arg Glu Ala Ala Arg Thr Thr Asp Gly Val Arg Val Tyr
   145                 150                 155                 160
   Glu Arg Met Arg Gln Val Ala Val Ala Tyr Gly Pro Ala Leu Phe Thr
                   165                 170                 175
   Val Gln Arg Thr Phe His Arg Asp Gly Glu Val Leu Gly Glu Leu Ala
               180                 185                 190
   Val Ala Glu Glu Ala Ser Val Gly Ser Asp Trp Leu Val Pro Pro Ala
           195                 200                 205
   Leu Leu Asp Gly Val Ile Val Ala Gly Ala Phe Glu Pro Leu Ala Ala
       210                 215                 220
   Arg Gly Arg Pro Cys Ile Pro Met Phe Val Glu Arg Ile Val Val His
   225                 230                 235                 240
   Gln Ala Pro Gly Pro Arg Cys Leu Ala Ser Ser Arg Val Arg Leu Ser
                   245                 250                 255
   Asn Asp Glu Val Leu Val Leu Asp Ala Arg Leu His Asp Ala Ser Gly
               260                 265                 270
   Arg Thr Leu Val Glu Leu Thr Gly Val Thr Leu Lys Asn Val Pro Ala
           275                 280                 285
   Leu Gly Asn Pro Phe Ser Ser Ser Ala Val Ala Ala Pro Pro Pro Ala
       290                 295                 300
   Arg Ala Pro Val Ala Pro Leu Ser Ala Gly Asp Glu Val Ala Arg Gly
   305                 310                 315                 320
   Val Glu Arg Arg Leu Arg Glu Arg Val Ala Ala Lys Leu Gly Val Ser
                   325                 330                 335
   Val Glu Gln Val Asp Pro Ala Arg Thr Phe Met Glu Ala Gly Leu Ser
               340                 345                 350
   Ser Val Ala Leu Val Glu Leu Met Thr Ala Leu Gly Thr Glu Leu Gly
           355                 360                 365
   Ala Ala Leu Ser Pro Thr Leu Val Phe Glu Phe Gln Ser Pro Arg Ala
       370                 375                 380
   Leu Ala Leu His Leu Ala Arg Glu His Ala Pro Ala Phe Ala Pro Ser
   385                 390                 395                 400
   Leu Pro Gln Arg Thr Ala Ser Val Pro Ala Ala Pro Pro Val Glu Arg
                   405                 410                 415
   Pro Arg Val Ala Ala Pro Val Ala Ala Pro Glu Pro Ala Arg Asp Glu
               420                 425                 430
   Pro Val Ala Ile Val Gly Leu Ala Gly Val Phe Pro Gly Ser Lys Asp
           435                 440                 445
   Leu Arg Glu Phe Trp Glu His Leu Ala Arg Gly Ala Asp Leu Val Thr
       450                 455                 460
   Pro Val Pro Ala Asp Arg Pro Tyr Leu Arg Ile Asp Gly Arg Glu Ser
   465                 470                 475                 480
   Trp Arg Ala Ser Phe Ile Ser Gly Val Asp Arg Phe Asp Ala Ala Phe
                   485                 490                 495
   Phe Gly Ile Thr Pro Arg Glu Ala Ala Leu Met Asp Pro Lys Gln Arg
               500                 505                 510
   Leu Leu Leu Glu Leu Val Trp Gln Ala Ile Glu Asp Ser Gly His Arg
           515                 520                 525
   Pro Ser Glu Leu Arg Gly Ser Asn Thr Gly Leu Phe Val Gly Val Gly
       530                 535                 540
   Ala Leu Glu Tyr Gly Ala Phe Gln Ala Arg Gln Gln Asp Ser Val Glu
   545                 550                 555                 560
   Ala Tyr Ser Ala Thr Gly Ile Ala Gly Thr Met Leu Pro Asn Arg Val
                   565                 570                 575
   Ser Tyr Leu Met Asp Trp His Gly Pro Ser Glu Ala Ile Asp Thr Ala
               580                 585                 590
   Cys Ser Ser Ser Leu Val Ala Ile His Arg Ala Leu Glu Ser Leu Arg
           595                 600                 605
   Asn Gly Asp Cys Glu Gln Ala Val Val Gly Gly Ile Asn Leu Ile Leu
       610                 615                 620
   Ser Pro Phe Leu Thr Leu Ala Phe Ser Arg Ala Gly Met Leu Ser Glu
   625                 630                 635                 640
   Asp Gly Arg Cys Lys Thr Phe Asp Arg Ser Ala Asp Gly Tyr Val Arg
                   645                 650                 655
   Gly Glu Gly Ala Gly Val Phe Val Leu Lys Pro Leu Ser Arg Ala Leu
               660                 665                 670
   Ala Asp Arg Asp Ala Ile His Ala Val Ile Arg Gly Ser Ala Ile Asn
           675                 680                 685
   His Gly Gly His Ala Asn Ser Leu Thr Ala Pro Asn Pro Arg Ala Gln
       690                 695                 700
   Glu Gln Leu Leu Thr Arg Ala Tyr Arg Lys Ala Arg Val Asp Pro Ala
   705                 710                 715                 720
   Thr Val Ser Tyr Val Glu Ala His Gly Thr Gly Thr Lys Leu Gly Asp
                   725                 730                 735
   Pro Val Glu Thr Arg Ala Leu Val Ser Ala Phe Gln Ala Leu Ala Ala
               740                 745                 750
   Glu Ser Gly Thr Pro Leu Ser Ala Arg Thr Cys Gly Leu Gly Ser Val
           755                 760                 765
   Lys Thr Asn Ile Gly His Leu Glu Ala Ala Ala Gly Val Ala Gly Val
       770                 775                 780
   Val Lys Val Leu Leu Ala Phe Lys His Arg Lys Leu Pro Ala Thr Val
   785                 790                 795                 800
   His Phe Arg Ala Gln Asn Glu Tyr Ile Ala Leu Glu Gly Ser Pro Phe
                   805                 810                 815
   Tyr Val Val Thr Glu Thr Thr Asp Trp Arg Arg Pro Ala Pro Ser Val
               820                 825                 830
   Pro Arg Arg Ala Gly Val Ser Ser Phe Gly Phe Gly Gly Val Asn Ala
           835                 840                 845
   His Val Val Leu Glu Glu Tyr Asp Gly Glu His Asp Ala Arg Ala Glu
       850                 855                 860
   Ala Gly Ser Thr Pro Glu Gly Pro Phe Val Phe Leu Leu Ser Ala Lys
   865                 870                 875                 880
   Thr Arg Gly Ala Leu Glu Arg Ser Ala Ala Ala Met Leu Glu Glu Leu
                   885                 890                 895
   Glu Arg Arg Pro Ser Thr Asp Arg Ala Glu Glu Gln Arg Trp Leu Gln
               900                 905                 910
   Ala Val Cys His Thr Leu Gln Val Gly Arg Glu Pro Met Pro His Arg
           915                 920                 925
   Leu Ala Val Val Val Ser Gln Val Asp Ala Leu Arg Ser Arg Leu Lys
       930                 935                 940
   Asp Phe Leu Ala Gly Lys Ala Pro Glu Ser Ala Phe Val Gly Asp Pro
   945                 950                 955                 960
   Gly Thr Arg Ser Pro Ala Leu Val Glu Ala Leu Glu Gly Glu Asp Gly
                   965                 970                 975
   Gln Asp His Val Trp Arg Leu Leu Gly Arg Gly Arg Leu Asp Lys Val
               980                 985                 990
   Ala Arg Leu Trp Val Ala Gly Leu Asp Val Asp Trp Gly Arg Leu His
           995                 1000                1005
   Thr Gly Pro Leu Pro Gln Arg Val His Leu Pro Thr Tyr Pro Phe Glu
       1010                1015                1020
   Arg Glu Arg His Trp Asp Ala Ser Leu Val Gln Gly Gly Ala Gly Arg
   1025                1030                1035                1040
   Ser Ser Ala Leu His Pro Leu Val Gly His Val Ile Pro Thr Leu Ala
                   1045                1050                1055
   Ala Asn Thr Leu Arg Tyr Glu Thr Thr Leu Thr Pro Asp Ala Pro Phe
               1060                1065                1070
   Leu Arg Asp His Val Val Gly Ser His Gly Val Leu Pro Gly Val Cys
           1075                1080                1085
   Tyr Leu Glu Met Ala Trp Arg Ala Phe Gln Leu Pro Glu Asn Glu Gly
       1090                1095                1100
   Arg Arg Phe Ser Leu Ser Arg Val Val Leu Gln Gln Arg Leu Glu Val
   1105                1110                1115                1120
   Ala Ser Pro Ala Arg Val Ser Ile Ala Leu Lys His Asp Ser Glu Gly
                   1125                1130                1135
   Thr Ala Ser Phe Ala Ile Ser Ser Val Arg Pro Asp Gly Asp Val Leu
               1140                1145                1150
   His Ala Thr Gly Glu Tyr Gln Leu Asp Ser Gly Ala Ala Pro Val Glu
           1155                1160                1165
   Arg Leu Asp Val Ala Ala Val Ala Ala Arg Cys Pro Gln Ala Leu Glu
       1170                1175                1180
   Gly Pro Glu Val Tyr Ala Arg Leu Glu Arg Val Gly Leu Arg Tyr Gly
   1185                1190                1195                1200
   Pro Phe Leu Arg Cys Leu Gln Arg Met Lys Thr Asp Gly Arg Glu Ser
                   1205                1210                1215
   Val Gly Trp Leu Ala Leu Pro Pro Glu Ala Leu Glu Gly Phe Ala Asp
               1220                1225                1230
   Tyr Gln Leu His Pro Ala Leu Leu Asp Gly Ala Leu His Val Val Leu
           1235                1240                1245
   Gly Gln Val Ala Ala Val Gln Gly Gly Ala Lys Arg Leu Leu Pro Phe
       1250                1255                1260
   Val Val Asp Ala Val Glu Val Leu Ala Pro Leu Thr Pro Ala Met Ala
   1265                1270                1275                1280
   Val Tyr Val Arg Ser Glu Asp Gly Arg Cys Phe Asp Val Ala Leu Val
                   1285                1290                1295
   Ala Asp Asp Gly Arg Val Ala Val Lys Val His Gly Leu Glu Ala Arg
               1300                1305                1310
   Glu Leu Lys Ala Glu Glu Ala Pro Leu Leu Tyr Ala Pro Tyr Phe Asp
           1315                1320                1325
   Ala Val Arg Val Asp Ala Pro Glu Ala Ser Pro Val Glu Lys Arg Gly
       1330                1335                1340
   Ala Leu Ile Val Tyr Pro Asp Asp Ala Gly Val Leu Ala Arg Ala Trp
   1345                1350                1355                1360
   Ala Asp Glu Ile Asp Glu Arg Tyr Ala Arg Thr Gln Ser Leu Ser Phe
                   1365                1370                1375
   Arg Asp Leu Glu Ala Gly Gly Leu Glu Pro Leu Leu Asp Gly Gly Phe
               1380                1385                1390
   Pro Leu Asp Ser Val Phe Phe Leu Ala Pro Gly Thr Glu Ala Lys Ser
           1395                1400                1405
   Leu Glu Ala Val Arg Ala Glu Asp Ser Arg Ala Leu Tyr Cys Leu Tyr
       1410                1415                1420
   Asp Val Val Arg Thr Leu Asp Ser Arg Gly Ala Leu Ala Gly Pro Leu
   1425                1430                1435                1440
   Asp Val Leu Val Phe Thr Val Gly Ala Leu Arg Thr Arg Ala Asp Glu
                   1445                1450                1455
   Pro Leu Leu Leu Ala Gly Ala Ala Leu Gly Gly Phe Leu Arg Ser Leu
               1460                1465                1470
   Ala Gly Glu Tyr Pro His Cys Arg Phe Arg Val Val Asp Leu Gly Ala
           1475                1480                1485
   Glu Gln Pro Leu Glu Asp Leu Arg Ser Glu Leu Leu Arg Arg Tyr Leu
       1490                1495                1500
   Gln Gly Ala Glu Leu Gln Ala Glu Val Ala Leu Arg Glu Gly Thr Leu
   1505                1510                1515                1520
   Leu Gln Arg Arg Leu Gly Ala Leu Glu Ala Pro Ala Ser Asn Ala Ala
                   1525                1530                1535
   Pro Ala Phe Arg Arg Ala Gly Thr Tyr Leu Ile Val Gly Gly Leu Gly
               1540                1545                1550
   Gly Ile Gly Leu Ala Leu Ala Glu His Leu Met Arg Asp His Gly Ala
           1555                1560                1565
   Arg Val Ala Leu Leu Gly Arg Ser Ala Pro Asp Glu Ala Met Glu Arg
       1570                1575                1580
   Gly Leu Gln Ala Leu Arg Ala Arg Gly Gly Asp Val Val Phe Val Gln
   1585                1590                1595                1600
   Ala Asp Val Thr Asp Ala Ala Ser Leu Arg Gln Ala Val Ala Arg Val
                   1605                1610                1615
   Lys Glu Arg Phe Gly Ala Ile His Gly Val Phe His Ser Ala Leu Val
               1620                1625                1630
   Leu Gln Asp Ala Leu Leu Lys Lys Leu Asp Arg Ala Arg Leu Arg Arg
           1635                1640                1645
   Val Leu Ala Pro Lys Val Ala Gly Ser Leu Val Leu Ala Glu Val Leu
       1650                1655                1660
   Ala Gly Glu Pro Leu Asp Phe Leu Val Phe Phe Ser Ser Ala Val Gly
   1665                1670                1675                1680
   Ile Ser Gly Gly Ala Gly Gln Ser His Tyr Ala Ala Ala Ser Arg Phe
                   1685                1690                1695
   Gln Asp Ala Leu Ala Leu Leu Leu Ser Gln Ala Phe Pro Phe Pro Val
               1700                1705                1710
   Lys Val Ile Asp Trp Gly Phe Trp Gly Thr Val Gly Ala Val Ala Thr
           1715                1720                1725
   Pro Glu Tyr Asn Gln Leu Leu Arg Glu Glu Gly Phe Gly Ser Ile Thr
       1730                1735                1740
   Pro Gln Glu Gly Phe Arg Ala Leu Glu Gln Val Leu Gly Leu Pro Leu
   1745                1750                1755                1760
   Pro Gln Val Val Ala Ile Lys Gly Asp Ala Arg Val Leu Gly Arg Ile
                   1765                1770                1775
   His Ala Ser Thr Arg Thr Val Val Ser Val Gln Arg Asp Gly Gly Ala
               1780                1785                1790
   Ala Leu Leu Ala Gly Val Arg Glu Gln Val Glu Ala Phe Ala Arg Ser
           1795                1800                1805
   Arg Pro Glu Glu Pro Ala Arg Arg Val Val Ala Gly Phe Glu Leu Phe
       1810                1815                1820
   Glu Glu Tyr Ala Arg Arg Asn Leu Arg Arg Ala Leu Val Asp Arg Glu
   1825                1830                1835                1840
   Pro Arg Ala Asp Glu Val Leu Pro Glu His Ser Arg Leu Tyr Arg Glu
                   1845                1850                1855
   Cys Met Ala Ile Arg Ala Arg Ala Ala Ala Gly Glu Arg Pro Leu Glu
               1860                1865                1870
   Val Leu Arg Ala Asp Val Leu Thr Arg Leu Pro Asp Leu Ala Ala Tyr
           1875                1880                1885
   Val Asp Leu Leu Asp Arg Cys Val Glu Ala Leu Pro Asp Val Trp Thr
       1890                1895                1900
   Gly Arg Arg Ser Ala Met Asp Val Met Phe Pro Gly Gly Ser Thr Gln
   1905                1910                1915                1920
   Leu Val Glu Asn Val Tyr Ala Ser Ser Leu His Ser Asp Leu Leu Asn
                   1925                1930                1935
   Ala Leu Thr Ala Glu Ala Val Arg Ala Phe Val Glu Ala Arg Leu Gly
               1940                1945                1950
   Glu Gln Pro Gly Arg Pro Leu Val Leu Val Glu Val Gly Ala Gly Thr
           1955                1960                1965
   Gly Gly Thr Thr Arg Phe Val Leu Glu Ala Leu Arg Pro Tyr Ala Ala
       1970                1975                1980
   His Val Arg Tyr Val Phe Thr Asp Leu Ser Val His Phe Leu His Lys
   1985                1990                1995                2000
   Ala Arg Glu Arg Phe Lys Asp Ser Ala Val Pro Val Glu Tyr Arg Thr
                   2005                2010                2015
   Leu Asp Leu Glu Arg Asp Ala Asp Leu Asp Gly Val Asp Leu Val Leu
               2020                2025                2030
   Gly Thr Asn Val Val His Ala Thr Ala Asp Leu Arg Ala Thr Leu Arg
           2035                2040                2045
   Arg Ile Lys Gly Met Leu Arg Arg Asn Gly Leu Leu Val Leu Asn Glu
       2050                2055                2060
   Thr Val Ala Ala Arg Asp Phe Ala Thr Leu Thr Phe Gly Thr Leu Lys
   2065                2070                2075                2080
   Gly Trp Trp Leu Tyr Arg Asp Ala Glu His Arg Ile Pro Gly Ser Pro
                   2085                2090                2095
   Val Val Ser Arg Glu Thr Trp Lys Arg Leu Leu Val Ser Glu Gly Phe
               2100                2105                2110
   Pro Gly Ala Ser Ala Cys Gly Leu Pro Gly Leu Ala Pro Gly Gln Glu
           2115                2120                2125
   Trp Gln Ser Val Leu Leu Ala Gln Ser Asp Gly Ile Thr Leu Arg Ala
       2130                2135                2140
   Arg Glu Glu Ala Arg Thr Pro Ala Pro Lys Pro Val Ala Arg Glu Ala
   2145                2150                2155                2160
   Ala Pro Val Pro Val Pro Val Pro Lys Thr Ala Pro Ala Glu Gly Gly
                   2165                2170                2175
   Thr Arg Ala Tyr Val Lys Arg Val Phe Ala Glu Val Leu Lys Gln Ser
               2180                2185                2190
   Pro Gly Ala Leu Ala Asp Asp Ala Thr Phe Asp Ala Tyr Gly Val Asp
           2195                2200                2205
   Ser Leu Leu Gly Leu Glu Val Arg Arg Arg Phe Glu Lys Asp Leu Gly
       2210                2215                2220
   Arg Leu Pro Ser Thr Leu Leu Phe Glu Gln Leu Thr Val Arg Ala Leu
   2225                2230                2235                2240
   Ser Gly Tyr Leu Glu Gln His Lys Ala Ala Glu Leu Ser Arg Val Leu
                   2245                2250                2255
   Gly Ala Ile Pro Ala Val Ala Pro Val Pro Glu Arg Arg Gln Glu Ser
               2260                2265                2270
   Val Ser Phe Glu Arg Arg Pro Ser Arg Gly Thr Pro Ala Glu Asp Arg
           2275                2280                2285
   Ser Pro Glu Pro Ala Ser Gln Asp Ile Ala Ile Thr Gly Leu Ala Gly
       2290                2295                2300
   Arg Tyr Pro Gly Ala Pro Thr Leu Asp Ala Leu Leu Asp Asn Leu Arg
   2305                2310                2315                2320
   Ala Gly Arg Ser Ala Phe Arg Thr Ile Pro Ala Glu Arg Trp Ser Gly
   2325 2330 2335
   Thr Glu Gly Gly Val Arg His Gly Ala Phe Leu Glu Asp Val Asp Arg
   2340 2345 2350
   Phe Asp Pro Leu Phe Phe Asn Ile Ser Pro Gly Glu Ala Glu Glu Met
   2355 2360 2365
   Asp Pro Gln Glu Arg Ile Leu Leu Glu Leu Ala Leu His Ala Leu Glu
   2370 2375 2380
   Asp Ala Gly Gln Pro Arg Gly Ala Leu Leu Arg Gln Pro Leu Lys Ala
   2385 2390 2395 2400
   Gly Val Phe Ile Gly Ala Met Asn Pro Asp Tyr Glu Trp Leu Gly Ala
   2405 2410 2415
   Arg Ala Ser Ala Ala Gly Thr Pro Asn Arg Ser Ser Ser Arg Phe Trp
   2420 2425 2430
   Ser Ile Ala Asn Arg Ile Ser Tyr Trp Phe Asn Leu Arg Gly Pro Ser
   2435 2440 2445
   Phe Ala Val Asp Ser Ala Cys Ser Ala Ser Leu Thr Ala Ile His Leu
   2450 2455 2460
   Ala Cys Gln Ser Leu Gln Arg Gly Glu Cys Glu Leu Ala Leu Ala Gly
   2465 2470 2475 2480
   Gly Val Asn Leu Ile Leu His Pro Asp His Leu Glu Arg Leu Ala His
   2485 2490 2495
   Ala Gly Leu Leu Thr Lys Gly Asp Ser Thr Arg Ser Phe Gly Ala Arg
   2500 2505 2510
   Ala Asp Gly Phe Val Asp Gly Glu Gly Ala Gly Leu Val Val Leu Lys
   2515 2520 2525
   Pro Leu Ala Arg Ala Arg Ala Asp Gly Asp Val Ile His Gly Val Ile
   2530 2535 2540
   Lys Gly Ser Ser Ile Asn Ala Gly Gly Lys Thr Ala Gly Tyr Leu Ala
   2545 2550 2555 2560
   Pro Asn Pro Gln Ala Gln Ala Asp Val Ile Asp Glu Ala Leu Arg Arg
   2565 2570 2575
   Ala Arg Val Pro Ala Arg Ser Ile Ser Tyr Val Glu Cys Ala Ala Ala
   2580 2585 2590
   Gly Ser Ser Met Gly Asp Ala Ile Glu Val Ser Gly Leu Lys Gln Ala
   2595 2600 2605
   Phe Ala Arg His Thr Pro Glu Arg Gly Phe Cys Ala Val Gly Ser Ile
   2610 2615 2620
   Lys Ala Asn Val Gly His Leu Glu Ser Ala Ser Gly Ile Ala Ala Leu
   2625 2630 2635 2640
   Thr Lys Val Leu Leu Gln Leu Arg Thr Arg Glu Leu Phe Pro Ser Pro
   2645 2650 2655
   His Ala Gly Thr Pro Asn Pro Glu Leu Glu Leu Ser Asp Ser Ala Phe
   2660 2665 2670
   His Leu Gln Ala Ala Leu Ala Pro Trp Arg Pro Gly Pro Ser Ala Asp
   2675 2680 2685
   Gly Gln Asp Ala Pro Leu Arg Ala Gly Ile Ser Ala Phe Gly Ala Gly
   2690 2695 2700
   Gly Ala Asn Ala His Leu Ile Val Glu Glu Tyr Arg Glu Thr Arg Pro
   2705 2710 2715 2720
   Arg Pro Glu Trp Ala Pro Gly Gln Ala Val Leu Val Pro Leu Ser Ala
   2725 2730 2735
   Lys Thr Ala Glu Arg Leu Gln Ala Leu Ala Lys Ala Leu Ala Glu His
   2740 2745 2750
   Leu Ala Arg His Arg Asp Thr Leu Phe Asp Ala Asp Val Ala Ser Ile
   2755 2760 2765
   Ala Tyr Thr Leu Gln Ile Ser Arg Glu Pro Leu Glu Glu Arg Ala Ala
   2770 2775 2780
   Phe Val Val Ala Ser Leu Gly Glu Leu Arg Glu Ala Leu Asp Gly Phe
   2785 2790 2795 2800
   Leu Ala Gly Gln Ser Leu Pro Gly Leu Arg Thr Gly His Val Arg Ala
   2805 2810 2815
   Leu Thr Glu Glu Gly Pro Ala Arg Asp Val Gly Ser Ala Arg Thr Arg
   2820 2825 2830
   Asp Ala Leu Leu Gln Val Ala Glu Gln Trp Val Ser Gly Thr Arg Val
   2835 2840 2845
   Asp Trp Arg Ser Leu His Ala Gly Thr Pro Pro Ala Arg Leu Pro Leu
   2850 2855 2860
   Pro Gly Tyr Pro Phe Ala Arg Arg Arg Cys Trp Leu Gly Asp Ala Pro
   2865 2870 2875 2880
   Val Ala Ala Ser Arg Pro Ala Ala Pro Val Glu Ala Ala Ala Pro Ala
   2885 2890 2895
   Arg Asp Ala Glu Ala Leu Val Thr Ala Val Gly Glu Asp Leu Arg Asp
   2900 2905 2910
   Ile Leu Cys His Val Leu Lys Val Arg Pro Glu Glu Leu Ser Val Glu
   2915 2920 2925
   Asp Asp Met Ser Gln Tyr Gly Leu Asp Ser Met Arg Val Val Gln Met
   2930 2935 2940
   Val Glu Ala Leu Asn Thr Arg Leu Gly Leu Asp Ala Thr Pro Pro Leu
   2945 2950 2955 2960
   Phe Tyr Gly His Val Ser Ile Gln Gly Phe Ala Arg Thr Leu Cys Gln
   2965 2970 2975
   Lys Phe Ala Asp Thr Leu Ala Thr His Tyr Gln Gly Arg Thr Pro Val
   2980 2985 2990
   Ala Pro Ala Glu Ala Pro Tyr Ala Pro Val Arg Glu Val Ala Val Ala
   2995 3000 3005
   Pro Ala Pro Arg Val Ser Thr Ser Glu Pro Ile Ala Ile Ile Gly Val
   3010 3015 3020
   Ser Gly Val Phe Pro Gly Ser Glu Asp Pro Gln Ala Phe Trp Glu His
   3025 3030 3035 3040
   Leu Leu Gln Gly His Asp Leu Val Thr Glu Val Pro Ala Gly Arg Trp
   3045 3050 3055
   Asn Ala Glu Val Leu Ala Lys Asp Ala Pro Ala Leu Arg Trp Gly Gly
   3060 3065 3070
   Phe Met Pro Gly Val Asp Gln Phe Asp Pro Glu Phe Phe Gly Leu Ser
   3075 3080 3085
   Pro Gln Glu Ala Arg Leu Met Asp Pro Gln His Arg Leu Phe Ile Glu
   3090 3095 3100
   Ala Val Trp Lys Ala Phe Glu Asp Ala Gly Gln Asp Pro Leu Ser His
   3105 3110 3115 3120
   Trp Gly Glu Arg Val Gly Val Phe Val Gly Val Ala Asn His Asp Tyr
   3125 3130 3135
   Thr Asn Leu Leu Leu Glu Arg Gln Ala Thr Ala His Met Ala Thr Gly
   3140 3145 3150
   Asn Ala His Ala Met Leu Pro Asn Arg Val Ser Tyr Leu Leu Asn Phe
   3155 3160 3165
   Thr Gly Pro Ser Glu Pro Val Asp Thr Ala Cys Ser Ser Ser Leu Val
   3170 3175 3180
   Ala Val Arg Lys Ala Val Glu Ala Leu Gln Arg Gly Asp Cys Thr Val
   3185 3190 3195 3200
   Ala Val Ala Gly Gly Val Asn Thr Leu Leu Ser Pro Asn Leu Tyr Val
   3205 3210 3215
   Ala Phe Asn Asp Ala Gly Met Leu Ser Pro Ala Gly Arg Cys Arg Thr
   3220 3225 3230
   Phe Asp Ser Arg Ala Asp Gly Tyr Ala Arg Gly Glu Gly Val Gly Ala
   3235 3240 3245
   Val Leu Leu Lys Pro Leu Arg Gln Ala Val Glu Asp Gly Asp Pro Ile
   3250 3255 3260
   Tyr Cys Val Ile Lys Gly Ala Ala Met Asn His Gly Gly His Thr Ala
   3265 3270 3275 3280
   Ser Ile Thr Ala Pro Asn Pro Met Ala Gln Arg Asp Val Ile Val Ala
   3285 3290 3295
   Ala Trp Glu Gln Ala Gly Leu Asp Pro Ser Ser Leu Gly Tyr Ile Glu
   3300 3305 3310
   Thr His Gly Thr Gly Thr Ala Leu Gly Asp Pro Ile Glu Leu Asn Ala
   3315 3320 3325
   Leu Lys Asp Val Phe Ala Glu Phe Ala Arg Arg Lys Gly Arg Pro Val
   3330 3335 3340
   Asp Thr Arg Lys His Cys Gly Ile Gly Ala Val Lys Thr Asn Ile Gly
   3345 3350 3355 3360
   His Leu Glu Thr Ala Ala Gly Ile Ala Gly Leu Val Lys Ala Ala Leu
   3365 3370 3375
   Ala Leu Lys His Gly Val Leu Pro Lys Asn Ala His Phe Glu Lys Leu
   3380 3385 3390
   Asn Pro Tyr Ile Gln Leu Asp Asp Ser Pro Leu Tyr Ile Leu Asp Asp
   3395 3400 3405
   Thr Lys Pro Trp Ala Pro Leu Val Asp Gln Asp Gly Ala Pro Leu Pro
   3410 3415 3420
   Arg Arg Ala Ala Val Ser Ser Phe Gly Phe Gly Gly Val Asn Ala His
   3425 3430 3435 3440
   Val Val Leu Glu Glu Tyr Val Ala Pro Ala Pro Ala Gln Ala Ala Lys
   3445 3450 3455
   Asp Ala Gly Pro Phe Val Val Pro Val Ser Ala Gln Thr Pro Glu Val
   3460 3465 3470
   Leu Lys Ala Ser Leu Gly Arg Leu Ala Ala His Leu Arg Ala Gly Val
   3475 3480 3485
   Gly Gly Thr Ala Ala Glu Pro Val Pro Ala Gln Glu Gly Arg Ala Val
   3490 3495 3500
   Leu Arg Asp Ile Ala Ala Arg Ile Leu Gly Val Ala Glu Ala Ala Leu
   3505 3510 3515 3520
   Asp Glu Asp Glu Leu Arg Val Asp Leu Gly Phe Gly Pro His Ala Leu
   3525 3530 3535
   Ser Arg Leu Val Glu Glu Ala Asn Ala Arg Leu Gly Thr Lys Gln Gln
   3540 3545 3550
   Val Lys Ala Leu Ala Ser Ala Glu Thr Leu Gly Ala Val Phe Asn Ala
   3555 3560 3565
   Phe Val Ala Pro Ala Ser Ser Gly Ser Ala Gly Gly Pro Ser Pro Arg
   3570 3575 3580
   Asp Leu Ala Tyr Thr Leu Gln Thr Gly Arg Met Ala Met Lys Glu Arg
   3585 3590 3595 3600
   Val Ala Phe Val Ala Arg Asp Val Pro Glu Leu Leu Arg Leu Met Asp
   3605 3610 3615
   Ala Phe Leu Arg Gly Gly Thr Gly Pro Ala Ile His Ala Gly Ser Ala
   3620 3625 3630
   Pro Ala Lys Gly Arg Phe Gln Gly Leu Arg Gly Arg Gly Thr Glu Ala
   3635 3640 3645
   Pro Glu Pro Thr Ala Glu Thr Ala Ser Pro Asp Val Leu Ala Ala Arg
   3650 3655 3660
   Trp Val Ala Gly Glu Asp Val Asp Trp Arg Ala Leu Tyr Arg Ser Gly
   3665 3670 3675 3680
   Pro Lys Pro Arg Arg Leu Ser Leu Pro Val Tyr Pro Phe Ala Arg Lys
   3685 3690 3695
   Arg Tyr Trp Phe Asp Glu Val Pro Phe Glu Pro Ser Leu Glu Pro Arg
   3700 3705 3710
   Ala Leu Gly Arg Ala Gly Thr Ala Val Glu Ala Pro Ala Lys Gln Glu
   3715 3720 3725
   Ala Ala Glu Glu Pro Gly Arg Glu Leu Gly Leu Ile Arg Val Ala Leu
   3730 3735 3740
   Ala Ala Ile Leu Gly Thr Gln Pro Asp Ser Ile Pro Asp Gly Val Glu
   3745 3750 3755 3760
   Phe Lys Asp Met Gly Leu Asp Ser Ile Met Thr Ile Lys Leu Val Lys
   3765 3770 3775
   Gln Leu Asn Gln Ala Leu Glu Leu Ser Ile Asn Pro Met Gln Val Ile
   3780 3785 3790
   Glu Asn Gln Thr Leu Gly Lys Leu Thr Arg Leu Leu Val Gly Met Arg
   3795 3800 3805
   Ser Asp Ala Gly Ala Ala Gly Ala Ala
   3810 3815
<210> 25
   <211> 4068
   <212> PRT
   <213> Corallococcus coralloides
<220>
   <221> CHAIN
   <222> 1..4068
   <223> CorK
<220>
   <221> CHAIN
   <222> 1663..1669
   <223> CorK-CP-1W
<220>
   <221> CHAIN
   <222> 3109..3115
   <223> CorK-CP-2W
<220>
   <221> CHAIN
   <222> 3900..3906
   <223> CorK-CP*-3W
<220>
   <221> CHAIN
   <222> 3995..4000
   <223> CorK-CP-3W
<220>
   <221> CHAIN
   <222> 942..954
   <223> CorK-DH-lW
<220>
   <221> CHAIN
   <222> 2319..2331
   <223> CorK-DH-2W
<400> 25
   Met Gly Ser Arg Glu Gln Ala Pro Gly Val Ala Ile Ile Gly Met Ala
   1               5                   10                  15
   Cys Arg Phe Pro Leu Ala Asp Asp Val Arg Ala Phe Trp Asp Asn Leu
               20                  25                  30
   Ala Ala Gly Arg Asp Cys Ile Arg Glu Ile Pro Pro Ser Arg Trp Asp
           35                  40                  45
   Thr Arg Lys Phe Tyr Ser Pro Asp Ile Asp Arg Pro Gly Thr Ser Val
       50                  55                  60
   Ser Arg Trp Cys Gly Leu Val Asp Gly Val Glu Arg Phe Asp His Arg
   65                  70                  75                  80
   Phe Phe Glu Ile Ser Pro Arg Glu Ala Lys Asn Met Asp Pro Gln Gln
                   85                  90                  95
   Arg Leu Leu Met Glu Glu Thr Leu His Cys Ile Glu Asp Ser Gly Val
               100                 105                 110
   Pro Leu Ser Glu Leu Arg Ala Lys Arg Thr Ser Val Phe Ala Gly Val
           115                 120                 125
   Met Ala Thr Asp Tyr His Gln Glu Ala Val His Pro Asp Val Gln Ala
       130                 135                 140
   Asp Ser Phe Ala Gly Thr Gly Asn Tyr Val Cys Ile Leu Ala Asn Arg
   145                 150                 155                 160
   Leu Ser Tyr Ala Phe Gly Leu Gln Gly Lys Ser Phe Thr Ile Asp Ala
                   165                 170                 175
   Ala Cys Ala Ser Ser Leu Val Ala Leu His Glu Ala Arg Cys Ser Leu
               180                 185                 190
   Glu Arg Gly Glu Ser Asp Tyr Ala Leu Val Ala Gly Val Ser Leu Asn
           195                 200                 205
   Leu His Pro Trp Lys Tyr Val Thr Trp Ser Lys Ser Arg Met Leu Ser
       210                 215                 220
   Pro Arg Gly Arg Cys Ser Thr Phe Asp Lys Asp Ala Asp Gly Tyr Val
   225                 230                 235                 240
   Pro Gly Asp Gly Val Gly Val Leu Leu Leu Gln Arg Arg Asp Asp Ala
                   245                 250                 255
   Arg Arg Asp Arg Asn Arg Ile His Gly Val Val Lys Gly Ser Ala Val
               260                 265                 270
   Asn His Gly Gly Arg Thr Ala Ser Met Thr Ala Pro Arg Ile Asp Ala
           275                 280                 285
   Gln Arg Asp Val Ile Leu Ala Ala Ile Ala Ser Ala Gly Ile Ser Ala
       290                 295                 300
   Arg Ser Ile Gly Tyr Val Glu Ala His Gly Thr Gly Thr Ser Leu Gly
   305                 310                 315                 320
   Asp Pro Ile Glu Val Glu Ala Leu Thr Arg Ala Phe Arg Gln Ser Thr
                   325                 330                 335
   Gln Asp Val Gln Phe Ala Lys Leu Gly Ser Val Lys Thr Asn Ile Gly
               340                 345                 350
   His Leu Glu Ala Ala Ala Gly Val Ala Gly Val Ile Lys Val Leu Leu
           355                 360                 365
   Met Met Arg His Gly Gln Val Pro Arg Ser Leu Asn Val Thr Thr Leu
       370                 375                 380
   Asn Pro Ile Ile Asp Phe Pro Gln Thr Pro Phe Ser Val Ala Leu Glu
   385                 390                 395                 400
   Pro Ser Asp Trp Pro Arg Pro Gly Lys Gly Glu Pro Arg Arg Ala Gly
                   405                 410                 415
   Val Ser Ser Phe Gly Ile Gly Gly Ala Asn Ser His Val Val Leu Glu
               420                 425                 430
   Glu His Leu Glu Asp Ala Ala Glu Pro Ala Arg Pro Ala Arg Asp Ala
           435                 440                 445
   Gly Arg Lys His Leu Phe Leu Leu Ser Ala Lys Ser Pro Gly Ser Phe
       450                 455                 460
   Ala Arg Arg Ala Glu Gln Trp Arg Glu Trp Leu Arg Gln Asn Gly Leu
   465                 470                 475                 480
   Glu Asp Ala Ala Leu Gly Asp Val Cys Val Thr Asn Ala Val Ala Ser
                   485                 490                 495
   Ala His His Pro Leu Arg Arg Gly Ala Leu Val Gly Ser Thr Glu Glu
               500                 505                 510
   Leu Arg Ala Phe Leu Asp Gly Ala Gly Arg Thr Ala Val Val Ala Ser
           515                 520                 525
   Thr Ala Pro Thr Thr Leu Ser Leu Arg Glu His Thr Val Thr Gly Phe
       530                 535                 540
   Arg Ser Val Arg Pro Leu Tyr Glu Ser Asn Ala Leu Phe Arg Asp Arg
   545                 550                 555                 560
   Val Asp Ala Leu Leu Lys Ala Cys Thr Val Pro Gly Leu Arg Gly Gly
                   565                 570                 575
   Leu Ala Thr Ala Phe Arg Ala Ala Ser Trp Ser Arg Lys Ala Arg Pro
               580                 585                 590
   Val Leu Ser Cys Leu Val Arg Tyr Ala Phe Trp Ala Thr Leu Gly Asp
           595                 600                 605
   Leu Gly Phe Gln Pro Arg Glu Val His Gly Gly Gln His Gly Phe Ala
       610                 615                 620
   Val Ala Leu Ala Leu Ser Gly Met Leu Pro Pro Glu Glu Ala Leu Gly
   625                 630                 635                 640
   Leu Val Ser Gly Ala Glu Val Gly Met Ala His Val Arg Ser Pro Ala
                   645                 650                 655
   Met Gly Val Phe Asp Pro Val Val Arg Arg Leu Ile Pro Pro Val Ser
               660                 665                 670
   Ile Arg Pro Glu Tyr Leu Ala His Leu Val Ala Asp Val Ala Leu Pro
           675                 680                 685
   Asp Glu Ala Leu Ala Ala Cys Val Thr Arg Ala Arg Gln Leu Phe His
       690                 695                 700
   Gly Gln Arg Thr Phe Gln Arg Phe Leu Glu Glu Trp Asp Gly Ala Leu
   705                 710                 715                 720
   Ala Pro Leu Gly Phe Thr Met Glu His Phe Leu His Asp Pro Arg Val
                   725                 730                 735
   Ser Gly Arg His Arg Leu Leu Leu Phe Val Ala Val Glu Ser Ala Phe
               740                 745                 750
   Arg Lys Leu Ser Thr Lys Trp Ser Phe Gln Ser Arg Pro Leu Ala Pro
           755                 760                 765
   Asp Ala Arg Leu Thr Glu Leu Ile Gly Leu Val Ala Glu Gly Val Val
       770                 775                 780
   Ser Arg Pro Ala Ala Val Glu Leu Leu Val Ala Asp Thr Pro Asp Leu
   785                 790                 795                 800
   Val Ser Leu Ala Arg Ala Ala Gln Ser Ala Ala Glu Ala Ala Pro Arg
                   805                 810                 815
   Leu Glu Pro His Pro Leu Leu Glu Ser Phe Ala Trp Gln Pro Asp Gly
               820                 825                 830
   Ala Gly Trp Leu Gln Ala Ala Leu Glu Ala Pro Pro Val Pro Ala Pro
           835                 840                 845
   Ser Gly Pro His Val Ile Glu Glu Ala Ala Leu Asp Ala Ala Glu Ala
       850                 855                 860
   Thr Asp Leu Gly Glu Leu Phe Asp Arg Thr Val Leu Ser Leu Trp Glu
   865                 870                 875                 880
   Gln Gly Val Ser Ile Thr Trp Glu Arg Leu His Pro Ala Gly Ser Tyr
                   885                 890                 895
   Ala Arg Ala Pro Leu Pro Pro Tyr Pro Phe Glu Gly Glu Ala Phe Trp
               900                 905                 910
   Ile Arg Asp Arg Asp Ala Ser Ala Pro Pro Ser Ser Thr Ala Ser Ala
           915                 920                 925
   Pro Ala Ala Arg Leu Ser Leu Ser Pro Glu Asp Pro Ile Leu Arg Asp
       930                 935                 940
   His Val Val Gly Gly His Arg Leu Ile Pro Gly Ala Leu Met Met Gly
   945                 950                 955                 960
   Leu Ser Leu Arg Ala Ala Arg Asp Ala Gly Leu Gly Glu Arg Ala Ser
                   965                 970                 975
   Leu Arg Asn Val Val Phe His Thr Pro Gly Arg Val Ser Arg Pro Val
               980                 985                 990
   Glu Leu Arg Ala Glu Leu Asp Gly Asp Val Ser Arg Val Ser Ala Asp
           995                 1000                1005
   Gly Val Gly Leu Ser Gln Ala Glu Val Ala Arg Glu Gly Glu Gly Ser
       1010                1015                1020
   Ala Arg Pro Asp Ala Pro Leu Ala Thr Asp Gly Leu Tyr Thr Val Gln
   1025                1030                1035                1040
   Asp Val Asn Arg Leu Tyr Ala Gly Leu Ala Ser Ser Gly Phe Gln Tyr
                   1045                1050                1055
   Gly Glu Ser Leu Arg Leu Leu Arg Arg Phe Gly Gly Gly Glu Ala Ala
               1060                1065                1070
   Cys Phe Ala Glu Leu Ser Thr Ala Gly Leu Arg Glu Gln Gly Asp Ala
           1075                1080                1085
   Leu Leu Pro His Val Leu Asp Thr Val Leu Gln Ala Gly Leu Leu Thr
       1090                1095                1100
   Gly Tyr Val Gly Gly Pro Leu Val Phe Gly Lys Asp Arg Phe Val Pro
   1105                1110                1115                1120
   Ala Phe Val Gly Ala Leu Arg Cys Phe Gly Thr Leu Pro Pro Val Leu
                   1125                1130                1135
   Arg Leu Leu Val Glu Arg Lys Pro Glu Lys Gly Gly Glu His Arg Phe
               1140                1145                1150
   His Val Asp Ile Ile Gly Gln Asp Ala Arg Gly Gln Arg Val Leu Glu
           1155                1160                1165
   Leu His Glu Val Val Tyr Gln Arg Ala Asp Ala Ala Pro Pro Ala Arg
       1170                1175                1180
   Ser Ala Ala Gly Trp Glu Met Lys Ala Pro Tyr Trp Arg Glu Leu Pro
   1185                1190                1195                1200
   Leu Pro Ala Ala His Ala Thr Ala Ser Gln Ala Ala Val Ile Gly Ala
                   1205                1210                1215
   Arg Val Gln Ala Phe Val Glu Arg Ala Arg Arg Val His Gly Ala Val
               1220                1225                1230
   Gln Ala Val Ser Pro Asp Gly Asp Val Gly Ala Leu Val Glu Ser Leu
           1235                1240                1245
   Thr Arg Ala Pro Gly Ala Ala Thr Leu Tyr Phe Leu Gly Ala Trp Glu
       1250                1255                1260
   Phe Ser Gly Leu Pro Asp Ala Glu Arg Ala Leu Arg Thr Leu Phe His
   1265                1270                1275                1280
   Leu Val Lys Ala His Leu Ser Arg Ala Ser Asp Arg Glu Leu Arg Ile
                   1285                1290                1295
   Val Val Ala Val Arg Asp Ala Phe Val Ala Thr Pro Glu Asp Arg Gly
               1300                1305                1310
   Ser Gly Phe Ala Ala Ala Ala Leu Leu Gly Leu Ala Lys Thr Ala Met
           1315                1320                1325
   Arg Glu Ser Pro Arg Leu Gln Val Val Leu Val Asp Val Asp Leu Ser
       1330                1335                1340
   Asn Asp Ala Gly Val Asp Ala Leu Leu Arg Glu Gly Thr Ala Gly Gln
   1345                1350                1355                1360
   Ala Ser Glu Glu Pro Val Ala Trp Arg His Gly Arg Arg Leu Val Arg
                   1365                1370                1375
   Glu Leu Arg Pro Val Pro Val Asp Gly Ala Gly Ala Glu Asn Pro Phe
               1380                1385                1390
   Arg Ala Gly Lys Asn Tyr Leu Leu Val Gly Gly Gly Gly Gly Ile Gly
           1395                1400                1405
   Arg Ala Leu Leu Asp Ala Leu Ala Ser Arg Val Ala Cys Arg Val Phe
       1410                1415                1420
   Val Leu Gly Arg Ser Glu Arg Ala Pro Leu Asp Gly Leu Ala Ser Arg
   1425                1430                1435                1440
   Ala Cys Glu Leu His Phe Leu Arg Cys Asp Val Thr Asp Gly Ala Gln
                   1445                1450                1455
   Val Asp Gly Val Val Arg Arg Ile Thr Arg Asp Phe Gly Pro Leu His
               1460                1465                1470
   Gly Val Leu His Leu Gly Gly Val Leu Ser Asp Arg Leu Leu Met Ala
           1475                1480                1485
   Lys Ser Trp Glu Asp Phe Gln Arg Val Leu Ala Pro Lys Leu Ala Gly
       1490                1495                1500
   Thr Leu Asn Leu His Gln Ala Thr Leu Ala Gln Pro Leu Asp Phe Phe
   1505                1510                1515                1520
   Val Gly Phe Ser Ser Ile Val Ala Leu Thr Gly Asn Val Gly Gln Ala
                   1525                1530                1535
   Asp Tyr Ala Ala Gly Asn Ser Phe Leu Asp Ala Phe Met Ala Phe Arg
               1540                1545                1550
   Asp Arg Ser Gly Ala Pro Gly Leu Ser Arg Val Val Asn Trp Pro Leu
           1555                1560                1565
   Trp Glu Gly Val Gly Met Ala Gln Gly Leu Thr Gly Glu Leu Arg His
       1570                1575                1580
   Leu Arg Pro Ile Ala Ala Ala Glu Gly Val Lys Ala Phe Thr Ala Ala
   1585                1590                1595                1600
   Leu Ser Gln Arg Glu His Ala Gln Val Leu Val Gln Gly Gln Glu Ala
                   1605                1610                1615
   Pro Ala Ala Pro Ala Arg Pro Ala Arg Ala Glu Gly Val Arg Lys Arg
               1620                1625                1630
   Asp Val Glu Gly Tyr Leu Arg Asp Leu Ile Ala Ala Arg Leu Gly Leu
           1635                1640                1645
   Pro Gly Pro Gly Phe Asp Ala Thr Glu Ser Phe Phe Thr Met Gly Leu
       1650                1655                1660
   Asp Ser Leu Ile Leu Gln Glu Leu Met Ala Glu Leu Asn Arg Thr Phe
   1665                1670                1675                1680
   Arg Glu Leu Pro Pro Thr Leu Leu Phe Glu His Pro Asn Leu Gln Lys
   1685 1690 1695
   Leu Val Ala Trp Phe Glu Lys Glu Gly Leu Ser Gly Pro Ala Glu Pro
   1700 1705 1710
   Glu Ala Pro Ala Ala Thr Pro Ala Leu Pro Glu Pro Ala Arg Ala Ala
   1715 1720 1725
   Pro Val Arg Gln Gly Tyr Asp Ile Ala Ile Ile Gly Met Ser Gly Arg
   1730 1735 1740
   Phe Pro Leu Ala Arg Thr Val Pro Glu Phe Trp Arg Asn Leu Val Asp
   1745 1750 1755 1760
   Gly Val Asp Cys Ile Gln Glu Ile Pro Pro Glu Arg Trp Asp Ala Asn
   1765 1770 1775
   Ala Ile Gln Gly Ser Arg Ser Asp Val Gly Ala Arg Ser Tyr Gly Arg
   1780 1785 1790
   Trp Gly Gly Phe Ile Glu Asp Val Asp Arg Phe Asp Pro Leu Phe Phe
   1795 1800 1805
   Asn Val Ser Pro Lys Glu Ala Glu Glu Met Asp Pro Gln Gln Arg Leu
   1810 1815 1820
   Phe Leu Glu Cys Val Trp Glu Thr Leu Glu Asp Ala Gly Tyr Ala Ala
   1825 1830 1835 1840
   Arg Lys Arg Tyr Ala His Gln Ser Val Gly Leu Phe Val Gly Ser Met
   1845 1850 1855
   Trp Asn Glu Tyr Ser Leu Leu Ala His Glu Gln Gly Leu Leu Gln Gly
   1860 1865 1870
   Asp Tyr Lys Gly Pro Gly Ser Leu Ser Trp Ala Ile Ala Asn Arg Val
   1875 1880 1885
   Ser Phe Phe Met Asp Phe Lys Gly Pro Ser Leu Ala Val Asp Thr Ala
   1890 1895 1900
   Cys Ser Ser Ser Leu Val Ser Ile His Leu Ala Cys Gln Ser Leu Leu
   1905 1910 1915 1920
   Thr Gly Glu Cys Asp Met Ala Val Ala Gly Gly Val Asn Leu Ser Ile
   1925 1930 1935
   His Pro Gly Lys Tyr Val Tyr Leu Ser Gln Ala Lys Phe Leu Ser Ser
   1940 1945 1950
   Asp Gly Arg Cys Arg Ser Phe Gly Glu Gly Gly Asp Gly Tyr Val Pro
   1955 1960 1965
   Gly Glu Gly Val Gly Ala Val Leu Leu Lys Pro Leu Ala Ala Ala Leu
   1970 1975 1980
   Ala Asp Gly Asp Arg Ile His Ala Val Ile Arg Gly Ser Ala Ser Asn
   1985 1990 1995 2000
   His Gly Gly Lys Ala Ala Gly Phe Thr Val Pro Asn Pro Glu Gln His
   2005 2010 2015
   Ala Arg Leu Ile Gln Lys Ser Met Ala Arg Ala Ala Val Gly Pro Glu
   2020 2025 2030
   Asp Ile Gly Tyr Val Glu Cys His Gly Thr Gly Thr Ser Leu Gly Asp
   2035 2040 2045
   Pro Ile Glu Ile Arg Gly Leu Gln Leu Ala Phe Gly Glu Gln Arg Gly
   2050 2055 2060
   Arg Glu His Pro Cys Pro Ile Gly Ser Val Lys Ser Asn Ile Gly His
   2065 2070 2075 2080
   Leu Glu Ala Ala Ala Gly Ile Ala Ala Val Ile Lys Val Ala Leu Ala
   2085 2090 2095
   Met Arg His Arg Val Leu Pro Arg Ser Leu His Gly Asp Val Glu Asn
   2100 2105 2110
   Pro Lys Leu Asp Leu Ala Gln Thr Pro Phe Gln Val Val Lys Gln Thr
   2115 2120 2125
   Arg Gly Trp Asp Val Pro Ala Gly Arg Thr Arg Ile Ala Ala Ile Ser
   2130 2135 2140
   Ser Phe Gly Ala Gly Gly Ser Asn Ala His Leu Val Leu Glu Glu Phe
   2145 2150 2155 2160
   Pro Arg Ala Glu Ala Val Arg Asp Glu Thr Pro Val Pro Arg Leu Phe
   2165 2170 2175
   Val Phe Ser Ala Arg Asp Ala Glu Arg Leu Glu Ala Gln Val Ala Arg
                   2180                2185                2190
   Leu Arg Asp Phe Leu Asp Thr Glu Glu Gly Arg Ala Cys Pro Leu Ser
           2195                2200                2205
   Asp Val Ala His Thr Leu Gln Val Gly Arg Gln Ala Phe Asn Thr Arg
       2210                2215                2220
   Trp Thr Val Val Ala Arg Asp Arg Asp Ser Leu Arg Ala Ala Leu Ala
   2225                2230                2235                2240
   Ala Tyr Pro Arg Glu Ala Met Pro Gly Glu Asp Ala Arg Ser Val Glu
                       2245                2250                2255
   Leu Ala Arg Leu Ala Glu Thr Trp Arg Gln Gly Gly Ser Val Asp Trp
                   2260                2265                2270
   Ala Arg Ala Ser Asp Gly Val Pro Gly Arg Ile Val Ser Leu Pro Thr
           2275                2280                2285
   Tyr Pro Phe Arg Arg Glu Arg Phe Trp Ile Asp Gly Pro Ala Pro Arg
       2290                2295                2300
   Pro Pro Ala Leu Thr Gln Met Val Ser Glu Gln Glu Pro Leu Leu Ala
   2305                2310                2315                2320
   Asp His Val Leu His Gly Glu Pro Thr Leu Pro Gly Val Phe Met Leu
                       2325                2330                2335
   Ala Lys Leu Leu Ala Thr Glu Gly Val Thr Gly Pro Gly Thr Arg Ala
                   2340                2345                2350
   Arg Arg Ile Arg Ser Ala Val Trp Thr Arg Pro Leu Arg Val Arg Gly
           2355                2360                2365
   Ser Ala Ala Val Glu Leu Ser Leu Glu Pro Lys Glu Asp Arg Ile Arg
       2370                2375                2380
   Phe Gly Leu Trp Ser Gly Thr Gly Gln Glu Arg Arg Cys Tyr Ala Glu
   2385                2390                2395                2400
   Gly Ser Met Leu Ala Gly Asp Ala Ala Pro Val Arg Pro Leu Asp Leu
                       2405                2410                2415
   Leu Ala Val Arg Glu Arg Cys Pro Ala Leu Lys Thr Gly Ala Ala Cys
                   2420                2425                2430
   Tyr Thr Ala Phe Thr Ala Leu Gly Leu Arg Tyr Gly Ala Arg Phe Gln
           2435                2440                2445
   Arg Val Gly Gln Val Ser Leu Gly Ala Gly Glu Ala Leu Ala Arg Leu
       2450                2455                2460
   Glu Leu Asp Pro Arg Glu Arg Gly Glu Ala Ala Trp Val Ser Leu Leu
   2465                2470                2475                2480
   Asp Gly Ala Leu Gln Ser Ala Leu Ala Leu Leu Leu His Thr Gly His
                       2485                2490                2495
   Ala Thr Glu Ala His Val Pro Phe Ser Leu Gly Ala Leu Asp Val Leu
                   2500                2505                2510
   Gly Glu Ala Gly Pro Glu Pro His Ala His Val Arg Met Gly Glu Ala
           2515                2520                2525
   Ser Arg Gly Gly Phe Arg Phe Asp Ile Asp Leu Thr Asp Gly Thr Gly
       2530                2535                2540
   Arg Val Phe Ala Thr Leu Arg Asp Val Phe Leu Gly Arg Met Ala Ala
   2545                2550                2555                2560
   Pro Lys Ala Ala Ala Gly Thr Gly Ala Gln Ala Pro Ala Thr Asp Gly
                       2565                2570                2575
   Asp Val Phe Gln Gly Ser Met Ala Ala Pro Glu Val Glu Ala Gly Thr
                   2580                2585                2590
   Gly Pro Tyr Phe Tyr Arg Pro Glu Trp His Glu Thr Pro Leu Pro Ala
           2595                2600                2605
   Ala Arg Ala Pro Thr Ala Gly Asp Val Leu Val Leu Gly Ala Asp Ala
       2610                2615                2620
   Ser Leu Val Ser Ala Leu Arg Ala Glu Leu Leu Ala Ala Gly Glu Gly
   2625                2630                2635                2640
   Ala Arg Leu Val Pro Val Ala Leu Ala Gly Gly Ala Gln Asp Gly Leu
                       2645                2650                2655
   Ser Val Asp Ala Glu Asp Pro Ala Ser Tyr Gln Ala Leu Val Asp Ala
                   2660                2665                2670
   Leu Val Arg Asp Gly Val Arg Pro Thr Arg Ile Leu Gln Val Ala Ser
           2675                2680                2685
   Leu Arg Pro Asp Val Ala His Gly Leu Ser Leu Asp Glu Arg Ile Ala
   2690 2695 2700
   Ala Ser Gly Leu Ser Leu Leu Leu Leu Phe Gln Ala Leu Ile Gln Ala
   2705 2710 2715 2720
   Arg Ala Ala Pro Gln Val Ser Leu Val Tyr Ala Tyr Pro Thr Gly Thr
   2725 2730 2735
   Asp Ala Ser Pro Val Leu Ala Ala Pro Gly Gly Leu Leu Arg Thr Leu
   2740 2745 2750
   Val Arg Glu Lys Ser Gly Leu Phe Cys Ala Ser Val Gly Leu Glu Ala
   2755 2760 2765
   Leu Pro Ala Arg Gln Thr Ala Ala Val Leu Trp Gln Glu Leu Ser Ser
   2770 2775 2780
   Val Ser Pro Gly Cys Ser Asp Val Ala Tyr Asp Gly Gly Leu Arg Leu
   2785 2790 2795 2800
   Thr Arg Arg Trp Ala Ser Val Glu Ala Pro Val Ser Ala Ser Pro Ala
   2805 2810 2815
   Leu Arg Arg Gly Gly Val His Leu Ile Thr Gly Gly Ala Gly Gly Leu
   2820 2825 2830
   Gly Leu Leu Phe Ala Glu His Leu Val Arg His His Asp Ala Arg Val
   2835 2840 2845
   Val Leu Thr Gly Arg Ser Ala Leu Asp Ala Ala Lys Gln Val Arg Leu
   2850 2855 2860
   Ser Arg Leu Gly Asp Ala Ser Arg Val Leu Tyr Leu Gln Gly Asp Val
   2865 2870 2875 2880
   Ser Arg Ala Glu Asp Val Thr Ala Trp Val Arg Thr Ala Arg Glu His
   2885 2890 2895
   Phe Gly Ala Leu His Val Val Leu His Gly Ala Gly Val Arg Arg Asp
   2900 2905 2910
   Ala Phe Leu Leu Lys Lys Thr Arg Arg Glu Leu Asp Glu Val Leu Arg
   2915 2920 2925
   Pro Lys Leu Leu Gly Ala Met His Leu Asp Ala Ala Thr Arg Asp Val
   2930 2935 2940
   Pro Leu Asp Ala Phe Val Leu Phe Ser Ser Val Ser Ala Val Leu Gly
   2945 2950 2955 2960
   Asn Thr Gly Gln Gly Asp Tyr Ala Tyr Ala Asn Ala Phe Leu Asp Ala
   2965 2970 2975
   Phe Ala Trp Gln Arg Glu Ala Arg Arg Gln Arg Gly Glu Arg Ser Gly
   2980 2985 2990
   Gln Thr Val Ser Leu Asp Trp Pro Leu Trp Arg Asp Gly Gly Met Thr
   2995 3000 3005
   Val Asp Pro Ala Ser Glu Ala Leu Leu Ala Thr Thr Met Gly Leu Arg
   3010 3015 3020
   Pro Leu Leu Arg Asp Glu Gly Leu Lys Ala Phe Glu Arg Ala Leu Thr
   3025 3030 3035 3040
   Phe Arg Glu Ser Gln Leu Met Val Ile Ala Gly Asn Lys Asp Arg Ile
   3045 3050 3055
   Asp Gly Leu Leu Gly Ala Gln Ala Pro Ala Arg Arg Ala Glu Ala Pro
   3060 3065 3070
   Ala Glu Asp Ala Ala Gly Gln Val Leu Ala Asp Met Ala Gly Ile Met
   3075 3080 3085
   Ser Arg Leu Leu Arg Val Asn Arg Arg Asp Ile His Pro Asn Glu Asp
   3090 3095 3100
   Leu Arg Glu Tyr Gly Phe Asp Ser Ile Thr Phe Thr Gln Phe Ala Asn
   3105 3110 3115 3120
   Ala Leu Asn Glu His Phe Arg Thr Glu Leu Ser Pro Ala Ile Phe Phe
   3125 3130 3135
   Glu His Ser Thr Leu Ala Ser Leu Ala Ala His Val Ala Ser Leu Arg
   3140 3145 3150
   Pro Ala Ala Pro Val Ala Pro Pro Thr Pro Ala Glu Pro Arg Arg Ala
   3155 3160 3165
   Pro Ala Met Thr Gly Pro Val Ala Thr Ala Ala Thr Gly Met Pro Pro
   3170 3175 3180
   Ile Ala Ala Ala Ala Ala Arg Thr Glu Leu Ala Pro Asp Ala Ile Ala
   3185 3190 3195 3200
   Ile Ile Gly Val Ser Gly Val Met Pro Arg Ser Glu Asn Leu Glu Gln
   3205 3210 3215
   Phe Trp Lys His Leu Glu Ala Gly Glu Asp Leu Val Thr Gly Ile Pro
   3220 3225 3230
   Glu Gln Arg Trp Asp Trp Trp Lys Lys Ala Tyr Gly Gln Ala Val Pro
   3235 3240 3245
   Gly Leu Leu Glu Ala Met Arg Gly Lys Arg Gly Gly Phe Ile Pro His
   3250 3255 3260
   Pro Glu His Phe Asp Ala Glu Phe Phe Gly Ile Ser Ala Arg Glu Ala
   3265 3270 3275 3280
   Asp Phe Met Asp Pro Gln Gln Arg Ile Phe Leu Arg Thr Leu Trp Gln
   3285 3290 3295
   Ser Val Glu Asp Ala Gly Tyr Arg Met Ser Asp Trp Trp Gly Ala Arg
   3300 3305 3310
   Val Gly Leu Phe Val Gly Val Ala Thr Ser Asp Tyr Thr Glu Leu Leu
   3315 3320 3325
   Arg Ala His Asn Ala Pro Ile Asp Gly Leu Thr Ser Thr Gly Val Phe
   3330 3335 3340
   His Ser Ile Leu Val Asn Arg Ile Ser Tyr Leu Phe Asn Phe Thr Gly
   3345 3350 3355 3360
   Pro Ser Glu Pro Val Asp Thr Ala Cys Ser Ser Ser Leu Val Ala Val
   3365 3370 3375
   Gln Arg Ala Val Ala Ala Leu Arg Asp Gly Gln Cys Asp Val Ala Ile
   3380 3385 3390
   Ala Gly Gly Val Asn Leu Leu Leu Ala Pro Thr Leu Phe Leu Ala Phe
   3395 3400 3405
   Asp Lys Ala Gly Met Leu Ser Pro Asp Gly Arg Cys Lys Thr Phe Asp
   3410 3415 3420
   Lys Asp Ala Asn Gly Tyr Val Arg Gly Glu Gly Cys Ala Ala Val Val
   3425 3430 3435 3440
   Leu Lys Pro Leu Arg Lys Ala Leu Glu Asp Gly Asp Thr Ile Arg Gly
   3445 3450 3455
   Ile Val Arg Ala Ala Val Val Gly His Ala Gly Arg Ala Asn Ser Leu
   3460 3465 3470
   Thr Ala Pro Asn Pro Asn Ala Gln Ala Asp Leu Leu Val Arg Ala Tyr
   3475 3480 3485
   Arg Glu Ala Gly Ile Pro Pro Ser Arg Val Ser Tyr Ile Glu Ala His
   3490 3495 3500
   Gly Thr Gly Thr Ser Leu Gly Asp Pro Ile Glu Val Asn Ala Leu Lys
   3505 3510 3515 3520
   Lys Ala Phe Arg Gln Leu Ser Gly Asp Ala Pro Ala Thr Asp Ala Arg
   3525 3530 3535
   Cys Ala Leu Gly Ala Val Lys Thr Asn Ala Gly His Leu Glu Ala Ala
   3540 3545 3550
   Ala Gly Met Ala Gly Leu Val Lys Val Leu Leu Ala Leu Lys His Arg
   3555 3560 3565
   Lys Val Pro Ala Asn Ala His Phe Arg Glu Arg Asn Pro Tyr Val Asp
   3570 3575 3580
   Leu Asp Gly Ser Pro Phe Tyr Leu Pro Gly Ser Leu Gln Ala Trp Glu
   3585 3590 3595 3600
   Ser Pro Arg Gly Pro Arg Val Ala Gly Leu Ser Ser Phe Gly Phe Gly
   3605 3610 3615
   Gly Val Asn Ala His Leu Val Ile Glu Glu His Val Glu Pro Ala Ala
   3620 3625 3630
   Pro Arg Gln Glu Asp Ala Glu Pro Gly Val Phe Val Leu Ser Ala Arg
   3635 3640 3645
   Thr Glu Ala Val Leu Arg Arg Tyr Ala Ala Asp Leu Leu Gln Asp Leu
   3650 3655 3660
   Glu Ala Arg Gly Ser Ala Val Ala Leu Gln Asp Val Ala Tyr Thr Thr
   3665 3670 3675 3680
   Gln Val Gly Arg Glu Ala Met Ala Val Arg Leu Ala Val Ile Ala Ser
   3685 3690 3695
   Asn Arg Gln Glu Leu Phe Ala Lys Leu Arg Ala Phe Thr Ser Gly Ala
   3700 3705 3710
   Pro Glu Thr Glu Gly Val Phe Leu Gly Arg Lys Ala Glu His Gly Ala
   3715 3720 3725
   Ala Val Gly Leu Leu Ile Glu Gly Glu Glu Gly Ala Leu Tyr Leu Arg
   3730 3735 3740
   Thr Leu Ala Arg Asn Gly Arg Leu Pro Lys Leu Cys Gln Leu Trp Thr
   3745 3750 3755 3760
   Leu Gly Val Asp Val Asp Trp Arg Leu Leu His Glu Gly Arg Thr Arg
   3765 3770 3775
   Arg Arg Val Pro Leu Pro Thr Tyr Pro Phe Val Glu Glu Ala His Trp
   3780 3785 3790
   Tyr Asp Leu His Val Pro Met Pro Thr Ala Ala Asn Arg Pro Val Met
   3795 3800 3805
   Glu Thr Ala Ala Pro Pro Val Gln Ala Pro Pro Pro Ala Pro Val Met
   3810 3815 3820
   Pro Val Ala Ala Glu Ala Gln Val Ala Arg Pro Gly Lys Leu Thr Leu
   3825 3830 3835 3840
   Arg Pro Pro Val Ala Gly Glu Val Val Lys Pro Val Pro Pro Val Pro
   3845 3850 3855
   Val Ala Ala Ala Pro Ala Asp Pro Ser Ser Ala Ala Leu Pro Ala Val
   3860 3865 3870
   Glu Arg Leu Arg Thr Leu Leu Ala Gln Val Leu Phe Val Asp Ala Ser
   3875 3880 3885
   Ala Val Gly Val Asn Glu Pro Leu Ser Asp Leu Gly Leu Asp Pro Val
   3890 3895 3900
   Leu Ala Asp Glu Phe Phe Arg Lys Val Asn Glu Ala Phe Gly Thr Ser
   3905 3910 3915 3920
   Leu Arg Thr Ala His Tyr Gln Gly Asp Gln Thr Leu Thr Ala Leu Ala
   3925 3930 3935
   Arg Gly Leu Asp Val Lys Ala Pro Pro Ala Thr Pro Pro Ala Pro Ala
   3940 3945 3950
   Pro Ala Ala Pro Ala Val Gly Pro Ser Ile Pro Glu Thr Leu Lys Val
   3955 3960 3965
   Leu Arg Ser Leu Leu Ala Gly Val Leu Phe Thr Glu Pro Glu Ala Ile
   3970 3975 3980
   Glu Ala Ser Arg Pro Leu Val Glu Leu Gly Leu Asp Ser Val Leu Gly
   3985 3990 3995 4000
   Ile Glu Phe Met Asp Lys Val Asn Gln Arg Phe Ala Ile Lys Val Ser
   4005 4010 4015
   Pro Ser Lys Val Tyr Glu His Pro Ser Leu Glu Arg Leu Gly Ala Tyr
   4020 4025 4030
   Ile Ala Gln Ala Ala Ala Ala Ala Gly Ala Pro Glu Pro Arg Gln Asp
   4035 4040 4045
   Lys His Leu Phe Phe Ala Val Ser Glu Ser Ser Arg Ser Ser Asn Gly
   4050 4055 4060
   Ala Gly Glu Ser
   4065
<210> 26
   <211> 4981
   <212> PRT
   <213> Corallococcus coralloides
<220>
   <223> CorL
<220>
   <221> CHAIN
   <222> 1443..1449
   <223> CorL-CP-4W
<220>
   <221> CHAIN
   <222> 2208..2214
   <223> CorL-CP-5W
<220>
   <221> CHAIN
   <222> 4116..4122
   <223> CorL-CP-6W
<220>
   <221> CHAIN
   <222> 4826..4832
   <223> CorL-CP*-7W
<220>
   <221> CHAIN
   <222> 4920..4926
   <223> CorL-CP-7W
<220>
   <221> CHAIN
   <222> 19..31
   <223> CorL-DH-4W
<220>
   <221> CHAIN
   <222> 2949..2961
   <223> CorL-DH-6W
<220>
   <221> CHAIN
   <222> 3865..3870
   <223> CorL-MT-6W
<400> 26
   Met Ser Gly Ser Leu Thr Phe Thr Phe Gln Thr Ser Ala Pro Glu Asn
   1               5                   10                  15
   Pro Cys Leu Asn Ala His Glu Val Phe Gly Arg Pro Leu Phe Pro Thr
               20                  25                  30
   Asp Ala Phe Leu Glu Leu Val Tyr Ser Val Ala Lys Thr Arg Leu Gly
           35                  40                  45
   Leu Ser Arg Val Gln Leu Asp Asp Leu Val Ile Ser Ala Pro Val Val
       50                  55                  60
   Gly Ala Tyr Asp Ala Pro Thr Ser Leu Thr Leu Met Phe Arg Glu Glu
   65                  70                  75                  80
   Pro Ala Gly Trp Arg Phe Ala Val Lys Ser Asp Ala Gly Pro Thr Gly
                   85                  90                  95
   Arg Leu His Leu Lys Gly Gln Val Arg Glu Leu Thr Gly Pro Ala Gly
               100                 105                 110
   Ala Ser Pro Val Pro Pro Pro Asp Phe Asp Gly Trp Pro Arg Thr Leu
           115                 120                 125
   Ser Lys Glu Asp Ile Tyr Gly Arg His Pro His Leu Gly Ile Gly Pro
       130                 135                 140
   Phe Tyr Arg Thr Met Asp Ala Leu Arg Val Gln Pro Gly Val Ser Val
   145                 150                 155                 160
   Ser Arg Ile Ala Pro Ser Pro Glu Gly Arg Pro Trp Glu Pro Arg Phe
                   165                 170                 175
   Leu Leu Gln Pro Pro Ile Val Asn Gly Leu Leu Met Thr Ala Leu Ser
               180                 185                 190
   Ser Ala Ile His Leu Ser Gly Asp Pro Ala Ser Val Tyr Val Pro Val
           195                 200                 205
   Gly Ala Arg Arg Val Asp Leu Pro Gly Asp Val Asp Gly Ala Pro His
       210                 215                 220
   Thr Ala Val Ala Arg Leu Leu Ser Asn Asp Gly Glu Ala Met Ala Phe
   225                 230                 235                 240
   Asp Met Asp Leu Leu Asp Ala Ser Ala Gln Val Val Leu Ala Ile His
                   245                 250                 255
   Glu Leu Arg Val Arg Arg Ile Arg Pro Asp Asp Leu Ser Val Ala Pro
               260                 265                 270
   Thr Gly Ala Ala Pro Val Ala Ser Arg Asp Gly Asp Glu Lys Ala Val
           275                 280                 285
   Ala Ile Ile Gly Tyr Ser Gly Arg Phe Pro Gly Ala Ala Asp Pro Glu
       290                 295                 300
   Ser Phe Trp Arg Asn Leu Met Glu Gly Val Asp Ser Ile Thr Glu Val
   305                 310                 315                 320
   Pro Pro Glu Arg Trp Asp Asn Ala Arg Tyr Tyr Asp Pro Asn Pro Arg
                   325                 330                 335
   Thr Pro Gly Arg Thr Val Ser Arg Trp Gly Gly Phe Leu Asp Gly Val
               340                 345                 350
   Asp Arg Phe Asp Pro Gly Phe Phe Gly Ile Ser Pro Met Glu Ala Glu
           355                 360                 365
   Ile Met Asp Pro Gln Gln Arg Leu Phe Leu Glu Glu Ser Tyr Arg Ala
       370                 375                 380
   Leu Glu Ser Ala Gly Leu Arg Pro Glu Asp Leu Gln Glu Arg Ser Cys
   385                 390                 395                 400
   Gly Val Phe Val Gly Ala Ser Val Ser Asp Tyr Glu Glu Thr Leu Ala
                   405                 410                 415
   Gln Ala Arg Gln Ser Thr Thr Ala Gln Ala Phe Thr Gly Leu Ala Pro
               420                 425                 430
   Ala Val Leu Ala Ala Arg Ile Ser Tyr Phe Leu Asp Leu Lys Gly Pro
           435                 440                 445
   Ala Leu Thr Leu Asp Thr Ala Cys Ser Ser Ser Leu Thr Ala Leu Ser
       450                 455                 460
   Met Ala Cys Gln Ser Ile Leu Ala Gly Glu Ser Glu Leu Ala Ile Ala
   465                 470                 475                 480
   Gly Gly Val His Val Met Val Thr Pro Lys Leu His Ile Lys Ala Ser
                   485                 490                 495
   Lys Ser Gly Met Leu Ser Arg Thr Gly Arg Cys Arg Thr Phe Asp Lys
               500                 505                 510
   Lys Ala Asp Gly Ile Ala Leu Gly Glu Gly Val Gly Ala Val Val Leu
           515                 520                 525
   Lys Arg Leu Asp Ala Ala Leu Arg Asp Gly Asp Val Ile Arg Gly Val
       530                 535                 540
   Val Arg Gly Phe Gly Val Asn Gln Asp Gly Arg Thr Asn Gly Ile Thr
   545                 550                 555                 560
   Ala Pro Asn Pro Ala Ala Gln Glu Gln Leu Gln Arg Asp Val Tyr Gln
                   565                 570                 575
   Arg Phe Gly Ile His Pro Arg Arg Ile Thr Tyr Val Glu Ala His Gly
               580                 585                 590
   Thr Gly Thr Ala Met Gly Asp Pro Ile Glu Leu Glu Ala Leu Thr Arg
           595                 600                 605
   Ala Phe Gln His Ser Thr Arg Asp Val Gly Phe Cys Thr Val Gly Ser
       610                 615                 620
   Val Lys Pro Asn Ile Gly His Thr Thr Phe Ser Ala Gly Ile Ala Ser
   625                 630                 635                 640
   Val Ile Lys Val Leu Leu Ala Leu Glu His Gly Ala Leu Pro Pro Ser
                   645                 650                 655
   Leu His Leu Glu Glu Leu Asn Pro Leu Ile Asp Phe Ser Gly Ser Pro
               660                 665                 670
   Phe Arg Val Thr Arg Glu Lys Thr Pro Trp Gln Gly His Asp Gly Gln
           675                 680                 685
   Pro Arg Met Ala Ala Ile Asn Ser Phe Gly Phe Ser Gly Thr Asn Cys
       690                 695                 700
   His Val Val Ile Glu Glu Ala Pro Arg Arg Glu Pro Arg Ala Ala Pro
   705                 710                 715                 720
   Leu Lys Pro Ala Trp Leu Val Pro Val Thr Ala Lys Thr Glu Asp Ser
                   725                 730                 735
   Leu Arg Ala Ala Leu Val Asp Leu Lys Ala Trp Leu Arg Gly His Asp
               740                 745                 750
   Val Pro Leu Gly Asp Val Ala Phe Thr Leu Leu Cys Lys Arg Ala His
           755                 760                 765
   Tyr Ala Arg Arg Ile Ala Phe Val Ala Ser Ser Arg Glu Glu Leu Asp
       770                 775                 780
   Ala Leu Ile Gly Thr Ala Leu Ala Ala Asp Arg Leu Pro Leu Val Ala
   785                 790                 795                 800
   Pro Arg Pro Ala Asp Ser Arg Pro Asp Ala Gly Ala Leu Leu Arg Lys
                   805                 810                 815
   Leu Thr Thr Gln Ala Leu Pro Asp Val Glu Tyr Arg Glu Val Leu Thr
               820                 825                 830
   Glu Leu Gly Arg Val Phe Ser Glu Gly Gly Thr Cys Asp Trp Ala Gln
           835                 840                 845
   Leu Ile Pro Ala Gly His His Gln Trp Val Ala Leu Pro Ala Tyr Pro
       850                 855                 860
   Phe Gln Arg Arg Val Cys Trp Val Ser Pro Ala Ala Gly Ala Glu Val
   865                 870                 875                 880
   Pro Arg Pro Pro Glu Pro Glu Arg Pro Ala Ala Val Pro Glu Ala Ala
                   885                 890                 895
   Ser Ser Ala Arg Leu Leu Arg Tyr Glu Trp Arg Arg Arg Asp Ala Thr
               900                 905                 910
   Ser Ser Lys Ser Pro Ala Val Pro Ala Gly Val Ala Val Ile Leu Thr
           915                 920                 925
   Arg Asp Pro Ala Leu Tyr Ala Glu Leu Gly Gln Arg Leu Arg Ser Arg
       930                 935                 940
   Thr Pro Gly Val Arg Thr Val Gln Val Arg Pro Gly Ala Ser Phe Arg
   945                 950                 955                 960
   Glu Leu Gly Pro Asp Thr Phe Glu Val Arg Pro Asp Asp Glu Glu Ser
                   965                 970                 975
   Tyr Ser Ala Leu Leu Thr Ala Cys Gly Ala Gly Pro Arg Asp Ala Val
               980                 985                 990
   Asp Leu Leu His Leu Trp Pro Leu Asp Ala Pro Pro Glu Ala Ser Ala
           995                 1000                1005
   Asp Leu Glu Arg Gln Lys Asp Glu Gly Phe Tyr Ser Leu Val Lys Ile
       1010                1015                1020
   Ala Gly Ala Met Ala Arg Ser Arg Leu Leu Gly Pro Ala Arg Leu Phe
   1025                1030                1035                1040
   Phe Phe His Pro Leu His Ala Asp Ala Thr Val Pro Val His Gly Gly
                   1045                1050                1055
   Val Asp Gly Leu Phe Ala Thr Leu Ala Thr Glu Asn Pro Ala Leu Arg
               1060                1065                1070
   Cys Arg Ser Ile Gly Val Thr Leu Ala Gly Thr Gly Ala Leu Ala Gly
           1075                1080                1085
   Leu Ile Ala Ser Glu Leu Leu Asp Gly Glu Gly Thr Ala Pro Val Leu
       1090                1095                1100
   His Arg Ala Gly Gly Arg Phe Val Arg Val Leu Gly Glu Leu Ser Pro
   1105                1110                1115                1120
   Glu Ala Arg Pro His Glu Gly Pro Thr Leu Arg Gln Gly Gly Ala Tyr
                   1125                1130                1135
   Leu Ile Thr Gly Gly Val Gly Ala Leu Gly Phe Val Phe Ala Arg His
               1140                1145                1150
   Leu Ala Ser Lys Phe Gln Ala Arg Leu Ala Leu Leu Gly Arg Ser Glu
           1155                1160                1165
   Pro Gly Ser Ala Gln Arg Glu Arg Ile Ala Glu Leu Glu Arg Leu Gly
       1170                1175                1180
   Ser Arg Val Leu Tyr Leu Arg Cys Asp Val Ala Asp Ala Asn Ala Val
   1185                1190                1195                1200
   Arg Glu Ala Val Glu Arg Ala Arg Thr Glu Leu Gly Gly Leu His Gly
                   1205                1210                1215
   Val Ile His Ala Ala Gly Asn Leu Arg Ser Leu Gln Ala Gly Arg Gly
               1220                1225                1230
   Arg Ala Ala Met Glu Glu Cys Leu Arg Pro Lys Leu Asp Gly Leu Phe
   1235 1240 1245
   Ala Leu Asp Gln Ala Thr Arg Asp Val Pro Leu Asp Phe Leu Ala Leu
   1250 1255 1260
   Cys Ser Ser Val Ser Ser Val Leu Gly Asn Ala Gly Gln Gly Asp Tyr
   1265 1270 1275 1280
   Ala Tyr Ala Asn Ala Val Met Asp His Phe Ala His His Arg Glu Arg
   1285 1290 1295
   Leu Arg Arg Asp Gly Phe Arg His Gly Arg Thr Val Ser Val Ser Trp
   1300 1305 1310
   Pro Leu Trp Arg Glu Gly Gly Leu Pro Val Thr Ala Glu Leu Glu Arg
   1315 1320 1325
   Leu Val Glu Lys Thr Leu Gly Met Arg Ala Leu Asp Thr Pro Ala Gly
   1330 1335 1340
   Leu Ala Leu Phe Glu Thr Thr Leu Arg Leu Ala Glu Pro His Val Val
   1345 1350 1355 1360
   Val Ala Tyr Gly Asp Pro Ala Arg Thr Ala Ser Leu Ala Glu Ala Ala
   1365 1370 1375
   Gly Pro Asp Glu Ala Pro Ala Ala Phe Met Asp Ala Pro Val Ser Thr
   1380 1385 1390
   Gly Gly Thr Ala Pro Ala Arg Ile Glu Glu Ser Val Val Glu Gly Ser
   1395 1400 1405
   Asp Val Val Glu Ala Leu Arg Gln Arg Ile Val Asp Glu Val Val Ala
   1410 1415 1420
   Leu Leu Lys Val Asp Arg Asp Ala Val Ser Val Asp Asp Glu Leu Val
   1425 1430 1435 1440
   Asp Tyr Gly Phe Asp Ser Ile Ile Leu Thr Glu Leu Ala Asn Arg Met
   1445 1450 1455
   Asn Asp His Tyr Gly Leu Asp Ile Thr Pro Ala Ser Phe Phe Glu His
   1460 1465 1470
   Ala Thr Ile Arg Glu Phe Ala Asp Phe Leu Met Lys Asn His Gly Asp
   1475 1480 1485
   Ala Ile Arg Lys His Ser Arg Pro Thr Thr Pro Pro Pro Ala Ala Pro
   1490 1495 1500
   Val Gln Thr Ala Pro Ala Ala Ser Ser Pro Gln Val Gln Ala Pro Ala
   1505 1510 1515 1520
   Ala Arg Pro Gly Ala Glu Gly Ile Ala Val Ile Gly Met Ala Gly Glu
   1525 1530 1535
   Leu Pro Arg Ser Ser Asp Leu Arg Glu Leu Trp Arg His Leu Glu Ala
   1540 1545 1550
   Gly Asp Val Leu Thr Gly Glu Val Pro Ser Asp Arg Trp Asp Thr Arg
   1555 1560 1565
   Gly Met Arg Ala Lys Trp Gly Gly Phe Leu Pro Arg Val Asp Gly Phe
   1570 1575 1580
   Asp Ala Ala Phe Phe Gly Ile Thr Pro Arg Glu Ala Glu Leu Met Asp
   1585 1590 1595 1600
   Pro Gln Gln Arg Leu Phe Leu Glu Thr Val Trp Lys Thr Met Glu Asp
   1605 1610 1615
   Ala Gly Tyr Arg Pro Ser Ser Leu Ser Gly Ser Arg Thr Gly Leu Phe
   1620 1625 1630
   Val Gly Val Ala Thr Ser Asp Tyr Gly Gly Leu Leu Lys Glu His Gly
   1635 1640 1645
   Leu Glu Gly Glu Ala Leu Ala Pro Thr Gly Thr Ala His Ala Leu Leu
   1650 1655 1660
   Ala Asn Arg Ile Ser Tyr Gln Phe Asn Phe His Gly Pro Ser Glu Pro
   1665 1670 1675 1680
   Cys Asn Thr Ala Cys Ser Ser Ser Leu Ile Ala Ile His Arg Ala Cys
   1685 1690 1695
   Glu Ala Leu Arg Glu Gly Ser Cys Asp Val Ala Leu Ala Gly Gly Val
   1700 1705 1710
   Asn Leu Leu Leu Ser Pro Ala Leu Phe Val Ala Phe Asp Ser Ala Gly
   1715 1720 1725
   Met Leu Ser Pro Asp Gly Arg Leu Arg Ala Phe Asp Ala Gly Ala Asn
   1730 1735 1740
   Gly Tyr Val Arg Gly Glu Gly Val Val Ala Leu Met Leu Lys Pro Leu
   1745 1750 1755 1760
   Ser Lys Ala Ile Ala Asp Gly Asp His Val Tyr Gly Val Ile Arg Ala
   1765 1770 1775
   Ser Ala Thr Asn His Gly Gly Lys Ala His Ser Leu Thr Ala Pro Asn
   1780 1785 1790
   Pro Asn Ala Gln Ala Glu Leu Leu Thr Thr Val Tyr Arg Lys Ala Gly
   1795 1800 1805
   Ile Ala Pro Asp Arg Val Ser Leu Ile Glu Ala His Gly Ser Gly Thr
   1810 1815 1820
   Pro Leu Gly Asp Pro Ile Glu Val Asn Gly Ile Lys Lys Ala Phe Asp
   1825 1830 1835 1840
   Thr Leu Tyr Arg Glu Trp Asn Leu Pro Tyr Gly Gly Gln Pro Arg Cys
   1845 1850 1855
   Ala Ile Gly Ser Leu Lys Gly Asn Thr Gly His Leu Glu Ala Ala Ala
   1860 1865 1870
   Gly Val Ala Gly Val Ala Lys Val Leu Leu Ala Leu Ala His Arg Arg
   1875 1880 1885
   Ile Pro Ala Asn Ala Thr Leu Asp Gln Val Asn Pro Gln Val Arg Leu
   1890 1895 1900
   Glu Asp Thr Pro Phe His Leu Ala Thr Arg Leu Glu Pro Trp Glu Ala
   1905 1910 1915 1920
   Arg Val Gly Ala Asp Gly Ser Ser Leu Arg Val Ala Gly Val Ser Ser
   1925 1930 1935
   Phe Gly Phe Gly Gly Ala Asn Ala His Val Ala Ile Glu Glu Tyr Val
   1940 1945 1950
   Ala Pro Pro Leu Pro Ala Arg Ala Ser Gly Pro Gln Ala Ile Leu Leu
   1955 1960 1965
   Ser Ala Arg Thr Pro Ala Ala Leu Thr Glu Tyr Ala Leu Arg Leu Arg
   1970 1975 1980
   Gly Asp Ala Arg Ser Gly Ala Leu Asp Gly Ala Arg Leu Glu Asp Leu
   1985 1990 1995 2000
   Ala Phe Thr Thr Gln Glu Gly Arg Glu Ser Met Thr Glu Arg Leu Ala
   2005 2010 2015
   Val Ile Ala Ser Asp Leu Arg Asp Leu Glu Arg Lys Leu Asp Ala Phe
   2020 2025 2030
   Ala Ala Gly Gly Thr Ala Gln Glu Gly Val Phe Arg Gly Thr Ala Gly
   2035 2040 2045
   Ala Arg Pro Ser Gly Ala Arg Ile Pro Gly Thr Gly Pro Leu Glu Met
   2050 2055 2060
   Leu Cys Arg Ala Trp Ala Asp Gly Ser Glu Val Asp Trp Ser Ala Leu
   2065 2070 2075 2080
   His Ala Gly Gln Pro Arg Arg Arg Val Ser Phe Ala Thr Tyr Pro Phe
   2085 2090 2095
   Glu Arg Lys Arg Phe Trp Cys Asp Ala Glu Arg Phe Ala Arg Pro Ala
   2100 2105 2110
   Ala Val Thr Thr Gln Gly Glu Ala Val Asp Thr Gly Lys Asp Lys Ala
   2115 2120 2125
   Val Met Lys Leu Lys Leu Lys Gln Pro Gln Ala Lys Glu Ser Pro Arg
   2130 2135 2140
   Val Leu Glu Glu Arg Ser Pro Ala Pro Val Ala Glu Ala Pro Pro Ala
   2145 2150 2155 2160
   Pro Ala Pro Ser Ala Pro Ala Pro Ala Pro Ala Pro Ala Val Ser Gln
   2165 2170 2175
   Gly Thr Leu Arg Arg Gln Leu Lys Gly Leu Leu Ala Ala Thr Leu His
   2180 2185 2190
   Val Pro Glu Glu Glu Leu Ser Glu Ser Arg Ser Phe Ala Glu Met Gly
   2195 2200 2205
   Leu Asp Ser Ile Leu Ala Leu Glu Phe Val Lys His Ile Asn Gln Met
   2210 2215 2220
   Leu Gly Thr Ser Leu Glu Ala Val Gln Leu Phe Asp Tyr Gly Asp Ile
   2225 2230 2235 2240
   Pro Arg Leu Ala Lys Ala Leu Gln Glu Leu Ala Pro Arg Ala Ala Ala
   2245 2250 2255
   Pro Thr Arg Lys Pro Glu Ala Val Val Ala Pro Asn Pro Ala Pro Gln
   2260 2265 2270
   Ser Thr Pro Glu Val Gln Ala Arg Val Asn Pro Pro Ala Leu Asp Glu
   2275 2280 2285
   Ala Leu Pro Gly Asp Glu Leu Leu Glu Leu Trp Arg Ala Pro Val Thr
   2290 2295 2300
   Ala Ala Pro Pro Ala Pro Pro Ala Gln Thr Leu Arg Glu Pro Ala Arg
   2305 2310 2315 2320
   Thr Ala Leu Ala Glu Ala Val Ser Glu Asp Ile Ala Val Ile Gly Met
   2325 2330 2335
   Ala Ala Arg Leu Pro Gly Ala Pro Asp Val Asp Ala Phe Trp Arg Asn
   2340 2345 2350
   Leu Lys Asp Gly Val Cys Ser Leu Arg Glu Val Pro Ser Glu Arg Trp
   2355 2360 2365
   Asp Ala Thr Arg Ala Gly His Arg Cys Arg Trp Gly Gly Phe Leu Asp
   2370 2375 2380
   Asp Val Asp Ala Phe Asp Pro Leu Phe Phe Asn Ile Ser Pro Asn Glu
   2385 2390 2395 2400
   Ala Ala Leu Met Asp Pro Gln Gln Arg Leu Phe Leu Glu Thr Ala Trp
   2405 2410 2415
   Ala Thr Leu Glu Asp Ala Gly Tyr Ser Ala Glu Arg Leu Glu Gly Gln
   2420 2425 2430
   Arg Cys Gly Val Tyr Val Gly Val Met Asn Asn Asp Tyr His Glu Leu
   2435 2440 2445
   Leu Gly Thr Gly Pro Ala Ala Val Arg Gln Ala Gln Ala Met Thr Gly
   2450 2455 2460
   Asn Ala Asn Ser Ile Leu Ala Ala Arg Ile Ala Tyr Val Leu Asn Leu
   2465 2470 2475 2480
   Lys Gly Pro Ala Met Gln Leu Asp Thr Ala Cys Ser Ser Ser Leu Val
   2485 2490 2495
   Ala Met His Leu Ala Arg Gln Ser Leu Val Ser Asp Glu Ala Asp Leu
   2500 2505 2510
   Met Leu Val Gly Gly Val Thr Leu Tyr Leu Ser Glu Ser Ser Tyr Ala
   2515 2520 2525
   Lys Met Asp Gly Ala Gly Met Leu Ser Pro Thr Gly Arg Cys His Thr
   2530 2535 2540
   Phe Asp Gln Gly Ala Asp Gly Phe Val Pro Gly Glu Gly Val Gly Ala
   2545 2550 2555 2560
   Val Leu Leu Lys Arg Leu Asp Lys Ala Arg Ala Asp Gly Asp Arg Ile
   2565 2570 2575
   Leu Gly Val Ile Lys Gly Thr Ala Ile Asn Gln Asp Gly Arg Thr Asn
   2580 2585 2590
   Gly Ile Thr Ala Pro Ser Ala Gln Ser Gln Thr Ala Leu Glu Ile Ser
   2595 2600 2605
   Leu Tyr Arg Arg Phe Gly Ile Asp Pro Glu Thr Leu Thr Tyr Val Glu
   2610 2615 2620
   Ala His Gly Thr Gly Thr Lys Leu Gly Asp Pro Ile Glu Val Lys Ala
   2625 2630 2635 2640
   Leu Thr Asp Ala Phe Arg Ala Phe Thr Pro Arg Lys Gln Phe Cys Gly
   2645 2650 2655
   Leu Gly Ser Val Lys Thr Asn Ile Gly His Thr Ser Ala Ala Ala Gly
   2660 2665 2670
   Val Ala Ser Leu Ile Lys Val Leu Leu Ala Leu Lys His Arg Gln Leu
   2675 2680 2685
   Pro Pro Ser Leu His Phe Thr Arg Glu Asn Gly Leu Ile Gly Phe Gln
   2690 2695 2700
   Asp Thr Pro Phe Arg Val Val Thr Gln Leu Ser Asp Trp Val Pro Ala
   2705 2710 2715 2720
   Pro Gly Ala Pro Arg Arg Ala Ala Ile Ser Ser Phe Gly Phe Ser Gly
   2725 2730 2735
   Thr Asn Ala His Leu Val Val Glu Glu Tyr Ala Gly Pro Ala Pro Ala
               2740                2745                2750
   Arg Ala Pro Ile Pro Ala Pro Ser Glu Val Leu Val Leu Ser Ala Arg
           2755                2760                2765
   Asp Glu Ala Arg Leu Lys Val Gln Ala Arg Arg Leu Ala Asp Phe Ile
       2770                2775                2780
   Gly Glu Asn Ala Glu Gly Leu Pro Leu Asp Ala Leu Ala Trp Thr Leu
   2785                2790                2795                2800
   Gln Thr Gly Arg Thr Ala Met Glu Glu Arg Leu Ala Phe Val Ala Ser
                   2805                2810                2815
   Ser Pro Gln Glu Ala Arg Glu Gln Leu Leu Arg Phe Ala Glu Gly Thr
               2820                2825                2830
   Gly Thr Gly Leu His His Arg Arg Leu Arg Glu Glu Gly Thr Asp Ala
           2835                2840                2845
   Asp Trp Leu Leu Glu Gly Arg Glu Gly Glu Gln Tyr Leu Glu Ala Leu
       2850                2855                2860
   Leu Ala Gly Gly Ala Leu Ala Lys Val Ala Lys Leu Trp Thr Met Gly
   2865                2870                2875                2880
   Val Arg Val Asp Trp Ala Arg Leu Arg Lys Gly Ala Arg Pro Gln Pro
                   2885                2890                2895
   Leu Ala Leu Pro Thr Tyr Pro Phe Ala Lys Glu Arg Cys Trp Phe Arg
               2900                2905                2910
   Lys Thr Glu Arg Ser Ala Thr Thr Thr Val Asp Ala Ser Cys Phe Gln
           2915                2920                2925
   Leu Gly Leu Glu Glu Gly Gly Leu Val Leu Lys Thr Arg Leu Leu Ala
       2930                2935                2940
   Lys Asp Val Leu Val Thr Gln His Gln Val His Gly Gln Pro Val Met
   2945                2950                2955                2960
   Pro Gly Thr Ala Trp Leu Glu Leu Met Met Glu Ala Gly Arg Leu Leu
                   2965                2970                2975
   Gly Glu Thr Gly Arg Thr Arg Leu Ala Gln Val Ala Phe His Gln Pro
               2980                2985                2990
   Leu Val Val Thr Gly Asp Arg Asp Val Glu Val Ala Val Thr Arg Gln
           2995                3000                3005
   Gly Ala Val Leu Arg Leu Val Leu Arg Ser Gln Glu Ala Ser Gly Thr
       3010                3015                3020
   Arg Ile Thr His Ala Ser Gly Glu Leu His Pro Glu Asp Ala Ala Gln
   3025                3030                3035                3040
   Val Pro Gly Pro Ile Ala Leu Gly Ala Val Arg Glu Arg Cys Arg Lys
                   3045                3050                3055
   Ser Leu Asp Ala Asp Ala Leu Tyr Ala Arg Ala Gln Ser Leu Gly Leu
               3060                3065                3070
   Thr Tyr Gly Pro Arg Phe Arg Gly Leu Lys Asp Cys Arg Val Gly Glu
           3075                3080                3085
   Gly Glu Ala Leu Ala Ala Phe Glu Ser Thr Pro Glu Thr Val Gly Glu
       3090                3095                3100
   Leu Ser Arg Arg Pro Leu His Pro Val Leu Leu Asp Ser Val Leu Gln
   3105                3110                3115                3120
   Ser Val Ser Leu Leu Ala Leu Gly Asp Ala Asn Ser Ala Thr Thr Thr
                   3125                3130                3135
   Ala Arg Ile Pro Phe Gly Ile Gly Ala Ala Arg Leu Thr Gly Ala Leu
               3140                3145                3150
   Pro Ala Lys Gly Phe Ala Tyr Val Arg Gln Gln Glu Ala Ser Phe Gln
           3155                3160                3165
   Val Leu Leu Leu Asp Asp Thr Gly Ala Val Arg Ala Thr Phe Asp Asp
       3170                3175                3180
   Val Val Leu Arg Pro Met Pro Glu Arg Thr Gly Gly Met Leu Phe Ala
   3185                3190                3195                3200
   Pro Arg Trp Arg Glu Gln Pro Arg Glu Ala Ala Ser Gly Ala Pro Arg
                   3205                3210                3215
   Ser Gly Val Ile Leu Tyr Arg Pro Asp Gln Pro Glu Glu Ala Ala Leu
               3220                3225                3230
   Ala Ala Glu Leu Val Ala Glu Ala Gly Arg Arg Gly Thr Glu Leu Gln
           3235                3240                3245
   Arg Leu Ala Leu His Glu Gly Gln Val Pro Ala Glu Leu Gly Glu Arg
       3250                3255                3260
   Ala Pro Glu Val Leu Tyr Val Leu Ser Ala Gly Gly Gly Asp Ala Leu
   3265                3270                3275                3280
   Ser Gly Ala Asn Ala Glu Ala Arg Val Leu Asp Val Lys Ala Arg Gln
                   3285                3290                3295
   Glu Gln His Leu Arg Ser Leu Phe Arg Cys Val Lys Arg Leu Leu Ser
               3300                3305                3310
   Arg Gly Phe Ala Glu Arg Pro Leu Glu Leu Lys Val Val Thr Arg Asp
           3315                3320                3325
   Ala His Arg Val Leu Gly Asp Glu Gln Val Arg Pro Met Ala Ala Ala
       3330                3335                3340
   Val Ser Gly Phe Val Arg Ser Leu Ser Arg Glu Leu Val Arg Trp Asn
   3345                3350                3355                3360
   Val Thr Gln Val Asp Val Gly Gly Gly Glu Pro Ala Ser Pro Ala Leu
                   3365                3370                3375
   Ala Arg Ala Ile Leu Asp Glu Arg Ala Arg Ala His Glu Ser Glu Val
               3380                3385                3390
   Ala Leu Arg Glu Gly Arg Arg Tyr Glu Leu Gly Leu Asp Arg Leu Val
           3395                3400                3405
   Leu Pro Ala Ala Thr Gly Ala Glu Pro Phe Lys Pro Arg Gly Val Tyr
       3410                3415                3420
   Phe Ile Val Gly Gly Leu Gly Gly Ile Gly His Ala Leu Gly Leu His
   3425                3430                3435                3440
   Leu Ala Arg Thr Val Lys Ala Arg Leu Val Leu Val Gly Arg Gly Gly
                   3445                3450                3455
   Val Asp Ala Arg Lys Gln Glu Gln Leu Arg Glu Leu Glu Ser Leu Gly
               3460                3465                3470
   Ala Glu Val Leu Tyr Val Pro Ala Asp Val Ser Asp Leu Ala Gln Leu
           3475                3480                3485
   Arg Asp Ala Tyr Ala Arg Ala Arg Ala Arg Phe Gly Ala Val Asp Gly
       3490                3495                3500
   Val Ile His Ser Ala Ile Val Leu Lys Asp Ala Thr Leu Ala Thr Leu
   3505                3510                3515                3520
   Glu Glu Pro Val Leu Asp Glu Val Leu Gly Pro Lys Val Leu Gly Thr
                   3525                3530                3535
   Gln Ala Leu Phe Glu Val Phe Arg Gly Ala Pro Leu Asp Phe Met Leu
               3540                3545                3550
   Phe Phe Ser Ser Ala Glu Ser Phe Ser Cys Asp Ala Gly Gln Ser Asn
           3555                3560                3565
   Tyr Ala Ala Ala Ser Arg Phe Glu Asp Ala Gln Ala Ala Leu Glu Arg
       3570                3575                3580
   Gly Arg Ala Gly Phe Pro Val Val Val Ile Asn Trp Gly Tyr Trp Gly
   3585                3590                3595                3600
   Ser Val Gly Ile Val Ala Ser Glu Arg Tyr Arg Arg Glu Leu Ala Lys
                   3605                3610                3615
   Arg Gly Val Gly Ser Ile Glu Pro Glu Glu Gly Trp Glu Val Val Arg
               3620                3625                3630
   Arg Val Leu Ala Ser Arg Glu His Leu Gly Gln Val Val Val Leu Lys
           3635                3640                3645
   Gly Asp Val Lys Gln Leu Gly Thr Val Arg Val His Asp Thr Gly Arg
       3650                3655                3660
   Gln Asp Leu Phe Ala Ala Glu His Gly Pro Val Ala Leu Glu Pro Val
   3665                3670                3675                3680
   Pro His Val Pro Ala Glu Leu Gly Thr Leu Leu Gln Leu Glu Ala Gly
                   3685                3690                3695
   Phe Gln Arg Leu Asp Val Leu Gly Gln Ala Leu Leu Leu Glu Thr Phe
               3700                3705                3710
   Gln Arg Leu Gly Val Phe Gln Arg Ala Gly Glu Arg Thr Ser Ala Glu
           3715                3720                3725
   Glu Leu Gly Ala Arg Leu Gly Val Val Asp Thr His Arg Lys Leu Phe
       3730                3735                3740
   Gln Ala Leu Leu Gly Ile Leu Glu Arg Gly Gly Phe Leu Met Ala Gln
   3745 3750 3755 3760
   Gly Arg Ser Leu Ile Ala Thr Pro Arg Val Glu Ser Ser Gly Arg Ala
   3765 3770 3775
   Asp Phe Ala Arg Leu Val Gln Glu His Pro Ala Leu Glu Pro His Val
   3780 3785 3790
   Arg Leu Leu Arg Thr Cys Val Gln Ala Tyr Pro Glu Val Leu Thr Gly
   3795 3800 3805
   Arg Val Asp Ala Thr Glu Val Met Phe Pro Gly Gly Ser Thr Ser Leu
   3810 3815 3820
   Val Glu Gly Val Tyr Arg Gly Ser Ala Leu Ser Asp Tyr Phe Asn His
   3825 3830 3835 3840
   Leu Cys Ala Glu Ala Ile Arg Ala Gln Val Glu Arg Arg Leu Ala Leu
   3845 3850 3855
   Glu Pro Thr Ser Thr Val Thr Val Leu Glu Val Gly Ala Gly Thr Gly
   3860 3865 3870
   Gly Thr Ser Glu Phe Val Met Lys Arg Leu Asp Gly Leu Gly Gly Arg
   3875 3880 3885
   Val Lys Tyr Leu Tyr Thr Asp Ile Ser Gln Ala Phe Ile Arg His Gly
   3890 3895 3900
   Ala Glu Arg Tyr Gly Ala Arg His Pro Phe Ala Val Phe Arg Thr Phe
   3905 3910 3915 3920
   Asp Val Glu Thr Pro Val Asp Arg Gln Gly Leu Arg Pro Gly Ser Val
   3925 3930 3935
   Asp Val Ala Phe Gly Ala Asn Val Leu His Ala Thr Arg Asp Ile Arg
   3940 3945 3950
   Arg Thr Val Ala Asn Leu Lys Arg Leu Leu Arg Pro Gly Gly Leu Leu
   3955 3960 3965
   Val Val Asn Asp Ala Thr Ala Val Gln Gly Tyr Val Thr Met Thr Phe
   3970 3975 3980
   Gly Leu Met Lys Gly Trp Trp Arg Phe Asp Asp Ala Gly Val Arg Met
   3985 3990 3995 4000
   Glu Gly Ser Pro Leu Leu Asp Ala Gly Gln Trp Leu Arg Val Leu Lys
   4005 4010 4015
   Ala Gly Gly Phe Gln Pro Ala Leu Ala Leu Gly Ser Pro Val Ala Ala
   4020 4025 4030
   Pro Glu Ala Ser Arg Gln Cys Leu Leu Met Ala Val Ser Asp Gly Leu
   4035 4040 4045
   Thr Trp Thr Glu Ser Glu Ala Arg Glu Thr Gln Pro Val Pro Pro Ala
   4050 4055 4060
   Arg Gln Ala Pro Val Pro Ser Glu Pro Arg Ser Ser Lys Ala Gly Thr
   4065 4070 4075 4080
   Gly Asp Leu Arg Ala Arg Val Leu Asp Tyr Val Arg Gly Val Phe Ser
   4085 4090 4095
   Glu Thr Leu Gly Leu Ala Lys Glu Arg Leu Pro Asp Asp Val Pro Leu
   4100 4105 4110
   Asp Arg Phe Gly Val Asp Ser Leu Val Asn Leu Lys Ile Val Ala Arg
   4115 4120 4125
   Phe Glu Ala Asp Leu Gly Gln Gln Pro Ser Thr Leu Leu Phe Glu His
   4130 4135 4140
   Leu Thr Leu Val Lys Leu Thr Asp His Phe Leu Ala Thr Ser Arg Asp
   4145 4150 4155 4160
   Ala Leu Glu Arg Leu Leu Gly Gly Thr Pro Thr Pro Pro Glu Pro Pro
   4165 4170 4175
   Gly Thr Arg Ala Pro Gln Pro Leu Pro Glu Pro Val Ala Pro Val Pro
   4180 4185 4190
   Gln Ala Ala Ala Asp Gly Asp Ile Ala Ile Ile Gly Cys Ala Gly Arg
   4195 4200 4205
   Tyr Pro Gln Ala Glu Asp Leu Glu Ala Phe Trp Thr Asn Leu Arg Glu
   4210 4215 4220
   Gly Arg His Ala Ile Thr Pro Val Pro Ala Glu Arg Trp Arg Pro Glu
   4225 4230 4235 4240
   Ala Gln Gly Ala Asp Val Tyr Ser Arg His Gly Gly Phe Ile Ser Gly
   4245 4250 4255
   Val Asp Arg Phe Asp Ala Ala Phe Phe Asn Met Val Pro Glu Asp Ala
   4260 4265 4270
   Asp Val Leu Asp Pro Gln Ala Arg Leu Phe Leu Glu Thr Ala Trp Gln
   4275 4280 4285
   Val Leu Glu Asp Ala Ala Gln Gly Pro Ala Ala Leu Asp Ala Val Asp
   4290 4295 4300
   Arg Arg Val Gly Val Phe Val Gly Ala Met Asn Gln Asp Tyr Glu Leu
   4305 4310 4315 4320
   Phe Ser Gly Glu Val His Ala Lys Gly Gly Phe Thr Arg Ala Asn Ala
   4325 4330 4335
   Ala His Trp Thr Leu Ser Asn Arg Val Ser Phe His Leu Asp Leu His
   4340 4345 4350
   Gly Pro Ser Leu Thr Val Asp Thr Ala Cys Ser Ser Ser Leu Thr Ala
   4355 4360 4365
   Leu His Leu Ala Val Glu Ser Leu Lys Arg Gly Glu Cys Arg Met Ala
   4370 4375 4380
   Ile Ala Gly Gly Val Asn Leu Ile Leu His Pro Leu His Tyr Gln Arg
   4385 4390 4395 4400
   Leu Cys Ser Val Asn Met Leu Ala Arg Asp Asp Arg Asn Lys Ala Phe
   4405 4410 4415
   Gly Ala Gly Ala Asp Gly Phe Val Asp Gly Glu Gly Val Gly Ala Val
   4420 4425 4430
   Leu Leu Lys Pro Leu Gln Glu Ala Leu Arg Asp Gly Asp Pro Ile His
   4435 4440 4445
   Ala Val Ile Lys Ala Thr Ala Ile Asn Thr Gly Gly Arg Thr Ser Gly
   4450 4455 4460
   Phe Thr Val Pro Asn Leu Asn Ala Gln Ala Asp Val Val Ala Thr Ala
   4465 4470 4475 4480
   Leu Gln Arg Ser Gly Val Ala Ala Asp Ser Ile Thr Tyr Val Glu Ala
   4485 4490 4495
   His Gly Thr Gly Thr Ala Leu Gly Asp Pro Ile Glu Val Ala Ala Leu
   4500 4505 4510
   Thr Lys Ala Phe Arg Thr Arg Thr Pro Ala Val Gly Phe Cys Ala Leu
   4515 4520 4525
   Gly Ser Val Lys Ser Asn Ile Gly His Leu Glu Ser Ala Ala Gly Ile
   4530 4535 4540
   Ala Gly Leu Thr Lys Val Val Leu Gln Leu Lys Glu Arg Met Leu Val
   4545 4550 4555 4560
   Pro Thr Leu His Ala Asp Thr Ala Asn Pro Lys Ile Ala Phe Glu Gly
   4565 4570 4575
   Ser Pro Phe Arg Val Gln Gln Arg Leu Thr Pro Trp Glu Leu Pro Ala
   4580 4585 4590
   Ser Ala Gly Ala His Ala Lys Arg Arg Ala Gly Leu Ser Ser Phe Gly
   4595 4600 4605
   Ala Gly Gly Ala Asn Ala His Val Ile Leu Glu Glu His Ala Ala Thr
   4610 4615 4620
   Trp Glu Ala Ala His Arg Gln Ala Pro Ala Ala Pro Gly Val Phe Val
   4625 4630 4635 4640
   Leu Ser Ala Lys Arg Pro Glu Ser Leu Arg Ala Ser Ala Glu Arg Met
   4645 4650 4655
   Arg Ala Phe Leu Arg Gln His Arg Glu Arg Leu Ser Leu Glu Asp Val
   4660 4665 4670
   Leu Tyr Thr Leu Gln Val Gly Arg Lys Ala Trp Pro Val Arg Leu Ala
   4675 4680 4685
   Leu Val Val Thr Ser Leu Asp Asp Leu Thr Gln Arg Leu Asp Ala Phe
   4690 4695 4700
   Leu Ser Gly Arg Ala Pro Val Asp Gly Leu Trp Glu Gly Thr Ala Thr
   4705 4710 4715 4720
   Gly Glu Gly Arg Asp Pro Glu Pro Ala His Asp Pro Arg Glu Val Ala
   4725 4730 4735
   Arg Arg Trp Val Gly Gly Ala Ala Phe Asp Trp Ala Ser Leu Tyr Arg
   4740 4745 4750
   Gly Gln Pro Arg Arg Ile His Leu Pro Thr Tyr Val Phe Asp Arg Lys
               4755                4760                4765
   Arg Cys Trp Phe Thr His Glu Glu Pro Glu Pro Leu His Val Pro Glu
           4770                4775                4780
   Pro Met His Thr Ala Pro Ala Pro Val Pro Asp Glu Pro Gln Ala Arg
   4785                4790                4795                4800
   Ile Leu Gln Val Ala Ala Glu Val Met Lys Arg Gln Ala Ser Ala Phe
                   4805                4810                4815
   Asp Pro Arg Gln Ser Leu Gln Asp Gln Gly Val Asp His Phe Asp Met
               4820                4825                4830
   Met Thr Ile Ile Leu Arg Leu Lys Ser Ala Phe Gly Ala Ala Phe Glu
           4835                4840                4845
   Pro Met Thr Val Val Ser Gly Gly Ser Ile Ala Glu Ile Ala Glu Ala
       4850                4855                4860
   Ile Arg Ala Arg Leu Pro Ser Gln Ala Glu Ser Pro Pro Ala Ser Pro
   4865                4870                4875                4880
   Ser Gly Gln Glu Ser Asp Val Gln Thr Arg Val Ala Pro Arg Val Arg
                   4885                4890                4895
   Ala Ala Ala Ala Arg Ile Leu Lys Val Gln Glu Ser Glu Ile Asp Met
               4900                4905                4910
   His Ala Asp Ile Ser Glu Tyr Gly Leu Asp Ser Ile Asn Val Met Phe
           4915                4920                4925
   Met Val Ala Lys Leu Arg Glu Asp Phe Ser Ile Gln Leu Glu Pro Gly
       4930                4935                4940
   Asp Val Val Ala His Arg Thr Leu Leu Glu Leu Ala His Tyr Val Ala
   4945                4950                4955                4960
   Gly Leu Thr Leu Pro Val Pro Pro Ala Pro Ala Arg Asn Gly Ile His
                   4965                4970                4975
   Glu Arg Glu Ser Ala
               4980
<210> 27
   <211> 573
   <212> PRT
   <213> Corallococcus coralloides
<220>
   <223> Orf1
<400> 27
   Met Thr Lys Ala Leu Ser Thr Ser Ser Asn Ser Arg Pro Gln Gln Leu
   1               5                   10                  15
   Leu Thr Arg Ile Leu Glu Glu Pro Glu Leu Val Ser Ile Val Gln Gly
               20                  25                  30
   Leu Glu Ala Pro Val Leu Ser Arg Leu Ile Asp His Ile Gly Leu Glu
           35                  40                  45
   Asp Ser Gly Glu Ile Val Ala Leu Ala Thr Thr Glu Gln Leu Lys Lys
       50                  55                  60
   Ile Phe Asp Glu Asp Leu Trp Arg Ser Ala Lys Pro Gly Lys Asp Gln
   65                  70                  75                  80
   Thr Phe Asp Ala Glu Arg Phe Ser Leu Trp Leu Gln Val Met Met Glu
                   85                  90                  95
   Ala Gly Val Glu Phe Ala Ala Gln Lys Leu Ala Glu Leu Asp Glu Asp
               100                 105                 110
   Leu Val Thr Leu Ala Val Cys Lys His Val Leu Val Ile Asn Ile Asp
           115                 120                 125
   Ala Leu Ala Glu Arg Met Ser Gly Asn Ser Arg Ser Asp Glu Asp Asp
       130                 135                 140
   Leu Thr Asp Lys Ala Leu Glu Ser Cys Leu Tyr Glu Glu Phe Ala Glu
   145                 150                 155                 160
   Tyr Gln Ile Ile Ala Lys Asp His Gln Ser Trp Asp Ala Leu Ile Ala
                   165                 170                 175
   Val Leu Val Glu Leu Asp Lys Asn His His Asp Phe Leu Met Arg Met
               180                 185                 190
   Leu Asp Arg Cys Cys Tyr Ile Ser Thr Glu Tyr Ile Asp Asp Asn Gly
           195                 200                 205
   Gly Leu Phe Asn Val Leu Asn Ser Glu Glu Met Leu Glu Ser Asp Val
       210                 215                 220
   Ala Ala Asp Arg Glu Asp Arg Arg Glu Ala Glu Gly Tyr Val Ala Pro
   225                 230                 235                 240
   Ser Ser Ala Ala Ser Phe Leu Asn Leu Ala Arg Val Thr Pro Thr Glu
                   245                 250                 255
   Glu Leu Ile Ala Asn Arg Ala Ala Asp Pro Ile Thr Gln Ser Tyr Phe
               260                 265                 270
   Lys Ser Leu Asp Arg Ala Ala Lys Glu Ala Ala Lys Lys Gly Pro Thr
           275                 280                 285
   Gln Ala Leu Pro Gln Gly Ala Pro Ser Gln Lys Val Val Lys Phe Leu
       290                 295                 300
   Gln Glu Leu Arg Glu Ala Glu Val Leu Glu Glu Pro Ala Gln His Pro
   305                 310                 315                 320
   Leu Leu Ala Ala Gly Glu Pro Gln Ala Ala His Thr Glu Pro Leu Phe
                   325                 330                 335
   Lys Leu Ala Val Leu Glu Val Arg Asp Arg Gln Pro Lys Ala Tyr Met
               340                 345                 350
   Asp Arg Leu Arg Glu Leu Ser Tyr Leu Ala Asn Val Leu Ile Ser Gly
           355                 360                 365
   Cys Ser Tyr Ala Gly Arg Ala Phe Arg Pro Leu Glu Ala Ala Asp Ala
       370                 375                 380
   Ala Val Ala Val Cys Asn Leu Gly Met Glu Arg Leu Leu Arg Asp Ala
   385                 390                 395                 400
   Ala Gly Asn Arg Pro Gly Asp Pro Thr Val Asp Asp Ala Ala Asp Leu
                   405                 410                 415
   Leu Glu Arg Gln Glu Leu Val Lys Leu Phe Gln Val Gly Trp Ser Leu
               420                 425                 430
   Leu His Arg Asp Val Gln Met Phe Thr Ala Arg Thr Leu Gln Glu Thr
           435                 440                 445
   Leu Thr Arg Met Ala Glu Gly Gln Arg Asp Ala Gln Lys Ser Lys Glu
       450                 455                 460
   Leu Thr Arg Met Ala Ala Thr Leu Lys Ala Asp Val Asn Ala Gly Lys
   465                 470                 475                 480
   Pro Thr Gln Ser Arg Gly Arg Leu Asp Gly Leu Gly His Val Leu Asp
                   485                 490                 495
   Arg Gln Val Ile Val Ala Val Lys Ala Leu Leu Ser Glu Tyr Pro Phe
               500                 505                 510
   Leu Pro Arg Ala Ile Ala Gly Gly Ser Lys Glu Thr Asp Lys Arg Asp
           515                 520                 525
   Ala Arg Phe Ile Phe Thr Gln Gln Gln Ile Gln Ala Val Gln Ala Phe
       530                 535                 540
   Val Gln Gly Ile Gly Ala Pro Ala Lys Lys Ala Ser Ala Pro Pro Ala
   545                 550                 555                 560
   Ser Pro Lys Asp Arg Pro Thr Gly Lys Asp Gly Arg Ser
                   565                 570
<210> 28
   <211> 240
   <212> PRT
   <213> Corallococcus coralloides
<220>
   <223> CorM
<400> 28
   Met Pro Arg Pro Gly Ala Arg Val Arg Leu Phe Phe Leu Pro Phe Ala
   1               5                   10                  15
   Gly Gly Gly Ala Ser Val Phe Arg Thr Trp His Arg Asp Leu Pro Glu
               20                  25                  30
   Thr Val Glu Gly Cys Ala Leu Glu Leu Pro Gly Arg Gly Ala Arg Ile
           35                  40                  45
   Arg Glu Leu Pro Ser Leu Arg Leu Glu Pro Leu Val Gln Gly Leu Ala
       50                  55                  60
   Asp Thr Leu Glu Pro Leu Leu Asp Arg Pro Phe Ala Leu Phe Gly His
   65                  70                  75                  80
   Ser Met Gly Gly Leu Ile Ser Phe Glu Leu Val Arg Glu Leu Arg Arg
                   85                  90                  95
   Arg His Ala Pro Leu Pro Cys His Leu Phe Ile Ser Ala Phe Ala Ala
               100                 105                 110
   Pro Gln His Ser Arg Trp Ser Pro Gln Val His Leu Met Ser Asn Glu
           115                 120                 125
   Ala Phe Leu Gln Phe Leu Val Arg Leu Glu Gly Thr Pro Pro Glu Val
       130                 135                 140
   Leu Ala Ser Pro Gly Leu Met Asp Met Leu Met Pro Ile Ile Arg Ala
   145                 150                 155                 160
   Asp Trp Ala Val Ile Glu Ser Tyr Thr Tyr Arg Arg Glu Pro Pro Leu
                   165                 170                 175
   Pro Val Pro Leu Thr Val Leu Gly Gly Glu Asp Asp Lys Ala Val Ala
               180                 185                 190
   Arg Val Ala Leu Glu Glu Trp Ala Glu Gln Thr Arg Glu Ser Phe Ala
           195                 200                 205
   Leu Lys Met Phe Pro Gly Gly His Phe Phe Leu Arg Thr Asn Ala Glu
       210                 215                 220
   Pro Leu Leu Gln Leu Phe Arg Gln Val Leu Gln Pro Trp Thr Gly Ser
   225                 230                 235                 240
<210> 29
   <211> 724
   <212> PRT
   <213> Corallococcus coralloides
<220>
   <223> CorN
<400> 29
   Met Val Ser Arg Ile Gln Arg Val Ala Val Leu Gly Thr Gly Ala Pro
   1               5                   10                  15
   Ala Leu Gly Leu Ala Ala His Leu Ala Asn Val Gly Val Pro Val Leu
               20                  25                  30
   Leu Leu Asp Ala Glu Arg Thr Ser Thr Ala Lys Glu Ala Leu Ile Arg
           35                  40                  45
   Ala Ile Glu Ala Arg Pro Ser Pro Phe Phe His Arg Ser Leu Ala Arg
       50                  55                  60
   Leu Val Thr Pro Gly Thr Leu Pro Gly Asp Ala Glu Ala Leu Arg Gly
   65                  70                  75                  80
   Cys Gly Leu Val Val Asp Ala Thr Arg Gly Thr Arg Ala Gly Thr Trp
                   85                  90                  95
   Glu Leu Leu Ala Arg Leu Glu Pro Leu Leu Ala Pro Asp Ala Leu Leu
               100                 105                 110
   Ala Val Asp Thr Ser Trp Leu Pro Leu Arg Asp Leu Val Ala Gly Arg
           115                 120                 125
   Ala Pro Asp Leu His Arg Arg Leu Leu Gly Met His Phe Val Ser Pro
       130                 135                 140
   Leu Arg Arg Thr Arg Leu Val Glu Leu Val Pro Gly Pro Gly Thr Glu
   145                 150                 155                 160
   Ala Gly Arg Leu Glu Glu Ala Thr Ala Phe Ala Arg Asp Val Leu Gly
                   165                 170                 175
   Leu Thr Val Val Val Ala Arg Glu Ser Pro His Leu Ile Ala Glu Arg
               180                 185                 190
   Val Ala Ala His Ala Phe Val Thr Cys Leu Gln Arg Met Glu Ala His
           195                 200                 205
   Gly Leu Ala Pro Gly Asp Val Glu Gly Leu Thr Ala Leu Leu Phe Ser
       210                 215                 220
   Arg Glu Arg Gly Val Phe Gln Ala Leu Glu Asp Trp Gly Ala Gly Ala
   225                 230                 235                 240
   Phe Met Asp Ala Val Asp Arg Cys His Ala Glu Leu Pro Glu Glu Arg
                   245                 250                 255
   Asn Gly Phe Gln Leu Pro Ser Pro Trp Arg Ala Gly Leu Pro Ser Pro
               260                 265                 270
   Glu Ala Arg Arg Glu Ala Asp Ala Thr Leu Thr Arg Thr Leu Arg Pro
           275                 280                 285
   Leu Leu Ala Glu Asp Asp Ala Ala Ala Arg Leu Arg Lys Leu Val Ala
       290                 295                 300
   Asp Glu Gly Arg Ala Gly Arg Phe Ala Trp Asp Val Val Ser Arg Ile
   305                 310                 315                 320
   Leu Ser His Ala Ala Arg Cys Val Gly Pro Val Ala Arg Ala Pro Gln
                   325                 330                 335
   Asp Val Asp Ala Ala Leu Thr Arg Gly Phe Gln Trp Asp Leu Gly Pro
               340                 345                 350
   Phe Gly Met Trp Ala Ala Leu Gly Val Thr Glu Ala Ala Gly Arg Met
           355                 360                 365
   Arg Arg Glu Gly Leu Pro Val Pro Glu Ala Val Glu Arg Arg Leu Arg
       370                 375                 380
   Glu Asp Ala Ala Pro Gly Ala Thr Pro Arg Ala Pro Val His Arg Ser
   385                 390                 395                 400
   Glu Arg Val Pro Val Leu Thr Ser Pro Gly Ala Glu Ala Trp Asp Leu
                   405                 410                 415
   Gly Asp Gly Val Leu Gly Val Gly Phe Lys Thr Lys His Asn Ser Ile
               420                 425                 430
   Asp Ala Asp Val Val Ala Thr Leu Gly Arg Ala Val Glu Arg Ala Glu
           435                 440                 445
   Ala Asp Phe Arg Ala Leu Val Leu Ala Asn Pro Gly Pro Asp Phe Ser
       450                 455                 460
   Val Gly Ala Asn Leu Met Leu Val Thr Met Trp Ala Ser Arg Lys Arg
   465                 470                 475                 480
   Trp Glu Asp Ile Arg Ala Leu Val Ala Ala Phe Gln Ala Ala Thr Gln
                   485                 490                 495
   Arg Leu Lys Tyr Ala Arg Val Pro Val Val Ala Val Pro Cys Gly Arg
               500                 505                 510
   Thr Leu Gly Gly Gly Leu Glu Leu Cys Leu Gly Ala Thr Ala Val Gln
           515                 520                 525
   Ala Ala Ala Glu Thr Gln Ala Gly Leu Val Glu Thr Glu Ile Gly Leu
       530                 535                 540
   Val Pro Gly Gly Gly Gly Thr Leu Asn Leu Leu Trp Arg Ala Leu Gln
   545                 550                 555                 560
   Gly Ile Pro Glu Gly Val Gln Val Asp Ala Leu Pro Tyr Val Ala Lys
                   565                 570                 575
   Val Phe Gln Asp Ile Ala Arg Ala Arg Val Ser Thr Ser Ala Val Glu
               580                 585                 590
   Ala Gln His Leu Gly Tyr Phe Arg Leu Thr Asp Gly Val Ser Val Arg
           595                 600                 605
   Pro Glu His Arg Leu Glu Glu Ala Arg Ala Arg Ala Leu Gly Leu Ala
       610                 615                 620
   Gly Ala Gly Tyr Arg Pro Pro Val Pro Arg Ala Phe Arg Leu Pro Gly
   625                 630                 635                 640
   Ala Pro Gly Ile Arg Ala Leu Glu Asp Gly Leu Ala Glu Gly Arg Ala
                   645                 650                 655
   Arg Gly Glu Leu Thr Ala His Asp Ala Leu Leu Gly Ala Arg Leu Ala
               660                 665                 670
   Ala Ile Leu Cys Gly Gly Gln Gly Gly Ala Gly His Glu Val Thr Glu
           675                 680                 685
   Glu Glu Val Leu Ala Leu Glu Gln Glu Ala Phe Val Ser Leu Cys Gly
       690                 695                 700
   Glu Pro Arg Ser Leu Glu Arg Met Gln Ser Leu Leu Met His Arg Arg
   705                 710                 715                 720
   Val Leu Arg Asn
<210> 30
   <211> 449
   <212> PRT
   <213> Corallococcus coralloides
<220>
   <223> CorO
<400> 30
   Met Ser Ser Arg Leu Pro Pro Gly Pro Arg Phe Pro Pro Leu Gln Met
   1               5                   10                  15
   Val Arg Phe Thr Lys Asp Ala Thr Arg Phe Phe Glu Asp Cys Ala Lys
               20                  25                  30
   Arg Cys Gly Asp Pro Phe Thr Met Thr Leu Pro Leu Gly Thr Val Val
           35                  40                  45
   Val Thr Gly Ser Pro Gln Val Thr Gln Gln Ile Phe Ala Ala Asp Pro
       50                  55                  60
   Asn Ile Phe Glu Pro Met Ala Lys Val Pro Met Glu Pro Leu Ile Gly
   65                  70                  75                  80
   Glu Asn Ser Leu Leu Leu Ile Gly Gly Asp Arg His Lys Arg Glu Arg
                   85                  90                  95
   Arg Leu Ile Met Pro Ala Phe His Gly Glu Arg Met Arg Thr Tyr Gly
               100                 105                 110
   Ala Leu Met Gln Glu Ala Ala Leu Arg Gln Leu Glu Gly Lys Lys Pro
           115                 120                 125
   Gly Ala Thr Leu Thr Ala Gln Gln Ile Thr Gln Ala Ile Ser Met Glu
       130                 135                 140
   Val Ile Ile Arg Ala Val Phe Gly Val Glu Gly Ala Ala Arg Val Glu
   145                 150                 155                 160
   Arg Phe Arg His Val Leu Glu Ala Tyr Leu Glu Asn Tyr Thr Pro Ser
                   165                 170                 175
   Leu Ala Met Met Pro Val Leu Arg Arg Gln Phe Gly Gly Met Gly Pro
               180                 185                 190
   Trp Ala Arg Phe Gln Arg His Ala Val Glu Leu Asp Val Met Leu Thr
           195                 200                 205
   Glu Glu Ile Ala Ala Arg Arg Ala Ser Gly Glu Lys Arg Glu Asp Val
       210                 215                 220
   Leu Ser Met Leu Leu Ala Ala Lys Asp Glu Asn Gly Glu Pro Met Thr
   225                 230                 235                 240
   Asp Val Glu Val Lys Asp Glu Leu Arg Thr Met Leu Val Ala Gly His
                   245                 250                 255
   Glu Thr Thr Ala Val Ser Met Thr Trp Gly Met Tyr Phe Leu His Arg
               260                 265                 270
   Ala Pro Glu Ala Leu Ala Lys Leu Arg Gln Glu Leu Ala Ala Phe Pro
           275                 280                 285
   Thr Ala Pro Asp Ala Glu Thr Leu Ser Lys Leu Pro Tyr Leu Ser Ala
       290                 295                 300
   Val Cys Asp Glu Ser Leu Arg Leu Asn Pro Val Phe Tyr Leu Val Ser
   305                 310                 315                 320
   Arg Lys Leu Arg Glu Pro Phe Thr Leu Ala Gly Tyr Glu Leu Pro Ala
                   325                 330                 335
   Gly Thr Gly Leu Met Val Ala Ile Thr Leu Thr His Ser Arg Pro Glu
               340                 345                 350
   Thr Phe Pro Glu Pro Glu Arg Phe Gln Pro Glu Arg Phe Leu Gly Arg
           355                 360                 365
   Arg Tyr Ser Pro Phe Glu Tyr Val Pro Phe Gly Gly Gly Ala Arg Arg
       370                 375                 380
   Cys Ile Gly Ala Ala Phe Ala Leu Tyr Glu Met Lys Val Thr Leu Gly
   385                 390                 395                 400
   Thr Leu Leu Trp Ala His Arg Phe Glu Leu Val Asn Asp Thr Glu Val
                   405                 410                 415
   Arg Pro Ile Arg Arg Asn Val Thr Ala Val Pro His Glu Asp Ile Pro
               420                 425                 430
   Leu Arg Tyr Thr Gly Leu Ala Val Pro Ala Arg Val Ala Ala Pro Ala
           435                 440                 445
   Ala
<210> 31
   <211> 24
   <212> DNA
   <213> Artificial Sequence
<220>
   <221> source
   <222> 1..24
   <223> /mol_type="unassigned DNA" /note= "mxn9" /organism="Artificial Sequence"
<400> 31
   gtcagacata tgaacaacag cggt 24
<210> 32
   <211> 29
   <212> DNA
   <213> Artificial Sequence
<220>
   <221> source
   <222> 1..29
   <223> /mol_type="unassigned DNA" /note="primer mxn10" /organism="Artificial Sequence"
<400> 32
   cgtcgtaagc tttcagtagg tgaaaacca 29
<210> 33
   <211> 30
   <212> DNA
   <213> Artificial Sequence
<220>
   <221> source
   <222> 1..30
   <223> /mol_type="unassigned DNA" /note="primer mxn214" /organism="Artificial Sequence"
<400> 33
   gatacgcata tggtcccctt cgagtcgagc 30
<210> 34
   <211> 30
   <212> DNA
   <213> Artificial Sequence
<220>
   <221> source
   <222> 1..30
   <223> /mol_type="unassigned DNA" /note="primer mxn215" /organism="Artificial Sequence"
<400> 34
   tctatgaagc tttcatggct tcgctcccgc 30
<210> 35
   <211> 27
   <212> DNA
   <213> Artificial Sequence
<220>
   <221> source
   <222> 1..27
   <223> /mol_type="unassigned DNA" /note="primer mxn230" /organism="Artificial Sequence"
<400> 35
   ctgaggcata tgcgtgcgtt cgagtcg 27
<210> 36
   <211> 30
   <212> DNA
   <213> Artificial Sequence
<220>
   <221> source
   <222> 1..30
   <223> /mol_type="unassigned DNA" /note="primer mxn231" /organism="Artificial Sequence"
<400> 36
   tctatgaagc tttcaggcac accactcatt 30
<210> 37
   <211> 45
   <212> DNA
   <213> Artificial Sequence
<220>
   <221> source
   <222> 1..45
   <223> /mol_type="unassigned DNA" /note="primer mxn30" /organism="Artificial Sequence"
<400> 37
   tgcttaatta atattcatat ggctagcgag ctcctggatg gcctt 45
<210> 38
   <211> 29
   <212> DNA
   <213> Artificial Sequence
<220>
   <221> source
   <222> 1..29
   <223> /mol_type="unassigned DNA" /note="primer mxn33" /organism="Artificial Sequence"
<400> 38
   caggtagata tctggacagc aagcgaacc 29
<210> 39
   <211> 20
   <212> DNA
   <213> Artificial Sequence
<220>
   <221> source
   <222> 1..20
   <223> /mol_type="unassigned DNA" /note="primer mxn34" /organism="Artificial Sequence"
<400> 39
   atcctgtctc ttgatcagat 20
<210> 40
   <211> 40
   <212> DNA
   <213> Artificial Sequence
<220>
   <221> source
   <222> 1..40
   <223> /mol_type="unassigned DNA" /note="primer mxn35" /organism="Artificial Sequence"
<400> 40
   agatctgatc aagagacagg atttcctgga gacgtcgtgg 40
<210> 41
   <211> 33
   <212> DNA
   <213> Artificial Sequence
<220>
   <221> source
   <222> 1..33
   <223> /mol_type="unassigned DNA" /note="primer mxn36" /organism="Artificial Sequence"
<400> 41
   catgtcggat cctcaccatc gtccacagct tcg 33
<210> 42
   <211> 35
   <212> DNA
   <213> Artificial Sequence
<220>
   <221> source
   <222> 1..35
   <223> /mol_type="unassigned DNA" /note="primer mxn41" /organism="Artificial Sequence"
<400> 42
   aatctgtacc tccttatcct gtctcttgat cagat 35
<210> 43
   <211> 57
   <212> DNA
   <213> Artificial Sequence
<220>
   <221> source
   <222> 1..57
   <223> /mol_type="unassigned DNA" /note="primer mxn42" /organism="Artificial Sequence"
<400> 43
   caagatctga tcaagagaca ggataaggag gtacagatta tgactttcac cgtcgtt 57
<210> 44
   <211> 18
   <212> DNA
   <213> Artificial Sequence
<220>
   <221> source
   <222> 1..18
   <223> /mol_type="unassigned DNA" /note="primer mxn48" /organism="Artificial Sequence"
<400> 44
   acgtcgacga gtactggc 18
<210> 45
   <211> 18
   <212> DNA
   <213> Artificial Sequence
<220>
   <221> source
   <222> 1..18
   <223> /mol_type="unassigned DNA" /note="primer mxn49" /organism="Artificial Sequence"
<400> 45
   aatgccttcg gagcctcg 18
<210> 46
   <211> 30
   <212> DNA
   <213> Artificial Sequence
<220>
   <221> source
   <222> 1..30
   <223> /mol_type="unassigned DNA" /note="primer mxn50" /organism="Artificial Sequence"
<400> 46
   catgtagata tcgaacggct ccggtacatc 30
<210> 47
   <211> 30
   <212> DNA
   <213> Artificial Sequence
<220>
   <221> source
   <222> 1..30
   <223> /mol_type="unassigned DNA" /note="primer mxn51" /organism="Artificial Sequence"
<400> 47
   tgtctaggat ccacagccgc tccaggattc 30
<210> 48
   <211> 30
   <212> DNA
   <213> Artificial Sequence
<220>
   <221> source
   <222> 1..30
   <223> /mol_type="unassigned DNA" /note="primer mxn52" /organism="Artificial Sequence"
<400> 48
   gatacacagc tgtccctcct gttcagctac 30
<210> 49
   <211> 29
   <212> DNA
   <213> Artificial Sequence
<220>
   <221> source
   <222> 1..29
   <223> /mol_type="unassigned DNA" /note="primer mxn53" /organism="Artificial Sequence"
<400> 49
   atcttaatta aggacgcgat ggatatgtt 29
<210> 50
   <211> 20
   <212> DNA
   <213> Artificial Sequence
<220>
   <221> source
   <222> 1..20
   <223> /mol_type="unassigned DNA" /note="primer mxn54" /organism="Artificial Sequence"
<400> 50
   gcgttgatgg atccacagca 20
<210> 51
   <211> 35
   <212> DNA
   <213> Artificial Sequence
<220>
   <221> source
   <222> 1..35
   <223> /mol_type="unassigned DNA" /note="primer mxn55" /organism="Artificial Sequence"
<400> 51
   tgtatcgcgg ccgctcagtg ccgctgaacc cgaac 35
<210> 52
   <211> 32
   <212> DNA
   <213> Artificial Sequence
<220>
   <221> source
   <222> 1..32
   <223> /mol_type="unassigned DNA" /note="primer mxn56" /organism="Artificial Sequence"
<400> 52
   gatacagcgg ccgcctttcc gagtccagca gc 32
<210> 53
   <211> 29
   <212> DNA
   <213> Artificial Sequence
<220>
   <221> source
   <222> 1..29
   <223> /mol_type="unassigned DNA" /note="primer mxn57" /organism="Artificial Sequence"
<400> 53
   gtatcgagct catctgctgt cctctgcct 29
<210> 54
   <211> 16
   <212> DNA
   <213> Artificial Sequence
<220>
   <221> source
   <222> 1..16
   <223> /mol_type="unassigned DNA" /note="primer mxn58" /organism="Artificial Sequence"
<400> 54
   gtacgtgccg ttggat 16
<210> 55
   <211> 18
   <212> DNA
   <213> Artificial Sequence
<220>
   <221> source
   <222> 1..18
   <223> /mol_type="unassigned DNA" /note="primer mxn59" /organism="Artificial Sequence"
<400> 55
   gaggggtact tgaagagc 18
<210> 56
   <211> 19
   <212> DNA
   <213> Artificial Sequence
<220>
   <221> source
   <222> 1..19
   <223> /mol_type="unassigned DNA" /note="primer mxn60" /organism="Artificial Sequence"
<400> 56
   tgccatgagc agacagtta 19
<210> 57
   <211> 18
   <212> DNA
   <213> Artificial Sequence
<220>
   <221> source
   <222> 1..18
   <223> /mol_type="unassigned DNA" /note="primer mxn64" /organism="Artificial Sequence"
<400> 57
   gctgagacgg tcttcctc 18
<210> 58
   <211> 45
   <212> DNA
   <213> Artificial Sequence
<220>
   <221> source
   <222> 1..45
   <223> /mol_type="unassigned DNA" /note="primer mxn65" /organism="Artificial Sequence"
<400> 58
   tgcttaatta atattcatat ggctagcacg cgattcactg tctca 45
<210> 59
   <211> 18
   <212> DNA
   <213> Artificial Sequence
<220>
   <221> source
   <222> 1..18
   <223> /mol_type="unassigned DNA" /note="primer mxn 68" /organism="Artificial Sequence"
<400> 59
   caacatctcg ccgagtga 18
<210> 60
   <211> 20
   <212> DNA
   <213> Artificial Sequence
<220>
   <221> source
   <222> 1..20
   <223> /mol_type="unassigned DNA" /note="primer mxn69" /organism="Artificial Sequence"
<400> 60
   atacgactca ctatagggcg 20
<210> 61
   <211> 18
   <212> DNA
   <213> Artificial Sequence
<220>
   <221> source
   <222> 1..18
   <223> /mol_type="unassigned DNA" /note="primer mxn70" /organism="Artificial Sequence"
<400> 61
   cttgcgccct gagtgctt 18
<210> 62
   <211> 18
   <212> DNA
   <213> Artificial Sequence
<220>
   <221> source
   <222> 1..18
   <223> /mol_type="unassigned DNA" /note="primer mxn71" /organism="Artificial Sequence"
<400> 62
   aacacctcac ggtccgac 18
<210> 63
   <211> 18
   <212> DNA
   <213> Artificial Sequence
<220>
   <221> source
   <222> 1..18
   <223> /mol_type="unassigned DNA" /note="primer mxn72" /organism="Artificial Sequence"
<400> 63
   cccatcgtcc acagcttc 18
<210> 64
   <211> 18
   <212> DNA
   <213> Artificial Sequence
<220>
   <221> source
   <222> 1..18
   <223> /mol_type="unassigned DNA" /note="primer mxn73" /organism="Artificial Sequence"
<400> 64
   gcactacaag cggctctg 18
<210> 65
   <211> 28
   <212> DNA
   <213> Artificial Sequence
<220>
   <221> source
   <222> 1..28
   <223> /mol_type="unassigned DNA" /note="primer mxn83" /organism="Artificial Sequence"
<400> 65
   gagcggccgc ctcgagatga aggtggat 28
<210> 66
   <211> 27
   <212> DNA
   <213> Artificial Sequence
<220>
   <221> source
   <222> 1..27
   <223> /mol_type="unassigned DNA" /note="primer mxn84" /organism="Artificial Sequence"
<400> 66
   atgactagtc tcctcgctct cgaagtc 27
<210> 67
   <211> 32
   <212> DNA
   <213> Artificial Sequence
<220>
   <221> source
   <222> 1..32
   <223> /mol_type="unassigned DNA" /note="primer mxn85" /organism="Artificial Sequence"
<400> 67
   gacacgatcg caacagcggt tctttctctt tg 32
<210> 68
   <211> 30
   <212> DNA
   <213> Artificial Sequence
<220>
   <221> source
   <222> 1..30
   <223> /mol_type="unassigned DNA" /note="primer mxn87" /organism="Artificial Sequence"
<400> 68
   caaggcggcc gccgggtgat cgcaggtgag 30
<210> 69
   <211> 28
   <212> DNA
   <213> Artificial Sequence
<220>
   <221> source
   <222> 1..28
   <223> /mol_type="unassigned DNA" /note="primer mxn88" /organism="Artificial Sequence"
<400> 69
   tctgggatcc ttcgacctcg gacagcag 28
<210> 70
   <211> 18
   <212> DNA
   <213> Artificial Sequence
<220>
   <221> source
   <222> 1..18
   <223> /mol_type="unassigned DNA" /note="primer mxn94" /organism="Artificial Sequence"
<400> 70
   tcacggcgaa gcacatcc 18
<210> 71
   <211> 18
   <212> DNA
   <213> Artificial Sequence
<220>
   <221> source
   <222> 1..18
   <223> /mol_type="unassigned DNA" /note="primer mxn95" /organism="Artificial Sequence"
<400> 71
   aggaggcagg gtccgaat 18
<210> 72
   <211> 18
   <212> DNA
   <213> Artificial Sequence
<220>
   <221> source
   <222> 1..18
   <223> /mol_type="unassigned DNA" /note="primer mxn98" /organism="Artificial Sequence"
<400> 72
   gactcagctc gtttggcg 18
<210> 73
   <211> 18
   <212> DNA
   <213> Artificial Sequence
<220>
   <221> source
   <222> 1..18
   <223> /mol_type="unassigned DNA" /note="primer mxn99" /organism="Artificial Sequence"
<400> 73
   taacccaggc gcagaatc 18
<210> 74
   <211> 18
   <212> DNA
   <213> Artificial Sequence
<220>
   <221> source
   <222> 1..18
   <223> /mol_type="unassigned DNA" /note="primer mxn100" /organism="Artificial Sequence"
<400> 74
   aggatgcgcg tcaagagt 18
<210> 75
   <211> 18
   <212> DNA
   <213> Artificial Sequence
<220>
   <221> source
   <222> 1..18
   <223> /mol_type="unassigned DNA" /note="primer mxn101" /organism="Artificial Sequence"
<400> 75
   gatacgaaga agcgcgag 18
<210> 76
   <211> 19
   <212> DNA
   <213> Artificial Sequence
<220>
   <221> source
   <222> 1..19
   <223> /mol_type="unassigned DNA" /note="primer mxn102" /organism="Artificial Sequence"
<400> 76
   cgtcattcat ccttgctcg 19
<210> 77
   <211> 18
   <212> DNA
   <213> Artificial Sequence
<220>
   <221> source
   <222> 1..18
   <223> /mol_type="unassigned DNA" /note="primer mxn103" /organism="Artificial Sequence"
<400> 77
   tccaggcctc gtagttcc 18
<210> 78
   <211> 18
   <212> DNA
   <213> Artificial Sequence
<220>
   <221> source
   <222> 1..18
   <223> /mol_type="unassigned DNA" /note="primer mxn104" /organism="Artificial Sequence"
<400> 78
   tttcctcacg tctcatgg 18
<210> 79
   <211> 18
   <212> DNA
   <213> Artificial Sequence
<220>
   <221> source
   <222> 1..18
   <223> /mol_type="unassigned DNA" /note="primer mxn105" /organism="Artificial Sequence"
<400> 79
   tgtcgctgtt gaaggcac 18
<210> 80
   <211> 18
   <212> DNA
   <213> Artificial Sequence
<220>
   <221> source
   <222> 1..18
   <223> /mol_type="unassigned DNA" /note="primer mxn106" /organism="Artificial Sequence"
<400> 80
   ttctcatcgc ggactacg 18
<210> 81
   <211> 18
   <212> DNA
   <213> Artificial Sequence
<220>
   <221> source
   <222> 1..18
   <223> /mol_type="unassigned DNA" /note="primer mxn107" /organism="Artificial Sequence"
<400> 81
   gagagagatt gacgcgac 18
<210> 82
   <211> 18
   <212> DNA
   <213> Artificial Sequence
<220>
   <221> source
   <222> 1..18
   <223> /mol_type="unassigned DNA" /note="primer mxn110" /organism="Artificial Sequence"
<400> 82
   gattctctgg gggctcct 18
<210> 83
   <211> 18
   <212> DNA
   <213> Artificial Sequence
<220>
   <221> source
   <222> 1..18
   <223> /mol_type="unassigned DNA" /note="primer mxn111" /organism="Artificial Sequence"
<400> 83
   caatcacaaa cgcagtcc 18
<210> 84
   <211> 18
   <212> DNA
   <213> Artificial Sequence
<220>
   <221> source
   <222> 1..18
   <223> /mol_type="unassigned DNA" /note="primer mxn112" /organism="Artificial Sequence"
<400> 84
   ttctcatcgc ggactacg 18
<210> 85
   <211> 28
   <212> DNA
   <213> Artificial Sequence
<220>
   <221> source
   <222> 1..28
   <223> /mol_type="unassigned DNA" /note="primer mxn113" /organism="Artificial Sequence"
<400> 85
   gatacgatcg cctggaggaa tacgtcgg 28
<210> 86
   <211> 30
   <212> DNA
   <213> Artificial Sequence
<220>
   <221> source
   <222> 1..30
   <223> /mol_type="unassigned DNA" /note="primer mxn114" /organism="Artificial Sequence"
<400> 86
   gtcgcggccg cctttcgtca tggtggctcc 30
<210> 87
   <211> 31
   <212> DNA
   <213> Artificial Sequence
<220>
   <221> source
   <222> 1..31
   <223> /mol_type="unassigned DNA" /note="primer mxn115" /organism="Artificial Sequence"
<400> 87
   gatacgcggc cgcttgaagg gactgggcaa g 31
<210> 88
   <211> 28
   <212> DNA
   <213> Artificial Sequence
<220>
   <221> source
   <222> 1..28
   <223> /mol_type="unassigned DNA" /note="primer mxn116" /organism="Artificial Sequence"
<400> 88
   ttgcggatcc acaatctatc gcgggtcg 28
<210> 89
   <211> 28
   <212> DNA
   <213> Artificial Sequence
<220>
   <221> source
   <222> 1..28
   <223> /mol_type="unassigned DNA" /note="primer mxn117" /organism="Artificial Sequence"
<400> 89
   gacacgatcg gcatgcatga ggcctaca 28
<210> 90
   <211> 31
   <212> DNA
   <213> Artificial Sequence
<220>
   <221> source
   <222> 1..31
   <223> /mol_type="unassigned DNA" /note="primer mxn118" /organism="Artificial Sequence"
<400> 90
   aatcgcggcc gcgcatcagg attccccaag g 31
<210> 91
   <211> 31
   <212> DNA
   <213> Artificial Sequence
<220>
   <221> source
   <222> 1..31
   <223> /mol_type="unassigned DNA" /note="primer mxn119" /organism="Artificial Sequence"
<400> 91
   tctggcggcc gcgcattgct cgcggctcag t 31
<210> 92
   <211> 40
   <212> DNA
   <213> Artificial Sequence
<220>
   <221> source
   <222> 1..40
   <223> /mol_type="unassigned DNA" /note="primer mxn120" /organism="Artificial Sequence"
<400> 92
   agatctgatc aagagacagg atgagttcat cgccttggag 40
<210> 93
   <211> 33
   <212> DNA
   <213> Artificial Sequence
<220>
   <221> source
   <222> 1..33
   <223> /mol_type="unassigned DNA" /note="primer mxn121" /organism="Artificial Sequence"
<400> 93
   catgtcggat cctcatcgct cttcacgtac acg 33
<210> 94
   <211> 25
   <212> DNA
   <213> Artificial Sequence
<220>
   <221> source
   <222> 1..25
   <223> /mol_type="unassigned DNA" /note="primer mxn124" /organism="Artificial Sequence"
<400> 94
   caatacgatc gctggactgg tgaag 25
<210> 95
   <211> 30
   <212> DNA
   <213> Artificial Sequence
<220>
   <221> source
   <222> 1..30
   <223> /mol_type="unassigned DNA" /note="primer mxn126" /organism="Artificial Sequence"
<400> 95
   gatacaccta ggtccctcct gttcagctac 30
<210> 96
   <211> 30
   <212> DNA
   <213> Artificial Sequence
<220>
   <221> source
   <222> 1..30
   <223> /mol_type="unassigned DNA" /note="primer mxn 127" /organism="Artificial Sequence"
<400> 96
   tcaggtccta ggggacgcga tggatatgtt 30
<210> 97
   <211> 88
   <212> DNA
   <213> Artificial Sequence
<220>
   <221> source
   <222> 1..88
   <223> /mol_type="unassigned DNA" /note="primer mxn128" /organism="Artificial Sequence"
<400> 97
   acatcgccta ggcgcgccct gagcgccctg cggaacaagg agtgtgatgc ggcgatcgga 60
   catacttaag ggatctgatc agcacgtg 88
<210> 98
   <211> 78
   <212> DNA
   <213> Artificial Sequence
<220>
   <221> source
   <222> 1..78
   <223> /mol_type="unassigned DNA" /note="primer mxn129" /organism="Artificial Sequence"
<400> 98
   tctatgccta ggtgcgcaaa ccaacccttg gcagaacata tccatcgcgt ccttaattaa 60
   tcagtcctgc tcctcggc 78
<210> 99
   <211> 30
   <212> DNA
   <213> Artificial Sequence
<220>
   <221> source
   <222> 1..30
   <223> /mol_type="unassigned DNA" /note="primer mxn132" /organism="Artificial Sequence"
<400> 99
   gatacacgat cgcgagcagg aggggtatgt 30
<210> 100
   <211> 30
   <212> DNA
   <213> Artificial Sequence
<220>
   <221> source
   <222> 1..30
   <223> /mol_type="unassigned DNA" /note="primer mxn133" /organism="Artificial Sequence"
<400> 100
   tctatggagc tccattccga agaactggga 30
<210> 101
   <211> 30
   <212> DNA
   <213> Artificial Sequence
<220>
   <221> source
   <222> 1..30
   <223> /mol_type="unassigned DNA" /note="primer mxn134" /organism="Artificial Sequence"
<400> 101
   gatacagagc tcttcctcaa tcaccacggt 30
<210> 102
   <211> 30
   <212> DNA
   <213> Artificial Sequence
<220>
   <221> source
   <222> 1..30
   <223> /mol_type="unassigned DNA" /note="primer mxn135" /organism="Artificial Sequence"
<400> 102
   tctatgggat cctgactgac gacgatgagc 30
<210> 103
   <211> 18
   <212> DNA
   <213> Artificial Sequence
<220>
   <221> source
   <222> 1..18
   <223> /mol_type="unassigned DNA" /note="primer mxn136" /organism="Artificial Sequence"
<400> 103
   aacatcggcc acctggag 18
<210> 104
   <211> 18
   <212> DNA
   <213> Artificial Sequence
<220>
   <221> source
   <222> 1..18
   <223> /mol_type="unassigned DNA" /note="primer mxn137" /organism="Artificial Sequence"
<400> 104
   aggacccagg cttctcgt 18
<210> 105
   <211> 18
   <212> DNA
   <213> Artificial Sequence
<220>
   <221> source
   <222> 1..18
   <223> /mol_type="unassigned DNA" /note="primer mxn140" /organism="Artificial Sequence"
<400> 105
   acatgttgga agccgacg 18
<210> 106
   <211> 18
   <212> DNA
   <213> Artificial Sequence
<220>
   <221> source
   <222> 1..18
   <223> /mol_type="unassigned DNA" /note="primer mxn141" /organism="Artificial Sequence"
<400> 106
   gctcagcgtg aagaggct 18
<210> 107
   <211> 18
   <212> DNA
   <213> Artificial Sequence
<220>
   <221> source
   <222> 1..18
   <223> /mol_type="unassigned DNA" /note="primer mxn142" /organism="Artificial Sequence"
<400> 107
   accgtggaac agctcagg 18
<210> 108
   <211> 18
   <212> DNA
   <213> Artificial Sequence
<220>
   <221> source
   <222> 1..18
   <223> /mol_type="unassigned DNA" /note="primer mxn143" /organism="Artificial Sequence"
<400> 108
   cgagatccag cacaccat 18
<210> 109
   <211> 30
   <212> DNA
   <213> Artificial Sequence
<220>
   <221> source
   <222> 1..30
   <223> /mol_type="unassigned DNA" /note="primer mxn144" /organism="Artificial Sequence"
<400> 109
   gatacattaa ttaaccggaa ttgccagctg 30
<210> 110
   <211> 32
   <212> DNA
   <213> Artificial Sequence
<220>
   <221> source
   <222> 1..32
   <223> /mol_type="unassigned DNA" /note="primer mxn145" /organism="Artificial Sequence"
<400> 110
   ctgttaatta atcagaagaa ctcgtcaaga ag 32
<210> 111
   <211> 30
   <212> DNA
   <213> Artificial Sequence
<220>
   <221> source
   <222> 1..30
   <223> /mol_type="unassigned DNA" /note="primer mxn147" /organism="Artificial Sequence"
<400> 111
   tatgtcctta agataattcg gctgcagggg 30
<210> 112
   <211> 32
   <212> DNA
   <213> Artificial Sequence
<220>
   <221> source
   <222> 1..32
   <223> /mol_type="unassigned DNA" /note="primer mxn148" /organism="Artificial Sequence"
<400> 112
   tatcttaatt aattaccaat gcttaatcag tg 32
<210> 113
   <211> 18
   <212> DNA
   <213> Artificial Sequence
<220>
   <221> source
   <222> 1..18
   <223> /mol_type="unassigned DNA" /note="primer mxn149" /organism="Artificial Sequence"
<400> 113
   gcttcgcgtg gaggtcat 18
<210> 114
   <211> 30
   <212> DNA
   <213> Artificial Sequence
<220>
   <221> source
   <222> 1..30
   <223> /mol_type="unassigned DNA" /note="primer mxn169" /organism="Artificial Sequence"
<400> 114
   gatacacgat cgcacgagtt ccgagtcctc 30
<210> 115
   <211> 18
   <212> DNA
   <213> Artificial Sequence
<220>
   <221> source
   <222> 1..18
   <223> /mol_type="unassigned DNA" /note="primer mxn170" /organism="Artificial Sequence"
<400> 115
   ctgcaggatg agggcgtc 18
<210> 116
   <211> 38
   <212> DNA
   <213> Artificial Sequence
<220>
   <221> source
   <222> 1..38
   <223> /mol_type="unassigned DNA" /note="primer mxn171" /organism="Artificial Sequence"
<400> 116
   tcgacgccct catcctgcag gagttgatgg cggagctg 38
<210> 117
   <211> 32
   <212> DNA
   <213> Artificial Sequence
<220>
   <221> source
   <222> 1..32
   <223> /mol_type="unassigned DNA" /note="primer mxn172" /organism="Artificial Sequence"
<400> 117
   tctatggcgg ccgcgagcgc gaccttgatg ac 32
<210> 118
   <211> 30
   <212> DNA
   <213> Artificial Sequence
<220>
   <221> source
   <222> 1..30
   <223> /mol_type="unassigned DNA" /note="primer mxn174" /organism="Artificial Sequence"
<400> 118
   gatacacgat cggacacaca ccggaagctg 30
<210> 119
   <211> 18
   <212> DNA
   <213> Artificial Sequence
<220>
   <221> source
   <222> 1..18
   <223> /mol_type="unassigned DNA" /note="primer mxn175" /organism="Artificial Sequence"
<400> 119
   attcaccagc gcgtcgac 18
<210> 120
   <211> 35
   <212> DNA
   <213> Artificial Sequence
<220>
   <221> source
   <222> 1..35
   <223> /mol_type="unassigned DNA" /note="primer mxn176" /organism="Artificial Sequence"
<400> 120
   cggcgtcgac gcgctggtga atctgagaat cgtcc 35
<210> 121
   <211> 30
   <212> DNA
   <213> Artificial Sequence
<220>
   <221> source
   <222> 1..30
   <223> /mol_type="unassigned DNA" /note="primer mxn177" /organism="Artificial Sequence"
<400> 121
   tctatggagc tcttgagctg caggatgacc 30
<210> 122
   <211> 17
   <212> DNA
   <213> Artificial Sequence
<220>
   <221> source
   <222> 1..17
   <223> /mol_type="unassigned DNA" /note="primer mxn185" /organism="Artificial Sequence"
<400> 122
   tcgctcatcc tgcagga 17
<210> 123
   <211> 17
   <212> DNA
   <213> Artificial Sequence
<220>
   <221> source
   <222> 1..17
   <223> /mol_type="unassigned DNA" /note="primer mxn187" /organism="Artificial Sequence"
<400> 123
   cgagtcgagt cccatgg 17
<210> 124
   <211> 17
   <212> DNA
   <213> Artificial Sequence
<220>
   <221> source
   <222> 1..17
   <223> /mol_type="unassigned DNA" /note="primer mxn189" /organism="Artificial Sequence"
<400> 124
   caacagcttc ctcgacg 17
<210> 125
   <211> 16
   <212> DNA
   <213> Artificial Sequence
<220>
   <221> source
   <222> 1..16
   <223> /mol_type="unassigned DNA" /note="primer mxn193" /organism="Artificial Sequence"
<400> 125
   ggagtcgacg ccgaac 16
<210> 126
   <211> 18
   <212> DNA
   <213> Artificial Sequence
<220>
   <221> source
   <222> 1..18
   <223> /mol_type="unassigned DNA" /note="primer mxn195" /organism="Artificial Sequence"
<400> 126
   tcggactgat gaaggggt 18
<210> 127
   <211> 18
   <212> DNA
   <213> Artificial Sequence
<220>
   <221> source
   <222> 1..18
   <223> /mol_type="unassigned DNA" /note="primer mxn206" /organism="Artificial Sequence"
<400> 127
   gaaggcctcc acgttctc 18
<210> 128
   <211> 18
   <212> DNA
   <213> Artificial Sequence
<220>
   <221> source
   <222> 1..18
   <223> /mol_type="unassigned DNA" /note="primer p15A-Tet1" /organism="Artificial Sequence"
<400> 128
   ggagaactgt gaatgcgc 18
<210> 129
   <211> 18
   <212> DNA
   <213> Artificial Sequence
<220>
   <221> source
   <222> 1..18
   <223> /mol_type="unassigned DNA" /note="primer p15A-Tet2" /organism="Artificial Sequence"
<400> 129
   actccgctag cgctgatg 18
<210> 130
   <211> 18
   <212> DNA
   <213> Artificial Sequence
<220>
   <221> source
   <222> 1..18
   <223> /mol_type="unassigned DNA" /note="primer p15A-Tet-3" /organism="Artificial Sequence"
<400> 130
   gagaactgtg aatgcgca 18
<210> 131
   <211> 20
   <212> DNA
   <213> Artificial Sequence
<220>
   <221> source
   <222> 1..20
   <223> /mol_type="unassigned DNA" /note="primer pTOPO-in" /organism="Artificial Sequence"
<400> 131
   cctctagatg catgctcgag 20
<210> 132
   <211> 20
   <212> DNA
   <213> Artificial Sequence
<220>
   <221> source
   <222> 1..20
   <223> /mol_type="unassigned DNA" /note="primer pTOPO-out" /organism="Artificial Sequence"
<400> 132
   ttggtaccga gctcggatcc 20
<210> 133
   <211> 18
   <212> DNA
   <213> Artificial Sequence
<220>
   <221> source
   <222> 1..18
   <223> /mol_type="unassigned DNA" /note="primer pSup_B" /organism="Artificial Sequence"
<400> 133
   gctcatgagc ccgaagtg 18
<210> 134
   <211> 18
   <212> DNA
   <213> Artificial Sequence
<220>
   <221> source
   <222> 1..18
   <223> /mol_type="unassigned DNA" /note="primer pSup_E" /organism="Artificial Sequence"
<400> 134
   cgctcatcgt catcctcg 18
<210> 135
   <211> 18
   <212> DNA
   <213> Artificial Sequence
<220>
   <221> source
   <222> 1..18
   <223> /mol_type="unassigned DNA" /note="primer pSWU41-F" /organism="Artificial Sequence"
<400> 135
   gtgcaaaaaa gcggttag 18
<210> 136
   <211> 16
   <212> PRT
   <213> Artificial Sequence
<220>
   <223> section of catalytic centre
<400> 136
   Ser Glu Ala Val Asp Ala Ala Cys Ala Ser Ser Leu Val Ala Leu His
   1               5                   10                  15
<210> 137
   <211> 8
   <212> PRT
   <213> Artificial Sequence
<220>
   <223> section of catalytic centre
<400> 137
   Val Glu Ala His Gly Thr Gly Thr
   1               5
<210> 138
   <211> 5
   <212> PRT
   <213> Artificial Sequence
<220>
   <223> section of catalytic centre
<400> 138
   Ile Gly His Ala Glu
   1 5
<210> 139
   <211> 16
   <212> PRT
   <213> Artificial Sequence
<220>
   <223> section of catalytic centre
<400> 139
   Ser Glu Ala Val Asp Thr Ala Cys Ser Ser Ser Leu Val Ala Ile His
   1 5 10 15
<210> 140
   <211> 8
   <212> PRT
   <213> Artificial Sequence
<220>
   <223> section of catalytic centre
<400> 140
   Ile Glu Thr His Gly Thr Gly Thr
   1 5
<210> 141
   <211> 5
   <212> PRT
   <213> Artificial Sequence
<220>
   <223> section of catalytic centre
<400> 141
   Ile Gly His Leu Glu
   1 5
<210> 142
   <211> 16
   <212> PRT
   <213> Artificial Sequence
<220>
   <223> section of catalytic centre
<400> 142
   Ser Glu Pro Val Asp Thr Leu Cys Ser Ser Ser Leu Val Ala Ile His
   1               5                   10                  15
<210> 143
   <211> 8
   <212> PRT
   <213> Artificial Sequence
<220>
   <223> section of catalytic centre
<400> 143
   Ile Glu Ala His Gly Thr Gly Thr
   1               5
<210> 144
   <211> 5
   <212> PRT
   <213> Artificial Sequence
<220>
   <223> section of catalytic centre
<400> 144
   Val Gly His Leu Glu
   1               5
<210> 145
   <211> 16
   <212> PRT
   <213> Artificial Sequence
<220>
   <223> section of catalytic centre
<400> 145
   Ser Glu Ala Ile Asp Thr Ala Cys Ser Ser Ser Leu Val Ala Ile His
   1               5                   10                  15
<210> 146
   <211> 8
   <212> PRT
   <213> Artificial Sequence
<220>
   <223> section of catalytic centre
<400> 146
   Ile Glu Ala His Gly Thr Gly Thr
   1               5
<210> 147
   <211> 5
   <212> PRT
   <213> Artificial Sequence
<220>
   <223> section of catalytic centre
<400> 147
   Ile Gly His Leu Glu
   1 5
<210> 148
   <211> 16
   <212> PRT
   <213> Artificial Sequence
<220>
   <223> section of catalytic centre
<400> 148
   Ser Phe Ala Val Asp Ser Ala Cys Ser Ala Ser Leu Thr Ala Ile His
   1 5 10 15
<210> 149
   <211> 8
   <212> PRT
   <213> Artificial Sequence
<220>
   <223> section of catalytic centre
<400> 149
   Val Glu Cys Ala Ala Ala Gly Ser
   1 5
<210> 150
   <211> 5
   <212> PRT
   <213> Artificial Sequence
<220>
   <223> section of catalytic centre
<400> 150
   Val Gly His Leu Glu
   1 5
<210> 151
   <211> 16
   <212> PRT
   <213> Artificial Sequence
<220>
   <223> section of catalytic centre
<400> 151
   Ser Glu Pro Val Asp Thr Ala Cys Ser Ser Ser Leu Ile Ala Val Gln
   1               5                   10                  15
<210> 152
   <211> 8
   <212> PRT
   <213> Artificial Sequence
<220>
   <223> section of catalytic centre
<400> 152
   Ile Glu Ala His Gly Thr Gly Thr
   1               5
<210> 153
   <211> 5
   <212> PRT
   <213> Artificial Sequence
<220>
   <223> section of catalytic centre
<400> 153
   Ile Gly His Leu Glu
   1               5
<210> 154
   <211> 16
   <212> PRT
   <213> Artificial Sequence
<220>
   <223> section of catalytic centre
<400> 154
   Ser Phe Thr Ile Asp Ala Ala Cys Ala Ser Ser Leu Val Ala Leu His
   1               5                   10                  15
<210> 155
   <211> 8
   <212> PRT
   <213> Artificial Sequence
<220>
   <223> section of catalytic centre
<400> 155
   Val Glu Ala His Gly Thr Gly Thr
   1               5
<210> 156
   <211> 5
   <212> PRT
   <213> Artificial Sequence
<220>
   <223> section of catalytic centre
<400> 156
   Ile Gly His Leu Glu
   1               5
<210> 157
   <211> 16
   <212> PRT
   <213> Artificial Sequence
<220>
   <223> section of catalytic centre
<400> 157
   Ser Leu Ala Val Asp Thr Ala Cys Ser Ser Ser Leu Ala Ala Ile His
   1               5                   10                  15
<210> 158
   <211> 8
   <212> PRT
   <213> Artificial Sequence
<220>
   <223> section of catalytic centre
<400> 158
   Val Glu Cys Gln Ala Thr Gly Thr
   1               5
<210> 159
   <211> 5
   <212> PRT
   <213> Artificial Sequence
<220>
   <223> section of catalytic centre
<400> 159
   Ile Gly His Leu Glu
   1               5
<210> 160
   <211> 16
   <212> PRT
   <213> Artificial Sequence
<220>
   <223> section of catalytic centre
<400> 160
   Ser Glu Pro Cys Asp Thr Ala Cys Ser Ser Ser Leu Ile Ala Ile His
   1               5                   10                  15
<210> 161
   <211> 8
   <212> PRT
   <213> Artificial Sequence
<220>
   <223> section of catalytic centre
<400> 161
   Ile Glu Ala His Gly Ser Gly Thr
   1               5
<210> 162
   <211> 5
   <212> PRT
   <213> Artificial Sequence
<220>
   <223> section of catalytic centre
<400> 162
   Ile Gly His Leu Glu
   1               5
<210> 163
   <211> 16
   <212> PRT
   <213> Artificial Sequence
<220>
   <223> section of catalytic centre
<400> 163
   Ala Met Gln Val Asp Thr Ala Cys Ser Ser Ser Leu Val Ala Val His
   1               5                   10                  15
<210> 164
   <211> 8
   <212> PRT
   <213> Artificial Sequence
<220>
   <223> section of catalytic centre
<400> 164
   Val Glu Ala His Gly Thr Gly Thr
   1               5
<210> 165
   <211> 5
   <212> PRT
   <213> Artificial Sequence
<220>
   <223> section of catalytic centre
<400> 165
   Ile Gly His Thr Ser
   1 5
<210> 166
   <211> 16
   <212> PRT
   <213> Artificial Sequence
<220>
   <223> section of catalytic centre
<400> 166
   Ser Leu Met Val Asp Thr Ala Cys Ser Ser Ser Leu Thr Ala Leu His
   1 5 10 15
<210> 167
   <211> 8
   <212> PRT
   <213> Artificial Sequence
<220>
   <223> section of catalytic centre
<400> 167
   Val Glu Ala His Gly Thr Gly Thr
   1 5
<210> 168
   <211> 5
   <212> PRT
   <213> Artificial Sequence
<220>
   <223> section of catalytic centre
<400> 168
   Ile Gly His Leu Glu
   1 5
<210> 169
   <211> 16
   <212> PRT
   <213> Artificial Sequence
<220>
   <223> section of catalytic centre
<400> 169
   Ser Glu Ala Val Asp Ala Ala Cys Ala Ser Ser Leu Val Ala Leu His
   1 5 10 15
<210> 170
   <211> 8
   <212> PRT
   <213> Artificial Sequence
<220>
   <223> section of catalytic centre
<400> 170
   Val Glu Ala His Gly Thr Gly Thr
   1 5
<210> 171
   <211> 5
   <212> PRT
   <213> Artificial Sequence
<220>
   <223> section of catalytic centre
<400> 171
   Ile Gly His Ala Glu
   1 5
<210> 172
   <211> 16
   <212> PRT
   <213> Artificial Sequence
<220>
   <223> section of catalytic centre
<400> 172
   Ser Glu Ala Val Asp Thr Ala Cys Ser Ser Ser Leu Val Ala Ile His
   1 5 10 15
<210> 173
   <211> 8
   <212> PRT
   <213> Artificial Sequence
<220>
   <223> section of catalytic centre
<400> 173
   Ile Glu Thr His Gly Thr Gly Thr
   1 5
<210> 174
   <211> 5
   <212> PRT
   <213> Artificial Sequence
<220>
   <223> section of catalytic centre
<400> 174
   Ile Gly His Leu Glu
   1               5
<210> 175
   <211> 16
   <212> PRT
   <213> Artificial Sequence
<220>
   <223> section of catalytic centre
<400> 175
   Ser Glu Pro Val Asp Thr Leu Cys Ser Ser Ser Leu Val Ala Ile His
   1               5                   10                  15
<210> 176
   <211> 8
   <212> PRT
   <213> Artificial Sequence
<220>
   <223> section of catalytic centre
<400> 176
   Ile Glu Ala His Gly Thr Gly Thr
   1               5
<210> 177
   <211> 5
   <212> PRT
   <213> Artificial Sequence
<220>
   <223> section of catalytic centre
<400> 177
   Val Gly His Leu Glu
   1               5
<210> 178
   <211> 16
   <212> PRT
   <213> Artificial Sequence
<220>
   <223> section of catalytic centre
<400> 178
   Ser Glu Ala Ile Asp Thr Ala Cys Ser Ser Ser Leu Val Ala Ile His
   1               5                   10                  15
<210> 179
   <211> 8
   <212> PRT
   <213> Artificial Sequence
<220>
   <223> section of catalytic centre
<400> 179
   Val Glu Ala His Gly Thr Gly Thr
   1               5
<210> 180
   <211> 5
   <212> PRT
   <213> Artificial Sequence
<220>
   <223> section of catalytic centre
<400> 180
   Ile Gly His Leu Glu
   1               5
<210> 181
   <211> 16
   <212> PRT
   <213> Artificial Sequence
<220>
   <223> section of catalytic centre
<400> 181
   Ser Phe Ala Val Asp Ser Ala Cys Ser Ala Ser Leu Thr Ala Ile His
   1               5                   10                  15
<210> 182
   <211> 8
   <212> PRT
   <213> Artificial Sequence
<220>
   <223> section of catalytic centre
<400> 182
   Val Glu Cys Ala Ala Ala Gly Ser
   1               5
<210> 183
   <211> 5
   <212> PRT
   <213> Artificial Sequence
<220>
   <223> section of catalytic centre
<400> 183
   Val Gly His Leu Glu
   1 5
<210> 184
   <211> 16
   <212> PRT
   <213> Artificial Sequence
<220>
   <223> section of catalytic centre
<400> 184
   Ser Glu Pro Val Asp Thr Ala Cys Ser Ser Ser Leu Val Ala Val Arg
   1 5 10 15
<210> 185
   <211> 8
   <212> PRT
   <213> Artificial Sequence
<220>
   <223> section of catalytic centre
<400> 185
   Ile Glu Thr His Gly Thr Gly Thr
   1 5
<210> 186
   <211> 5
   <212> PRT
   <213> Artificial Sequence
<220>
   <223> section of catalytic centre
<400> 186
   Ile Gly His Leu Glu
   1 5
<210> 187
   <211> 16
   <212> PRT
   <213> Artificial Sequence
<220>
   <223> section of catalytic centre
<400> 187
   Ser Phe Thr Ile Asp Ala Ala Cys Ala Ser Ser Leu Val Ala Leu His
   1 5 10 15
<210> 188
   <211> 8
   <212> PRT
   <213> Artificial Sequence
<220>
   <223> section of catalytic centre
<400> 188
   Val Glu Ala His Gly Thr Gly Thr
   1 5
<210> 189
   <211> 5
   <212> PRT
   <213> Artificial Sequence
<220>
   <223> section of catalytic centre
<400> 189
   Ile Gly His Leu Glu
   1 5
<210> 190
   <211> 16
   <212> PRT
   <213> Artificial Sequence
<220>
   <223> section of catalytic centre
<400> 190
   Ser Leu Ala Val Asp Thr Ala Cys Ser Ser Ser Leu Val Ser Ile His
   1 5 10 15
<210> 191
   <211> 8
   <212> PRT
   <213> Artificial Sequence
<220>
   <223> section of catalytic centre
<400> 191
   Val Glu Cys His Gly Thr Gly Thr
   1 5
<210> 192
   <211> 5
   <212> PRT
   <213> Artificial Sequence
<220>
   <223> section of catalytic centre
<400> 192
   Ile Gly His Leu Glu
   1 5
<210> 193
   <211> 16
   <212> PRT
   <213> Artificial Sequence
<220>
   <223> section of catalytic centre
<400> 193
   Ser Glu Pro Val Asp Thr Ala Cys Ser Ser Ser Leu Val Ala Val Gln
   1 5 10 15
<210> 194
   <211> 8
   <212> PRT
   <213> Artificial Sequence
<220>
   <223> section of catalytic centre
<400> 194
   Ile Glu Ala His Gly Thr Gly Thr
   1 5
<210> 195
   <211> 5
   <212> PRT
   <213> Artificial Sequence
<220>
   <223> section of catalytic centre
<400> 195
   Ala Gly His Leu Glu
   1 5
<210> 196
   <211> 16
   <212> PRT
   <213> Artificial Sequence
<220>
   <223> section of catalytic centre
<400> 196
   Ala Leu Thr Leu Asp Thr Ala Cys Ser Ser Ser Leu Thr Ala Leu Ser
   1 5 10 15
<210> 197
   <211> 8
   <212> PRT
   <213> Artificial Sequence
<220>
   <223> section of catalytic centre
<400> 197
   Val Glu Ala His Gly Thr Gly Thr
   1 5
<210> 198
   <211> 5
   <212> PRT
   <213> Artificial Sequence
<220>
   <223> section of catalytic centre
<400> 198
   Ile Gly His Thr Thr
   1 5
<210> 199
   <211> 16
   <212> PRT
   <213> Artificial Sequence
<220>
   <223> section of catalytic centre
<400> 199
   Ser Glu Pro Cys Asn Thr Ala Cys Ser Ser Ser Leu Ile Ala Ile His
   1 5 10 15
<210> 200
   <211> 8
   <212> PRT
   <213> Artificial Sequence
<220>
   <223> section of catalytic centre
<400> 200
   Ile Glu Ala His Gly Ser Gly Thr
   1 5
<210> 201
   <211> 5
   <212> PRT
   <213> Artificial Sequence
<220>
   <223> section of catalytic centre
<400> 201
   Thr Gly His Leu Glu
   1 5
<210> 202
   <211> 16
   <212> PRT
   <213> Artificial Sequence
<220>
   <223> section of catalytic centre
<400> 202
   Ala Met Gln Leu Asp Thr Ala Cys Ser Ser Ser Leu Val Ala Met His
   1 5 10 15
<210> 203
   <211> 8
   <212> PRT
   <213> Artificial Sequence
<220>
   <223> section of catalytic centre
<400> 203
   Val Glu Ala His Gly Thr Gly Thr
   1 5
<210> 204
   <211> 5
   <212> PRT
   <213> Artificial Sequence
<220>
   <223> section of catalytic centre
<400> 204
   Ile Gly His Thr Ser
   1 5
<210> 205
   <211> 16
   <212> PRT
   <213> Artificial Sequence
<220>
   <223> section of catalytic centre
<400> 205
   Ser Leu Thr Val Asp Thr Ala Cys Ser Ser Ser Leu Thr Ala Leu His
   1               5                   10                  15
<210> 206
   <211> 8
   <212> PRT
   <213> Artificial Sequence
<220>
   <223> section of catalytic centre
<400> 206
   Val Glu Ala His Gly Thr Gly Thr
   1               5
<210> 207
   <211> 5
   <212> PRT
   <213> Artificial Sequence
<220>
   <223> section of catalytic centre
<400> 207
   Ile Gly His Leu Glu
   1               5
<210> 208
   <211> 8
   <212> PRT
   <213> Artificial Sequence
<220>
   <223> section of catalytic centre
<400> 208
   Thr Gly Gly Ala Gly Ala Leu Gly
   1               5
<210> 209
   <211> 9
   <212> PRT
   <213> Artificial Sequence
<220>
   <223> section of catalytic centre
<400> 209
   His Ala Ala Gly Gln Arg Gln Asp Gln
   1               5
<210> 210
   <211> 21
   <212> PRT
   <213> Artificial Sequence
<220>
   <223> section of catalytic centre
<400> 210
   Phe Ser Ser Leu Val Ala Leu Thr Gly Asn Val Gly Gln Thr Asp Tyr
   1               5                   10                  15
   Ala Phe Ala Asn Ala
               20
<210> 211
   <211> 8
   <212> PRT
   <213> Artificial Sequence
<220>
   <223> section of catalytic centre
<400> 211
   Val Gly Gly Thr Gly Gly Ile Gly
   1               5
<210> 212
   <211> 9
   <212> PRT
   <213> Artificial Sequence
<220>
   <223> section of catalytic centre
<400> 212
   His Ser Ala Leu Val Leu Gln Asp Ala
   1               5
<210> 213
   <211> 21
   <212> PRT
   <213> Artificial Sequence
<220>
   <223> section of catalytic centre
<400> 213
   Phe Ser Ser Ala Val Gly Ile Ala Gly Gly Ala Gly Gln Ser Asn Tyr
   1               5                   10                  15
   Ala Ala Ala Ser Arg
               20
<210> 214
   <211> 8
   <212> PRT
   <213> Artificial Sequence
<220>
   <223> section of catalytic centre
<400> 214
   Val Gly Gly Gly Gly Gly Leu Gly
   1               5
<210> 215
   <211> 9
   <212> PRT
   <213> Artificial Sequence
<220>
   <223> section of catalytic centre
<400> 215
   His Leu Gly Gly Val Leu Ser Asp Lys
   1               5
<210> 216
   <211> 21
   <212> PRT
   <213> Artificial Sequence
<220>
   <223> section of catalytic centre
<400> 216
   Phe Ser Ser Ile Val Ser Leu Leu Gly Asn Val Gly Gln Ala Asp Tyr
   1               5                   10                  15
   Ala Ala Gly Asn Ser
               20
<210> 217
   <211> 8
   <212> PRT
   <213> Artificial Sequence
<220>
   <223> section of catalytic centre
<400> 217
   Val Gly Gly Leu Gly Gly Leu Gly
   1               5
<210> 218
   <211> 9
   <212> PRT
   <213> Artificial Sequence
<220>
   <223> section of catalytic centre
<400> 218
   His Ser Ala Ile Val Leu Lys Asp Ala
   1 5
<210> 219
   <211> 21
   <212> PRT
   <213> Artificial Sequence
<220>
   <223> section of catalytic centre
<400> 219
   Phe Ser Ser Ala Glu Ser Phe Ile Cys Glu Ala Gly Gln Ser Asn Tyr
   1 5 10 15
   Ala Ala Ala Ser Arg
   20
<210> 220
   <211> 8
   <212> PRT
   <213> Artificial Sequence
<220>
   <223> section of catalytic centre
<400> 220
   Thr Gly Gly Ala Gly Ala Leu Gly
   1 5
<210> 221
   <211> 9
   <212> PRT
   <213> Artificial Sequence
<220>
   <223> section of catalytic centre
<400> 221
   His Ala Ala Gly Glu Arg Arg Asp Ala
   1 5
<210> 222
   <211> 21
   <212> PRT
   <213> Artificial Sequence
<220>
   <223> section of catalytic centre
<400> 222
   Phe Ser Ser Leu Val Ala Arg Thr Gly Asn Val Gly Gln Thr Asp Tyr
   1 5 10 15
   Ala Phe Ala Asn Ala
   20
<210> 223
   <211> 8
   <212> PRT
   <213> Artificial Sequence
<220>
   <223> section of catalytic centre
<400> 223
   Val Gly Gly Leu Gly Gly Ile Gly
   1               5
<210> 224
   <211> 9
   <212> PRT
   <213> Artificial Sequence
<220>
   <223> section of catalytic centre
<400> 224
   His Ser Ala Leu Val Leu Gln Asp Ala
   1               5
<210> 225
   <211> 21
   <212> PRT
   <213> Artificial Sequence
<220>
   <223> section of catalytic centre
<400> 225
   Phe Ser Ser Ala Val Gly Ile Ser Gly Gly Ala Gly Gln Ser His Tyr
   1               5                   10                  15
   Ala Ala Ala Ser Arg
               20
<210> 226
   <211> 8
   <212> PRT
   <213> Artificial Sequence
<220>
   <223> section of catalytic centre
<400> 226
   Val Gly Gly Gly Gly Gly Ile Gly
   1               5
<210> 227
   <211> 9
   <212> PRT
   <213> Artificial Sequence
<220>
   <223> section of catalytic centre
<400> 227
   His Leu Gly Gly Val Leu Ser Asp Arg
   1               5
<210> 228
   <211> 21
   <212> PRT
   <213> Artificial Sequence
<220>
   <223> section of catalytic centre
<400> 228
   Phe Ser Ser Ile Val Ala Leu Thr Gly Asn Val Gly Gln Ala Asp Tyr
   1               5                   10                  15
   Ala Ala Gly Asn Ser
               20
<210> 229
   <211> 8
   <212> PRT
   <213> Artificial Sequence
<220>
   <223> section of catalytic centre
<400> 229
   Thr Gly Gly Ala Gly Gly Leu Gly
   1               5
<210> 230
   <211> 9
   <212> PRT
   <213> Artificial Sequence
<220>
   <223> section of catalytic centre
<400> 230
   His Gly Ala Gly Val Arg Arg Asp Ala
   1               5
<210> 231
   <211> 21
   <212> PRT
   <213> Artificial Sequence
<220>
   <223> section of catalytic centre
<400> 231
   Phe Ser Ser Val Ser Ala Val Leu Gly Asn Thr Gly Gln Gly Asp Tyr
   1               5                   10                  15
   Ala Tyr Ala Asn Ala
               20
<210> 232
   <211> 8
   <212> PRT
   <213> Artificial Sequence
<220>
   <223> section of catalytic centre
<400> 232
   Thr Gly Gly Val Gly Ala Leu Gly
   1               5
<210> 233
   <211> 5
   <212> PRT
   <213> Artificial Sequence
<220>
   <223> section of catalytic centre
<400> 233
   His Ala Ala Gly Asn
   1               5
<210> 234
   <211> 21
   <212> PRT
   <213> Artificial Sequence
<220>
   <223> section of catalytic centre
<400> 234
   Cys Ser Ser Val Ser Ser Val Leu Gly Asn Ala Gly Gln Gly Asp Tyr
   1               5                   10                  15
   Ala Tyr Ala Asn Ala
               20
<210> 235
   <211> 8
   <212> PRT
   <213> Artificial Sequence
<220>
   <223> section of catalytic centre
<400> 235
   Val Gly Gly Leu Gly Gly Ile Gly
   1               5
<210> 236
   <211> 9
   <212> PRT
   <213> Artificial Sequence
<220>
   <223> section of catalytic centre
<400> 236
   His Ser Ala Ile Val Leu Lys Asp Ala
   1               5
<210> 237
   <211> 21
   <212> PRT
   <213> Artificial Sequence
<220>
   <223> section of catalytic centre
<400> 237
   Phe Ser Ser Ala Glu Ser Phe Ser Cys Asp Ala Gly Gln Ser Asn Tyr
   1               5                   10                  15
   Ala Ala Ala Ser Arg
               20
<210> 238
   <211> 10
   <212> PRT
   <213> Artificial Sequence
<220>
   <223> section of catalytic centre
<400> 238
   Gly Gly Ala His Phe Asp Ser Ile Ser Leu
   1               5                   10
<210> 239
   <211> 6
   <212> PRT
   <213> Artificial Sequence
<220>
   <223> section of catalytic centre
<400> 239
   Phe Thr Ser Val Thr Leu
   1               5
<210> 240
   <211> 6
   <212> PRT
   <213> Artificial Sequence
<220>
   <223> section of catalytic centre
<400> 240
   Tyr Asp Ser Ile Ser Phe
   1               5
<210> 241
   <211> 6
   <212> PRT
   <213> Artificial Sequence
<220>
   <223> section of catalytic centre
<400> 241
   Leu Thr Ser Leu Ser Leu
   1               5
<210> 242
   <211> 6
   <212> PRT
   <213> Artificial Sequence
<220>
   <223> section of catalytic centre
<400> 242
   Val Asp Ser Leu Ile Gly
   1               5
<210> 243
   <211> 6
   <212> PRT
   <213> Artificial Sequence
<220>
   <223> section of catalytic centre
<400> 243
   Leu Asp Ser Ile Arg Val
   1               5
<210> 244
   <211> 6
   <212> PRT
   <213> Artificial Sequence
<220>
   <223> section of catalytic centre
<400> 244
   Leu Asp Ser Ile Met Thr
   1 5
<210> 245
   <211> 6
   <212> PRT
   <213> Artificial Sequence
<220>
   <223> section of catalytic centre
<400> 245
   Phe Asp Gln Leu Ala Leu
   1 5
<210> 246
   <211> 6
   <212> PRT
   <213> Artificial Sequence
<220>
   <223> section of catalytic centre
<400> 246
   Leu Asp Ser Leu Ile Leu
   1 5
<210> 247
   <211> 6
   <212> PRT
   <213> Artificial Sequence
<220>
   <223> section of catalytic centre
<400> 247
   Phe Asp Ser Ile Thr Phe
   1 5
<210> 248
   <211> 6
   <212> PRT
   <213> Artificial Sequence
<220>
   <223> section of catalytic centre
<400> 248
   Leu Asp Ser Ile Leu Ala
   1 5
<210> 249
   <211> 6
   <212> PRT
   <213> Artificial Sequence
<220>
   <223> section of catalytic centre
<400> 249
   Val Asp Ser Leu Val Asn
   1               5
<210> 250
   <211> 6
   <212> PRT
   <213> Artificial Sequence
<220>
   <223> section of catalytic centre
<400> 250
   Val Asp His Leu Asp Met
   1               5
<210> 251
   <211> 6
   <212> PRT
   <213> Artificial Sequence
<220>
   <223> section of catalytic centre
<400> 251
   Leu Asp Ser Ile Asn Val
   1               5
<210> 252
   <211> 10
   <212> PRT
   <213> Artificial Sequence
<220>
   <223> section of catalytic centre
<400> 252
   Ser Gly Ala His Phe Asp Ser Ile Ser Leu
   1               5                   10
<210> 253
   <211> 6
   <212> PRT
   <213> Artificial Sequence
<220>
   <223> section of catalytic centre
<400> 253
   Phe Asn Ser Val Thr Leu
   1 5
<210> 254
   <211> 6
   <212> PRT
   <213> Artificial Sequence
<220>
   <223> section of catalytic centre
<400> 254
   Tyr Asp Ser Ile Ser Phe
   1 5
<210> 255
   <211> 6
   <212> PRT
   <213> Artificial Sequence
<220>
   <223> section of catalytic centre
<400> 255
   Leu Ser Ser Val Ala Leu
   1 5
<210> 256
   <211> 6
   <212> PRT
   <213> Artificial Sequence
<220>
   <223> section of catalytic centre
<400> 256
   Val Asp Ser Leu Leu Gly
   1 5
<210> 257
   <211> 6
   <212> PRT
   <213> Artificial Sequence
<220>
   <223> section of catalytic centre
<400> 257
   Leu Asp Ser Met Arg Val
   1 5
<210> 258
   <211> 6
   <212> PRT
   <213> Artificial Sequence
<220>
   <223> section of catalytic centre
<400> 258
   Phe Gly Pro His Ala Leu
   1 5
<210> 259
   <211> 6
   <212> PRT
   <213> Artificial Sequence
<220>
   <223> section of catalytic centre
<400> 259
   Leu Asp Ser Ile Met Thr
   1 5
<210> 260
   <211> 6
   <212> PRT
   <213> Artificial Sequence
<220>
   <223> section of catalytic centre
<400> 260
   Leu Asp Ser Leu Ile Leu
   1 5
<210> 261
   <211> 6
   <212> PRT
   <213> Artificial Sequence
<220>
   <223> section of catalytic centre
<400> 261
   Phe Asp Ser Ile Thr Phe
   1 5
<210> 262
   <211> 6
   <212> PRT
   <213> Artificial Sequence
<220>
   <223> section of catalytic centre
<400> 262
   Leu Asp Pro Val Leu Ala
   1 5
<210> 263
   <211> 6
   <212> PRT
   <213> Artificial Sequence
<220>
   <223> section of catalytic centre
<400> 263
   Leu Asp Ser Val Leu Gly
   1 5
<210> 264
   <211> 6
   <212> PRT
   <213> Artificial Sequence
<220>
   <223> section of catalytic centre
<400> 264
   Phe Asp Ser Ile Ile Leu
   1 5
<210> 265
   <211> 6
   <212> PRT
   <213> Artificial Sequence
<220>
   <223> section of catalytic centre
<400> 265
   Leu Asp Ser Ile Leu Ala
   1 5
<210> 266
   <211> 6
   <212> PRT
   <213> Artificial Sequence
<220>
   <223> section of catalytic centre
<400> 266
   Val Asp Ser Leu Val Asn
   1 5
<210> 267
   <211> 6
   <212> PRT
   <213> Artificial Sequence
<220>
   <223> section of catalytic centre
<400> 267
   Val Asp His Phe Asp Met
   1 5
<210> 268
   <211> 6
   <212> PRT
   <213> Artificial Sequence
<220>
   <223> section of catalytic centre
<400> 268
   Leu Asp Ser Ile Asn Val
   1 5
<210> 269
   <211> 13
   <212> PRT
   <213> Artificial Sequence
<220>
   <223> section of catalytic centre
<400> 269
   Leu Arg Asp His Val Ile Gly Gly Lys Lys Leu Leu Pro
   1 5 10
<210> 270
   <211> 13
   <212> PRT
   <213> Artificial Sequence
<220>
   <223> section of catalytic centre
<400> 270
   Leu Arg Asp His Thr Val Phe Gly Gln Arg Val Leu Leu
   1 5 10
<210> 271
   <211> 13
   <212> PRT
   <213> Artificial Sequence
<220>
   <223> section of catalytic centre
<400> 271
   Leu Arg Asp His Val Val Gly Ser Arg Gly Val Leu Pro
   1 5 10
<210> 272
   <211> 13
   <212> PRT
   <213> Artificial Sequence
<220>
   <223> section of catalytic centre
<400> 272
   Ile Arg Asp His Val Val Gly Gly Asn Arg Leu Ile Pro
   1 5 10
<210> 273
   <211> 13
   <212> PRT
   <213> Artificial Sequence
<220>
   <223> section of catalytic centre
<400> 273
   Val Ser Gln His Gln Val His Gly Arg Pro Val Phe Pro
   1 5 10
<210> 274
   <211> 13
   <212> PRT
   <213> Artificial Sequence
<220>
   <223> section of catalytic centre
<400> 274
   Leu Arg Asp His Glu Ile Gly Gly Lys Arg Ile Leu Pro
   1 5 10
<210> 275
   <211> 13
   <212> PRT
   <213> Artificial Sequence
<220>
   <223> section of catalytic centre
<400> 275
   Leu Arg Asp His Thr Val Leu Gly Gln Arg Val Leu Leu
   1 5 10
<210> 276
   <211> 13
   <212> PRT
   <213> Artificial Sequence
<220>
   <223> section of catalytic centre
<400> 276
   Leu Arg Asp His Val Val Gly Ser His Gly Val Leu Pro
   1               5                   10
<210> 277
   <211> 13
   <212> PRT
   <213> Artificial Sequence
<220>
   <223> section of catalytic centre
<400> 277
   Leu Arg Asp His Val Val Gly Gly His Arg Leu Ile Pro
   1               5                   10
<210> 278
   <211> 13
   <212> PRT
   <213> Artificial Sequence
<220>
   <223> section of catalytic centre
<400> 278
   Leu Ala Asp His Val Leu His Gly Glu Pro Thr Leu Pro
   1               5                   10
<210> 279
   <211> 13
   <212> PRT
   <213> Artificial Sequence
<220>
   <223> section of catalytic centre
<400> 279
   Leu Asn Ala His Glu Val Phe Gly Arg Pro Leu Phe Pro
   1               5                   10
<210> 280
   <211> 13
   <212> PRT
   <213> Artificial Sequence
<220>
   <223> section of catalytic centre
<400> 280
   Val Thr Gln His Gln Val His Gly Gln Pro Val Met Pro
   1 5 10
<210> 281
   <211> 6
   <212> PRT
   <213> Artificial Sequence
<220>
   <223> section of catalytic centre
<400> 281
   Val Glu Val Gly Ala Gly
   1 5
<210> 282
   <211> 6
   <212> PRT
   <213> Artificial Sequence
<220>
   <223> section of catalytic centre
<400> 282
   Leu Glu Val Gly Ala Gly
   1 5
<210> 283
   <211> 6
   <212> PRT
   <213> Artificial Sequence
<220>
   <223> section of catalytic centre
<400> 283
   Val Glu Val Gly Ala Gly
   1 5
<210> 284
   <211> 6
   <212> PRT
   <213> Artificial Sequence
<220>
   <223> section of catalytic centre
<400> 284
   Leu Glu Val Gly Ala Gly
   1 5
<210> 285
   <211> 41
   <212> DNA
   <213> Artificial Sequence
<220>
   <221> source
   <222> 1..41
   <223> /mol_type="unassigned DNA" /note="primer mxn125" /organism="Artificial Sequence"
<400> 285
   tgctacttaa gttatcaact agtcatggct tcgctcccgc c 41

## Description

The present invention relates to a process for the production of the myxobacterial compound myxopyronin and of derivatives of myxopyronin, e.g. for the production of a pharmaceutical composition containing myxopyronin and/or its derivative. Further, the invention relates to the derivatives obtainable by the production process, especially for use of the derivative in a medical treatment, e.g. in the medical treatment of a bacterial infection. The production process uses enzymes from the natural myxopyronin synthetic pathway for producing derivatives of myxopyronin and uses the enzymes constituting the entire myxopyronin synthetic pathway for producing myxopyronin. The production process uses the enzymes expressed in a microorganism, which can be the source organism of the genes encoding the enzymes, or uses the enzymes expressed in a heterologous host microorganism, e.g. a bacterium or fungus, including a yeast. For expression of the enzymes in a microorganism, the genes encoding the enzymes are contained in the microorganism. In addition to or in the alternative to expression of the enzymes in a microorganism, the production process can use the enzymes in a cell-free medium, e.g. following disruption and/or isolation of the enzymes from a microorganism expressing the enzymes, optionally with immobilization of the enzymes to a carrier, and/or preferably with addition of at least one precursor compound to myxopyronin or its derivative.

### State of art

Erol et al., ChemBioChem 2010, 11, 1253-1265 describe the genes of the putative corallopyronin A biosynthetic gene cluster from *Corallococcus coralloides* and feeding experiments using ¹³C- and ¹⁵N-labelled acetate, sodium bicarbonate, methyl-methionine, and glycine to deduce the natural synthetic pathway for corallopyronin A from NMR analyses. Irschik et al., J. Antibiot. 1983 describe that *Myxococcus fulvus* produces myxopyronin. Myxopyronin can be described as two half molecules, also termed Western chain and Eastern chain, which are fused in a pyrone ring moiety :

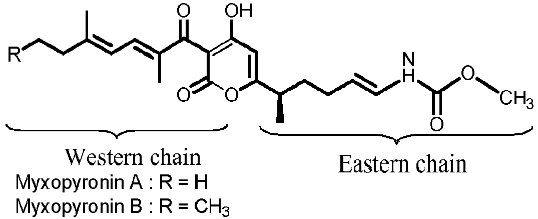

Kohl et al., Liebigs Ann. Chem. 1983 describe the isolation and structure elucidation of myxopyronin A and B from *Myxococcus fulvus* Mx f50.

Kohl et al., Liebigs Ann. Chem. 1984 describe the biosynthetic origin of myxopyronin A based on feeding experiments with ¹³C- and ¹⁵N-labeled precursors and analysis of the labelling pattern by NMR spectroscopy.

Sucipto et al., ChemBioChem 2013, describes the production of myxopyronin and the involved genes.

### Object of the invention

It is an object of the invention to provide an alternative process for the production of myxopypronin and of its derivatives, and preferably to provide new derivatives of myxopyronin and a process for their production.

### General description of the invention

The invention achieves the object by the features of the claims. Generally, the invention provides a process for producing myxopyronin and derivatives of myxopyronin by providing synthetic pathway enzymes in a microorganism or in vitro, e.g. extracellularly, for instance following expression of the enzymes in a microorganism and separating the enzymes from the microorganism. Myxopyronin and derivatives of myxopyronin produced according to the invention are compounds in which a first chain, also called Western chain, and a second chain, also called Eastern chain, are condensed forming a pyrone ring. Generally, the embodiments of the invention can be combined with one another. The synthetic pathway enzymes can be produced by expression from at least one expression cassette comprising the nucleic acid sequences encoding the enzymes in a microorganism, wherein one or more coding sequences can be contained in one expression cassette, e.g. each coding sequence can be contained in a separate expression cassette or at least two coding sequences can be contained in one expression cassette.

In detail, there is described a process for producing myxopyronin by the enzymes constituting the synthetic pathway, wherein preferably these enzymes are expressed in a microorganism which is genetically manipulated to contain genes encoding the enzymes, wherein the microorganism preferably is a heterologous host microorganism, or by providing the enzymes in a cell-free medium. These enzymes are MxnA-MxnB-MxnC-MxnD-MxnE-MxnF-MxnG-MxnH-MxnI-MxnJ-MxnK-MxnL-MxnM, which in the process preferably are encoded in 5' to 3' by genes mxnA-mxnB-mxnC-mxnD-mxnE-mxnF-mxnG-mxnH-mxnI-mxnJ-mxnK-mxnL-mxnM. Enzymes MxnA, MxnI and MxnJ, preferably additionally MxnH, catalyze the synthesis of the Eastern chain molecule portion, and enzymes MxnA and MxnK, preferably additionally MxnC-MxnD-MxnE-MxnF-MxnG, catalyze the biosynthetic pathway for synthesis of the Western chain molecule portion. Preferably, the enzyme MxnA is provided, e.g. to catalyse loading of the carrier protein module of MxnI and/or of the carrier protein module of MxnK and/or of the carrier protein module of MxnJ.

Generally, the enzymes are described herein as comprising as their portions modules or consisting of modules as their portions. The modules comprise as their portions catalytic domains containing catalytic centers. Nucleic acid sequences are given in 5'-3', amino acid sequences are given from N-terminus to C-terminus.

For the condensation reaction, the Eastern chain molecule portion and the Western chain molecule portion are both enzyme-linked, e.g. to a carrier protein domain, via a sulfur atom (S), and are condensed at their respective S-linked termini, resulting in a pyrone ring, which condensation reaction is catalyzed by the enzyme MxnB. In the alternative, the Eastern chain molecule portion and/or the Western chain molecule portion can be activated by being linked to the sulfur atom of an auxiliary group, and the enzyme MxnB can be present in a cell-free medium, e.g. as an isolated protein, to catalyze the condensation reaction to the pyrone ring.

The enzymes MxnA-MxnB-MxnC-MxnD-MxnE-MxnF-MxnG-MxnH-MxnI-MxnJ-MxnK-MxnL-MxnM which constitute the natural synthetic pathway can be expressed in a heterologous host microorganism.

In the invention, at least one enzyme thereof can be inactivated, e.g. mutated to obtain disfunctionality, or deleted, for producing myxopyronin or a derivative thereof. For example, enzyme MxnL and/or MxnM and/or MxnC-MxnG can be inactivated and the active enzymes can consist of
MxnA-MxnB-MxnC-MxnD-MxnE-MxnF-MxnG-MxnH-MxnI-MxnJ-MxnK-MxnM,
MxnA-MxnB-MxnC-MxnD-MxnE-MxnF-MxnG-MxnH-MxnI-MxnJ-MxnK-MxnL,
MxnA-MxnB- MxnH-MxnI-MxnJ-MxnK-MxnL-MxnM,
MxnA-MxnB -MxnH-MxnI-MxnJ-MxnK-MxnM,
MxnA-MxnB -MxnH-MxnI-MxnJ-MxnK-MxnL or
MxnA-MxnB-MxnC-MxnD-MxnE-MxnF-MxnG-MxnH-MxnI-MxnJ-MxnK.

Alternatively or additionally to the inactivation of enzyme MxnL and/or MxnM and/or MxnC-MxnG, at least one catalytic center of at least one of MxnK and MxnI-MxnJ can be inactivated, which catalytic center preferably is a ketosynthase (KS), ketoreductase (KR), dehydrogenase (DH), methyltransferase (MT), hydroxymethylglutaryl-CoA synthase (HMG-CoA), enoyl-CoA hydratase/isomerase (ECH).

In case of inactivation of at least one catalytic activity of the synthetic pathway enzymes, the enzymes can be expressed in an original myxopyronin producer strain which e.g. has been genetically manipulated, e.g. using homologous recombination, for inactivation of at least one enzyme or catalytic center, e.g. when providing the enzymes by homologous expression. In the first embodiment, the process comprises cultivating a microorganism expressing the enzymes and isolating the myxopyronin or its derivative from the cultivation medium and/or from the microorganism.

In a first embodiment, the invention provides a process for producing myxopyronin or a process for producing at least one derivative of myxopyronin by cultivating a microorganism expressing at least partially modified enzymes, which are modified e.g. by inactivation and/or change of activity of at least one enzyme of the enzymes MxnA-MxnB-MxnC-MxnD-MxnE-MxnF-MxnG-MxnH-MxnI-MxnJ-MxnK-MxnL-MxnM and/or of at least one domain of one of these enzymes and/or of at least one catalytic center of one of these domains. Inactivation can be by at least partial deletion and/or exchange, e.g. of at least one amino acid or by complete deletion of an enzyme, domain or catalytic center.

Insertion or exchange of at least one catalytic center, domain, module or enzyme can be by insertion or replacement of at least one additional catalytic center, domain or enzyme into the synthetic pathway enzymes comprising or consisting of MxnA-MxnB-MxnC-MxnD-MxnE-MxnF-MxnG-MxnH-MxnI-MxnJ-MxnK-MxnL-MxnM or a portion of these, which catalytic center, domain, module or enzyme is selected from MxnA-MxnB-MxnC-MxnD-MxnE-MxnF-MxnG-MxnH-MxnI-MxnJ-MxnK-MxnL-MxnM, resulting e.g. in at least one additional or exchanged homologous catalytic center, domain, module or enzyme, and/or which catalytic center, domain, module or enzyme is selected from enzymes CorJ, CorK, CorL, Orfl, CorM, CorL, CorM, CorN and CorO and their catalytic centers, domains and modules, respectively.

Generally, changes to the amino acid sequence are preferably made by changes to the nucleic acid sequence encoding the amino acid sequence of the enzyme, domain or catalytic center, respectively. The amino acid sequences of the enzymes are given in Table 1.

**Table 1: Enzymes and functions**

| origin Myxococcus (amino acids) | origin Corallococcus (amino acids) | function [Catalytic domains] |
|---|---|---|
| MxnA (798) SEQ ID NO: 1 | CorA (763) SEQ ID NO: 15 | Acyltransferase - Enoylreductase [AT-ER] |
| MxnB (335) SEQ ID NO: 2 | CorB (335) SEQ ID NO: 16 | Ketosynthase [KS] |
| MxnC (83) SEQ ID NO: 3 | CorC (83) SEQ ID NO: 17 | Acyl carrier protein [CP] |
| MxnD (423) SEQ ID NO: 4 | CorD (423) SEQ ID NO: 18 | Ketosynthase [KS] |
| MxnE (410) SEQ ID NO: 5 | CorE (410) SEQ ID NO: 19 | Hydroxymethylglutaryl-CoA (HMG-CoA) synthase |
| MxnF (253) SEQ ID NO: 6 | CorF (261) SEQ ID NO: 20 | Enoyl-CoA hydratase/isomerase [ECH] |
| MxnG (254) SEQ ID NO: 7 | CorG (254) SEQ ID NO: 21 | Enoyl-CoA hydratase/isomerase [ECH] |
| MxnH (286) SEQ ID NO: 8 | CorH (286) SEQ ID NO: 22 | Carboxyl-methyltransferase [MT] |
| MxnI (3906) SEQ ID NO: 9 | CorI (3883) SEQ ID NO: 23 | Hybrid polyketide synthase (PKS, *trans-*AT type), nonribosomal peptide synthetase (NRPS) [PM-CP-KS-A-CP-KS-DH-KR-CP-KS] |
| MxnJ (3862) SEQ ID NO: 10 | CorJ (3817) SEQ ID NO: 24 | Polyketide synthase (PKS, *trans-*AT type) [DH-CP-KS-DH-KR-MT-CP-KS-CP-KS-CP*-CP] |
| MxnK (6045) SEQ ID NO: 11 | CorK (4068) SEQ ID NO: 25 | Polyketide synthase (PKS, *trans-AT* type) [KS-DH-KR-CP-KS-CP-KS-CP-KS-DH-KR-MT-CP-KS-CP*-CP] |
| | CorL (4981) SEQ ID NO: 26 | |
| MxnL (582) SEQ ID NO: 12 | ORF1 (573) SEQ ID NO: 27 | Hypothetical protein (SorT) |
| MxnM (326) SEQ ID NO: 13 | ./. | Acyltransferase (AT) |
| ./. | CorM (240) SEQ ID NO: 28 | Thioesterase |
| ./. | CorN (724) SEQ ID NO: 29 | enoyl-CoA-hydratase |
| ./. | CorO (449) SEQ ID NO: 30 | cytochrome P450 |

Catalytic domains are: PM, phosphomutase; CP, carrier protein; KS, ketosynthase; A, adenylation; DH, dehydratase; KR, ketoreductase; AT, acyltransferase; MT, methyl transferase; ER, enoyl reductase; KS, ketosynthase. A "*" denotes inactivity of the domain. In the alternative to enzymes from Myxococcus, the indicated enzyme from Corallococcus can be used. Reference to a Myxococcus gene therefore includes reference to the Corallococcus gene indicated in the same row of table 1.

The C-terminal elongation module of MxnJ is included in the domain 6E of MxnJ.

The entire set of synthetic pathway enzymes of Myxococcus is encoded by SEQ ID NO: 14. The amino acid sequences of functional domains are given in Table 2.

**Table 2 : Modules of enzymes**

| enzyme : inactivated modules | sequence (amino acids (aa) of enzyme sequence) to be deleted or mutated for inactivation | inactivation results in |
|---|---|---|
| MxnK : 1W-2W | aa 1 to 2531, preferably aa 1 to 2467 of SEQ ID NO: 11 | MxnK consisting of modules 3W to 5W (aa 2532 to 6023, preferably aa 2468 to 6023 of SEQ ID NO: 11) |
| MxnK : 1W-3W | aa 1 to 3348, preferably aa 1 to 3272 of SEQ ID NO: 11 | MxnK consisting of modules 4W to 5W (aa 3349 to 6023, preferably aa 3273 to 6023 of SEQ ID NO: 11) |
| MxnK : 1W-4W | aa 1 to 5251, preferably aa 1 to 5203 of SEQ ID NO: 11 | MxnK consisting of modules 5W (aa 5252 to 6023, preferably aa 5204 to 6023 of SEQ ID NO: 11) |
| MxnI : LM (loading module) | aa 1 to 530, preferably aa 1 to 466 of SEQ ID NO: 9 | MxnI consisting of modules 1E to 6E (aa 533 to 3717, preferably aa 467 to 3717 of SEQ ID NO:9 and aa 25 to 3849 of SEQ ID NO : 10) |
| MxnI : 1E | aa 1 to 1781, preferably aa 1 to 1759 of SEQ ID NO: 9 | MxnI consisting of modules 2E to 6E (aa 1786 to 3717, preferably aa 1760 to 3717 of SEQ ID NO:9 and aa 25 to 3849 of SEQ ID NO : 10) |
| MxnJ : 3E | aa 1 to 405, preferably aa 1 to 450 of SEQ ID NO: 10, optionally additionally SEQ ID NO: 9 | MxnI-MxnJ consisting of modules 4E to 6E (aa 25 to 3849 of SEQ ID NO : 10) (MxnI inactivated) |
| MxnJ : 4E | aa 451 to 2276 of SEQ ID NO: 10 | MxnJ consisting of modules 5E to 6E (aa 2325 to 3849 preferably aa 2277 to 3849 of SEQ ID NO : 10) |
| MxnJ : 5E | aa 2325 to 3006 of SEQ ID NO: 10 | MxnJ consisting of modules 6E, preferably consisting of aa 3007 to 3849, more preferably aa 3053 to 3849 of SEQ ID NO: 10 |

wherein aa is amino acids, followed by the numbering of the amino acids in the indicated SEQ ID NO. The amino acid sequences of catalytic centers of domains are given in Table 3 below.

In a second embodiment, a precursor compound for the Western chain, also termed Western chain precursor, and/or a precursor compound for the Eastern chain, also termed Eastern chain precursor, is added to a microorganism expressing the synthetic pathway enzymes of the first and/or second embodiment, in which at least one catalytic domain is inactivated in the synthetic pathway enzymes MxnK and/or MxnI-MxnJ, wherein the inactivated catalytic domain preferably is a catalytic domain the inactivation of which in the absence of precursor results in a stop of synthesis by the enzyme when expressed in a microorganism. For the third embodiment, a preferred inactivated catalytic domain is selected from a carrier protein (CP) and a ketoreductase (KR). The respective amino acid sequence of catalytic domains for MxnK and MxnI-MxnJ are given in Table 3 below.

In the second embodiment, a portion of the entire set of synthetic enzymes is provided in a microorganism, which portion catalyzes the condensation of the pyrone ring, the portion e.g. comprising or consisting of enzymes MxnB, optionally of MxnA-MxnB, and a Western chain precursor is provided and at least a portion of MxnK for binding the Western chain precursor and catalyzing the conversion of the Western chain precursor to a converted Western chain precursor and MxnB, optionally MxnH and/or MxnA, and further optionally additionally MxnC-MxnD-MxnE-MxnF-MxnG. This embodiment allows the production of compounds in which the converted Western chain precursor is condensed to the natural Eastern chain molecule portion. Additionally or alternatively, an Eastern chain precursor is provided and a portion of MxnI-MxnJ is provided for binding the Eastern chain precursor and catalyzing the conversion of the Eastern chain precursor to a converted Eastern chain precursor, which is condensed to the converted Western chain precursor or to the Western molecule portion. When adding a Western chain precursor, the following enzymes are provided by genetic manipulation in a microorganism with the genes encoding the enzymes and expression of the enzymes, in combination with providing a Western chain precursor. The enzymes which are provided can e.g. comprise or consist of: MxnA-MxnB, MxnI-MxnJ, preferably MxnH, and MxnK in which at least one catalytic domain is inactivated, the inactivation resulting in the interruption of the synthetic pathway of MxnK, e.g. MxnK optionally consisting of modules 3W-5W, modules 4W-5W or module 5W of MxnK, and further optionally additionally MxnC-D-E-F-G.

In the second embodiment, in a first variant, in the wild-type enzyme MxnK (SEQ ID NO: 1) which consists of modules 1 W-5W, at least one module can be functionally absent, e.g. be deleted or inactivated, modules 1W-2W (amino acids No. 1 to 2531, preferably amino acids No. 1 to 2467 in SEQ ID NO: 11) can be absent or MxnK can consist of modules 3W to 5W, respectively. In a second variant, the moduleslW to 3W (amino acids No. 1 to 3348, preferably amino acids No. 1 to 3272 in SEQ ID NO: 11) can be functionally absent, e.g. be deleted or inactivated, or the portion of enzyme MxnK can consist of modules 4W to 5W, respectively. In a third variant, the modules 1W to 4W (amino acids No. 1 to 5251, preferably amino acids No. 1 to 5203 in SEQ ID NO: 11) can be functionally absent, e.g. be deleted or inactivated, or the portion of enzyme MxnK can consist of module 5W (amino acids No.5252 to 6023, preferably amino acids No. 5204 to 6023 in SEQ ID NO: 11), respectively. In variants, functionally absent modules can e.g. be modules in which the amino acids forming at least one of its catalytic domains are mutated or deleted.

When providing a Western chain precursor, preferably a portion of enzyme MxnK is present and the Western chain precursor can have a structure that becomes bound to a domain of MxnK, preferably to the carrier protein (CP), e.g. the Western chain precursor preferably has a structure comprising a keto-group linked to a sulfur atom of an auxiliary group. Preferably, the structure of the Western chain precursor mimics a biosynthetic intermediate. The auxiliary group can e.g. be comprise or consist of a β-keto-*N*-acetylcysteamine group (SNAC) or a thiophenol group, the sulfur atom of which is bound to the C of the keto-group.

Especially in the first variant, especially for incorporation by domain 3W, the Western chain precursor can have the following structure: R_{W}-CH₂-CH₂-CH₂-C(O)-X. Especially in the second variant, especially for incorporation by domain 4W, the Western chain precursor can have the following structure: R_{W}-CH₂-CH₂-C(CH₃)=CH₂-C(O)-X. Especially in the third variant, the Western chain precursor can have the following structure: R_{W}-CH₂-CH₂-C(CH₃)=CH-CH=C(CH₃)-C(O)-X. In each variant, R can e.g. be any alkyl, aryl or aromatic hydrocarbon, each optionally containing a heteroatom, or R can be H. X is an auxiliary group having a sulfur atom that is bound to the carbon atom. Further examples of the Western chain precursor are given below, wherein each of X₁-X₁₀ and R_{W} can independently be any heteroatom, any methine group (=CH or -CH-R with R e.g. be an alkyl, aryl or alcohol group), a methylene group, a carbonyl group.

Examples for Western chain precursor:

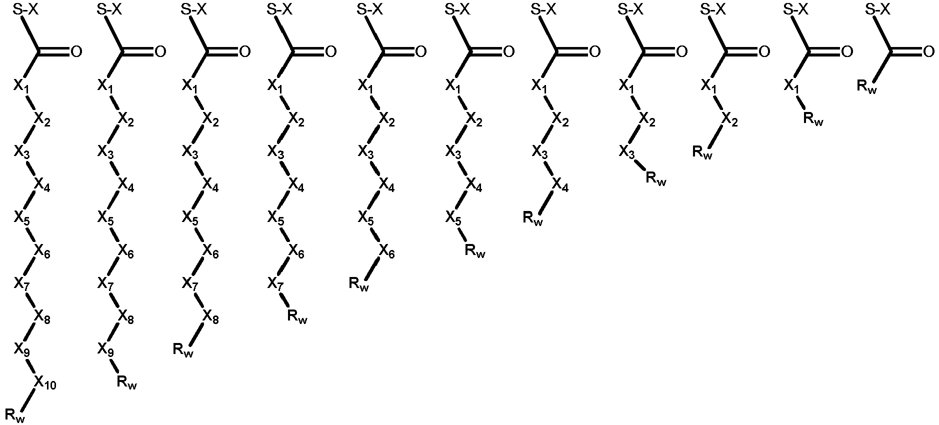

Generally, in embodiments comprising the synthesis of the natural Eastern chain molecule portion, the process can optionally be in the absence of MxnH and include the provision of a methylated precursor compound. In this embodiment, the process can comprise the provision of enzymes comprising or consisting of MxnI and MxnJ, preferably additionally MxnA, for synthesis of the Eastern chain molecule portion and the provision of at least a part of the enzymes for synthesis of the Western chain molecule portion, optionally with provision of a Western chain precursor and of enzyme MxnB. The methylated precursor compound can e.g. be selected from the following compounds: A methylcarbonate (R1-O-C(O)-OCH₃), especially an alkyl or aryl methylcarbonate (R1 is an alkyl or aryl), e.g. C1 to C12, especially C6, alternatively R1-S-C(O)-OCH₃.

In the second embodiment, a portion of the entire set of synthetic enzymes is provided, e.g. in a microorganism, which portion of the entire set of synthetic enzymes catalyzes the condensation of the pyrone ring. The portion can comprise or consist of MxnB, preferably MxnA-MxnB. In this embodiment, an Eastern chain precursor is provided, and at least a portion of MxnI-MxnJ is provided, which portion catalyzes the conversion of an added Eastern chain precursor to a converted Eastern chain precursor and MxnA, optionally MxnA-MxnB, preferably MxnK, and optionally MxnC- MxnD-MxnE-MxnF-MxnG are provided. This allows the production of compounds, in which the converted Eastern chain precursor is condensed to the natural Western chain molecule portion or to a converted Western chain precursor.

The enzymes which are provided can e.g. comprise or consist of MxnA-MxnB, and in a first variant modules 1E-6E of MxnI-MxnJ, in a second variant modules 2E-6E of MxnI-MxnJ, in a third variant modules 4E to 6E or MxnI-MxnJ, or in a fourth variant module 6E of MxnJ. In combination with providing a Western chain precursor when at least one catalytic domain or a module of MxnK is inactivated as described above, further optionally in combination with MxnC-MxnD-MxnE-MxnF-MxnG.

In a first variant, MxnI-MxnJ consisting of modules 1E-6E can be provided, e.g. by inactivating or deleting at least the loading domain (LM, amino acids No. 1 to 530, preferably amino acids No. 1 to 466, in SEQ ID NO: 9 (MxnI)), in a second variant, MxnI-MxnJ consisting of modules 2E-6E can be provided by inactivating at least module 1E (amino acids No. 1 to 1781, preferably amino acids No. 1 to 1759, in SEQ ID NO: 9 (MxnI)), in a third variant, MxnJ consisting of modules 4E-6E can be provided by inactivating at least module 3E, e.g. by deleting or inactivating MxnI (SEQ ID NO: 9), and optionally amino acids No. 1 to 405, preferably No. 1 to 450, of SEQ ID NO: 10, optionally MxnI (SEQ ID NO: 9), and further optionally the part of MxnJ comprised of amino acids No. 1 to 405, in SEQ ID NO: 10) can be functionally absent, e.g. deleted or inactivated, in a fourth variant, MxnI-MxnJ consisting of modules 5E and 6E can be provided, e.g. by inactivating at least module 4E. In the third and fourth variant, MxnI (SEQ ID NO: 9) can be functionally absent, e.g. mutated, especially deleted, resulting in the loading domain to module 4E being functionally absent, optionally part of MxnJ, e.g. amino acids No. 1 to 3052, preferably complete MxnI and the part of MxnJ comprised of amino acids No. 1 to 3006 in SEQ ID NO :10 can be functionally absent, e.g. deleted or inactivated. Preferably, an enzyme comprising or consisting of module 6E (amino acids No. 3007 to 3849, preferably amino acids No. 3053 to 3849 in SEQ ID NO: 10) of MxnJ is provided for binding the Eastern chain precursor, optionally in combination with providing MxnA.

In the third embodiment, the Eastern chain precursor can have the structure R_{E}-O-C(O)-X. Especially in the second variant, for incorporation by module 2E, the Eastern chain precursor can have the following structure: R_{E}-O-C(O)-NH-CH₂-C(O)-X. Especially in the third variant, for incorporation by module 4E, the Eastern chain precursor can have the following structure: R_{E}-O-C(O)-NH-CH=CH-CH₂-C(O)-X. Especially in the fourth variant, for incorporation by module 6E, the Eastern chain precursor can have the following structure: R_{E}-O-C(O)-NH-CH=CH-CH₂-CH₂-CH(CH₃)-C(O)-X. In each variant, R_{E} can e.g. be any alkyl, aryl or aromatic hydrocarbon, each optionally containing a heteroatom, or R_{E} can be H. X is an auxiliary group having a sulfur atom that is bound to the carbon atom.

In the first and second embodiments, the set of synthetic enzymes can be provided by genetic manipulation in an original myxopyronin producer microorganism by inactivating or deleting the genes encoding synthetic enzymes, modules or domains thereof which are in excess of the genes encoding the portion of the natural set of enzymes used in the process. Accordingly, the portion of the natural set of enzymes used in the process of the invention forms synthetic enzymes.

Accordingly, in the first embodiment of the process for producing a myxopyronin or a derivative, the natural steps for the synthesis of the Western chain molecule portion, e.g. the provision of the enzymes catalysing the Western chain molecule portion, can be replaced by providing a Western chain precursor and preferably at least a part of the enzymes catalysing the synthesis of the Western chain molecule portion, e.g. a part of the modules or domains of MxnK. In this embodiment, the precursor is not necessarily bound to the synthetic enzyme, but the precursor can have an auxiliary group, preferably bound via a sulfur atom of the auxiliary group, e.g. the precursor can be bound to the sulfur atom of a thioalkyl or a thioaryl which may be substituted further by heteroatoms, e.g. by an amine group, an alkyl amine, an alkyl amide, e.g. to the sulfur atom of an *N*-2-thioalkyl carbon acid amide, wherein the carbon chain of the alkyl and of the acid moiety can be a linear or branched C1 to C6 group, especially *N*-2-thioethylacetamide. As an example for a thioaryl, the precursor can be bound to the sulfur atom of a thiophenol group.

In the second embodiment of the process for producing a myxopyronin or a derivative, the natural steps for the synthesis of the Eastern chain molecule portion, e.g. the provision of the enzymes catalysing the Eastern chain molecule portion is replaced by providing an Eastern chain precursor and preferably an enzyme comprising or consisting of module 6E of MxnI-MxnJ, modules 4E-6E of MxnI-MxnJ, modules2E-6E of MxnI-MxnJ, or modules1E-6E of MxnI-MxnJ.

For the purposes of this invention, the Western chain molecule portion and the Eastern chain molecule portion, respectively, are synthesized by the host microorganism, whereas the Western chain precursor and the Eastern chain precursor, respectively, are compounds which are added to the culture of the microorganism. Western chain precursor and Eastern chain precursor can be produced synthetically.

The precursor preferably contains or consists of R1-(C=O)-CH₂-(C=O)- which is bound to the S of the auxiliary group X, wherein preferably the R1 is R2(CH₃)C- or R2(CH₃CH₂)C-, with R2 e.g. selected from a C1 to C12 branched or linear alkyl or aryl or aromatic compound, e.g containing one or two double bonds, preferably two conjugated double bonds, and optionally further substituted with an aryl or a heteroatom containing group, e.g. a phenyl, hydroxyphenyl, hydroxyl or amine group, preferably at the terminus. R1 can be R_{W} for the Western chain precursor and R_{E} for the Eastern chain precursor, respectively. Generally, the precursor can have the following structure 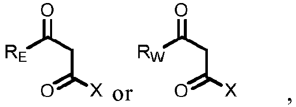 wherein X is the auxiliary group, e.g.

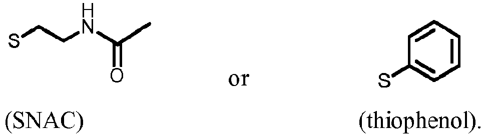

In embodiments in which both a Western chain precursor and an Eastern chain precursor are provided, the process comprises the provision of the enzyme MxnB or CorB, optionally of MxnA-MxnB or CorA-CorB, as it was found that MxnB is sufficient for catalyzing the condensation of both precursors to the pyrone ring moiety carrying the substituents to the (C=O)-CH₂-(CO) group of each of the Western chain and of the Eastern chain precursor, preferably of module 6E of MxnJ, especially for binding the Eastern chain precursor to the enzyme, and preferably for introducing an intermediate group into the Eastern chain precursor (elongation step introducing the group -CH₂-CH₂-), respectively.

Accordingly, MxnC-MxnM, or CorC-ORF1, respectively, including the intermediate enzymes given in Table 1 or modules or domains thereof can be absent or be inactivated in this embodiment. Preferably, in addition to provision of enzyme MxnB, preferably of MxnA-MxnB, the process includes provision of enzyme MxnL, MxnM or MxnM-MxnL. In this embodiment, at least a portion of enzymes MxnK and MxnJ are absent or inactive, preventing synthesis of Western chain molecule portion and of the Eastern chain molecule portion, and enzymes MxnC-MxnG and/or MxnH can optionally be present or absent. Addition of a Western chain precursor and of an Eastern chain precursor results in production of myxopyronin derivatives. Schematically, this embodiment in which both MxnK and MxnI are inactivated, can be represented by 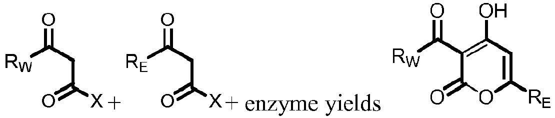 wherein the enzyme preferably is MxnB and X is an auxiliary group which is bound by an S (sulfur atom). The auxiliary group X is e.g. SNAC or thiophenol. Similarly, the product was obtained when both MxnK and MxnJ were inactivated, again with the other enzymes optionally active.

In embodiments for producing a myxopyronin or a myxopyronin derivative having e.g. the natural Eastern chain or an Eastern chain derived from an Eastern chain precursor, the process can comprise the provision of a Western chain precursor and the provision of enzyme MxnB, preferably of MxnA-MxnB, and of enzymes MxnI-MxnJ, optionally in addition MxnL and/or MxnM in order to provide the synthetic pathway enzymes for synthesis of the Eastern chain molecule portion in the host microorganism. In the alternative to MxnB, CorB can be provided, and in the alternative to MxnA-MxnB, CorA-CorB can be provided, in the alternative to MxnI-MxnJ, CorI-CorJ can be provided, in the alternative to MxnL and/or MxnM, ORF1 can be provided. In this embodiment, enzyme MxnK is absent or inactive, preventing synthesis of Western chain molecule portion, and enzymes MxnC-MxnG and/or MxnH can optionally be provided or absent. Optionally, the Eastern chain precursor can be a methylcarbonate, preferably activated by binding to the S of an auxiliary group. In the presence of enzyme MxnH, the Eastern chain precursor is methylated by MxnH.

Schematically, this embodiment can be represented by

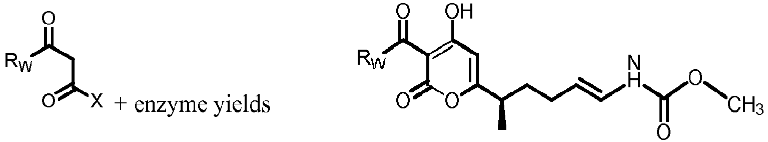

In embodiments for producing a myxopyronin or a derivative myxopyronin having e.g. the natural Western chain or a Western chain derived from a Western chain precursor, the process can comprise the provision of a Eastern chain precursor and the provision of enzymes MxnA-MxnB, alternatively CorA-CorB, preferably in combination with a C-terminal elongation module of MxnJ, e.g. module 6E of MxnJ, alternatively a C-terminal elongation module of CorJ, and of enzyme MxnK, optionally in addition MxnL and/or MxnM, alternatively CorK-CorL, optionally with ORF1, for synthesis of the Western chain molecule portion in the host microorganism. In this embodiment, enzymes MxnI-MxnJ are absent or inactive, preventing synthesis of Eastern chain molecule portion, preferably an enzyme comprising or consisting of module 6E of MxnJ is provided, and enzymes MxnC-MxnG and/or MxnH can optionally be provided or absent.

Schematically, this embodiment can be represented by

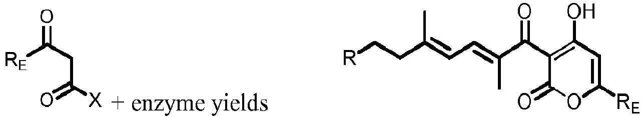

For the Western chain precursor, R2 can e.g. be a branched or linear alkyl chain, optionally having one or two double bonds, and/ or further optionally bound to an aryl, e.g. to a phenyl ring, which is optionally substituted with a hydroxyl or amine group.

For the Eastern chain precursor, R2 preferably is a C1 to C24, e.g. C1 to C12, each branched or linear alkyl or aryl, optionally containing one or two double bonds, preferably a C4 linear chain, optionally carrying a terminal amine or amide group, e.g a methoxy amide group.

Preferably, the Western chain precursor and/or the Eastern chain precursor can have the following structure : 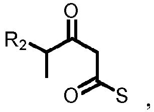 wherein only the sulfur atom (S) of the auxiliary group X is shown.

The process can provide a part of or all of the enzymes which catalyse the synthesis of the Eastern chain molecule portion, and providing a precursor for the Western chain only. In this embodiment, the Eastern chain of the product corresponds to the natural Eastern chain of myxopyronin and the Western chain corresponds to the Western chain precursor which is bound to the auxiliary molecule.

Alternatively or additionally, the process can provide a portion of or all of the enzymes which catalyse the synthesis of the Western chain molecule portion, and providing a precursor for the Eastern chain only. In this embodiment, the Western chain of the product is produced by the microorganism and corresponds e.g. to the natural Western chain of myxopyronin, e.g. when enzymes MxnA-MxnK are provided or to a derivative of the natural Western chain, e.g. when MxnC-MxnG and/or MxnH are functionally absent from the microorganism, and the Eastern chain corresponds to that of the Eastern chain precursor which is bound to the auxiliary molecule with the pyrone ring produced by MxnB.

In a specific embodiment, the process provides the complete synthetic pathway enzymes constituting the natural biosynthetic pathway of myxopyronin synthesis, from which optionally genes are inactivated, e.g. genes encoding MxnC-MxnG and/or MxnH and/or MxnL and/or MxnM are deleted, resulting e.g. in an alteration of the product synthesized. In this embodiment, the pathway enzymes are expressed in a microorganism, and myxopyronin is synthesized entirely by the microorganism. Accordingly, in this embodiment, the process excludes the provision of specific precursor compounds.

From analyses of production of myxopyronin or derivatives from *M. fulvus* in which specific genes had been inactivated, it is found that production of myxopyronin occured when all enzymes MxnA-MxnB-MxnC-MxnD-MxnE-MxnF-MxnG-MxnH-MxnI-MxnJ-MxnK-MxnL-MxM were present.

When enzymes were inactivated, the following effects on myxopyronin production were found:
- that inactivation of MxnC- MxnD-MxnE-MxnF-MxnG with active MxnA-MxnB and MxnH-MxnI-MxnJ-MxnM-MxnL-MxnM resulted in production of the derivatve of myxopyronin 21 -desmethyl-myxopyronin A, 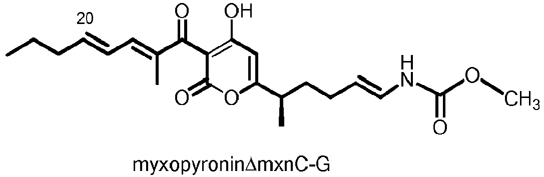
- that inactivation of MxnH only resulted in no production,
- that inactivation of MxnC-MxnG and inactivation of MxnH resulted in no production,
- that inactivation of MxnA only resulted in no production of myxopyronin,
- that inactivation of MxnB only resulted in no production of myxopyronin,
- that inactivation of MxnL only resulted in production of myxopyronin A and B,
- that inactivation of MxnM only resulted in production of myxopyronin A and B,
- inactivation of MxnI only, i.e. active MxnA-MxnH and MxnJ-MxnM with addition of Eastern chain presursor containing R_{E} resulted in production of 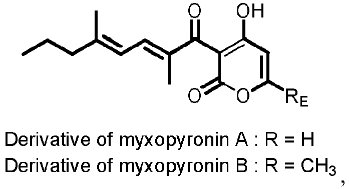
- that inactivation of both MxnK and MxnI only resulted in no production of myxopyronin, and
- that inactivation of MxnK and MxnJ resulted in no production of myxopyronin.

When feeding a Western and/or Eastern chain precursor, the following production was found for inactivated enzymes, wherein the enzymes are denoted by their respective coding sequences and precursors are indicated, with products for each row given in the right hand column. In the following table enzymes connected by a hyphen (-) denote the indicated enzymes including the intermediate enzymes

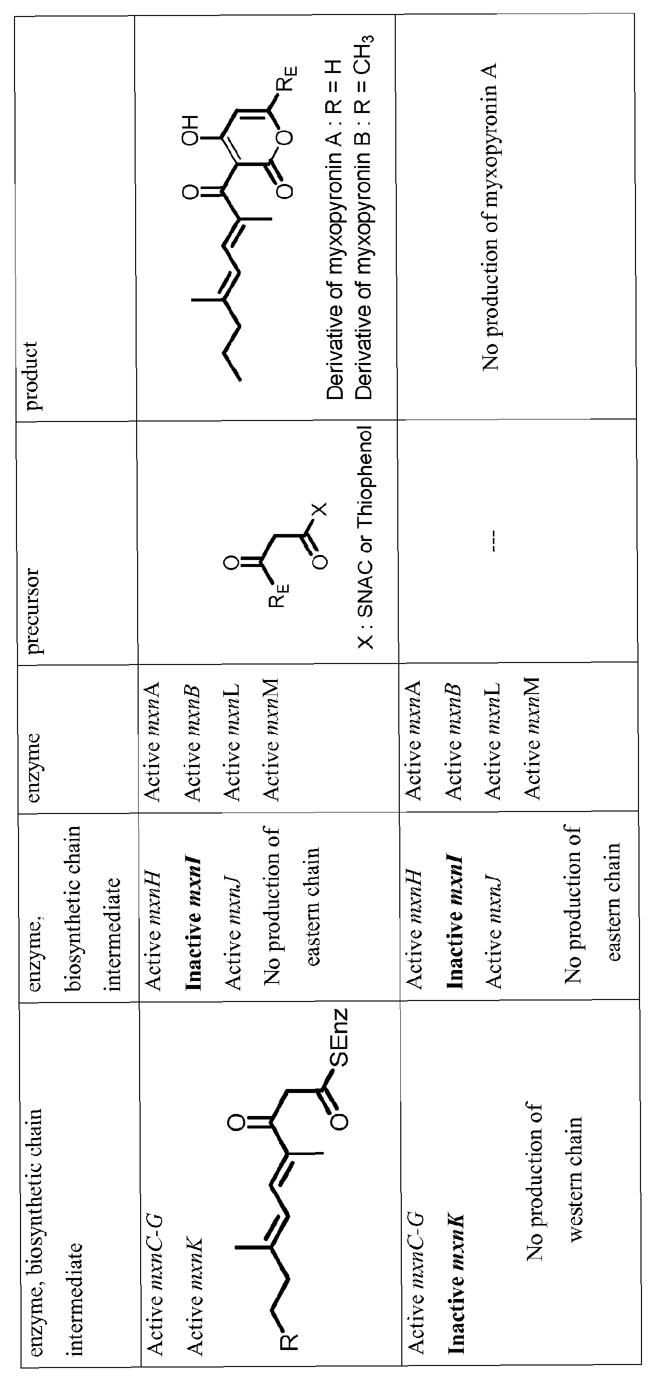

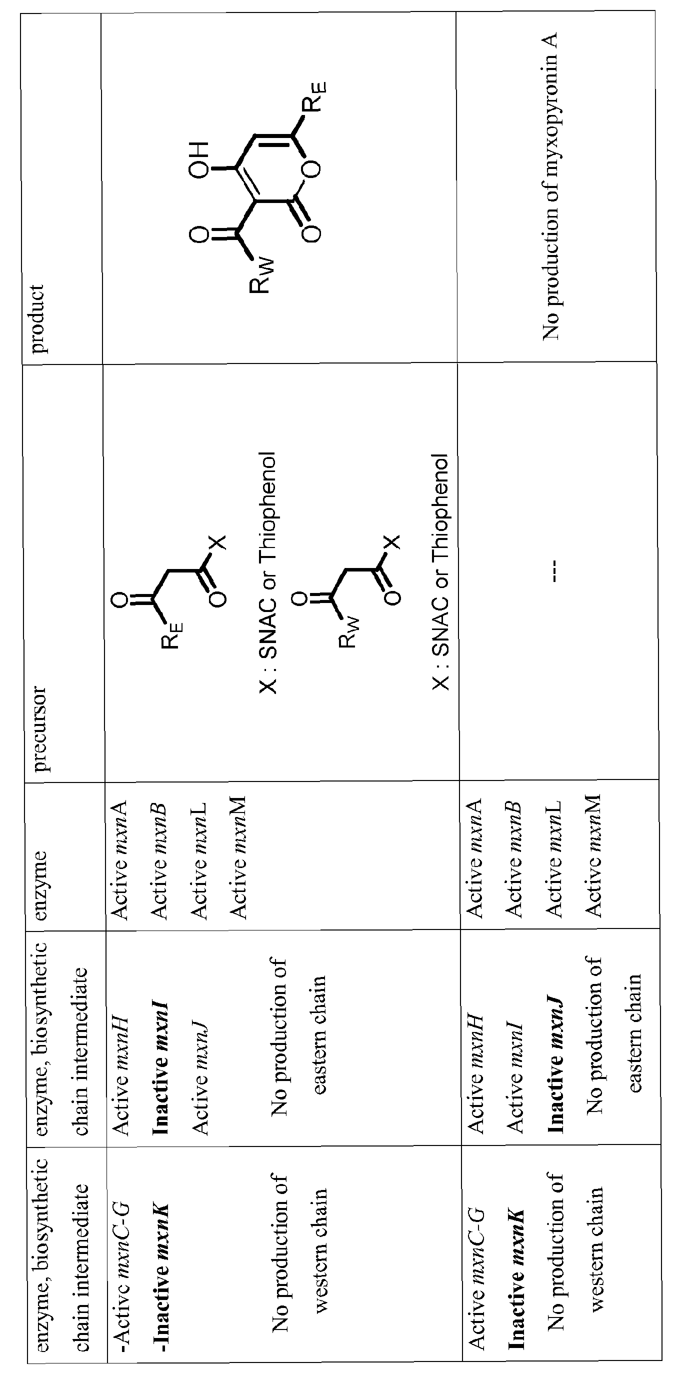

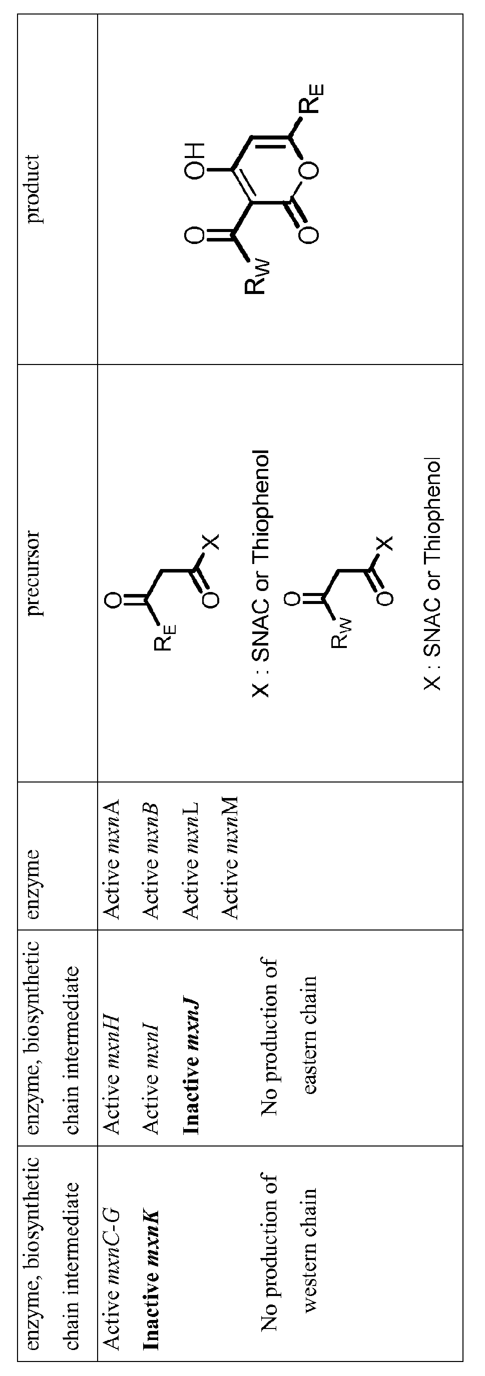

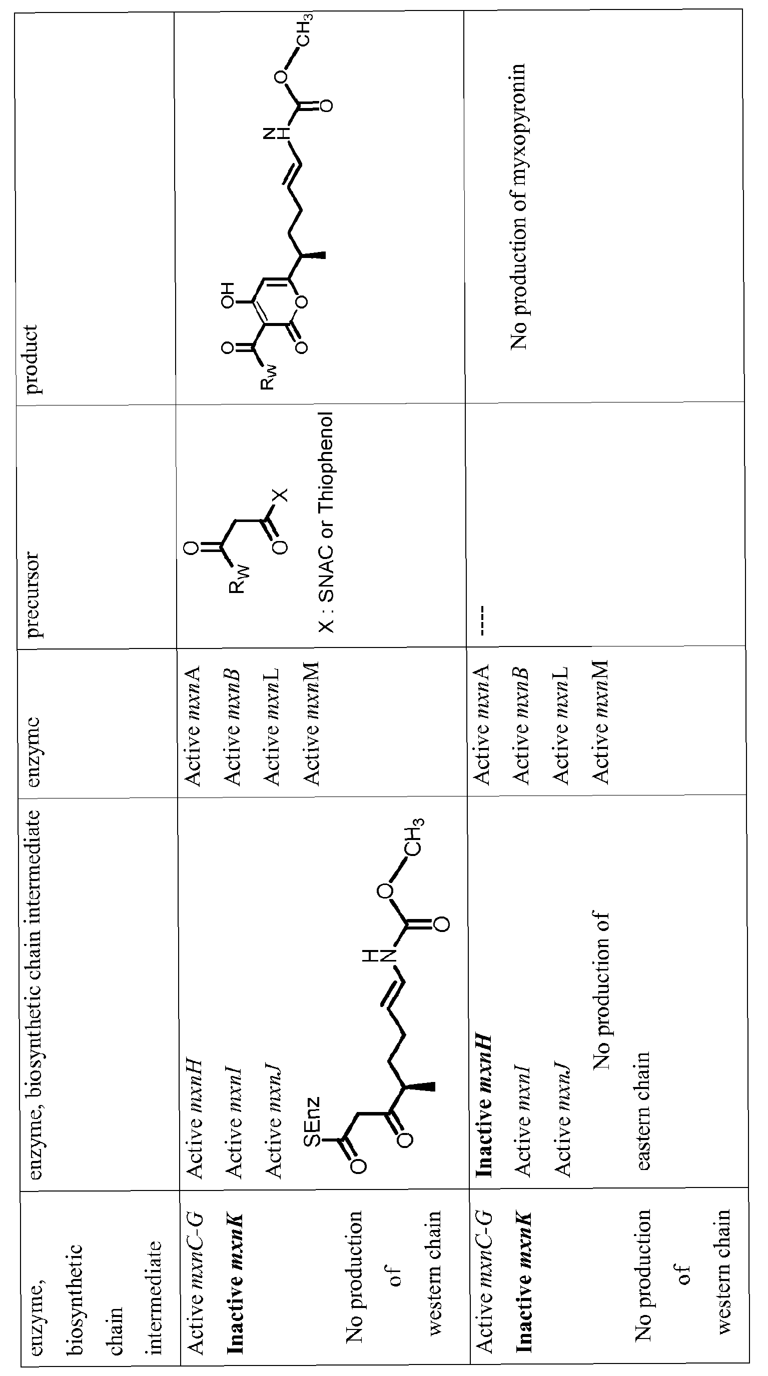

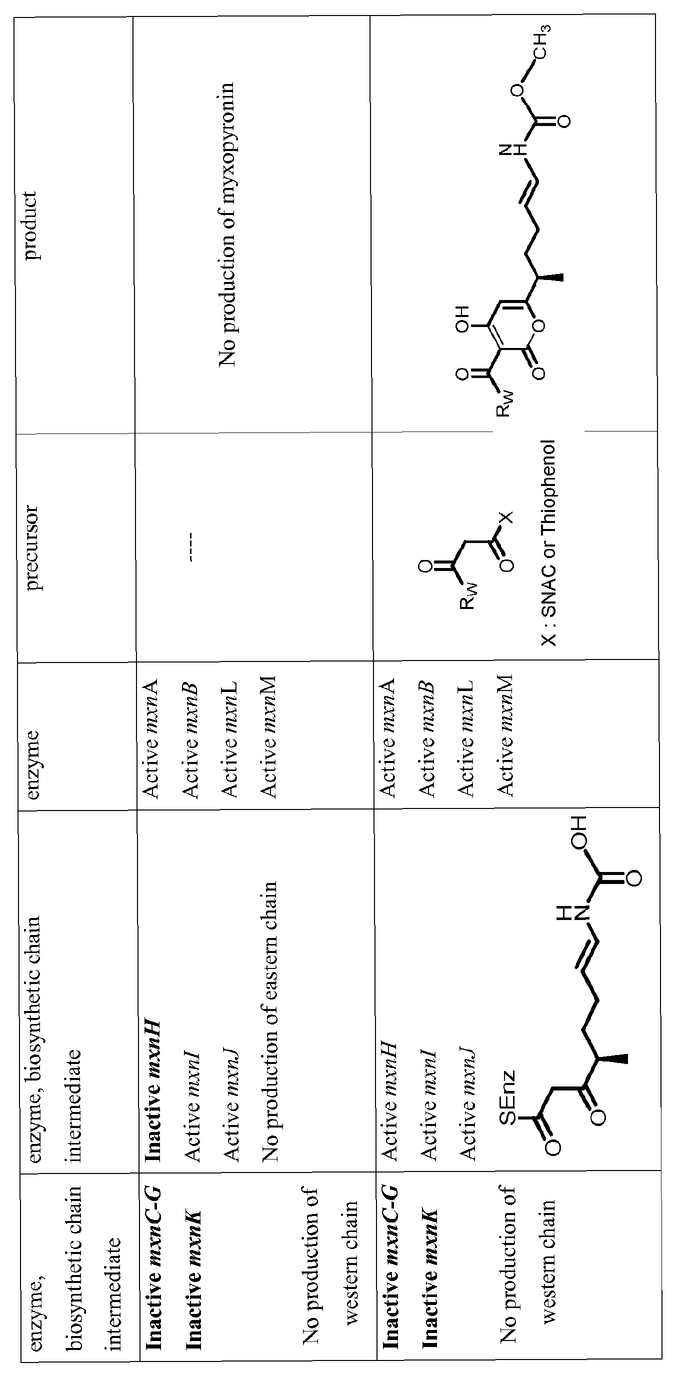

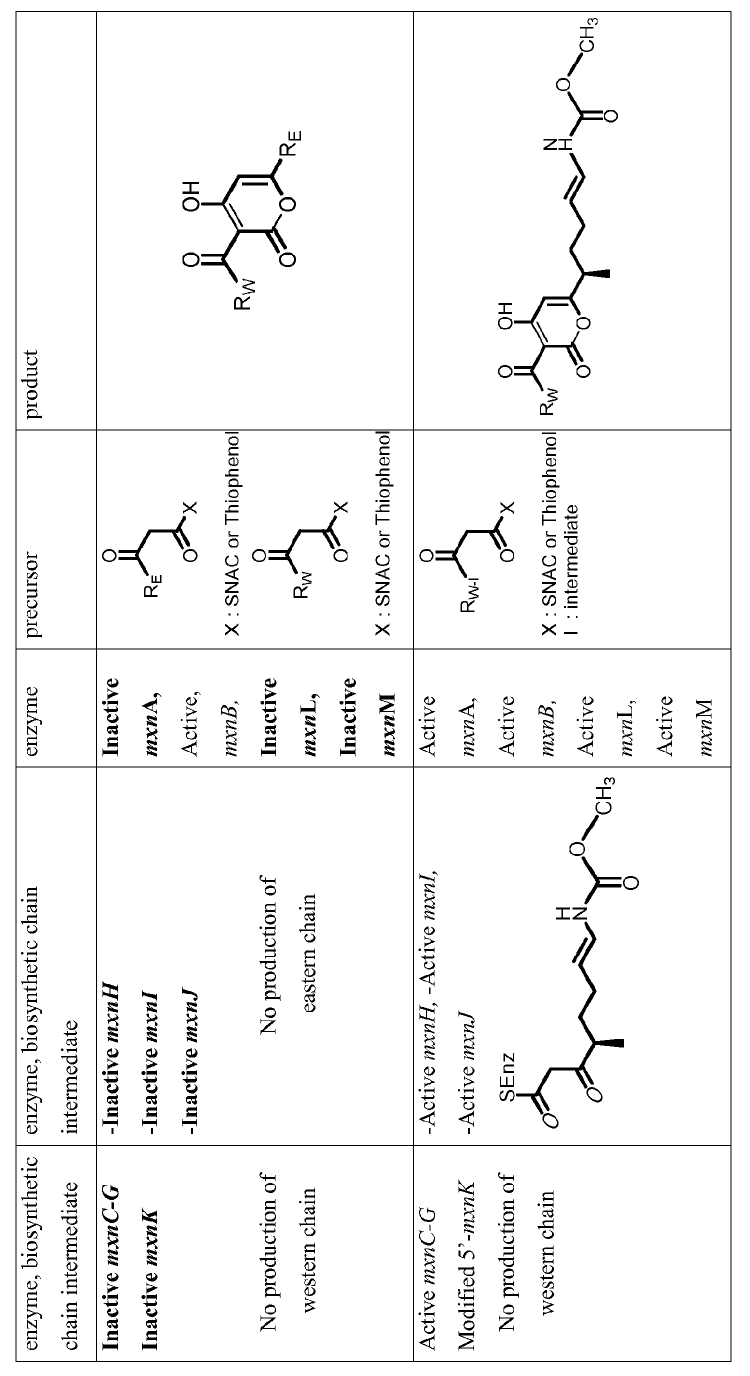

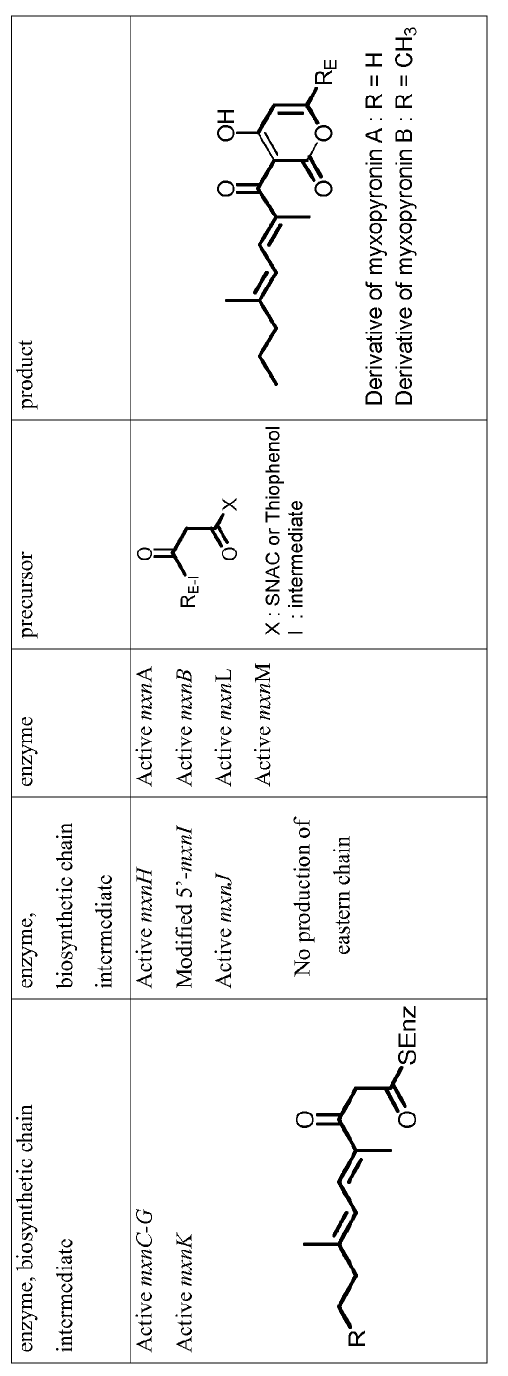

These results show that a process for production of myxopyronin can comprise expression of MxnA-MxnK, with expression of MxnL-MxnM an option at least in Myxococcus (*M. fulvus* Mx f50), as deletion of MxnL-MxnM did not prevent synthesis of myxopyronin in *M. fulvus.*

A process for production of myxopyronin using synthesis of the Western chain molecule portion by expression of MxnK but without expression of MxnC-MxnG results in myxopyronin derivatives having no methyl group on C21 of the Western chain. Optionally, production of myxopyronin using biosynthesis of the Eastern chain molecule portion by expression of MxnI-MxnJ, and/or using biosynthesis of the Western chain molecule portion by expression of at least a portion of MxnK, optionally in the presence or absence of MxnC-MxnG, can optionally be without expression of MxnH, preferably in the presence of MxnH with addition of the precursor in the form of a carbon acid monoalkyl ester, wherein the carbon acid contains a C1 to C12 linear or branched alkyl linked to the acid group, and wherein the alkyl e.g. is a linear or branched C1 to C12, preferably a carbon acid monomethyl ester. The carbon acid monoalkyl ester in this embodiment is integrated into the Eastern chain.

Generally, the enzyme or enzymes catalyzing the synthesis can be provided by expression from a nucleic acid sequence encoding the enzyme and enzymes, resp, wherein the nucleic acid sequence is contained in at least one expression cassette. The nucleic acid sequence can encode the enzyme or enzymes by the codon usage of the host microorganism. As a host microorganism, bacteria are preferred, most preferably Gram-negative bacteria, e.g. Pseudomonas spp, Myxococcus spp. Generally in the process, the enzymes can be provided in vivo by the microorganism or can be provided in vitro, e.g. produced and obtained by the microorganism, e.g. extracted or isolated from the microorganism.

Generally, incubating the microorganism can comprise cultivating the microorganism in cultivating medium under agitation and/or aeration.

The invention is now described in greater detail by way of examples with reference to the figures, which show in
- Figure 1 a scheme of the natural arrangement of synthetic genes in Myxococcus fulvus Mx f50 (mxnA to mxnM),
- Figure 2 in A) a scheme of the natural synthesis of the Eastern molecule portion, at B) a scheme of the natural synthesis of the Western molecule portion and at C) a scheme of the mechanism of the condensation of the molecule portions to the pyrone ring,
- Figures 3 and 4 schemes for subcloning of pathway enzymes,
- Figure 5 a scheme for cloning of an expression construct for expressing the synthetic pathway enzymes from one expression cassette,
- Figure 6 a scheme for cloning coding sequences of enzymes in separate expression cassettes,
- Figure 7 HPLC-MS results of extracts of non-producer Myxococcus xanthus DK 1622 ΔmchA::tet, of transformant *M. xanthus* DK1622 ΔmchA-tet:: pHSU-mxn43 (*M. xanthus* DK1622 ΔmchA-tet::myxopyronin A), and of Myxococcus fulvus Mx f50 wild-type (WT),
- Figure 8 a scheme for cloning for inactivating enzyme MxnI,
- Figure 9 a scheme for cloning for inactivating enzyme MxnJ,
- Figure 10 a scheme for cloning for inactivating enzyme MxnK,
- Figure 11 a scheme for cloning for inactivating enzyme MxnK,
- Figure 12 a scheme for cloning for inactivating enzyme MxnH,
- Figure 13 a scheme for cloning for inactivating enzymes MxnC-MxnD-MxnE-MxnG-MxnH,
- Figure 14 a scheme for cloning for inactivating enzyme MxnC-MxnD-MxnE-MxnG,
- Figure 15 HPLC-MS results of extracts of cultures from microorganisms in which at least one enzyme of the myxopyronin A synthetic pathway enzymes is inactivated,
- Figure 16 a scheme for cloning for inactivating enzyme MxnL,
- Figure 17 a scheme for cloning for inactivating enzyme MxnM,
- Figure 18 analytical results for myxopyronin A production during cultivation of a) Myxococcus fulvus Mx f50, b) Myxococcus fulvus Mx f50 having MxnL inactivated, c) Myxococcus fulvus Mx f50 having MxnM inactivated (ΔmxnM),
- Figure 19 a scheme of the effect of inactivation (X) of the KR of module 2E (KR-2E) on synthesis,
- Figure 20 a scheme of the effect of inactivation (X) of the DH of module 2E on synthesis,
- Figure 21 a scheme of the effect of inactivation (X) of the MT of module 4E on synthesis,
- Figure 22 HPLC-MS analytical results for in vitro synthesis using Western chain and Eastern chain precursors,
- Figure 23 MS² spectra of product in culture supernatant extracts,
- Figure 24 a cloning scheme for inactivation of the nucleic acid sequence encoding catalytic domain CP of module 1W of enzyme MxnK,
- Figure 25 a cloning scheme for inactivation of the nucleic acid sequence encoding catalytic domain CP of module 4W of enzyme MxnK,
- Figure 26 a scheme in a) of the wild-type synthesis of the Western molecule portion by MxnK, in b) the conversion of an added Western chain precursor by MxnK having catalytic domain CP of module 1W inactivated, in c) the conversion of an added Western chain precursor by MxnK having catalytic domain CP of module 3W inactivated, in d) the conversion of an added Western chain precursor by MxnK having catalytic domain CP of module 4W inactivated, dark circles indicating active domains, white circles indicating inactive domains,

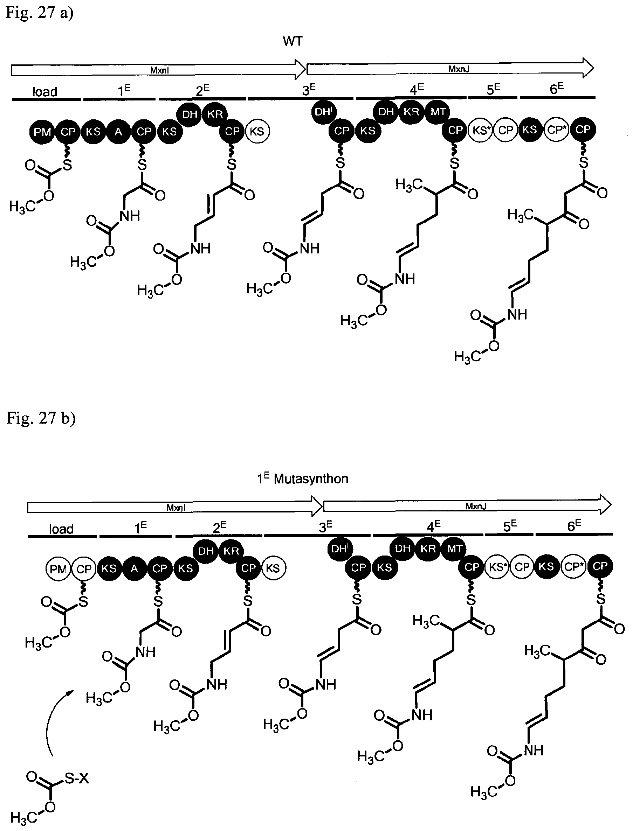

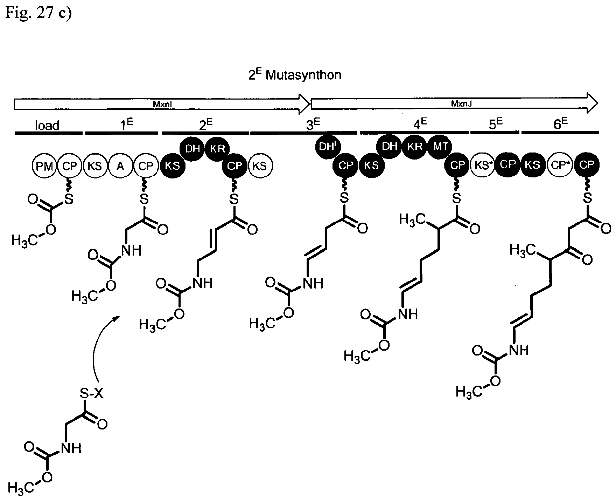

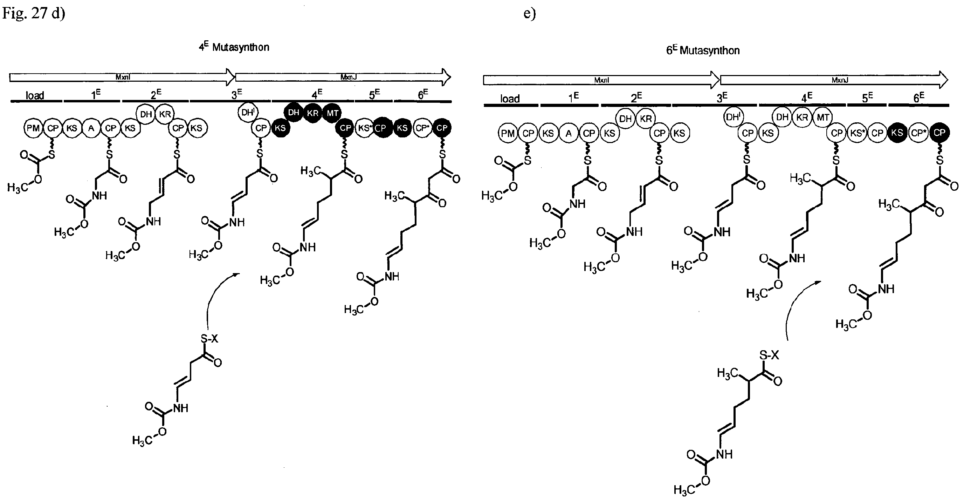

- Figure 27 a scheme in a) of the wild-type synthesis of the Eastern molecule portion by MxnI-MxnJ, in b) the conversion of an added Eastern chain precursor by MxnI-MxnJ having catalytic domain CP of the load module (load) inactivated, in c) the conversion of an added Eastern chain precursor by MxnI-MxnJ having catalytic domain CP of module 1E inactivated, in d) the conversion of an added Eastern chain precursor by MxnI-MxnJ having catalytic domain CP of module 3E inactivated, in e) the conversion of an added Eastern chain precursor by MxnI-MxnJ having catalytic domain CP of module 4E inactivated, dark circles indicating active domains, white circles indicating inactive domains,
- Figure 28 schematically shows the conversion of an added Western chain precursor by MxnK having catalytic domain CP of the module 2W inactivated and the synthesis of the Eastern molecule portion by MxnI-MxnJ having the catalytic domain MT of module 4E inactivated, followed by condensation of the altered Eastern molecule portion with the converted Western chain precursor by MxnB, dark circles indicating active domains, white circles indicating inactive domains, and
- Figure 29 schematically shows the synthesis of the Western molecule portion by MxnK having catalytic domain MT of the module 4W inactivated and the conversion of an added Eastern chain precursor by MxnI-MxnJ having the catalytic domain CP of module 1E inactivated, followed by condensation of the altered Western molecule portion with the converted Eastern chain precursor by MxnB, dark circles indicating active domains, white circles indicating inactive domains.

Generally, in the Figures depicting enzyme encoding genes or enzymes, wherein e.g. modules, domains or catalytic centers are indicated, in combination with the synthesis of myxopyronin or of a derivative, the sequence of catalytic steps is from left to right, corresponding to the direction from the N-terminus to the C-terminus of proteins indicated.

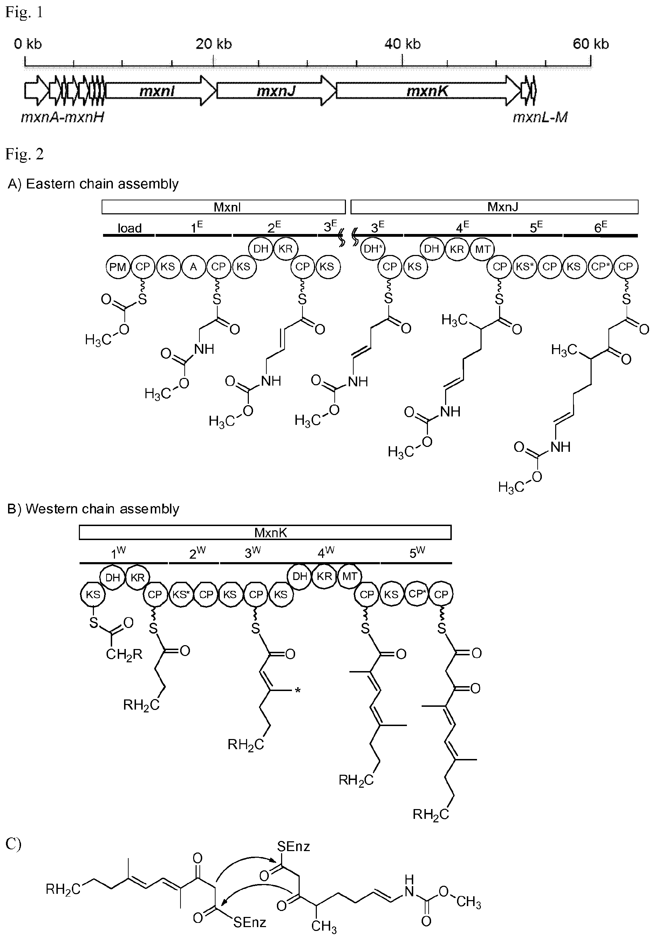

As shown in Figure 1, the genes encoding the enzymes for myxopyronin synthesis in Myxococcus are arranged adjacent one another. In the figures, * denotes catalytic domains which may not entirely have the catalytic function indicated due to diversions, e.g. from known catalytic domains. Figure 2 A) schematically shows the catalytic domains of the enzymes encoded by the respective genes participating in myxopyronin synthesis. From analysis, MxnI is assumed to provide an N-terminal phosphomutase (PM) domain which could be involved in the activation of sodium biscarbonate as a starter unit by phosphoryl group transfer to enable subsequent attachment to the CP domain of the loading module. Synthesis of the Eastern chain molecule portion comprises elongation of the starter unit with glycine and of three malonyl-CoA extender units by a mixed polyketide synthetase/nonribosomal peptide megasynthetase consisting of two subunits (enzymes MxnI and MxnJ) and harbouring a total of six elongation modules (1E-6E) in addition to the loading module (PM-CP) indicated at the N-terminus of module 1E of MxnI. MxnI-MxnJ are PKS/NPRS (polyketide synthetase/non-ribosomal peptide synthase) comprising one loading module and six extension modules. Module 1E (KS-A-CP) catalyses glycine incorporation, which is then elongated by module 2E (KS-DH-KR-CP) with malonyl-CoA. Module 3E represents a split module (KS from MxnI and DH*-CP from MxnJ), which is not assumed to incorporate an extender unit, but wherein the dehydrogenase domain DH might catalyse the shift of a double bond from α, β position to β, γ position. Module 4E (KS-DH-KR-MT-CP) incorporates another malonyl-CoA extender unit, which gets methylated by the MT domain. Complete reduction of the β-carbonyl functionality is assumed to be catalysed by internal DH-KR activities plus the ER activity from MxnA, which is provided in *trans.* The ketosynthase domain of module 5E (KS*-CP) possibly does not catalyse chain elongation, so that no extender unit can be incorporated from this module. A third malonyl-CoA extender unit is incorporated by module 6E (KS-CP*-CP), in which one of the tandem-CP domains is assumed to be inactive, to generate the final eastern chain intermediate with a β-carbonyl functionality, which is essential for the chain condensation for pyrone ring formation. For all three elongation steps with malonyl-CoA (by modules 2E, 4E and 6E) the *trans* AT-activity of MxnA is required. MxnM might have an editing/proofreading activity to remove aberrant acyl units from blocked MxnI/MxnJ modules, and optionally also from blocked MxnK modules.

As shown in Fig. 2 B), the Western chain molecule portion is catalysed by MxnK. It is assumed that initiation is with acetyl-CoA for myxopyronin A or with propionyl-CoA for myxopyronin B, which is directly loaded onto the N-terminal KS domain of module 1W; a typical loading module was not found. After elongation with the first malonyl-CoA unit by module 1W (KS-DH-KR-CP), the resulting β-keto compound is completely reduced by internal DH-KR activities plus the *trans*-ER activity of MxnA. From analysis, it is assumed that the domain KS* of module 2W, KS*-CP, is inactive and accordingly module 2W is not involved in chain elongation. Subsequent modules 3W, 4W, 5W are assumed to catalyse incorporation of three additional malonyl-CoA extender units to yield the Western chain molecule portion. The chain elongation by module 3W includes β-branching to introduce a methyl group in β-position, catalysed by MxnC-MxnG. Chain elongation on module 4W includes a complete reduction of the reduction of the β-carbonyl functionality by internal DH-KR activities plus the *trans-ER* activity from MxnA. Similar to eastern chain biosynthesis, the *trans-AT* activity from MxnA is required for all four elongation steps with malonyl-CoA by modules 1W, 3W, 4W and 5W. MxnM might have an editing/proofreading activity to remove aberrant acyl units from blocked MxnK modules, and also from blocked MxnI/MxnJ modules.

Fig. 2 C) shows the condensation of the enzyme-bound Western chain molecule portion and the enzyme-bound Eastern chain molecule portion to the pyrone ring that is substituted with the R_{W} and R_{E} as provided by the natural biosynthetic enzymes.

The following nucleic acid sequences were used as primers:

| Name | Sequence (5'→3') restriction sites underlined | Restriction sites |
|---|---|---|
| mxn9 | GTCAGACATATGAACAACAGCGGT (SEQ ID NO: 31) | NdeI |
| mxn10 | CGTCGTAAGCTTTCAGTAGGTGAAAACCA (SEQ ID NO: 32) | HindIII |
| mxn214 | GATACGCATATGGTCCCCTTCGAGTCGAGC (SEQ ID NO: 33) | NdeI |
| mxn215 | TCTATGAAGCTTTCATGGCTTCGCTCCCGC (SEQ ID NO: 34) | HindIII |
| mxn230 | CTGAGGCATATGCGTGCGTTCGAGTCG (SEQ ID NO: 35) | NdeI |
| mxn231 | TCTATGAAGCTTTCAGGCACACCACTCATT (SEQ ID NO: 36) | HindIII |
| mxn30 | | PacI, SspI, NdeI, NheI |
| mxn33 | CAGGTAGATATCTGGACAGCAAGCGAACC (SEQ ID NO: 38) | EcoRV |
| mxn34 | ATCCTGTCTCTTGATCAGAT (SEQ ID NO: 39) | |
| mxn35 | | |
| mxn36 | CATGTCGGATCCTCACCATCGTCCACAGCTTCG (SEQ ID NO: 41) | BamHI |
| mxn41 | AATCTGTACCTCCTTATCCTGTCTCTTGATCAGAT (SEQ ID NO: 42) | |
| mxn42 | | |
| mxn48 | ACGTCGACGAGTACTGGC (SEQ ID NO: 44) | |
| mxn49 | AATGCCTTCGGAGCCTCG (SEQ ID NO: 45) | |
| mxn50 | CATGTAGATATCGAACGGCTC CGGTACATC (SEQ ID NO: 46) | EcoRV |
| mxn51 | TGTCTAGGATCCACAGCCGCTCCAGGATTC (SEQ ID NO: 47) | BamHI |
| mxn52 | GATACACAGCTGTCCCTCCTGTTCAGCTAC (SEQ ID NO: 48) | PvuI |
| mxn53 | ATCTTAATTAAGGACGCGATGGATATGTT (SEQ ID NO: 49) | PacI |
| mxn54 | GCGTTGATGGATCCACAGCA (SEQ ID NO: 50) | BamHI |
| mxn55 | TGTATCGCGGCCGCTCAGTGCCGCTGAACCCGAAC (SEQ ID NO: 51) | NotI |
| mxn56 | GATACAGCGGCCGCCTTTCCGAGTCCAGCAGC (SEQ ID NO: 52) | NotI |
| mxn57 | GTATCGAGCTCATCTGCTGTCCTCTGCCT (SEQ ID NO: 53) | SacI |
| mxn58 | GTACGTGCCGTTGGAT (SEQ ID NO: 54) | |
| mxn59 | GAGGGGTACTTGAAGAGC (SEQ ID NO: 55) | |
| mxn60 | TGCCATGAGCAGACAGTTA (SEQ ID NO: 56) | |
| mxn64 | GCTGAGACGGTCTTCCTC (SEQ ID NO: 57) | |
| mxn65 | | PacI, SspI, NdeI, NheI |
| mxn68 | CAACATCTCGCCGAGTGA (SEQ ID NO: 59) | |
| mxn69 | ATACGACTCACTATAGGGCG (SEQ ID NO: 60) | |
| mxn70 | CTTGCGCCCTGAGTGCTT (SEQ ID NO: 61) | |
| mxn71 | AACACCTCACGGTCCGAC (SEQ ID NO: 62) | |
| mxn72 | CCCATCGTCCACAGCTTC (SEQ ID NO: 63) | |
| mxn73 | GCACTACAAGCGGCTCTG (SEQ ID NO: 64) | |
| mxn83 | GAGCGGCCGCCTCGAGATGAAGGTGGAT (SEQ ID NO: 65) | NotI |
| mxn84 | ATGACTAGTCTCCTCGCTCTCGAAGTC (SEQ ID NO: 66) | SpeI |
| mxn85 | GACACGATCGCAACAGCGGTTCTTTCTCTTTG (SEQ ID NO: 67) | PvuI |
| mxn87 | CAAGGCGGCCGCCGGGTGATCGCAGGTGAG (SEQ ID NO: 68) | NotI |
| mxn88 | TCTGGGATCCTTCGACCTCGGACAGCAG (SEQ ID NO: 69) | BamHI |
| mxn94 | TCACGGCGAAGCACATCC (SEQ ID NO: 70) | |
| mxn95 | AGGAGGCAGGGTCCGAAT (SEQ ID NO: 71) | |
| mxn98 | GACTCAGCTCGTTTGGCG (SEQ ID NO: 72) | |
| mxn99 | TAACCCAGGCGCAGAATC (SEQ ID NO: 73) | |
| mxn100 | AGGATGCGCGTCAAGAGT (SEQ ID NO: 74) | |
| mxn101 | GATACGAAGAAGCGCGAG (SEQ ID NO: 75) | |
| mxn102 | CGTCATTCATCCTTGCTCG (SEQ ID NO: 76) | |
| mxn103 | TCCAGGCCTCGTAGTTCC (SEQ ID NO: 77) | |
| mxn104 | TTTCCTCACGTCTCATGG (SEQ ID NO: 78) | |
| mxn105 | TGTCGCTGTTGAAGGCAC (SEQ ID NO: 79) | |
| mxn106 | TTCTCATCGCGGACTACG (SEQ ID NO: 80) | |
| mxn107 | GAGAGAGATTGACGCGAC (SEQ ID NO: 81) | |
| mxn110 | GATTCTCTGGGGGCTCCT (SEQ ID NO: 82) | |
| mxn111 | CAATCACAAACGCAGTCC (SEQ ID NO: 83) | |
| mxn112 | TTCTCATCGCGGACTACG (SEQ ID NO: 84) | |
| mxn113 | GATACGATCGCCTGGAGGAATACGTCGG (SEQ ID NO: 85) | PvuI |
| mxn114 | GTCGCGGCCGCCTTTCGTCATGGTGGCTCC (SEQ ID NO: 86) | NotI |
| mxn115 | GATACGCGGCCGCTTGAAGGGACTGGGCAAG (SEQ ID NO: 87) | NotI |
| mxn116 | TTGCGGATCCACAATCTATCGCGGGTCG (SEQ ID NO: 88) | BamHI |
| mxn117 | GACACGATCGGCATGCATGAGGCCTACA (SEQ ID NO: 89) | PvuI |
| mxn118 | AATCGCGGCCGCGCATCAGGATTCCCCAAGG (SEQ ID NO: 90) | NotI |
| mxn119 | TCTGGCGGCCGCGCATTGCTCGCGGCTCAGT (SEQ ID NO: 91) | NotI |
| mxn120 | AGATCTGATCAAGAGACAGGATGAGTTCATCGCCTTG GAG (SEQ ID NO: 92) | |
| mxn121 | CATGTCGGATCCTCATCGCTCTTCACGTACACG (SEQ ID NO: 93) | BamHI |
| mxn124 | CAATACGATCGCTGGACTGGTGAAG (SEQ ID NO: 94) | PvuI |
| mxn125 | | AflII, SpeI |
| mxn126 | GATACACCTAGGTCCCTCCTGTTCAGCTAC (SEQ ID NO: 95) | AvrII |
| mxn127 | TCAGGTCCTAGGGGACGCGATGGATATGTT (SEQ ID NO: 96) | AvrII |
| mxn128 | | AvrII, PvuI, AflII |
| mxn129 | | AvrII |
| mxn132 | GATACACGATCGCGAGCAGGAGGGGTATGT (SEQ ID NO: 99) | PvuI |
| mxn133 | TCTATGGAGCTCCATTCCGAAGAACTGGGA (SEQ ID NO: 100) | SacI |
| mxn134 | GATACAGAGCTCTTCCTCAATCACCACGGT (SEQ ID NO: 101) | SacI |
| mxn135 | TCTATGGGATCCTGACTGACGACGATGAGC (SEQ ID NO: 102) | BamHI |
| mxn136 | AACATCGGCCACCTGGAG (SEQ ID NO: 103) | |
| mxn137 | AGGACCCAGGCTTCTCGT (SEQ ID NO: 104) | |
| mxn140 | ACATGTTGGAAGCCGACG (SEQ ID NO: 105) | |
| mxn141 | GCTCAGCGTGAAGAGGCT (SEQ ID NO: 106) | |
| mxn142 | ACCGTGGAACAGCTCAGG (SEQ ID NO: 107) | |
| mxn143 | CGAGATCCAGCACACCAT (SEQ ID NO: 108) | |
| mxn144 | GATACATTAATTAACCGGAATTGCCAGCTG (SEQ ID NO: 109) | PacI |
| mxn145 | CTGTTAATTAATCAGAAGAACTCGTCAAGAAG (SEQ ID NO: 110) | PacI |
| mxn147 | TATGTCCTTAAGATAATTCGGCTGCAGGGG (SEQ ID NO: 111) | AflII |
| mxn148 | TATCTTAATTAATTACCAATGCTTAATCAGTG (SEQ ID NO: 112) | PacI |
| mxn149 | GCTTCGCGTGGAGGTCAT (SEQ ID NO: 113) | |
| mxn169 | GATACACGATCGCACGAGTTCCGAGTCCTC (SEQ ID NO: 114) | PvuI |
| mxn170 | CTGCAGGATGAGGGCGTC (SEQ ID NO: 115) | |
| mxn171 | TCGACGCCCTCATCCTGCAGGAGTTGATGGCGGAGCTG (SEQ ID NO: 116) | |
| mxn172 | TCTATGGCGGCCGCGAGCGCGACCTTGATGAC (SEQ ID NO: 117) | NotI |
| mxn174 | GATACACGATCGGACACACACCGGAAGCTG (SEQ ID NO: 118) | PvuI |
| mxn175 | ATTCACCAGCGCGTCGAC (SEQ ID NO: 119) | |
| mxn176 | CGGCGTCGACGCGCTGGTGAATCTGAGAATCGTCC (SEQ ID NO: 120) | |
| mxn177 | TCTATGGAGCTCTTGAGCTGCAGGATGACC (SEQ ID NO: 121) | NotI |
| mxn185 | TCGCTCATCCTGCAGGA (SEQ ID NO: 122) | |
| mxn187 | CGAGTCGAGTCCCATGG (SEQ ID NO: 123) | |
| mxn189 | CAACAGCTTCCTCGACG (SEQ ID NO: 124) | |
| mxn193 | GGAGTCGACGCCGAAC (SEQ ID NO: 125) | |
| mxn195 | TCGGACTGATGAAGGGGT (SEQ ID NO: 126) | |
| mxn206 | GAAGGCCTCCACGTTCTC (SEQ ID NO: 127) | |
| p15A-Tet1 | GGAGAACTGTGAATGCGC (SEQ ID NO: 128) | |
| p15A-Tet-2 | ACTCCGCTAGCGCTGATG (SEQ ID NO: 129) | |
| p15A-Tet-3 | GAGAACTGTGAATGCGCA (SEQ ID NO: 130) | |
| pTOPO-in | CCTCTAGATGCATGCTCGAG (SEQ ID NO: 131) | |
| pTOPO-out | TTGGTACCGAGCTCGGATCC (SEQ ID NO: 132) | |
| pSup_B | GCTCATGAGCCCGAAGTG (SEQ ID NO: 133) | |
| pSup_E | CGCTCATCGTCATCCTCG (SEQ ID NO: 134) | |
| pSWU41-F | GTGCAAAAAAGCGGTTAG (SEQ ID NO: 135) | |

### Comparative Example: Production of myxopyronin by heterologous expression of synthetic pathway enzymes

For heterologous expression, genes encoding synthetic pathway enzymes for synthesis of myxopyronin were isolated from Myxococcus fulvus Mxf50 genomic DNA and subcloned to generate expression constructs that were transferred into host microorganisms, preferably a heterologous host, e.g. by electroporation. For integration of the expression constructs into the genome of the host microorganism, suicide plasmids were constructed as described below.

In detail, three different suicide plasmids were constructed for integration at different sites of the myxopyronin pathway region: (1) The Tn5 promoter was amplified by PCR using primers mxn33/ mxn41 with pCRII-TOPO vector as a template to obtain fragment 1. A 1191 bp fragment of mxnA was amplified from M. fulvus Mxf50 genomic DNA by PCR using primers mxn42/mxn30 to obtain fragment 2. Fragment 1 and fragment 2 were linked together using overlap PCR with primers mxn33/mxn30. The joined fragments were then subcloned into pJET1.2 vector to obtain plasmid pTn5-mxnA/pJET. In parallel, a minimal linear cloning vector p15A-Tet was obtained by PCR using primers mxn52/mxn53 with pACYC184 as a template. pTn5-mxnA/pJET plasmid was hydrolyzed with PacI/EcoRV and ligated to the PacI/PvuII hydrolyzed cloning vector p15A-Tet to generate pHSU-mxn16. (2) A 1491 bp fragment right after the 3'-end of the myxopyronin A gene cluster was amplified from M. fulvus Mxf50 genomic DNA using primers mxn64/mxn65. The amplified fragment was cloned into pCRII-TOPO vector to create pHSU-mxn19. (3) A 1380 bp fragment of mxnJ was amplified from M. fulvus Mxf50 genomic DNA using primers mxn124 and mxn125. A zeocin resistance cassette was amplified from pCDNA-Zeo as the template using primers mxn128/mxn129 and it was subcloned to a minimal linear cloning vector p15A-Tet obtained by PCR using primers mxn126/mxn127 with pACYC184 as a template to obtain p15A-Tet-Zeo plasmid. The 1380 bp fragment of mxnJ was then hydrolyzed with PvuI and subsequently cloned to p15A-Tet-Zeo hydrolyzed with the same restriction enzyme to create pHSU-mxn32.

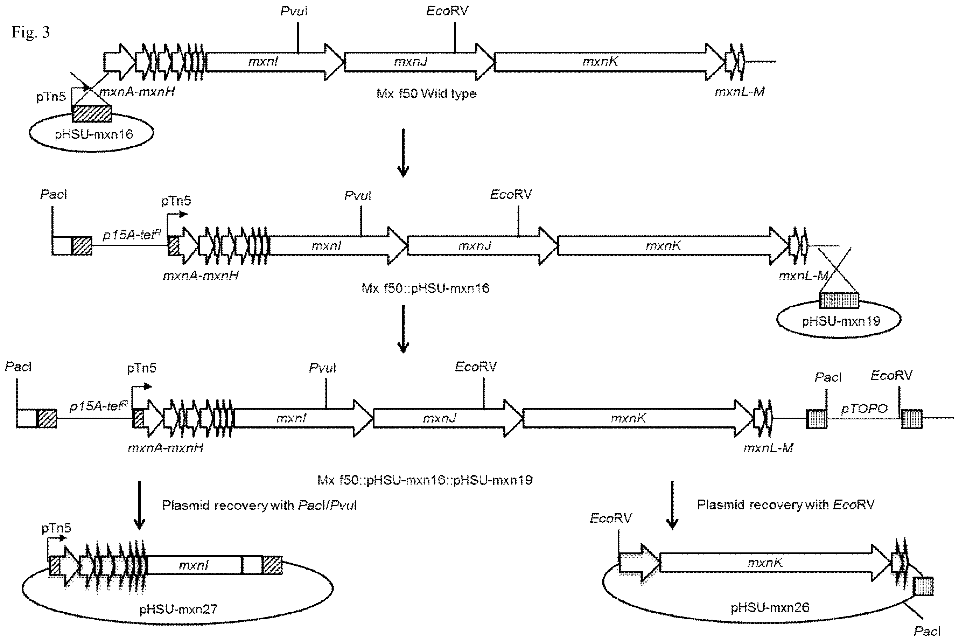

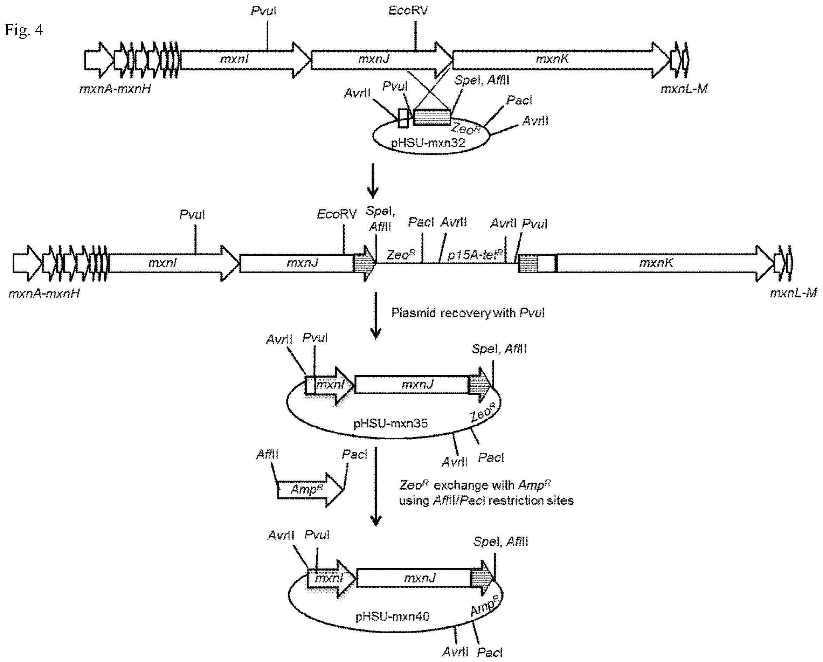

First, pHSU-mxn16 was transformed into M. fulvus Mx f50 to obtain mutant Mx f50::pHSU-mxn16. For genotypic analysis of the single cross-over, a set of different PCRs using the primer combinations mxn60/p15A-Tet1 and mxn61/p15A-Tet2 was carried out to verify the correct integration of the inactivation plasmid. Subsequently, the next single crossover was performed by transforming pHSU-mxn19 into Mx f50::pHSU-mxn16 to obtain Mx f50::pHSU-mxn16::pHSU-mxn19 as depicted in Fig. 3. The mutant was confirmed by PCR using the primer combinations mxn94/pTOPO-out and mxn95/pTOPO-in. Further confirmation of this mutant was also performed by Southern Blot analysis. Second, pHSU-mxn32 was transformed into M. fulvus Mxf50 to obtain mutant Mx f50::pHSU-mxn32 as depicted in Fig. 4. For genotypic analysis of the single cross-over, a set of different PCRs using the primer combinations mxn136/p15A-Tet2 and p15A-Tet3/mxn137 was carried out to verify the correct integration of the inactivation plasmid.

The recovery of pathway fragments was performed as follows: genomic DNA of mutant Mx f50::pHSU-mxn16::pHSU-mxn19 was hydrolyzed with EcoRV and subsequently re-ligated to generate pHSU-mxn26 (Fig. 3). Independently, it was also hydrolyzed with PacI/PvuI and subsequently re-ligated to generate pHSU-mxn27 (Fig. 3). The genomic DNA of mutant Mx f50::pHSU-mxn32 was hydrolyzed with PvuI and then re-ligated to generate pHSU-mxn35 (Fig. 4). An ampicillin cassette was amplified by PCR using primers mxn147/mxn148 with pJET1.2 vector as the template. pHSU-mxn35 was hydrolyzed with AflIIIPacI to replace the zeocin cassette with the amplified ampicillin cassette hydrolyzed with the same set of restriction enzymes to generate pHSU-mxn40 (Fig. 4).

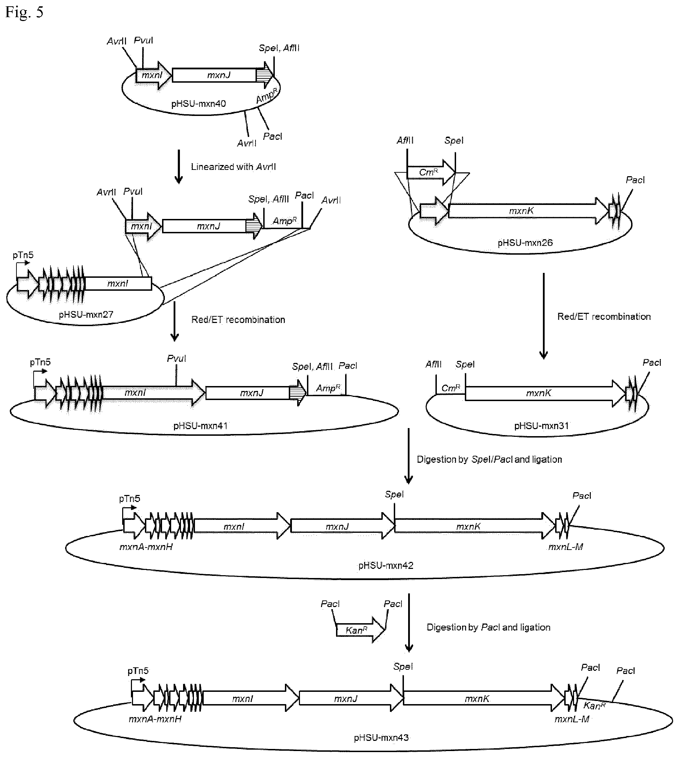

The cloning method for the generation of an expression construct harbouring the complete pathway is shown in Fig. 5. First, the Western chain biosynthesis pathway was completed by integration of the 18.5 kb AvrII fragment from pHSU-mxn40 into pHSU-mxn27 via Red/ET recombination. Another round of Red/ET recombineering was performed to exchange the 6.2 kb 3' end of mxnI in pHSU-mxn26 with a chloramphenicol resistance (CmR) cassette to generate pHSU-mxn31. Both, pHSU-mxn31 and pHSU-mxn41, were then hydrolyzed with SpeI/PacI to ligate the mxnK-M fragment into pHSU-mxn41 to generate pHSU-mxn42. A kanamycin resistance cassette was amplified from pCRII-TOPO vector as a template using primers mxn144/mxn145. The amplified cassette was then subcloned into pJET1.2 vector to obtain pHSU-mxn37. Both, pHSU-mxn37 and pHSU-mxn42 were hydrolyzed with PacI to ligate the kanamycin resistance cassette into pHSU-mxn42 to generate pHSU-mxn43.

For heterologous expression, the expression construct pHSU-mxn43 described above and shown in Fig. 5 was transformed into the expression host M. xanthus DK1622 ΔmchA-tet by the following procedure : M. xanthus DK1622 ΔmchA-tet was grown in baffle flasks with CTT medium supplemented with 6.25 µg/ml oxytetracycline at 30 °C until an OD600 between 0.6-0.9 was reached. Cells were then harvested from 2-4 ml culture (1-2 x 10⁹ cells/ml) by centrifugation at 12.500 rpm for 1 min at room temperature. After two washing steps with 1 ml H₂O at room temperature, cells were resuspended in 65 µl H₂O. 1-2 µg plasmid DNA was mixed with the cell suspension, and electroporation was carried out under the following conditions: 25 µF, 400 Ω, 650 V using 0.1 cm electroporation cuvettes and a GenePulser XCell device (Bio-Rad). 1 ml CTT medium was directly added to the cell suspension immediately after electroporation and the cells were transferred into a 2 ml centrifuge tube. After 5 h cultivation at 30 °C and 850 rpm on a thermomixer, the cells were mixed with 2 ml CY soft agar (CTT medium with 0.7 % agar) and plated on CTT agar plates (CTT medium with 1.5 % agar) supplemented with 50 µg/ml kanamycin. The plates were incubated at 30 °C for 7-10 days until colonies became visible. Selection was performed onto CTT agar plates containing 50 µg/ml kanamycin to isolate M. xanthus DK1622 ΔmchA-tet:: pHSU-mxn43 expression strain.

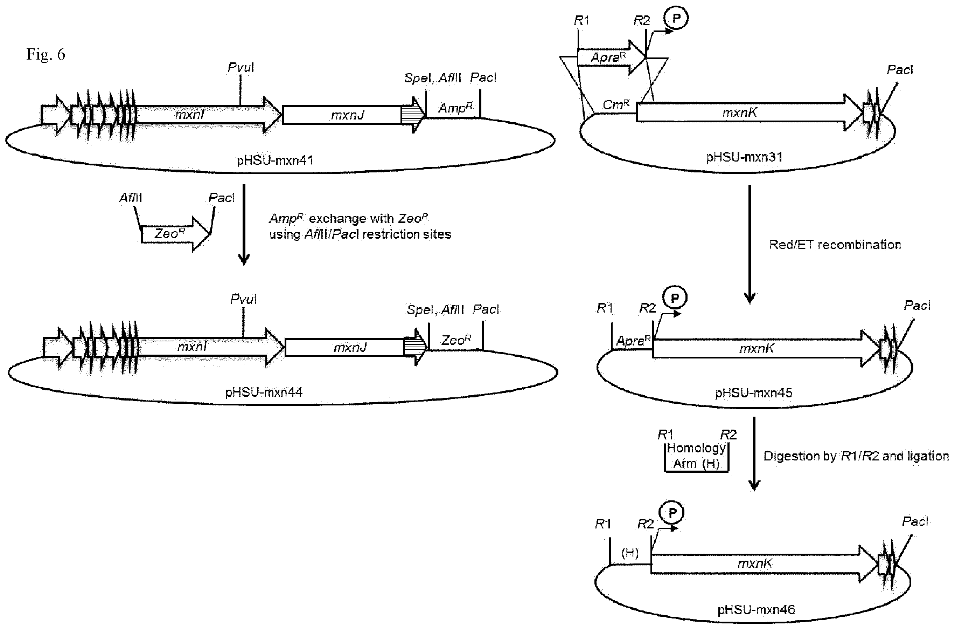

Alternatively, for construction of expression constructs harbouring parts of the synthetic pathway enzymes, a multiplasmid approach could be used. The cloning method for the generation of two compatible expression constructs harbouring two portions of the pathway is schematically depicted in Fig. 6. First, a suitable selection marker, exemplified by the zeocin resistance gene, zeoR, was ligated into pHSU-mxn41 using AflII/PacI restriction sites to generate the expression construct pHSU-mxn44. In parallel, construct pHSU-mxn31 was modified by Red/ET recombination to insert a suitable promotor upstream of mxnK together with another selection marker, exemplified here by the apramycin resistance cassette (ApraR), which was flanked by two unique restriction sites (R1 and R2) to generate plasmid pHSU-mxn45. The plasmid was further modified by ligation of a suitable homology region via the restriction sites R1/R2 to yield the second expression plasmid pHSU-mxn46.

For the multiplasmid method, the expression constructs pHSU-mxn44 and pHSU-mxn46 described above were transformed consecutively into M. xanthus DK1622 ΔmchA-tet according to the following procedure M. xanthus DK1622 ΔmchA-tet was grown in baffle flasks with CTT medium supplemented with 6.25 µg/ml oxytetracycline at 30 °C until an OD600 between 0.6-0.9 was reached. Cells were then harvested from 2-4 ml culture (1-2 x 10⁹ cells/ml) by centrifugation at 12.500 rpm for 1 min at room temperature. After two washing steps with 1 ml H₂O at room temperature, cells were resuspended in 65 µl H₂O. 1-2 µg plasmid DNA was mixed with the cell suspension, and electroporation was carried out under the following conditions: 25 µF, 400 Ω, 650 V using 0.1 cm electroporation cuvettes and a GenePulser XCell device (Bio-Rad). 1 ml CTT medium was directly added to the cell suspension immediately after electroporation and the cells were transferred into a 2 ml centrifuge tube. After 5 h cultivation at 30 °C and 850 rpm on a thermomixer, the cells were mixed with 2 ml CY soft agar (CTT medium with 0.7 % agar) and plated on CTT agar plates (CTT medium with 1.5 % agar) supplemented with 50 µg ml-1 kanamycin. The plates were incubated at 30 °C for 7-10 days until colonies became visible.

Selection was performed on CTT agar plates amended with 12.5 µg/ml zeocin for pHSU-mxn44 or 50 µg/ml kanamycin for pHSU-mxn46. Then, the respective second expression construct was transformed into the mutants to generate *M. xanthus* DK1622 ΔmchA-tet:: pHSU-mxn44/pHSU-mxn46 expression strains.

For heterologous production of myxopyronin, *M. xanthus* DK1622 ΔmchA-tet:: pHSU-mxn43 and *M. xanthus* DK1622 ΔmchA-tet:: pHSU-mxn44/pHSU-mxn46 were cultivated in CTT medium at 30 °C, e.g. in 300 ml baffle flasks with 50 ml medium for 2 days at 105 rpm. Following centrifugation, supernatant was extracted twice with an equal volume ethyl acetate, from the extract the ethyl acetate was evaporated, the residue was dissolved in 1 ml methanol to give the extract. A 5 µl aliquot of the extract was analyzed by HPLC-MS (Thermo Ultimate 3000 RSLC, coupled to a Bruker Daltonics Amazon ESI-MS ion trap instrument) operating in positive ionization mode. Compounds were separated on Waters Acquity BEH C18 (50 x 2.1 mm; 1.7 µm particle diameter; flow rate 600 µl/min and 45 °C) with a mobile phase of water/acetonitrile each containing 0.1 % formic acid, using a gradient from 5 % - 95 % acetonitrile over 9 min. Detection was by both diode array and ESI-MS. High Performance Liquid Chromatography-Mass Spectrometry (HPLC-MS) (Thermo Ultimate 3000 RSLC, coupled to a Bruker Daltonics Amazon electrospray ionization (ESI)-MS ion trap instrument) was used operating in positive ionization mode. Compounds were separated on a Waters Acquity BEH C18 column (50 x 2.1 mm; 1.7 µm particle diameter) at a flow rate of 600 µl min⁻¹ and 45 °C by a linear gradient with (A) H₂O + 0.1 % formic acid (FA) to (B) acetonitrile (ACN) + 0.1 % FA at a flow rate of 600 µl min⁻¹ and 45 °C. The gradient was initiated by a 0.33 min isocratic step at 5 % B, followed by an increase to 95 % B in 9 min to end up with a 1 min flush step at 95 % B before re-equilibration with initial conditions. Detection was carried out by both diode array (DAD) and ESI-MS. For high resolution mass spectrometry analysis, measurements were performed on a Dionex Ultimate 3000 RSLC system using a Waters BEH C18 column (50 x 2.1 mm, 1.7 µm dp) by injecting 5 µl of the methanolic extract. Separation was achieved by the same gradient as above with 0.33 min isocratic step at 5 % B. UV and MS detection were performed simultaneously. Coupling the HPLC to the MS was supported by an Advion Triversa Nanomate nano-ESI system attached to a Thermo Fisher Orbitrap. Mass spectra were acquired in centroid mode ranging from 200-2000 m/z at a resolution of R = 30000.

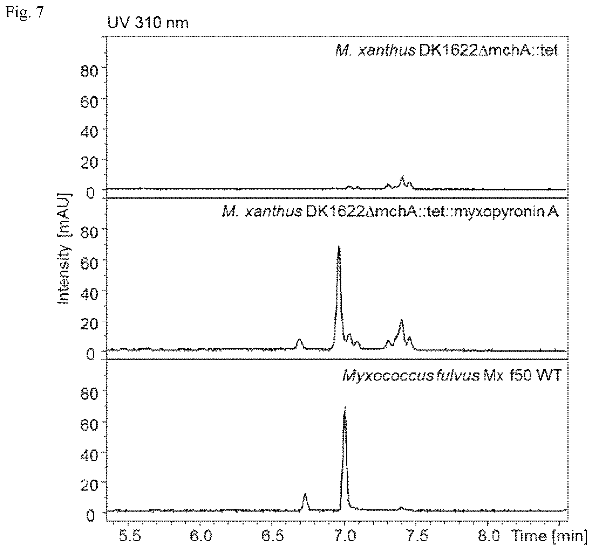

For comparison, the natural producer strain Myxococcus fulvus Mx f50 (wild-type, wt) and the non-transformed Myxococcus xanthus DK1622 ΔmchA-tet were cultivated and extracted in parallel. The results shown in Fig. 7 make it clear that non-transformed *M. xanthus* DK1622 ΔmchA-tet does not produce myxopyronin and that the transformant *M. xanthus* DK1622 ΔmchA-tet:: pHSU-mxn43 which harbours genes mxnA-mxnM (*M. xanthus* DK1622 ΔmchA-tet::myxopyronin A) produces myxopyronin A like the Myxococcus fulvus Mx f50 wild-type.

### Example 1 : Deletion of nucleic acid sequences in Myxococcus for inactivation of synthetic pathway genes

For introduction of nucleic acid sequences into Myxococcus or for deletion of nucleic acid sequences from Myxococcus, the following markerless procedure was preferably used: *M. fulvus* Mx f50, which was shown in Example 1 to contain the genes encoding the entire synthetic pathway enzymes, was grown in baffle flask with CY medium (0.3 % casitone, 0.1 % yeast extract, 0.1 % CaCl₂ x 2 H₂O) supplemented with 5 x 10⁻⁴ µg/L vitamin B12 at 30 °C until an OD600 between 0.6-0.9 was reached. Cells were then harvested from 2-4 ml culture (1-2 x 10⁹ cells/ml) by centrifugation at 12500 rpm for 1 min at room temperature. After two washing steps with 1 ml H₂O at room temperature, cells were resuspended in 65 µl H₂O. Plasmid DNA (1-2 µg) was mixed with the cell suspension, and electroporation was carried out under the following conditions: 25 µF, 400 Ω, 650 V using 0.1 cm electroporation cuvettes and a GenePulser XCell device (Bio-Rad). 1 ml CY medium was directly added to the cell suspension immediately after electroporation and the cells were transferred into a 2 ml centrifuge tube. After 6-8 h cultivation at 30 °C and 800 rpm on a thermomixer, the cells were mixed with 2 ml CY soft agar (CY medium with 0.7 % agar) and plated on CY agar plates (CY medium with 1.7 % agar) supplemented with 50 µg ml⁻¹ kanamycin or 6.25 µg ml⁻¹ oxytetracycline. The plates were incubated at 30 °C for 7-10 days until colonies became visible.

For the construction of markerless double cross-over mutants the following procedure was used: After verification of the single cross-over mutants (kanamycin-resistant mutants) by PCR, because integration via two different homology regions was possible, a selected single cross-over mutant was grown in CY medium in the absence of antibiotic. After 3-4 days 1 ml of the well-grown culture was transferred into 50 ml fresh medium to start another cultivation cycle at 30 °C for 3-4 days. This procedure was repeated three times to increase the possibility for a second cross-over event, which would result in the loss of the inactivating plasmid. Depending on the region of homology used for the second cross-over, this can yield either the wild-type genotype (revertant) or the expected mutant strain, in which the targeted region is being deleted (double cross-over mutant). To select for clones, in which this second cross-over reaction took place, a counter-selection system based on the sacB gene was used. For this, different dilutions of the cell population were plated on CY agar supplemented with 6 % sucrose for counter-selection. After 7-10 days, first colonies became visible, which were then grown in CY medium to isolate genomic DNA for genotypic verification and to extract the cultures for analysis for production of myxopyronin.

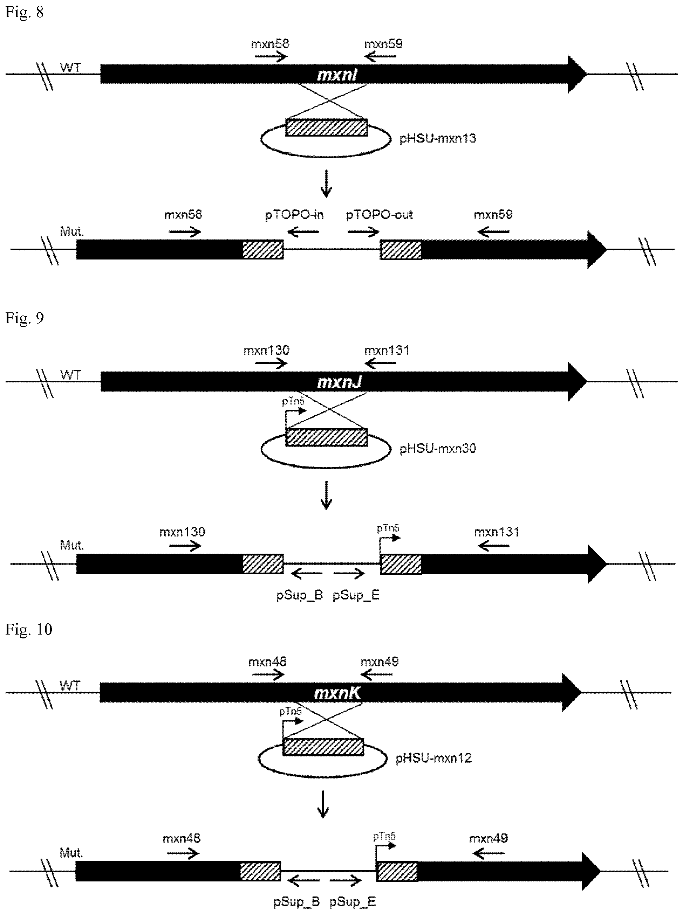

For inactivation of MxnI, insertional mutagenesis of its coding gene *mxnI* was employed, using knock-out plasmid pHSU-mxn13, which was constructed by amplification of a 1474 bp fragment for homologous recombination from *M. fulvus* Mx f50 genomic DNA by PCR using the primers mxn50 (SEQ ID NO: 29) and mxn51 (SEQ ID NO: 30) containing BamHI and EcoRV restriction sites. The resulting fragment was cloned into pCRII-TOPO vector to generate pHSU-mxn13. The plasmid was transformed into *M. fulvus* Mx f50 by electroporation. For genotypic analysis, a set of different PCR experiments using the primer combinations mxn58 (SEQ ID NO: 31)/pTOPO-in (SEQ ID NO: 32) and mxn59 (SEQ ID NO: 33)/pTOPO-out (SEQ ID NO: 34) was carried out to verify the correct integration of the inactivation plasmid. The cloning method is schematically depicted in Fig. 8. The resultant microorganism contained the synthetic pathway enzymes of MxnA-MxnB-MxnC-MxnD-MxnE-MxnF-MxnG-MxnH-MxnJ-MxnK-MxnL-MxnM.

For inactivation of MxnJ, insertional mutagenesis of its coding gene *mxnJ* was employed, using knock-out plasmid pHSU-mxn30, which was constructed by amplification of a 1439 bp fragment for homologous recombination was amplified from *M. fulvus* Mx f50 genomic DNA by PCR using the primers mxn120 (SEQ ID NO: 35) and mxn121 (SEQ ID NO: 36). In another PCR, the Tn5 promoter was amplified from the pCRII-TOPO vector backbone using the primers mxn33 (SEQ ID NO: 37) and mxn34 (SEQ ID NO: 38). After gel purification, both PCR products were fused by overlapping PCR using the primers mxn33 and mxn121. The resulting product (Tn5*-mxnJ* homologous fragment) was hydrolyzed by *Eco*RV and *Bam*HI and ligated into the pSupKan vector hydrolyzed by the same enzymes to generate pHSU-mxn30. The plasmid was transformed into *M. fulvus* Mx f50 by electroporation. For genotypic analysis, a set of different PCRs using the primer combinations mxn130 (SEQ ID NO: 39) /pSubB (SEQ ID NO: 40) and mxn131 (SEQ ID NO: 41) /pSupE (SEQ ID NO: 42) was carried out to verify the correct integration of the inactivation plasmid. The cloning method is schematically depicted in Fig. 9. The resultant microorganism contained the synthetic pathway enzymes of MxnA-MxnB-MxnC-MxnD-MxnE-MxnF-MxnG-MxnH-MxnI- MxnK-MxnL-MxnM.

For inactivation of MxnK, insertional mutagenesis of its coding gene *mxnK* was employed, using knock-out plasmid pHSU-mxn12, which was constructed by amplification of a 1448 bp fragment for homologous recombination from *M. fulvus* Mx f50 genomic DNA by PCR using the primers mxn35 (SEQ ID NO: 43) and mxn36 (SEQ ID NO: 44). In another PCR, the Tn5 promoter was amplified from the pCRII-TOPO vector backbone using the primers mxn33 and mxn34. After gel purification, both PCR products were fused by overlapping PCR using the primers mxn33 and mxn36. The resulting product (Tn5-mxnK homologous fragment) was hydrolyzed by EcoRV and BamHI and ligated into the pSupKan vector hydrolyzed by the same enzymes to generate pHSU-mxn12. For genotypic analysis, a set of different PCRs using the primer combinations mxn48/pSubB and mxn49/pSupE was carried out to verify the correct integration of the inactivation plasmid. This procedure is schematically shown in Fig. 10, indicating the position of the primers on the wild-type (WT) genome prior to the integration by cross-over, and following the integration in the mutant genome (Mut.). The resultant microorganism contained the synthetic pathway enzymes of MxnA-MxnB-MxnC-MxnD-MxnE-MxnF-MxnG-MxnH-MxnI-MxnJ-MxnL-MxnM.

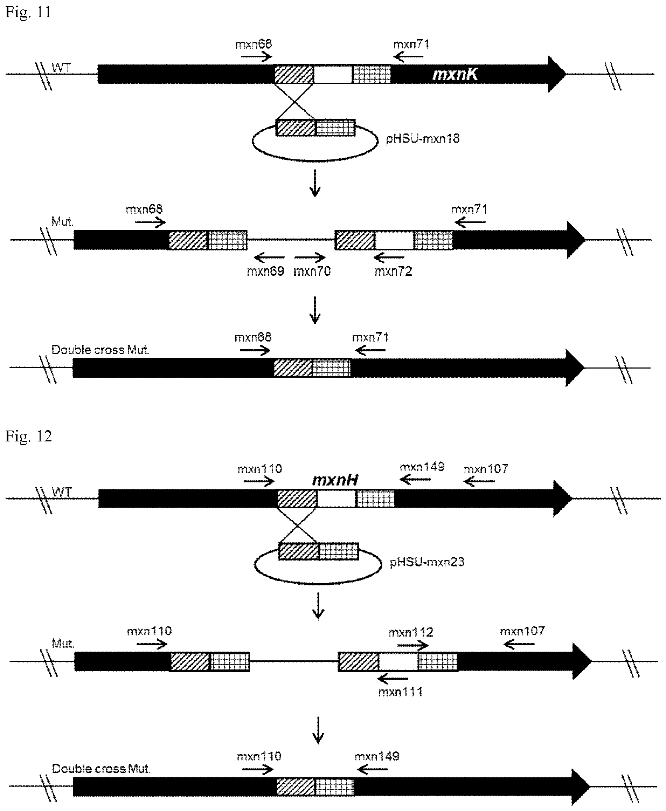

In the alternative, an enzyme was inactivated by inactivating the gene encoding it by in-frame deletion. This is shown on the example of *mxnK.* To disrupt *mxnK* by in-frame deletion, a gene inactivation plasmid harbouring two fragments was constructed. The fragments are 1001 bp and 1262 bp in size and homologous to the upstream and downstream area of the chromosomal target region. These fragments were amplified from *M. fulvus* Mx f50 genomic DNA by PCR using the primers mxn54 (SEQ ID NO: 45)/mxn55 (SEQ ID NO: 46) and mxn56 (SEQ ID NO: 47)/mxn57 (SEQ ID NO: 48). After hydrolysis of the upstream fragment (mxn54/mxn55 product) by BamHI and NotI, and of the downstream fragment (mxn56/57 product) by NotI and SacI, the fragments were ligated into pSWU41 previously hydrolyzed by BamHI and SacI to generate pHSU-mxn18. For genotypic analysis of the single cross-over, a set of different PCRs using the primer combinations mxn68 (SEQ ID NO: 49)/mxn69 (SEQ ID NO: 50), mxn70 (SEQ ID NO: 51)/mxn72 (SEQ ID NO: 52) and mxn73 (SEQ ID NO: 53)/mxn71 (SEQ ID NO: 54) was carried out to verify for correct integration of the inactivation plasmid. For genotypic analysis of the putative double cross-over mutants PCRs using the primers mxn68/mxn71 were carried out. Further confirmation of the deletion mutant was obtained by Southern Blot analysis. This cloning procedure is schematically shown in Fig. 11, indicating the position of the primers in the wild-type (WT) genome, in the genome mutated by the integration of pHSU-mxn18 (Mut.), and in the genome following the double cross-over (Double cross Mut.). The resultant microorganism contained the synthetic pathway enzymes of MxnA-MxnB-MxnC-MxnD-MxnE-MxnF-MxnG-MxnH-MxnI-MxnJ-MxnL-MxnM.

Inactivation of of MxnH could be obtained by in frame deletion of its coding gene *mxnH*. In detail, a gene inactivation plasmid harbouring two fragments, which are 1121 bp and 1250 bp in size and homologous to the upstream and downstream area of the chromosomal target region, was constructed. These fragments were amplified from *M. fulvus* Mx f50 genomic DNA by PCR using the primers mxn117/mxn118 and mxn83/mxn84. After hydrolysis of the upstream fragment (mxn117/mxn118 product) with *Pvu*I and *Not*I, and the downstream fragment (mxn83/84 product) with *Not*I and *Spe*I, the fragments were ligated into pSWU41 hydrolyzed with *Pvu*I and *Spe*I to generate plasmid pHSU-mxn23. For genotypic analysis of the single cross-over, a set of different PCRs using the primer combinations mxn110/mxn111 and mxn112/mxn107 was carried out to verify the correct integration of the inactivation plasmid. For genotypic analysis of the putative double cross-over mutants, PCRs using the primers mxn110/mxn111, mxn112/mxn107 and mxn110/mxn149 were carried out as schematically depicted in Fig. 12. The resultant microorganism contained the synthetic pathway enzymes of MxnA-MxnB-MxnC-MxnD-MxnE-MxnF-MxnG-MxnI-MxnJ-MxnK-MxnL-MxnM.

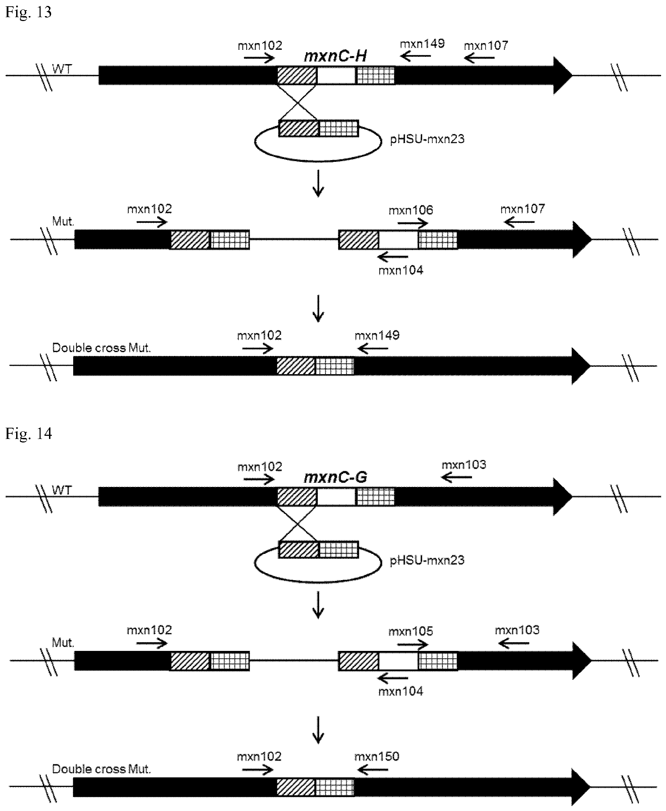

Inactivation of MxnC-MxnH could be obtained by in frame deletion of its coding genes *mxnC-mxnH*. In detail, for such a disruption of genes *mxnCDEFGH* by in-frame deletion, a gene inactivation plasmid harbouring two fragments, which are 1016 bp and 1237 bp in size and homologous to the upstream and downstream area of the chromosomal target region, was constructed. These fragments were amplified from *M. fulvus* Mx f50 genomic DNA by PCR using the primers mxn85/mxn119 and mxn83/mxn84. After hydrolysis of the upstream fragment (mxn85/mxn119 product) with *Pvu*I and *Not*I, and the downstream fragment (mxn83/84 product) with *Not*I and *Spe*I, the fragments were ligated into pSWU41 hydrolyzed with *Pvu*I and *Spe*I to generate pHSU-mxn22. For genotypic analysis of the single cross-over, a set of different PCRs using the primer combinations mxn102/mxn104 and mxn106/mxn107 was carried out to verify the correct integration of the inactivation plasmid. For genotypic analysis of the putative double cross-over mutants, PCRs using the primers mxn102/mxn104, mxn106/mxn107 and mxn102/mxn149 were carried out. The cloning method is schematically depticted in Fig.13. The resultant microorganism contained the synthetic pathway enzymes of MxnA-MxnB-MxnI-MxnJ-MxnK-MxnL-MxnM.

Inactivation of MxnC-MxnG could be obtained by in frame deletion of its coding genes *mxnC - mxnG*. In detail, for such a disruption of *mxnCDEFG* by in-frame deletion, a gene inactivation plasmid harbouring two fragments, which are 1016 bp and 1250 bp in size and homologous to the upstream and downstream area of the chromosomal target region, was constructed. These fragments were amplified from *M. fulvus* Mx f50 genomic DNA by PCR using the primers mxn85/mxn119 and mxn87/mxn88. After hydrolysis of the upstream fragment (mxn85/mxn119 product) with *Pvu*I and *Not*I, and the downstream fragment (mxn87/88 product) with *Not*I and *Bam*HI, the fragments were ligated into pSWU41 hydrolyzed with *Pvu*I and *Bam*HI to generate pHSU-mxn21. For genotypic analysis of the single cross-over, a set of different PCRs using the primer combinations mxn102/mxn104 and mxn105/mxn103 was carried out to verify the correct integration of the inactivation plasmid. For genotypic analysis of the putative double cross-over mutants, PCRs using the primers mxn102/mxn104, mxn105/mxn103 and mxn102/mxn150 were carried out. The cloning method is schematically depicted in Fig.14. The resultant microorganism contained the synthetic pathway enzymes of MxnA-MxnB-MxnH-MxnI-MxnJ-MxnK-MxnL-MxnM.

Production of myxopyronin was analysed from cultivation broth. Well-grown pre-cultures (OD₆₀₀ = 0.7-1.0) of *M. fulvus* Mx f50 or of mutants were used to inoculate production cultures in 300 ml baffled flask containing 50 ml CY medium (1 % inoculum). After cultivation for 2 days at 30 °C and 105 rpm, the cultures were harvested by centrifugation. Myxopyronin is almost exclusively present in the supernatant, which was extracted twice with an equal volume of ethyl acetate. After evaporation of the organic phase, the residue was dissolved in 1 ml methanol. A 5 µl aliquot of the extract was analyzed by HPLC-MS (Thermo Ultimate 3000 RSLC, coupled to a Bruker Daltonics Amazon ESI-MS ion trap detector) operating in positive ionization mode. Compounds were separated on a Waters Acquity BEH C18 column (50 x 2.1 mm; 1.7 µm particle diameter) at a flow rate of 600 µl/min and 45 °C by a linear gradient with (A) H₂O + 0.1 % FA (formic acid) to (B) ACN (aceto nitrile) + 0.1 % FA at a flow rate of 600 µl/min and 45 °C. The gradient was initiated by a 0.5 min isocratic step at 5 % B, followed by an increase to 95 % B in 9 min to end up with a 1 min flush step at 95 % B before re-equilibration with initial conditions. Detection was by both diode array and ESI-MS. For high resolution mass spectrometry, measurements were performed on a Dionex Ultimate 3000 RSLC system using a Waters BEH C18, 50 x 2.1 mm, 1.7 µm dp column by injection of 2 µl methanolic sample. Separation was achieved by a linear gradient with (A) H₂O + 0.1 % FA to (B) ACN + 0.1 % FA at a flow rate of 600 µl/min and 45 °C. The gradient was initiated by a 0.33 min isocratic step at 5 % B, followed by an increase to 95 % B in 9 min to end up with a 1 min flush step at 95 % B before re-equilibration with initial conditions. UV and MS detection were performed simultaneously. Coupling the HPLC to the MS was supported by an Advion Triversa Nanomate nano-ESI system attached to a Thermo Fisher Orbitrap. Mass spectra were acquired in centroid mode ranging from 200 - 2000 m/z at a resolution of R = 30000.

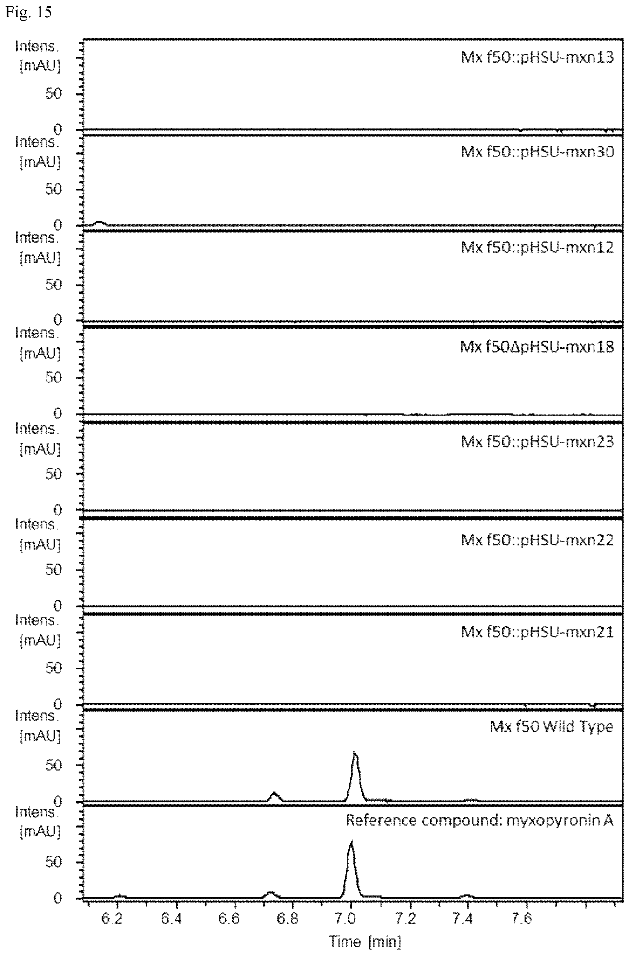

Results from HPLC-MS are shown in Fig. 15. In the wild-type producer strain (Mx f50 Wild Type), myxopyronin could be detected, but in extracts of the gene inactivation mutant strains obtained e.g. by insertional inactivation of *mxnI* (Mx f50::pHSU-mxn13), inactivation of *mxnJ* (Mx f50::pHSU-mxn30), inactivation of *mxnK* (Mx f50::pHSU-mxn12), inactivation by in frame deletion of *mxnK* (Mx f50::pHSU-mxn18), inactivation of *mxnH* by in frame deletion (Mx f50::pHSU-mxn23), inactivation of *mxnC-mxnH* by in frame deletion (Mx f50::pHSU-mxn22) no myxopyronin was found. For inactivation of *mxnC-mxnG* by in frame deletion (Mx f50::pHSU-mxn21), the myxopyronin derivative 21-desmethyl-myxopyronin A was found. This shows that the biosynthesis of native myxopyrin is interrupted by the inactivation of enzymes MxnI, MxnK, MxnC-MxnH of the biosynthetic pathway, whereas inactivation of MxnC-MxnG results in production of the 21-desmethyl derivative of myxopyronin A.

### Example 2: Deletion of nucleic acid sequences in Myxococcus for inactivation of synthetic pathway enzymes

For inactivation of enzymes, the Myxococcus producer *M. fulvus* Mx f50 of Example 2 was used, alternatively the M. xanthus DK1622 ΔmchA-tet::pHSU-mxn43 expression strain of Example 1 was used.

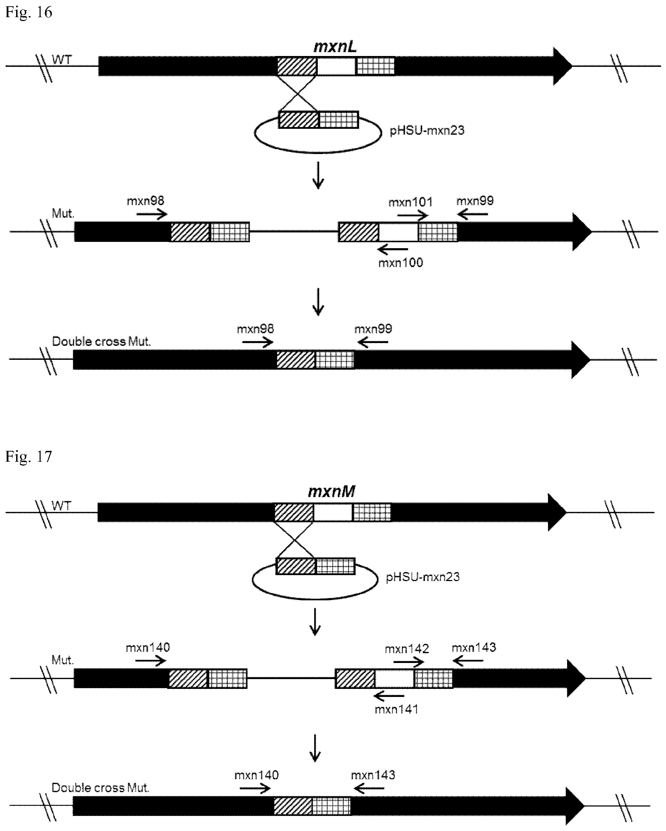

Inactivation of mxnL could be obtained by in frame deletion. In detail, a gene inactivation plasmid harbouring two fragments, which are 1232 bp and 1109 bp in size and homologous to the upstream and downstream area of the chromosomal target region, was constructed. These fragments were amplified from M. fulvus Mx f50 genomic DNA by PCR using the primers mxn113/mxn1 14 and mxn115/mxn1 16. After hydrolysis of the upstream fragment (mxn1 13/mxn1 14 product) with PvuI and NotI, and the downstream fragment (mxn115/116 product) with NotI and BamHI, the fragments were ligated into pSWU41 hydrolyzed with PvuI and BamHI to generate pHSU-mxn20. For genotypic analysis of the single cross-over, a set of different PCRs using the primer combinations mxn98/mxn100 and mxn101/mxn99 was carried out to verify the correct integration of the inactivation plasmid. The cloning process is schematically depicted in Fig. 16. Confirmation of the deletion mutant was obtained by Southern Blot analysis. The resultant microorganism contained the synthetic pathway enzymes of MxnA-MxnB-MxnC-MxnD-MxnE-MxnF-MxnG-MxnH-MxnI-MxnJ-MxnK-MxnM.

Inactivation of MxnM could be obtained by in frame deletion. In detail, a gene inactivation plasmid harbouring two fragments, which are 1110 bp and 1199 bp in size and homologous to the upstream and downstream area of the chromosomal target region, was constructed. These fragments were amplified from M. fulvus Mx f50 genomic DNA by PCR using the primers mxn132/mxn133 and mxn134/mxn135. After hydrolysis of the upstream fragment (mxn132/mxn133 product) with PvuI and SacI, and the downstream fragment (mxn134/135 product) with SacI and BamHI, the fragments were ligated into pSWU41 hydrolyzed with PvuI and BamHI to generate pHSU-mxn34. For genotypic analysis of the single cross-over, a set of different PCRs using the primer combinations mxn140/mxn141 and mxn142/mxn143 was carried out to verify the correct integration of the inactivation plasmid. For genotypic analysis of the putative double cross-over mutants, PCRs using the primers mxn140/mxn141, mxn142/mxn143 and mxn140/mxn143 were carried out. The cloning process is schematically depicted in Figure 17. The resultant microorganism contained the synthetic pathway enzymes of MxnA-MxnB-MxnC-MxnD-MxnE-MxnF-MxnG-MxnH-MxnI-MxnJ-MxnK-MxnL.

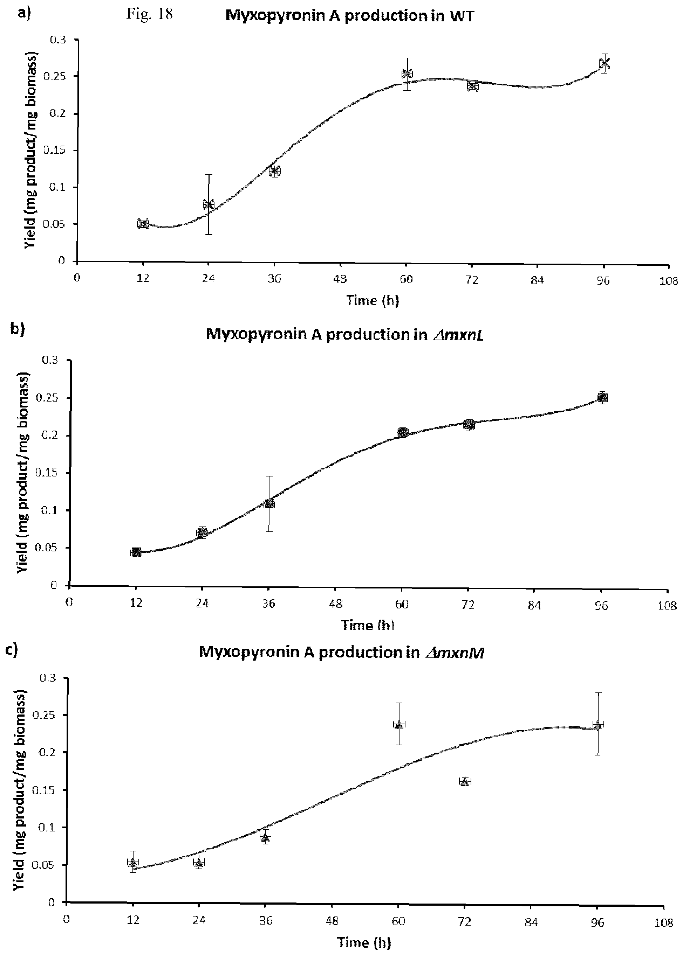

Extracts from supernatants of cultures of these microorganisms were produced and analysed on samples taken during the cultivation as described in Example 2. The results measured for myxopyronin A during the cultivation are shown in Fig. 18 a) for the wild-type *M. fulvus* Mx f50 (WT), in Fig. 18 b) for the microorganism containing the synthetic pathway enzymes of MxnA-MxnB-MxnC-MxnD-MxnE-MxnF-MxnG-MxnH-MxnJ-MxnJ-MxnK-MxnM (ΔmxnL), and in Fig. 18 c) for the microorganism containing the synthetic pathway enzymes of MxnA-MxnB-MxnC-MxnD-MxnE-MxnF-MxnG-MxnH-MxnI-MxnJ-MxnK-MxnL, with the gene mxnM encoding MxnM being deleted (ΔmxnM). These results show that these synthetic pathway enzymes suffice for synthesis of myxopyronin or its derivatives.

### Example3: Inactivation of catalytic centers of domains of synthetic pathway genes

Using the cloning methods described herein, preferably the double cross-over method using an inactivating nucleic acid construct, single or multiple catalytic centers of domains can be inactivated in enzymes, preferably by mutating the coding sequence of amino acids defining the catalytic center, e.g. catalytically active amino acids. The preferred amino acids to be changed for inactivation of a catalytic center, e.g. deleted or exchanged for a catalytically inactive amino acid, e.g. for Leucine, Isoleucine, Alanine, or Valine, are given in Table 3 below in bold face, which amino acids participate in the catalytic activity.

**Table 3: amino acids of catalytic centers**

| enzyme - catalytic center - domain | amino acids |
|---|---|
| keto synthase (KS) | |
| MxnI-KS-1E | SEAV**D**AA**C**ASSLVAL**H** (SEQ ID NO: 136)-----V**E**A**H**GTGT (SEQ ID NO: 137**)**-----**IGH**AE (SEQ ID NO: 138) , amino acids No. 694-885 of SEQ ID NO: 9 |
| MxnI-KS-2E | SEAV**D**TA**C**S**SS**LVA**IH** (SEQ ID NO: 139)-----I**ETH**GTGT(SEQ ID NO: 140)-----I**GH**LE **(**SEQ ID NO: 141) , amino acids No. 1945-2135 of SEQ ID NO: 9 |
| MxnI-KS-3E | SEPV**D**TL**C**S**SS**LVAI**H** (SEQ ID NO: 142)-----I**E**A**H**GTGT (SEQ ID NO: 143)-----V**GH**LE (SEQ ID NO: 144) , amino acids No. 3442-3633 of SEQ ID NO: 10 |
| MxnJ-KS-4E | SEA**ID**TA**C**S**SS**LVA**IH** (SEQ ID NO: 145)-—I**E**A**H**GTGT (SEQ ID NO: 146)-----I**GH**LE (SEQ ID NO: 147) , amino acids No. 605-795 of SEQ ID NO: 10 |
| MxnJ-KS-5E | SFAV**D**SA**C**SA**S**LTAI**H** (SEQ ID NO: 148)-----V**E**CAAAGS (SEQ ID NO: 149)-----V**GH**LE (SEQ ID NO: 150) , amino acids No. 2476-2660 of SEQ ID NO: 10 |
| MxnJ-KS-6E | SEPV**D**TA**C**S**SS**LIAVQ (SEQ ID NO: 151)-----I**E**A**H**GTGT (SEQ ID NO: 152)-----I**GH**LE (SEQ ID NO: 153) , amino acids No. 3208-3399 of SEQ ID NO: 10 |
| MxnK-KS-1W | SFTI**D**AA**C**A**SS**LVAL**H** (SEQ ID NO: 154)-----V**E**A**H**GTGT (SEQ ID NO: 155)-----I**GH**LE (SEQ ID NO: 156) , amino acids No. 180-364 of SEQ ID NO: 11 |
| MxnK-KS-2W | SLAV**D**TA**C**S**SS**LAA**IH** (SEQ ID NO: 157)-----V**E**CQATGT (SEQ ID NO: 158)-----I**GH**LE (SEQ ID NO: 159) , amino acids No. 1926-2110 of SEQ ID NO: 11 |
| MxnK-KS-3W | SEPC**D**TACS**SS**LIAI**H** (SEQ ID NO: 160)-----I**E**A**H**GSGT (SEQ ID NO: 161)-----I**GH**LE **(**SEQ ID NO: 162) , amino acids No. 2684-2875 of SEQ ID NO: 11 |
| MxnK-KS-4W | AMQV**D**TA**C**S**SS**LVAV**H** (SEQ ID NO: 163)-----V**E**A**H**GTGT (SEQ ID NO: 164)-----I**GH**TS (SEQ ID NO: 165) , amino acids No. 3504-3688 of SEQ ID NO: 11 |
| MxnK-KS-5W | SLMV**D**TACS**SS**LTAL**H** (SEQ ID NO: 166)-----V**E**A**H**GTGT (SEQ ID NO: 167)-----I**GH**LE (SEQ ID NO: 168) , amino acids No. 5409-5593 of SEQ ID NO: 11 |
| CorI-KS-1E | SEAV**D**AA**C**A**SS**LVAL**H** (SEQ ID NO: 169)-----V**E**A**H**GTGT (SEQ ID NO: 170)-----I**GH**AE (SEQ ID NO: 171) , amino acids No. 688-880 of SEQ ID NO: 23 |
| CorI-KS-2E | SEAV**D**TACS**SS**LVA**IH** (SEQ ID NO: 172)-----I**E**T**H**GTGT (SEQ ID NO: 173)-----I**GH**LE (SEQ ID NO: 174) , amino acids No. 1925-2115 of SEQ ID NO: 23 |
| CorI-KS-3E | SEPV**D**TLCS**SS**LVAI**H** (SEQ ID NO: 175)-----I**E**A**H**GTGT (SEQ ID NO: 176)-----V**GH**LE (SEQ ID NO: 177) , amino acids No. 3416-3607 of SEQ ID NO: 23 |
| CorJ-KS-4E | SEAI**D**TACS**SS**LVAI**H** (SEQ ID NO: 178)-----V**E**A**H**GTGT (SEQ ID NO: 179)-----I**GH**LE (SEQ ID NO: 180) , amino acids No. 586-776 of SEQ ID NO: 24 |
| CorJ-KS-5E | SFAV**D**SA**C**SA**S**LTAI**H** (SEQ ID NO: 181)-----V**E**CAAAGS (SEQ ID NO: 182)-----V**GH**LE **(**SEQ ID NO: 183) , amino acids No. 2448-2632 of SEQ ID NO: 24 |
| CorJ-KS-6E | SEPV**D**TA**C**S**SS**LVAVR (SEQ ID NO: 184)-----I**E**T**H**GTGT (SEQ ID NO: 185)-----I**GH**LE **(**SEQ ID NO: 186) , amino acids No. 3172-3363 of SEQ ID NO: 24 |
| CorK-KS-1W | SFTI**D**AA**C**A**SS**LVAL**H** (SEQ ID NO: 187)-----V**E**A**H**GTGT (SEQ ID NO: 188)-----I**GH**LE (SEQ ID NO: 189) , amino acids No. 171-355 of SEQ ID NO: 25 |
| CorK-KS-2W | SLAV**D**TA**C**S**SS**LVSI**H** (SEQ ID NO: 190)-----V**E**C**H**GTGT (SEQ ID NO: 191)-----I**GH**LE (SEQ ID NO: 192) , amino acids No. 1898-2082 of SEQ ID NO: 25 |
| CorK-KS-3W | SEPV**D**TA**C**S**SS**LVAVQ (SEQ ID NO: 193)-----I**E**A**H**GTGT (SEQ ID NO: 194)-----A**GH**LE (SEQ ID NO: 195) , amino acids No. 3362-3550 of SEQ ID NO: 25 |
| CorL-KS-4W | ALTL**D**TA**C**S**SS**LTALS (SEQ ID NO: 196)-----V**E**A**H**GTGT (SEQ ID NO: 197)-----I**GH**TT (SEQ ID NO: 198) , amino acids No. 449-633 of SEQ ID NO: 26 |
| CorL-KS-5W | SEPCNTACSSSLIA**IH** (SEQ ID NO: 199)-----I**E**A**H**GSGT (SEQ ID NO: 200)-----T**GH**LE (SEQ ID NO: 201) , amino acids No. 1678-1869 of SEQ ID NO: 26 |
| CorL-KS-6W | AMQL**D**TA**C**S**SS**LVAM**H** (SEQ ID NO: 202)-----V**E**A**H**GTGT (SEQ ID NO: 203)-----I**GH**TS (SEQ ID NO: 204) , amino acids No. 2484-2668 of SEQ ID NO: 26 |
| CorL-KS-7W | SLTV**D**TACS**SS**LTAL**H** (SEQ ID NO: 205)-----V**E**A**H**GTGT (SEQ ID NO: 206)-----I**GH**LE (SEQ ID NO: 207) , amino acids No. 4355-4539 of SEQ ID NO: 26 |

| ketoreductase (KR) | amino acids |
|---|---|
| MxnI-KR-2E | T**G**GA**GA**L**G** (SEQ ID NO: 208)-----HAAGQRQ**D**Q (SEQ ID NO: 209)-----FS**S**LVALTGNVGQTD**YA**FA**N**A (SEQ ID NO: 210) , amino acids No. 2903-3050 of SEQ ID NO: 9 |
| MxnJ-KR-4E | V**G**GT**GG**I**G** (SEQ ID NO: 211)-----HSALVLQ**D**A (SEQ ID NO: 212)----FS**S**AVGIAGGAGQSN**YA**AA**S**R (SEQ ID NO: 213) , amino acids No. 1568-1715 of SEQ ID NO: 10 |
| MxnK-KR-1W | V**G**GG**GG**L**G** (SEQ ID NO: 214)-----HLGGV**L**S**D**K (SEQ ID NO: 215)-----FS**SI**VSLLGNVGQAD**YA**AG**N**S (SEQ ID NO: 216) , amino acids No. 1418-1560 of SEQ ID NO: 11 |
| MxnK-KR-4W | V**G**GL**GG**L**G** (SEQ ID NO: 217)-----HSAIV**L**K**D**A (SEQ ID NO: 218)----FS**S**AESFICEAGQSN**YA**AA**S**R (SEQ ID NO: 219) , amino acids No. 4470-4617 of SEQ ID NO: 11 |
| CorI-KR-2E | T**G**GA**G**AL**G** (SEQ ID NO: 220)-----HAAGERR**D**A (SEQ ID NO: 221)-----FS**S**LVARTGNVGQTD**YA**FA**N**A (SEQ ID NO: 222) , amino acids No. 2874-3021 of SEQ ID NO: 23 |
| CorJ-KR-4E | V**G**GL**GG**I**G** (SEQ ID NO: 223**)**-----HSALVLQ**D**A **(**SEQ ID NO: 224**)-**---FS**S**AVGISGGAGQSH**YA**AA**S**R **(**SEQ ID NO: 225**)** , amino acids No. 1548-1695 of SEQ ID NO: 24 |
| CorK-KR-1W | V**G**GG**GG**I**G (**SEQ ID NO: 226**)**-----HLGGVLS**D**R **(**SEQ ID NO: 227**)-**---FS**S**IVALTGNVGQAD**YA**AG**N**S **(**SEQ ID NO: 228**)** , amino acids No. 1401-1543 of SEQ ID NO: 25 |
| CorK-KR-2W | T**G**GA**GG**LG (SEQ ID NO: 229)-----HGAGVRR**D**A (SEQ ID NO: 230)-----FS**S**VSAVLGNTGQGD**YA**YA**N**A (SEQ ID NO: 231) , amino acids No. 2826-2972 of SEQ ID NO: 25 |
| CorL-KR-4W | T**G**GV**GA**L**G** (SEQ ID NO: 232)-----HAAGN-LRS (SEQ ID NO: 233)----CS**S**VSSVLGNAGQGD**YA**YA**N**A (SEQ ID NO: 234) , amino acids No. 1139-1285 of SEQ ID NO: 26 |
| CorL-KR-6W | V**G**GL**GG**I**G** (SEQ ID NO: 235)-----HSAIVLK**D**A (SEQ ID NO: 236)---FS**S**AESFSCDAGQSN**YA**AA**S**R (SEQ ID NO: 237) , amino acids No. 3427-3574 of SEQ ID NO: 26 |

| carrier protein (CP) | amino acids |
|---|---|
| MxnI-CP-L | GGAHFD**S**ISL (SEQ ID NO: **238)** , amino acids No. 424-433 of SEQ ID NO: 9 |
| MxnI-CP-1E | G---FT**S**VTL (SEQ ID NO: 239) , amino acids No. 1720-1726 of SEQ ID NO: 9 |
| MxnI-CP-2E | G---YD**S**ISF (SEQ ID NO: 240) , amino acids No. 3192-3198 of SEQ ID NO: 9 |
| MxnJ-CP-3E | G---LT**S**LSL (SEQ ID NO: 241) , amino acids No. 366-372 of SEQ ID NO: 9 |
| MxnJ-CP-4E | G---VD**S**LIG (SEQ ID NO: 242) , amino acids No. 2238-2244 of SEQ ID NO: 10 |
| MxnJ-CP-5E | G---LD**S**IRV (SEQ ID NO: 243) , amino acids No. 2967-2973 of SEQ ID NO: 10 |
| MxnJ-CP-6E | G---LD**S**IMT (SEQ ID NO: 244) , amino acids No. 3810-3816 of SEQ ID NO: 10 |
| MxnJ-CP*-6E | G---FDQLAL (SEQ ID NO: 245) , amino acids No. 3566-3572 of SEQ ID NO: 10 |
| MxnK-CP-1W | G---LD**S**LIL (SEQ ID NO: 246) , amino acids No. 1683-1689 of SEQ ID NO: 11 |
| MxnK-CP-2W | G---FD**S**ITF (SEQ ID NO: 247) , amino acids No. 2429-2435 of SEQ ID NO: 11 |
| MxnK-CP-3W | G---LD**S**ILA (SEQ ID NO: 248) , amino acids No. 3234-3240 of SEQ ID NO: 11 |
| MxnK-CP-4W | G---VD**S**LVN (SEQ ID NO: 249) , amino acids No. 5165-5171 of SEQ ID NO: 11 |
| MxnK-CP*-5W | G---VDHLDM (SEQ ID NO: 250) , amino acids No. 5894-5900 of SEQ ID NO: 11 |
| MxnK-CP-5W | G---LD**S**INV (SEQ ID NO: 251) , amino acids No. 5984-5990 of SEQ ID NO: 11 |
| CorI-CP-L | SGAHFD**S**ISL (SEQ ID NO: 252) , amino acids No. 416-425 of SEQ ID NO: 23 |
| CorI-CP-1E | G---**F**N**S**VTL (SEQ ID NO: 253) , amino acids No. 1706-1712 of SEQ ID NO: 23 |
| CorI-CP-2E | G---YD**S**ISF (SEQ ID NO: 254) , amino acids No. 3166-3172 of SEQ ID NO: 23 |
| CorJ-CP-3E | G---LS**S**VAL (SEQ ID NO: 255) , amino acids No. 350-356 of SEQ ID NO: 24 |
| CorJ-CP-4E | G---VD**S**LLG (SEQ ID NO: 256) , amino acids No. 2206-2212 of SEQ ID NO: 24 |
| CorJ-CP-5E | G---LD**S**MRV (SEQ ID NO: 257) amino acids No. 2935-2941 of SEQ ID NO: 24 |
| CorJ-CP*-6E | G---FGPHAL (SEQ ID NO: 258) , amino acids No. 3530-3536 of SEQ ID NO: 24 |
| CorJ-CP-6E | G---LD**S**IMT (SEQ ID NO: 259) , amino acids No.3765-3771 of SEQ ID NO: 24 |
| CorK-CP-1W | G---LD**S**LIL (SEQ ID NO: 260) , amino acids No. 1663-1669 of SEQ ID NO: 25 |
| CorK-CP-2W | G---FD**S**ITF (SEQ ID NO: 261) , amino acids No. 3109-3115 of SEQ ID NO: 25 |
| CorK-CP*-3W | G---LDPVLA (SEQ ID NO: , amino acids No. 3900-3906 of SEQ ID NO: 25 |
| CorK-CP-3W | G---LD**S**VLG (SEQ ID NO: 263) , amino acids No. 3995-4000 of SEQ ID NO: 25 |
| CorL-CP-4W | G---FD**SI**IL (SEQ ID NO: 264) , amino acids No. 1443-1449 of SEQ ID NO: 26 |
| CorL-CP-5W | G---LD**SI**LA (SEQ ID NO: 265) , amino acids No. 2208-2214 of SEQ ID NO: 26 |
| CorL-CP-6W | G---VD**S**LVN (SEQ ID NO: 266) , amino acids No. 4116-4122 of SEQ ID NO: 26 |
| CorL-CP*-7W | G---VDHFDM (SEQ ID NO: 267) , amino acids No. 4826-4832 of SEQ ID NO: 26 |
| CorL-CP-7W | G---LD**S**INV (SEQ ID NO: 268) , amino acids No. 4920-4926 of SEQ ID NO: 26 |

| dehydratase (DH) | amino acids |
|---|---|
| MxnI-DH-2E | **L**RD**H**VIG**G**KKLL**P** (SEQ ID NO: 269) , amino acids No. 2402-2414 of SEQ ID NO: 9 |
| MxnJ-DH-3E | **L**RD**H**TVF**G**QRVLL (SEQ ID NO: 270) , amino acids No. 53-65 of SEQ ID NO: 9 |
| MxnJ-DH-4E | **L**RD**H**VVGSRGVL**P** (SEQ ID NO: 271) , amino acids No. 1091-1103 of SEQ ID NO: 10 |
| MxnK-DH-1W | **I**RD**H**VVG**G**NRLI**P** (SEQ ID NO: 272) , amino acids No. 955-967 of SEQ ID NO: 11 |
| MxnK-DH-4W | VSQ**H**QVH**G**RPVF**P** (SEQ ID NO: 273) , amino acids No. 3980-3992 of SEQ ID NO: 11 |
| CorI-DH-2E | **L**RD**H**EIG**G**KRIL**P** (SEQ ID NO: 274) , amino acids No. 2380-2392 of SEQ ID NO: 23 |
| CorJ-DH-3E | **L**RD**H**TVL**G**QRVLL (SEQ ID NO: 275) , amino acids No. 44-56 of SEQ ID NO: 24 |
| CorJ-DH-4E | **L**RD**H**VVGSHGVL**P** (SEQ ID NO: 276) , amino acids No. 1073-1085 of SEQ ID NO: 24 |
| CorK-DH-1W | **L**RD**H**VVG**G**HRLI**P** (SEQ ID NO: 277) , amino acids No. 942-954 of SEQ ID NO: 25 |
| CorK-DH-2W | **L**AD**H**VLH**G**EPTL**P** (SEQ ID NO: 278) , amino acids No. 2319-2331 of SEQ ID NO: 25 |
| CorL-DH-4W | **L**NA**H**EVF**G**RPLF**P** (SEQ ID NO: 279) , amino acids No. 19-31 of SEQ ID NO: 26 |
| CorL-DH-6W | VTQ**H**QVH**G**QPVM**P** (SEQ ID NO: 280) , amino acids No. 2949-2961 of SEQ ID NO: 26 |

| methyltransferase (MT) | amino acids |
|---|---|
| MxnJ-MT-4E | V**E**V**G**A**G** (SEQ ID NO: 281) , amino acids No. 1982-1987 of SEQ ID NO: 10 |
| MxnK-MT-4W | L**E**V**G**A**G** (SEQ ID NO: 282) , amino acids No. 4914-4919 of SEQ ID NO: 11 |
| CorJ-MT-4E | V**E**V**G**A**G** (SEQ ID NO: 283) , amino acids No. 1962-1967 of SEQ ID NO: 24 |
| CorL-MT-6W | L**E**V**G**A**G** (SEQ ID NO: 284) , amino acids No. 3865-3870 of SEQ ID NO: 26 |

In Table 3, --- denotes gaps inserted into the amino acid sequences for better alignment of similar amino acids during computer-assisted analysis. For KS and KR alignments, the gaps inserted comprise more than 3 amino acids. In Table 3, the amino acids sections given as amino acid Nos. of the amino acid sequence of an enzyme comprises or defines the respective enzyme section containing the domain. The amino acid alignment for catalytic centers shows the importance of conserved amino acids, which are printed in bold. Accordingly, mutation of the coding sequence to change, e.g. elemination at least one of these amino acids, preferably two or three of these conserved amino acids or all of the amino acids given for a catalytic center, can be used for inactivation of the respective domain, preferably of the module indicated.

Examples for cloning methods for inactivating one specific catalytic center are given in Example 7 below for the domain CP of modules 1W and 4W, respectively.

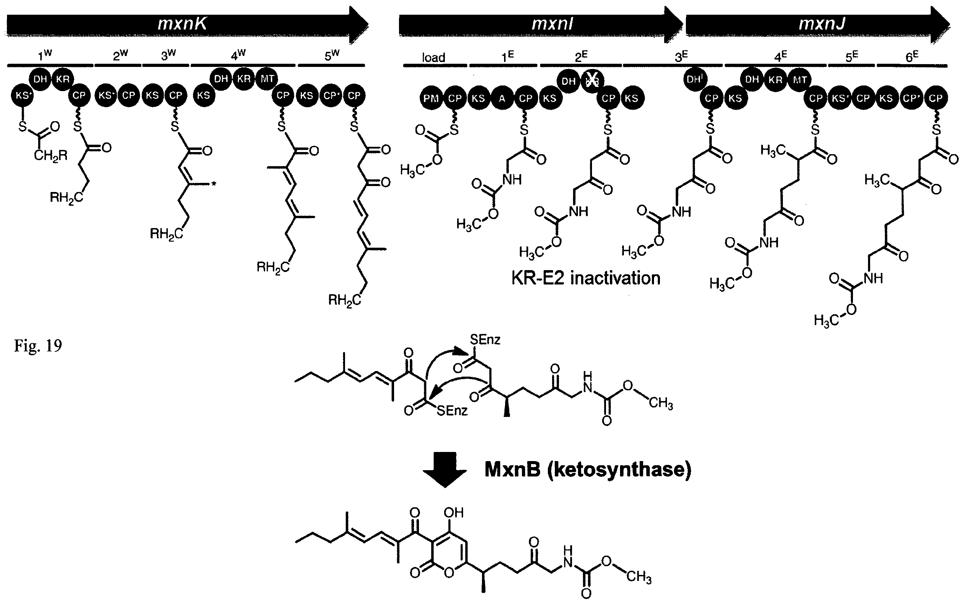

Preferably, for production of a myxopyronin derivative, at least one ketoreductase (KR) catalytic center is inactivated. As an example Fig. 19 schematically shows the effect of inactivation of the KR domain of module 2E results in the production of a myxopyronin derivative. The inactivation of the KR domain of module 2E results in the β-ketoester intermediate not being reduced to the hydroxy ester intermediate, and accordingly the β-ketoester group occurs in the side chain to the pyrone ring.

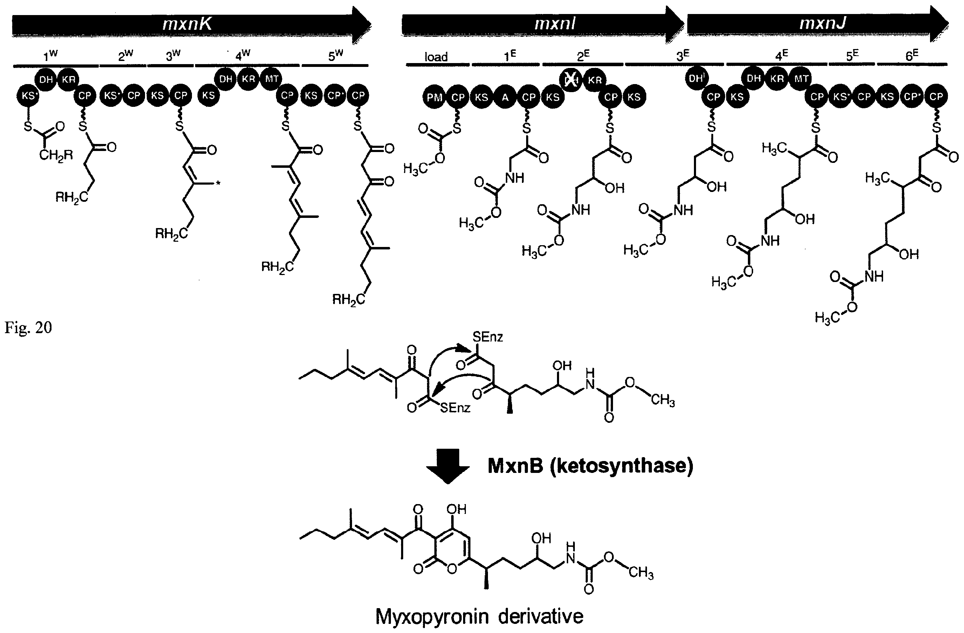

Preferably, for production of a myxopyronin derivative, at least one catalytic center of a dehydrogenase (DH) catalytic domain is inactivated. As an example Fig. 20 schematically shows the effect of inactivation of the DH domain of module 2E results in the production of a myxopyronin derivative. The inactivation of the DH domain of module 2E results in the hydroxyester intermediate not being dehydrated, and accordingly the β-hydroxyl group occurs in the side chain to the pyrone ring.

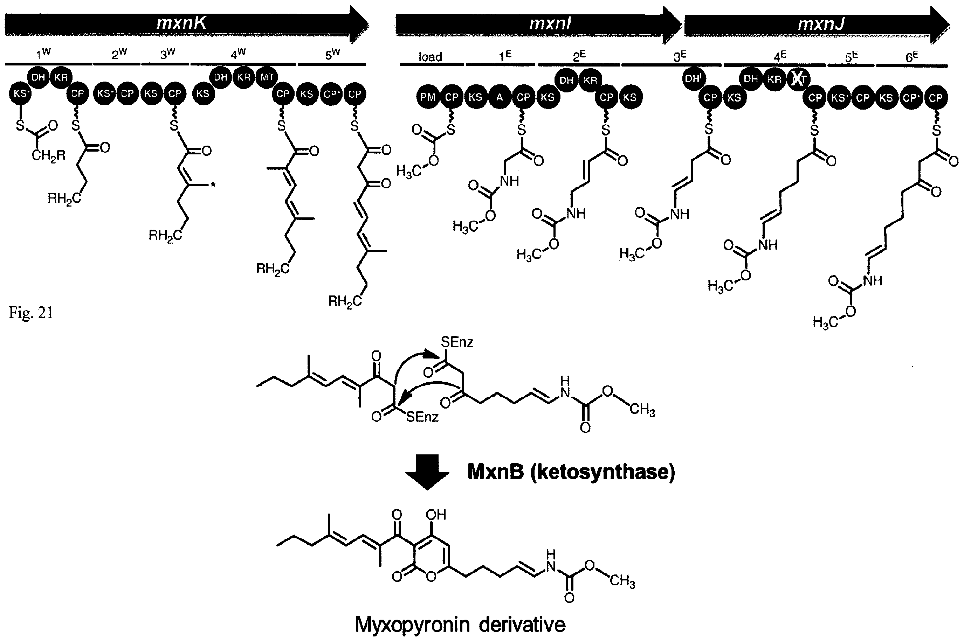

Preferably, for production of a myxopyronin derivative, the catalytic center of at least one methyltransferase (MT) domain is inactivated. As an example Fig. 21 schematically shows the effect of inactivation of the MT domain of module 4E results in the production of a the effect of inactivation of the MT domain of module 4E results in the production of a myxopyronin derivative. The inactivation of an MT domain, e.g. of module 4W results in the absence of a methyl group in the side chain to the pyrone ring, in this case in the absence of the methyl group bound to the α-carbon.

### Example 4: Production of myxopyronin derivatives using enzymes in in vitro process

Production of myxopyronin or a derivative was catalysed in vitro by MxnB in combination with the catalytic center of domain CP of module 6E of MxnJ (CP-6E) or with the catalytic center CP of domain 5W of MxnK (CP-5W) from precursors JHS486 and JHS274. This shows that MxnB catalyses the condensation of precursors to form the pyrone ring moiety having the chains of the precursors. In presence of the catalytic center of the CP domain of module 6E of MxnJ and in presence of the catalytic center of the CP o domain of module 5W of MxnK, respectively, the transfer of the diketo moiety of each precursor to MxnB forming the pyrone ring occurs, indicating that derivatives of myxopyronin can be produced by these peptides from precursors which are activated, e.g. activated by carrying an auxiliary group, e.g. like the SNAC group, linked to the diketo group.

The peptides were expressed from separate expression cassettes: For expression of MxnB, the coding nucleic acid sequence *mxnB* was amplified from *M. fulvus* Mx f50 genomic DNA by PCR using primers mxn9 and mxn10. The PCR products were cloned into into pJET1.2 blunt (Thermo Fisher Scientific) vector resulting in pJET-MxnB and sequenced. The resultant pJET-HSU-mxn5 was digested with *Nde*I and *Hind*III and the DNA fragment harboring the MxnB encoding region was cloned into the *Nde*I and *Hind*III site of pColdI resulting in pColdI-HSU-mxn5, encoding MxnB with an N-terminal hexahistidine tag.

The coding sequence for CP-6E (CP domain of module 6E) was amplified from *M. fulvus* Mx f50 genomic DNA by PCR using the primers mxn214 and mxn215. The PCR products were cloned into into pJET1.2 blunt (Thermo Fisher Scientific) vector resulting in pJET-CP-6E and sequenced. pJET-HSU-mxn59 was digested with *Nde*I and *Hind*III, and the DNA fragment harboring CP-6E encoding region was cloned into the *Nde*I and *Hind*III site of pColdI, resulting in pColdI-HSU-mxn59 with an N-terminal hexahistidine tag. To obtain soluble CP-6E, a chaperone was co-expressed using pGro7 to obtain pColdI-pGro7-mxn59. Independently, pColdI-HSU-mxn59 was co-expressed with pSUMtaA to obtain pColdI-pSUMtaHSU-mxn64.

The coding sequence for CP-5W (CP domain of module 5W) was amplified from *M. fulvus* Mx f50 genomic DNA by PCR using the primers mxn230 and mxn231. The PCR products were cloned into into pJET1.2 blunt (Thermo Fisher Scientific) vector resulting in pJET-MxnB and sequenced using the primers pJET1.2For/pJET1.2Rev, respectively. pJET-HSU-mxn63 was digested with *Nde*I and *Hind*III and the DNA fragment harboring CP-5W encoding region was cloned into the *Nde*I and *Hind*III site of pColdI resulting in pColdI-HSU-mxn63 with an N-terminal hexahistidine tag. Furthermore, pColdI-HSU-mxn63 was co-expressed with pSUMtaA to obtain pColdI-pSUMtaHSU-mxn65.

MxnB, the catalytic center of the CP domain of module 6E of MxnJ (CP-6E) and the catalytic center of the CP domain of module 5W of MxnK (CP-5W) were each separately produced by heterologous expression. The respective expression plasmids pHSU-mxn5, pHSU-mxn57, pHSU-mxn59, and pHSU-mxn63, were introduced into Escherichia coli BL21(DE3) in separate transformations. The E. coli BL21(DE3) carrying the expression plasmids were cultured in LB medium containing 100 µg/mL of ampicillin (for pColdI expression) or modified LB medium containing 100 µg/mL of ampicillin, 25 µg/mL of chloroamphenicol and 2 mg/mL 1-arabinose (for pColdI-pGro7 expression) at 37°C until OD₆₀₀ = 0.5 to 0.6. The expression was induced by addition of isopropyl-β-d-thiogalactopyranoside (IPTG, 0.15 mM) and cultivation was continued at 16°C for 20 h. The cells were harvested by centrifugation (8,000 rpm ; 20 min) and resuspended in lysis buffer (40 mM Tris-HCl buffer pH 7.5 containing glycerol 10%, 300 mM NaCl and 10 mM imidazole). Cells were lyzed by sonication and a cell-free extract was prepared by removing cell debris by centrifugation at 8,000 rpm for 10 min at 4°C. The cell-free extract was purified by using Ni-NTA Superflow resin (Qiagen), washed with buffer containing imidazole (20 mm), and eluted by imidazole (200 mm) in the same buffer. The elution buffer was exchanged with buffer (40 mm Tris-HCl buffer pH 7.5 containing glycerol 10%, 300 mM NaCl) using ultrafiltration (Amicon, Milipore). Protein concentrations were estimated by the Bradford assay (Bio-Rad) with bovine serum albumin as a standard. The purified proteins were flash frozen in liquid nitrogen and stored at -80°C.

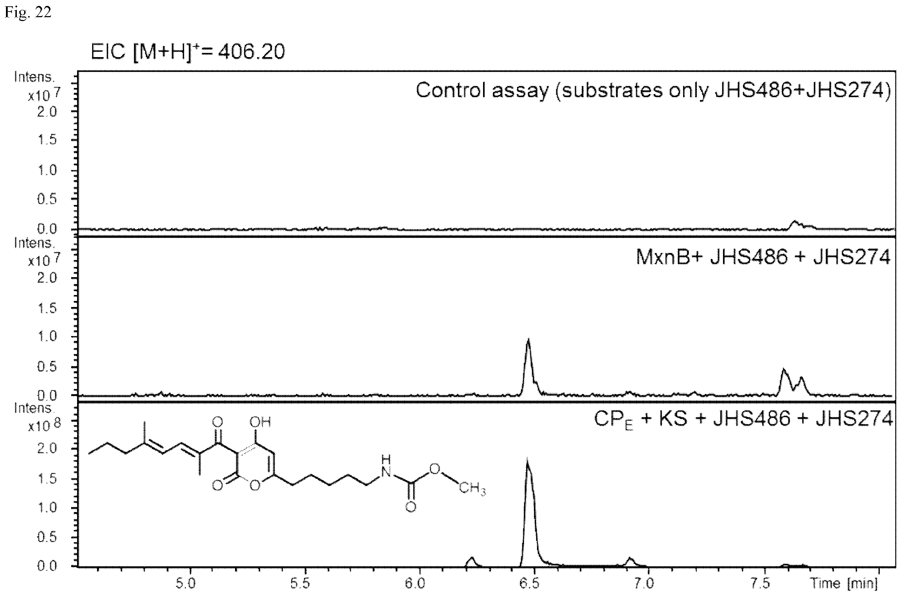

For the reaction a mixture (total of 100 µL) containing 100 µM CP-6E or CP-5W, 30 µM MxnB, 1 mM JHS274 and 1 mM JHS486 was prepared and incubated at 37°C for 8 h. The reaction mixtures were extracted with ethyl acetate and the organic layer was evaporated. The residue was dissolved in 100 µl methanol and a 5 µl aliquot of the extract was analyzed by residue was dissolved in 100 µl methanol and a 5 µl aliquot of the extract was analyzed by HPLC/MS as described in Example 2. Results from HPLC-MS are shown in Fig. 22, indicating that the precursors in the presence of MxnB only are condensed to a pyrone ring, and that in the presence of MxnB and domain CP of module 6E and/or domain CP of module 5W, the precursors are condensed to a pyrone ring (structural formula inset) carrying the side chain linked to SNAC in JHS486 as the Western chain and carrying the side chain linked to SNAC in JHS274 as the Eastern chain.

### Example 5: Production of myxopyronin derivatives using enzymes with MxnK inactivated and Western chain precursor feeding

The block mutant resulting from inactivation of MxnK by markerless mutagenesis of *mxnK* (*M.fulvus* Mx f50 ΔpHSU-mxn18, containing pHSU-mxn18 described in Example 2), has been found not to produce myxopyronin A. For the mutasynthesis experiments, a pre-culture of *M*. *fulvus* Mx f50ApHSU-mxn18 was grown in CY medium at 30 °C until an OD₆₀₀ between 0.3 to 0.6. The pre-culture was used to inoculate 20 mL CY medium (7.2 x 10⁶ cells/mL). Cultivation was carried out for further 12-24 hours before feeding precursor acting as a substrate mimic. *N*-acetyl-cysteamine (SNAC)-esters that were used as a Western chain precursor each were fed to the different cultures to a final concentration of 100 µM. They were further cultivated for another 48 h before the cells were harvested.

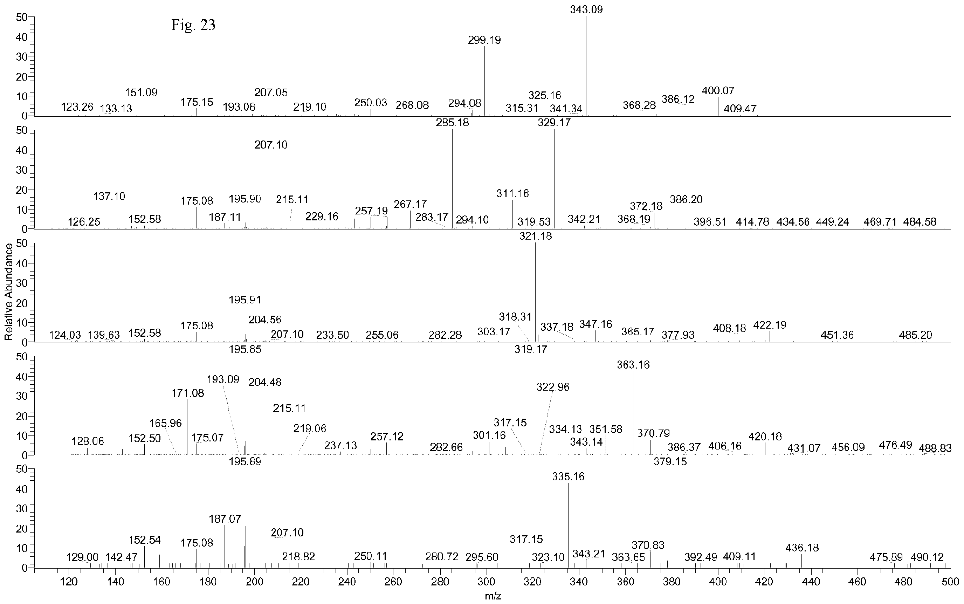

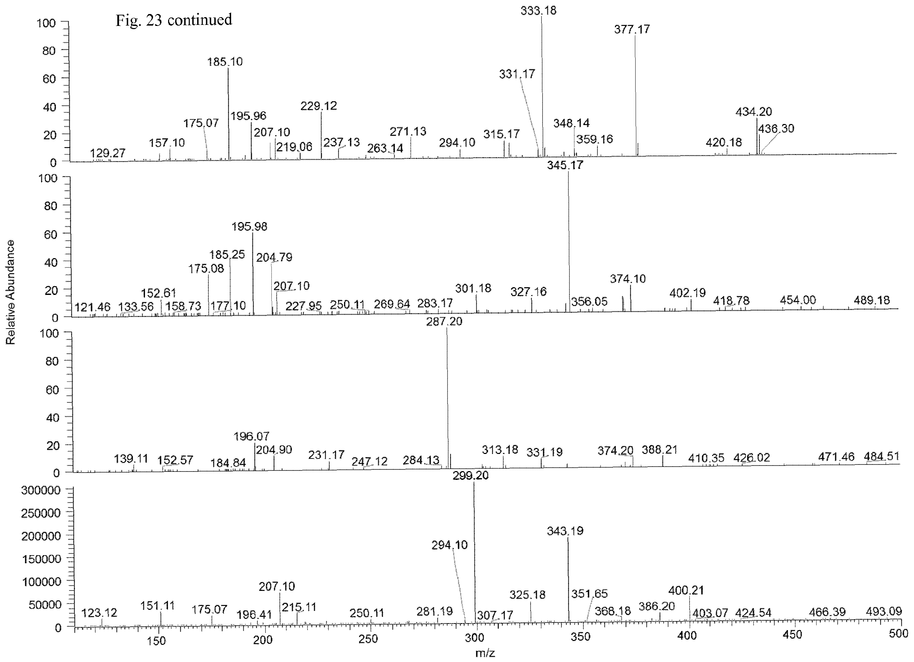

The precursors and the respective myxopyronin derivatives found are given in Table 4 below. MS² spectra of product in culture supernatant extracts are shown in Fig. 23. Analysis of the crude extract was performed by HPLC/MS as described above. Measurement by high-resolution mass spectrometry confirmed the incorporation of the chains of the precursors by showing the same manner of fragmentation pattern.

**Table 4: The following Western precursor compounds were added to separate cultures each, and the following myxopyronin derivatives were identified due to their fragmentation losses in HPLC-MS:**

| Western chain precursor added (name) | product (*m*/*z*) | Fragmentation due to losses of | | |
|---|---|---|---|---|
| | | H₂O (*m*/*z* = 18) | CH₃OH (*m*/*z* = 32) | H₂N-CO₂CH₃, CO₂ (*m*/*z* = 119) |
| (comparative: wild-type M. fulvus) | myxopyronin A (418.22) | 400.07 | 386.12 | 299.19 |

| **Feeding to *Myxococcus fulvus* Mx f50 ΔpHSU-mxn18 (Δ*mxnK*)** | | | | |
|---|---|---|---|---|
| 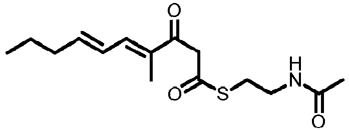 | 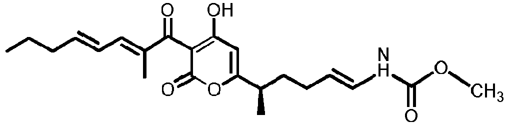 | 386.20 | 372.18 | 285.18 |
| (mgr41) | (404.18) | | | |
| 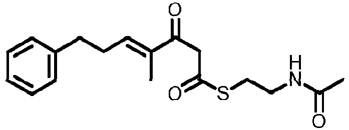 | 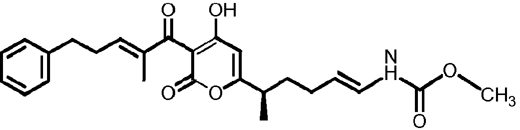 | 422.19 | 408.18 | 321.18 |
| (mgr171) | (440.20) | | | |
| 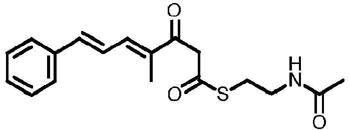 | 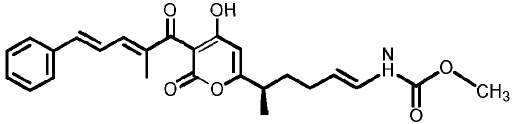 | 420.18 | 406.16 | 319.17 |
| (JHS206) | (438.18) | | | |
| 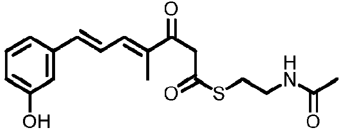 | 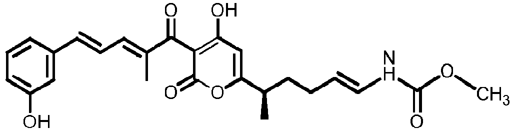 | 436.18 | 422.43 | 335.16 |
| (JHS226) | (454.18) | | | |
| 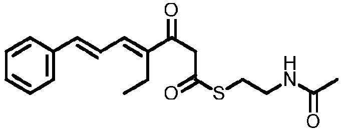 | 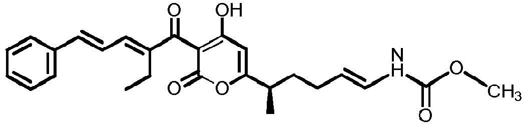 | 434.20 | 420.18 | 333.18 |
| (JHS246) | (452.20) | | | |
| 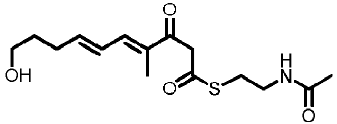 | 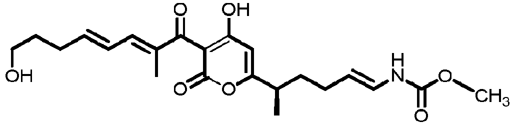 | 402.19 | 388.17 | 345.1 |
| (JHS266) | (420.20) | | | |
| 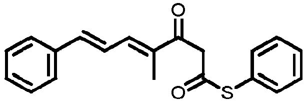 | 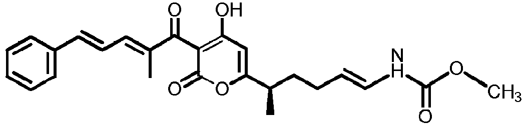 | 420.18 | 406.16 | 319.17 |
| (JHS289) | (438.18) | | | |
| 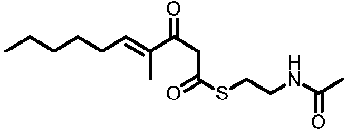 | 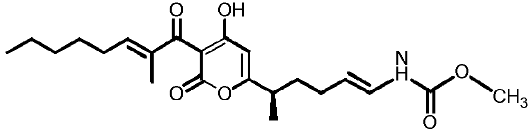 | 388.21 | 374.20 | 331.19 |
| (JHS291) | (406.22) | | | |
| 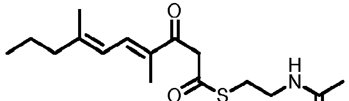 | myxopyronin A (418.22) | 400.07 | 386.12 | 299.19 |
| (JHS486) | | | | |

| **Feeding to *Myxococcus fulvus* Mx f50 ΔpHSU-mxn48 (Δ*mxnK-CP-1 W*)** | | | | |
|---|---|---|---|---|
| 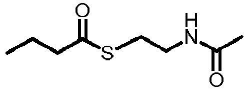 | myxopyronin A (418.22) | 400.07 | 386.12 | 299.19 |
| (JHS401) | | | | |
| 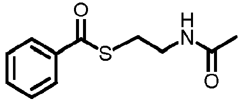 | 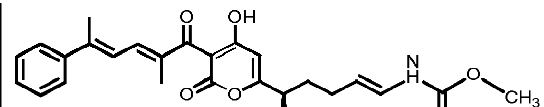 | 434.2 | 420.18 | 333.18 |
| (JHS417) | (452.2) | | | |

| **Feeding to *Myxococcus fulvus* Mx f50 ΔpHSU-mxn49 (*ΔmxnK-CP-4 W*)** | | | | |
|---|---|---|---|---|
| 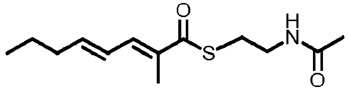 | 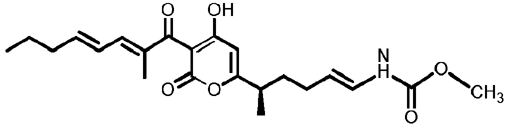 | 386.20 | 372.18 | 285.18 |
| (JHS282) | (404.18) | | | |
| 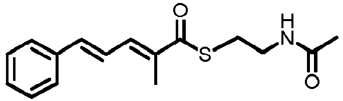 | 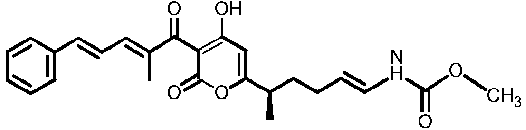 | 420.18 | 406.16 | 319.17 |
| (JHS283) | (438.18) | | | |
| 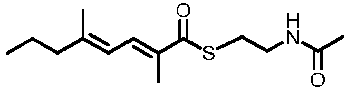 | myxopyronin A (418.22) | 400.07 | 386.12 | 299.19 |
| (JHS415) | | | | |

These results show that myxopyronin derivatives can be produced by expression of the synthetic enzymes, wherein MxnK is inactivated, e.g. the enzymes provided consist of MxnA, MxnI, MxnJ, MxnK, and optionally comprising MxnL and MxnM, e.g. by expression in a microorganism, in combination with adding a Western chain precursor.

For production of myxopyronin derivatives, MxnK could be inactivated at one of its catalytic domains, e.g. at a CP domain, in combination with feeding a Western chain precursor. In this embodiment, the remaining portion of MxnK converts the Western chain precursor to a converted Western chain precursor which is then condensed to the Eastern chain by MxnB, resulting in a myxopyronin derivative having a Western chain originating from the converted Western chain precursor.

### Example 6: Production of myxopyronin derivatives using enzymes with one catalytic center of MxnK inactivated and Western chain precursor feeding

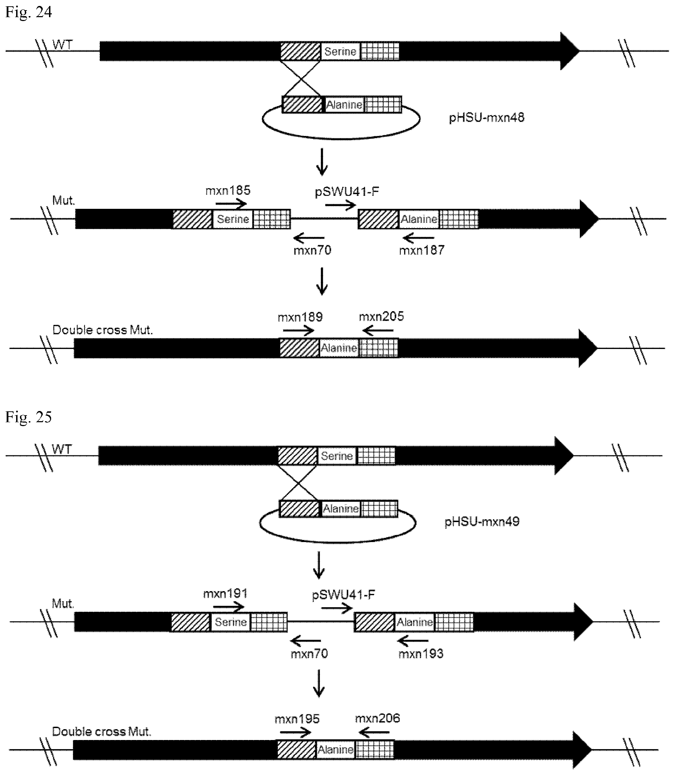

As an example for inactivation of one catalytic center, a CP (carrier protein) catalytic domain was inactivated in different modules of MxnK. MxnK could be partially inactivated, e.g. by inactivating the CP catalytic domain of module 1W of MxnK. For inactivating this catalytic domain of MxnK, a point mutation was induced in its coding sequence mxnK using a gene inactivation plasmid harbouring 2445 bp fragment. The fragment was amplified from pHSU-mxn43 plasmid of Example 1 by overlap PCR using the primers mxn169/mxn170 and mxn171/mxn172. After hydrolysis of the fragment with PvuI and NotI, it was ligated into pSWU41 hydrolyzed with PvuI and NotI to generate pHSU-mxn48. For genotypic analysis of the single cross-over, a set of different PCRs using the primer combinations mxn185/mxn70 and pSWU41-F/mxn187 was carried out to verify the correct integration of the inactivation plasmid. For genotypic analysis of the double cross-over, a PCR using primer combination mxn189/mxn205 was performed and the amplified product was sequenced. The cloning method is schematically shown in Fig. 24.

MxnK could be partially inactivated at catalytic domain CP of its module 4W by point mutation. For inactivating mxnK at domain CP of module 4W, a gene inactivation plasmid harbouring a 2486 bp fragment was constructed. The fragment was amplified from pHSU-mxn43 plasmid by overlap PCR using the primers mxn174/mxn175 and mxn176/mxn177. After hydrolysis of the fragment with PvuI and NotI, it was ligated into pSWU41 hydrolyzed with PvuI and NotI to generate pHSU-mxn49. For genotypic analysis of the single cross-over, a set of different PCRs using the primer combinations mxn191/mxn70 and pSWU41-F/mxn193 was carried out to verify the correct integration of the inactivation plasmid. For genotypic analysis of the double cross-over, PCR using primer combination mxn195/mxn206 was performed and the amplified product was sequenced. The cloning method is schematically shown in Fig. 25.

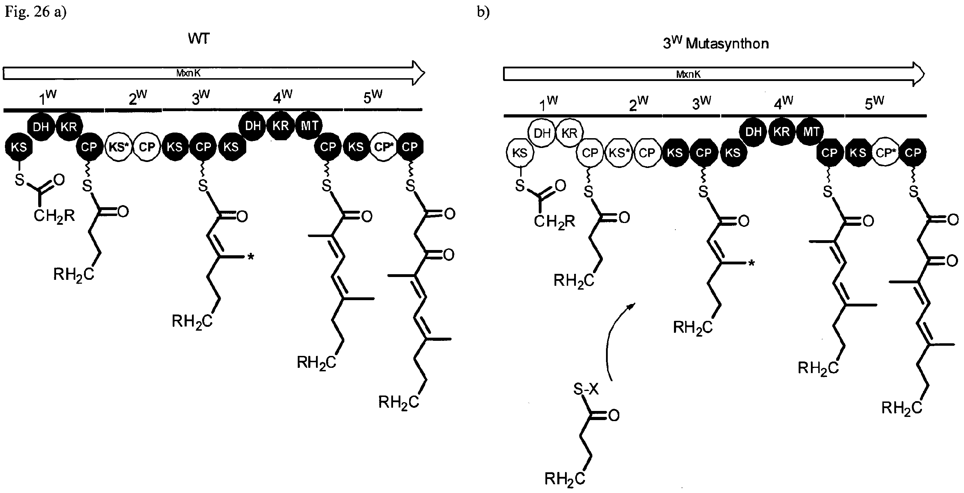

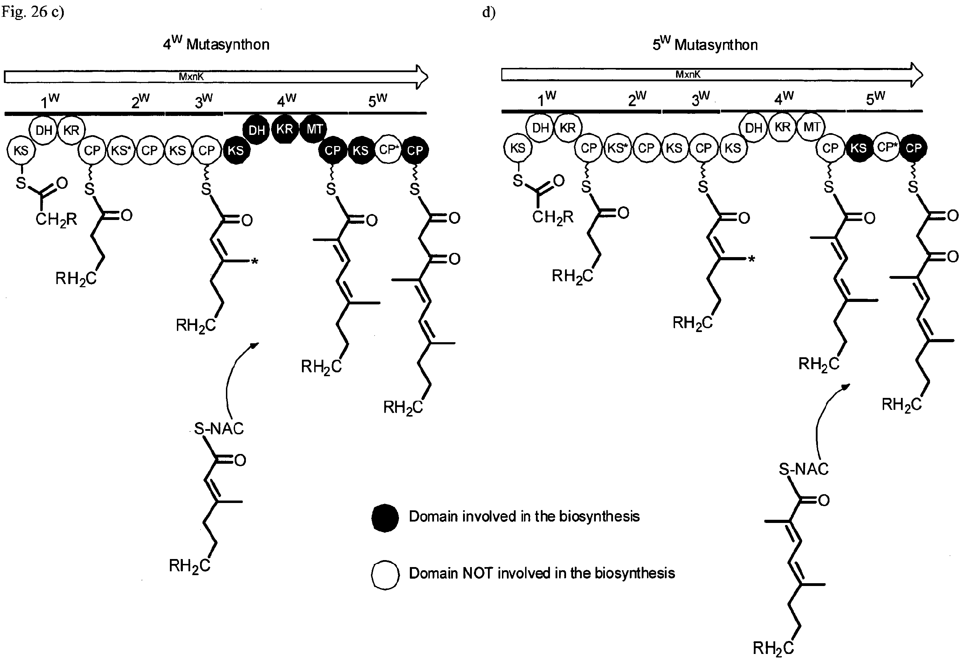

For production of myxopyronin derivatives, a block mutant of at least one carrier protein (CP) catalytic domain of MxnK inactivated was cultivated with feeding a Western chain precursor. The Western chain precursor can be converted by catalytic centers arranged C-terminally to the inactivated catalytic center. As examples, Fig. 26 in a) schematically shows the wild-type synthesis of the Western chain molecule portion, in b) MxnK with the CP domain of module 1W inactivated with feeding of a Western chain precursor that is bound to the CP domain of module 3W and is subsequently converted by modules 3W to 5W before being condensed into the pyrone ring, in c) MxnK with the CP domain of module 3W inactivated with feeding of a Western chain precursor that is bound to the CP domain of module 4W and is subsequently converted by modules 4W to 5W before being condensed into the pyrone ring, and in d) MxnK with the CP domain of module 4W inactivated with feeding of a Western chain precursor that is bound to the CP domain of module 5W and is subsequently converted by module 5W before being condensed into the pyrone ring. Generally, especially in Figures 26 - 28, enzymes in which at least one catalytic domain is inactivated can be termed Mutasynthon.

### Example 7: Production of myxopyronin derivatives using enzymes with one catalytic center of MxnI-MxnJ inactivated and Eastern chain precursor feeding

As shown above, inactivation of MxnJ in *M. fulvus* Mx f50 ::pHSU-mxn30 abolishes production of myxopyronin A. As an example for inactivation of one catalytic domain, a CP (carrier protein) catalytic domain was inactivated in different modules of MxnI-MxnJ.

Fig. 27 schematically shows in a) the wild-type (wt) synthesis of the Eastern chain molecule portion, in b) MxnI-MxnJ having an inactivated CP domain in the N-terminal load module and the binding of the fed Eastern chain precursor by the CP domain of module 1E and its subsequent conversion by the following modules of MxnI-MxnJ, in c) MxnI-MxnJ having an inactivated CP domain in module 1E and the binding of the fed Eastern chain precursor by the CP domain of module 2E and its subsequent conversion by the following catalytic centers of MxnI-MxnJ, in d) MxnI-MxnJ having an inactivated CP domain in module 3E and the binding of the fed Eastern chain precursor by the CP domain of module 4E and its subsequent conversion by the following modules of MxnI-MxnJ, and in e) MxnI-MxnJ having an inactivated CP domain in the module 4E and the binding of the fed Eastern chain precursor by the CP domain of module 6E and its subsequent condensation to the pyrone ring, i.e. its condensation without further conversion. In the alternative to inactivation of the CP domain in module 4E, the CP domain in module 5E can be inactivated.

For production, a pre-culture of *M*. *fulvus* Mx f50::pHSU-mxn30 was grown in CY medium at 30 °C until an OD₆₀₀ between 0.3-0.6. The pre-culture was used to inoculate 20 mL CY medium (7.2 x 10⁶ cells/mL). Cultivation was carried out for further 12-24 hours before feeding the substrate mimics. *N*-acetyl-cysteamine (SNAC)-esters mimicking Eastern chain to the cultures to the final 100 µM. They were further grown for another 48 hours before the cells were harvested. Analysis of the crude extract was performed by HPLC/MS.

### Example 8: Production of myxopyronin derivatives using enzymes with one CP catalytic center of MxnK inactivated and Western chain precursor feeding and one catalytic center of MxnI-MxnJ inactivated

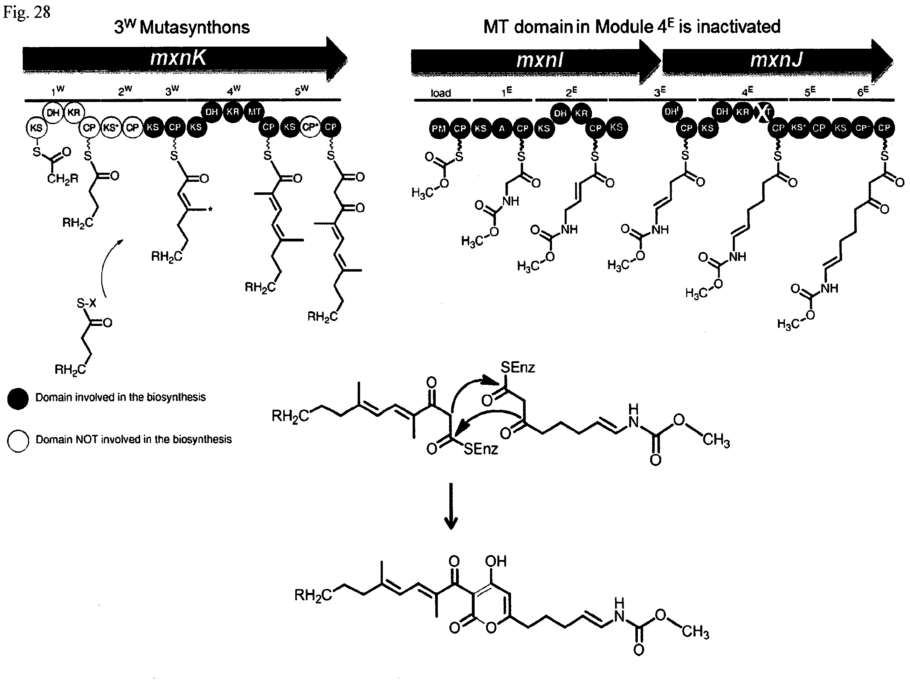

As an example for the combination of the second and third embodiments, *M. fulvus* Mx f50 ::pHSU-mxn48, wherein the CP catalytic domain of module 1W of MxnK is inactivated, is used in combination with inactivation of at least one catalytic domain of MxnI-MxnJ. The inactivation of at least one catalytic domain of MxnI-MxnJ is exemplified by the inactivation of the MT catalytic domain in module 4E of MxnJ. Fig. 28 schematically shows the synthesis of the Western chain starting from the Western chain precursor that is bound by the CP catalytic domain of module 3 W and subsequently converted by the following catalytic domains of modules 4W-5W, whereas the synthesis of the Eastern chain molecule portion is altered by the lack of the activity of the MT domain in module 4E. The condensation of the converted Western chain precursor to the altered Eastern chain molecule portion is catalysed by MxnB.

### Example 9: Production of myxopyronin derivatives using enzymes with one catalytic center of MxnK inactivated and one CP catalytic center of MxnI-MxnJ inactivated with Eastern chain precursor feeding

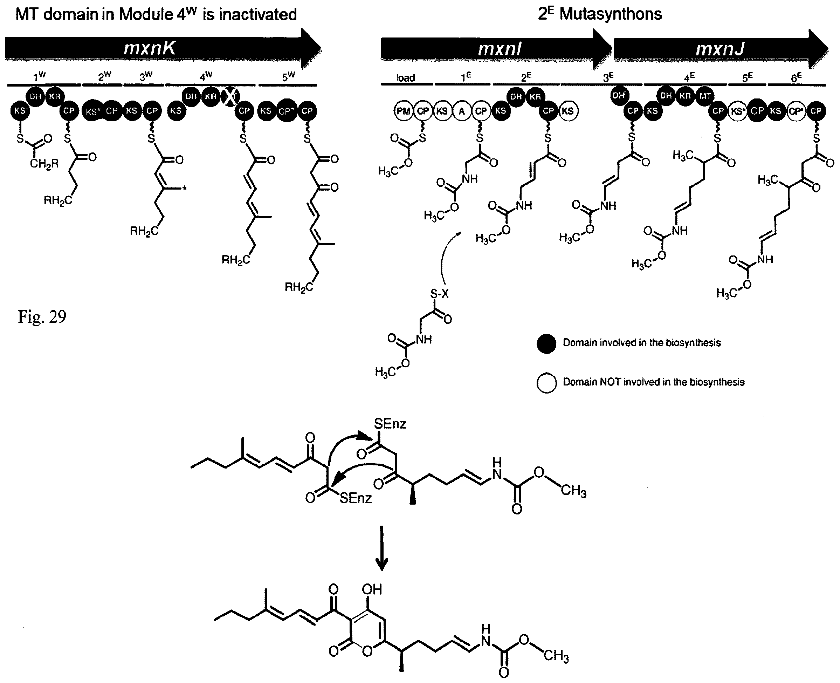

As an example for the combination of the second and third embodiments, *M. fulvus* Mx f50, in which the MT catalytic domain of module 4W of MxnK is inactivated is described in combination with inactivation of the CP catalytic domain of module 1E of MxnI-MxnJ, with feeding of an Eastern chain precursor during cultivation. The synthetic pathway of the resultant myxopyronin derivative is schematically depicted in Fig. 29, wherein the Western chain molecule portion is altered due to the absence of the activity of the methyl transferase and wherein the Eastern chain precursor is bound by CP catalytic domain of module 2E and is subsequently converted by the following modules of MxnI-MxnJ, followed by the condensation to the pyrone ring as catalysed by MxnB.

### Example 10: Production of myxopyronin derivatives using enzymes, wherein at least one catalytic center or domain or enzyme of MxnI-MxnJ-MxnK is exchanged for at least one catalytic center or domain or enzyme of CorI-CorJ-CorK-CorL

Heterologous production of corallopyronins in Myxococcus xanthus DK1622 The cloning method for the generation of an expression construct harbouring the "hybrid" pathway is illustrated in Figure 8. The eastern chain biosynthesis pathway is pHSU-mxn41 (consisting of mxnA-mxnJ) from myxopyronin pathway and the western part including tailoring genes (corK-corO) generated from a corallopyronin cosmid library AM24 and BA5.

First, an apramycin resistance cassette was amplified by PCR using primers mxn200/mxn201 with pKC1132 vector as the template. The fragment Z (∼13 kb) in cosmid BA5 was exchanged with the apramycin resistance cassette (ApraR) via Red/ET recombineering to generate pHSU-mxn51. In parallel, another apramycin resistance cassette amplified by PCR using primers mxn202/mxn203 with pKC1132 vector as the template. The fragment Y in cosmid AM24 was then exchanged with the apramycin resistance cassette (ApraR) via Red/ET recombineering to generate pHSU-mxn52. Another round of Red/ET recombineering was performed to exchange fragment X in cosmid AM24 with a chloroamphenicol resistance (CmR) cassette to generate pHSU-mxn53. The next round of Red/ET recombination was then performed to integrate corK* fragment from pHSU-mxn53 into pHSU-mxn51 to generate pHSU-mxn54. Both pHSU-mxn41 and pHSU-mxn54 were then hydrolyzed with SpeI/PacI to ligate the corK-O fragment into pHSU-mxn41 to generate pHSU-mxn55.

Subsequently, both pHSU-mxn37 and pHSU-mxn42 are to be hydrolyzed with PacI to ligate the kanamycin resistance cassette into pHSU-mxn55 to generate pHSU-mxn56. The expression construct pHSU-mxn56 described above shown in Figure 8 can then be transformed into an expression host, e.g. M. xanthus DK1622 ΔmchA-tet by electroporation as described above.

### Precursors could be obtained as follows :

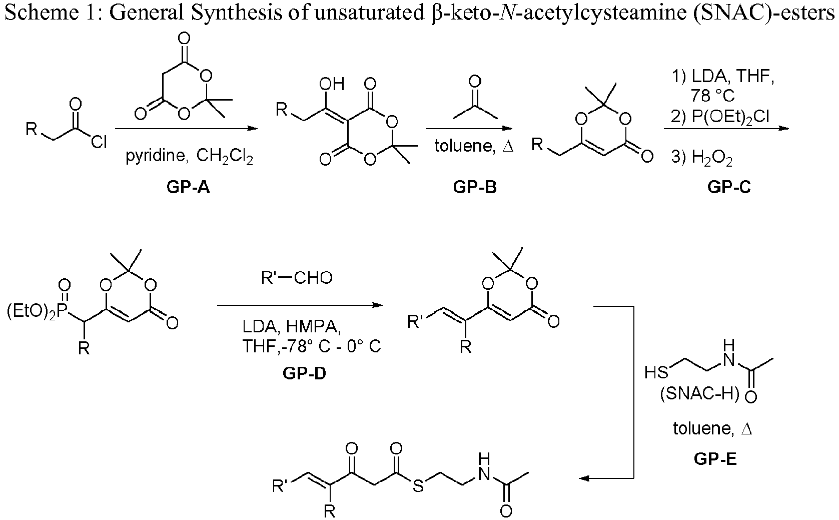

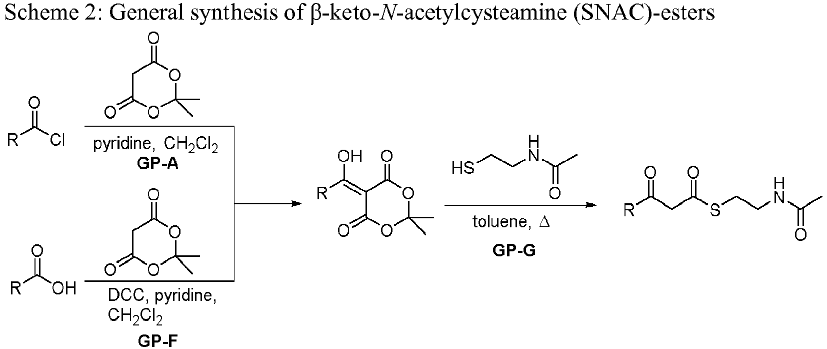

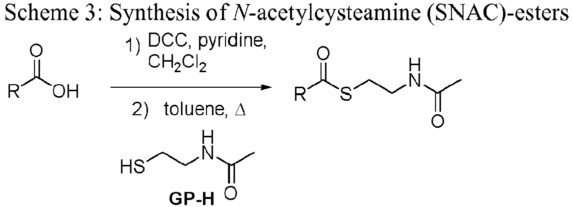

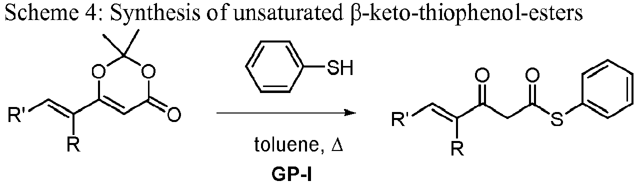

The following precursors are preferred:

| Lab-Code | Formula |
|---|---|
| MAGR169 | 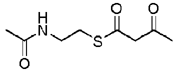 |
| MAGR170/ JHS201 | 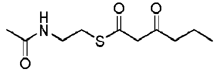 |
| JHS493 | 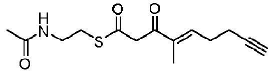 |
| JHS210 | 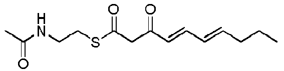 |
| MAGR41 / MAGR168/ | 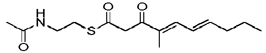 |
| JHS486 | 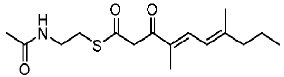 |
| MAGR42 | 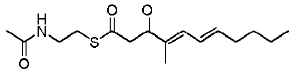 |
| MAGR171 | 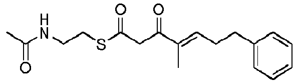 |
| JHS206 | 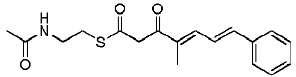 |
| JHS226 | 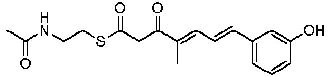 |
| JHS247B | 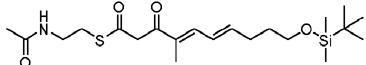 |
| JHS266 | 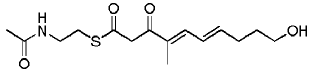 |
| JHS291 | 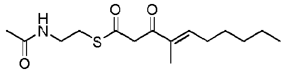 |
| JHS246 | 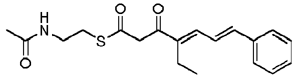 |
| JHS274 | 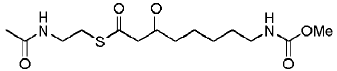 |
| JHS287 | 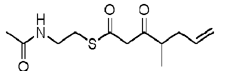 |
| JHS285 | 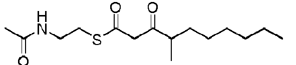 |
| JHS328 | 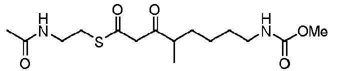 |
| JHS282 | 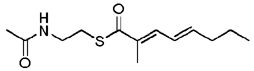 |
| JHS283 | 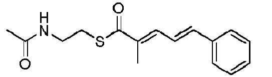 |
| JHS401 | 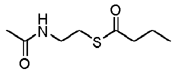 |
| JHS410 | 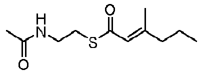 |
| JHS415 | 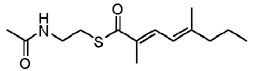 |
| JHS417 | 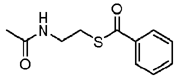 |
| JHS427 | 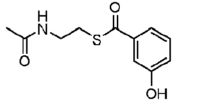 |
| JHS289 | 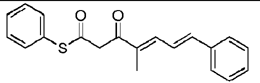 |

Precursor compounds could be synthesized by the following methods:

### General Procedure for synthesis of acylmeldrums acids from acyl chlorides: General procedure A:

To a solution of meldrums acid (10.8 g, 75.0 mmol) and pyridine (12.0 g, 0.15 mol) in anhydrous dichloromethane (50 mL) at 0 °C was added dropwise a solution of the appropriate acyl chloride (80.0 mmol) in anhydrous dichloromethane (20 mL). The resulting orange mixture was stirred for 1 h at 0 °C and then for 1 h at room temperature. The reaction mixture was diluted with dichloromethane (50 mL) and washed with aqueous 2M HCl (3 x 50 mL), brine (2 x 50 mL), dried (MgSO₄) and concentrated under reduced pressure. The crude material was filtered over a short pad of SiO₂ eluting with *n*-hexane/ EtOAc 2:1.

### General Procedure for synthesis of oxinones from acylmeldrums acids: General procedure B

The appropriate acylmeldrums acid (20.0 mmol) was dissolved in toluene (25 mL) and acetone (2.0 mL). The solution was heated at reflux for 2 h. The solvents were removed under reduced pressure and the crude material was purified by flash chromatography (SiO₂, *n-*hexane/EtOAc) or vacuum distillation.

General Procedure for synthesis of dioxinon phosphonates: General procedure C: To a solution of diisopropylamine (7.18 mL, 51.1 mmol) in THF (30 mL) was added dropwise a solution of n-BuLi (2.5M in *n*-hexane, 20.5 mL, 51.1 mmol) at 0 °C. After stirring for 30 minutes, the reaction mixture was cooled to -78 °C and the appropriate 2,2-dimethyl-4*H*-1,3-dioxin-4-one (36.5 mmol) was added over 5 min. After 40 min chlorodiethylphosphite (7.62 mL, 53.0 mmol) was added and the cooling bath was removed. When the mixture reached room temperature, it was diluted with Et₂O (200 mL), washed with half-saturated brine (100 mL), dried (MgSO₄) and concentrated *in vacuo.* The resulting yellow oil was dissolved in CH₂Cl₂ (60 mL) and aqueous H₂O₂ (30%, 13 mL) was carefully added at 0 °C. The solution was stirred for 1 h and diluted with EtOAc (200 mL). After separation, the organic layer was washed with brine (2 x 100 mL), dried (MgSO₄) and concentrated *in vacuo.* The crude material was purified by flash chromatography (SiO₂, EtOAc).

### General Procedure for the Horner-Wadsworth-Emmons olefination for the preparation of unsaturated dioxinones: General procedure D

To a solution of diisopropylamine (1.04 mL, 7.13 mmol) in THF (20 mL) was added a solution of n-BuLi (2.5M in *n*-hexane, 2.85 mL, 7.13 mmol) at 0 °C. After stirring for 30 min, the reaction mixture was cooled to -78 °C. A solution of the appropriate phosphonate (6.84 mmol) in THF (10 mL) was added to the reaction mixture, and the resulting mixture was warmed to 0 °C and stirred for 15 min. After cooling to -78 °C, HMPA (2.10 mL, 12.1 mmol) was added and stirring was continued for 30 min. A solution of the appropriate aldehyde (6.84 mmol) in THF (10 mL) was added slowly. The reaction mixture was allowed to warm slowly to 0 °C and stirred for 1 h. After diluting with EtOAc (80 mL), the mixture was washed with saturated aqueous NH₄Cl (3 x 40 mL) and brine (50 mL). The organic layer was dried (MgSO₄) and concentrated. The crude material was purified by flash chromatography (SiO₂, *n*-hexane/EtOAc).

### General procedure for synthesis of β-keto-N-acetyleysteamine (SNAC)-esters from dioxinones: General procedure E:

A degassed solution of the appropriate dioxinone (0.95 mmol), *N*-acetylcysteamine (SNAC-H, 0.15 g, 1.25 mmol) in toluene (6 mL) was refluxed for 16 h under a nitrogen atmosphere. After cooling to room temperature the solvent was evaporated under reduced pressure and the crude product was purified by flash column chromatography (SiO₂, *n*-hexane/EtOAc= 1:1 → EtOAc) to afford the desired SNAC ester.

### General procedure for synthesis of acylmeldrums acids from carboxylic acids via DCC coupling: General procedure F:

A solution of the appropriate acid (10.6 mmol) and DCC (2.19 g, 10.6 mmol) in anhydrous CH₂Cl₂ (20 mL) was stirred for 30 min at 0°C. A white solid precipitated. DMAP (1.94 g, 15.9 mmol) and meldrums acid (1.52 g, 10.6 mmol) were subsequently added. The cooling bath was removed and the reaction mixture was stirred for 2 h at room temperature. The insoluble urea was filtered off and the filter-cake was washed with ice-cold CH₂Cl₂ (10 mL). The combined filtrates were washed with 1M HCl (2 x 50 mL), H₂O (2 x 50 mL) and brine (75 mL), dried (MgSO₄) and concentrated. To remove last residues of urea, the crude material was filtered over cotton.

### General procedure for synthesis of β-keto-N-acetylcysteamine (SNAC)-esters from acylmeldrums acids: General procedure G:

A degassed solution of the appropriate acylmeldrums acid (0.80 mmol), *N*-acetylcysteamine (SNAC-H, 0.12 g, 1.00 mmol) in toluene (6 mL) was refluxed for 16 h under a nitrogen atmosphere. After cooling to room temperature the solvent was evaporated under reduced pressure and the crude product was purified by flash column chromatography (SiO₂, n-hexane/EtOAc = 1:1 → EtOAc) to afford the desired β-keto-SNAC ester.

### General procedure for synthesis of N-acetylcysteamine (SNAC)-esters from carboxylic acids via DCC coupling: General procedure H:

A solution of the appropriate acid (1.06 mmol) and DCC (0.41 g, 1.06 mmol) in anhydrous CH₂Cl₂ (5 mL) was stirred for 30 min at 0°C. A white solid precipitated. DMAP (0.37 g, 1.59 mmol) and SNAC-H (0.24 g, 1.06 mmol) were subsequently added. The cooling bath was removed and the reaction mixture was stirred for 2 h at room temperature. The insoluble urea was filtered off and the filter-cake was washed with ice-cold CH₂Cl₂ (10 mL). The combined filtrates were evaporated *in vacuo* and the crude product was purified by flash column chromatography (SiO₂, *n*-hexane/EtOAc= 1:1 → EtOAc) to afford the desired SNAC ester.

### General procedure for synthesis of thiophenol-esters from acylmeldrums acids: General procedure I:

A degassed solution of the dioxinone (0.92 mmol), thiophenol (0.13 g, 1.16 mmol) in toluene (6 mL) was refluxed for 16 h under a nitrogen atmosphere. After cooling to room temperature the solvent was evaporated under reduced pressure and the crude product was purified by flash column chromatography (SiO₂, *n*-hexane/EtOAc = 12:1 → 8:1) to afford the desired thioester.

### Synthesis and spectroscopic data of intermediates. (E)-3-(3-Hydroxyphenyl)acrylaldehyde (IM-1) (JHS216)

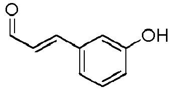

The title compound was prepared from 3-hydroxybenzaldehyde according to the following procedure:
A mixture of 3-hydroxybenzaldehyde (5.00 g, 41.0 mmol) and vinyl acetate (4.15 mL, 45.1 mmol) in acetonitrile (15 mL) was added at room temperature to a suspension of potassium carbonate (6.80 g, 49.2 mmol) in acetonitrile (50 mL) and water (0.2 mL). The reaction mixture was refluxed for 40 h and cooled to room temperature. The reaction mixture was diluted with water (50 mL) and EtOAc (50 mL). The aqueous layer was separated and extracted with EtOAc (2 x 50 mL). The combined organic layers were dried (MgSO₄) and concentrated *in vacuo* to dryness. The residue was purified by flash column chromatography (SiO₂, *n*-hexane/EtOAc 8:1 → 2:1) to afford the title compound.
Yield: 25%, orange solid. ¹H NMR (DMSO-d₆): δ = 9.68 (br. s, 1H), 9.65 (d, *J* = 7.7 Hz, 1H), 7.65 (d, *J* = 15.8 Hz, 1H), 7.27 (t, *J* = 8.0 Hz, 1H), 7.17 (d, *J* = 8.0 Hz, 1H), 7.08 (t, *J* = 2.4 Hz, 1H), 6.89 (ddd, *J* = 8.0, 2.4, 1.0 Hz, 1H), 6.73 (dd, *J* = 15.8, 7.7 Hz, 1H) ppm. ¹³C NMR (DMSO-d₆): δ = 194.4, 157.8, 153.5, 135.3, 130.1, 128.3, 119.6, 118.4, 115.0 ppm.

### (E)-3-(3-((tert-Butyldimethylsilyl)oxy)phenyl)acrylaldehyde (IM-2) (JHS221)

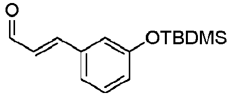

The title compound was prepared from IM-1 according to the following procedure:
Imidazole (0.86 g, 12.7 mmol) was added to a solution of IM-1 (0.76 g, 5.13 mmol) and *tert-*butyldimethylchlorosilane (0.92 g, 6.10 mmol) in anhydrous *N*,*N*-dimethylformamide (10 mL). The mixture was stirred for 3 h, diluted with 5% aqueous NaHCO3 (50 mL), and extracted with EtOAc (4 x 50 mL). The combined organic layers were washed with brine (100 mL), dried (MgSO₄), and evaporated *in vacuo* to yield the title compound.
Yield: 82%, yellow oil. ¹H NMR (DMSO-d₆): δ = 9.66 (d, *J* = 7.7 Hz, 1H), 7.71 (d, *J* = 15.9 Hz, 1H), 7.34-7.38 (m, 2H), 7.19-7.22 (m, 1H), 6.94-6.99 (m, 1H), 6.84 (dd, *J* = 15.9, 7.7 Hz, 1H), 0.96 (s, 9H), 0.21 (s, 6H) ppm. ¹³C NMR (DMSO-d₆): δ = 194.3, 155.5, 152.9, 135.6, 130.2, 128.7, 122.7, 121.9, 119.8, 25.5, 17.9, -4.6 ppm.

### (E)-3-(3-Bromophenyl)acrylaldehyde (IM-3) (JHS487)

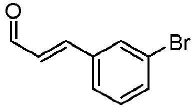

The title compound was prepared from 3-bromobenzaldehyde according to the following procedure:
A solution of 3-bromobenzaldehyde (5.00 g, 27.0 mmol) in H₂O (6 mL) was filled into a 100 mL three-necked round bottom flask equipped with an thermometer. Solid NaOH (54.0 mg, 1.35 mmol) was added and dissolved. A solution of vinylacetate (0.41 g, 10.4 mmol) in H₂O (1 mL) was added dropwise. The temperature was kept at 25 °C and NaOH (270 mg, 0.68 mmol) in H₂O (1 mL) was added *via* a syringe. Afterwards another vinylacetate (0.41 g, 10.4 mmol) in H₂O (1 mL) and the reaction mixture was stirred over night at room temperature. The reaction was neutralized with acetic acid, diluted with 50 mL H₂O and extracted with Et₂O (2 x 100 mL). The organic layers were combined, washed with brine (100 mL), dried (MgSO₄), and evaporated *in vacuo.* The residue was purified by flash chromatography (SiO₂, *n*-hexane/EtOAc = 20:1 → 15:1).
Yield: 12%, yellow oil. ¹H NMR (CDCl₃): δ = 9.72 (d, *J* = 7.5 Hz, 1H), 7.71 (s, 1H), 7.57 (d, *J* = 7.7 Hz, 1H), 7.50 (d, *J* = 7.7 Hz, 1H), 7.40 (d*, J* = 15.9 Hz, 1H), 7.32 (t, *J* = 7.7 Hz, 1H), 6.70 (dd, *J* = 15.9, 7.5 Hz, 1H) ppm. ¹³C NMR (CDCl₃): δ = 193.2, 150.6, 136.1, 134.0, 131.2, 130.6, 129.7, 126.9, 123.2 ppm.

### Pent-4-yn-1-ol (IM-4) (JHS490)

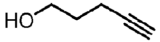

The title compound was prepared according to the following procedure:
4-pentynoyc acid (3.00 g, 30.6 mmol) was dissolved in anhydrous THF (90 mL) and cooled to 0 °C. Triethylamine (4.69 mL, 33.6 mL) and methylchloroformate (2.60 mL, 33.6 mmol) were subsequently added and the reaction was stirred at 0 °C for 20 min. The occurring white precipitate was filtered off, afterwards under a nitrogen atmosphere and the filtrate was added to a solution of NaBH4 (1.85 g, 49.9 mmol) in water (60 mL) at 0 °C. The resulting mixture was stirred for 1 h at 0 °C and afterwards for 2 h at room temperature. The reaction was carefully quenched with 1M HC1 (20 mL) until bubbling ceased. The mixture was extracted with EtOAc (3 x 100 mL). The combined organic layers were washed with saturated NaHCO₃ solution (100 mL) and brine (100 mL), dried (MgSO₄) and concentrated *in vacuo* to yield the title compound.
Yield: 39%, colorless liquid. ¹H NMR (CDCl₃): δ = 3.75 (t, *J* = 6.4 Hz, 2H), 2.31 (dt, *J* = 7.0, 2.7 Hz, 2H), 1.97 (t, *J* = 2.7 Hz, 1H), 1.85 (s, 1H), 1.77 (quin, *J* = 6.4 Hz, 2H) ppm. ¹³C NMR (CDCl₃): δ = 83.8, 68.7, 61.4, 31.0, 14.9 ppm.

### Pent-4-ynal (IM-5) (JHS491)

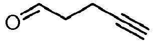

The title compound was prepared from IM-4 according to the general procedure shown for IM-7 and used in the next step without further purification.

### 4-(tert-Butyldimethylsilyloxy)butan-1-ol (IM-6) (JHS225)

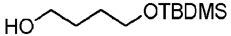

The title compound was prepared according to the following procedure:
To a suspension of potassium hydride (1.32 g, 33.0 mmol) in THF (40 mL) 1,4-butanediol (3.00 g, 33.3 mmol) was added at room temperature and stirred for 45 min. A voluminous white precipitate formed. To the reaction mixture was added *tert*-butyldimethylsilyl chloride (4.97 g, 33.0 mmol) and stirring was continued for 45 min. The mixture was diluted with Et₂O (200 mL), washed with aqueous K₂CO₃ (10%, 50 mL) and brine (2 x 100 mL), dried (MgSO₄) and concentrated *in vacuo.*
Yield: 99%, colorless oil. ¹H NMR (CDCl₃): δ = 3.63-3.69 (m, 4H), 2.51 (t, *J* = 5.6 Hz, 1H), 1.62-1.69 (m, 4H), 0.91 (s, 9H), 0.08 (s, 6H) ppm.¹³C NMR (CDCl₃): δ = 63.3, 62.7, 30.2, 29.8, 25.9, 18.3, -5.4 ppm.

### 4-(tert-Butyldimethylsilyloxy)butanal (IM-7) (JHS227)

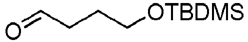

The title compound was prepared from IM-6 according to the following procedure:
To a stirred suspension of PCC (10.6 g, 49.2 mmol) and NaOAc (0.49 g, 9.84 mmol) in CH₂Cl₂ (100 mL) a solution of the alcohol IM-6 (6.70 g, 32.8 mmol) in CH₂Cl₂ (100 mL) was added. After 7 h the reaction mixture was diluted with Et₂O (100 mL) and the mixture was filtered over short pad of celite. The solvents were evaporated *in vacuo* and the residue was purified by flash chromatography (SiO₂, *n*-hexane/EtOAc = 20:1 → 10:1).
Yield: 30%, colorless oil. ¹H NMR (CDCl₃): δ = 9.79 (t, *J* = 1.7 Hz, 1H), 3.65 (t, *J* = 6.0 Hz, 2H), 2.51 (dt, *J* = 7.1, 1.7 Hz, 2H), 1.86 (quin, *J* = 6.5 Hz, 2H), 0.89 (s, 9H), 0.05 (s, 6H) ppm. ¹³C NMR (CDCl₃): δ = 202.6, 62.1, 40.8, 25.9, 25.5, 18.3, -5.4.

### (E)-6-(tert-Butyldimethylsilyloxy)hex-2-enal (IM-8) (JHS228)

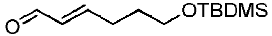

The title compound was prepared from IM-7 according to the following procedure:
To a solution formylmethylenetriphenylphosphorane (3.01 g, 9.90 mmol) in benzene (80 mL), a solution of the aldehyde IM-7 (2.00 g, 9.90 mmol) in benzene (15 mL) was added and the mixture was heated at reflux for 24 h. After removal of the solvent, the crude material was purified by flash chromatography (SiO₂, *n*-hexane/EtOAc = 12:1 → 10:1).
Yield: 58%, light yellow oil. ¹H NMR (CDCl₃): δ = 9.46 (d, *J* = 7.9 Hz, 1H), 6.84 (dd, *J* = 15.7, 6.8 Hz, 1H), 6.08 (ddt, *J* = 15.7, 7.9, 1.5 Hz, 1H), 3.61 (t, *J* = 6.1 Hz, 2H), 2.34-2.41 (m, 2H), 1.63-1.72 (m, 2H), 0.84 (s, 9H), 0.00 (s, 6H) ppm. ¹³C NMR (CDCl₃): δ = 194.0, 158.6, 133.0, 62.0, 30.9, 29.3, 25.9, 18.3, 15.4 ppm.

### 3-((tert-Butyldimethylsilyl)oxy)benzoic acid (IM-9) (JHS423)

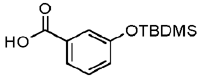

The title compound was prepared from 3-hydroxybenzoic acid according to the following procedure:
Imidazole (1.36 g, 20.0 mmol) was added to a solution of 3-hydroxybenzoic acid (1.00 g, 7.24 mmol) and *tert*-butyldimethylchlorosilane (1.31 g, 8.69 mmol) in anhydrous *N,N-*dimethylformamide (15 mL). The mixture was stirred for 3 h at room temperature and then diluted with acetone (50 mL). The solvents were evaporated *in vacuo* and the residue was dissolved in EtOAc (50 mL). The organic layer was washed with H₂O (2 x 40 mL) and brine (30 mL), dried (MgSO₄), and evaporated *in vacuo* to yield the title compound.
Yield: 38%, colorless crystals. ¹H NMR (CDCl₃) δ 7.73 (td, *J* = 8.0, 1.1 Hz, 1H), 7.58 (t, *J* = 2.5 Hz, 1H), 7.34 (t, *J* = 8.0 Hz, 1H), 7.10 (ddd, *J* = 8.0, 2.5, 1.1 Hz, 1H), 1.01 (s, 9H), 0.24 (s, 6H) ppm. ¹³C NMR (CDCl₃) 171.7, 155.8, 130.7, 129.5, 125.7, 123.2, 121.5, 25.6, 18.2, -4.5 ppm.

### (S)-(2-Acetamidoethyl) 3-((tert-butyldimethylsilyl)oxy)benzothioate (IM-10) (JHS425)

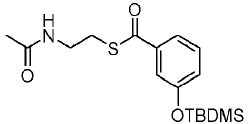

The title compound was prepared from IM-9 according to the general procedure H:
Yield 51%, white solid. ¹H NMR (CDCl₃) δ = 7.57 (d, *J* = 7.7 Hz, 1H), 7.39-7.44 (m, 1H), 7.32 (t, *J* = 7.70 Hz, 1H), 7.03-7.12 (m, 1H), 5.92 (br. s., 1H), 3.50-3.60 (m, 1H), 3.23 (t*, J* = 6.38 Hz, 1H), 1.98 (s, 3H), 1.01 (s, 9H), 0.23 (s, 6H) ppm. ¹³C NMR (CDCl₃) δ = 191.6, 169.9, 155.6, 137.6, 129.3, 125.1, 119.9, 118.1, 76.8, 76.6, 76.2, 39.3, 28.2, 25.2, 22.8, -4.9 ppm.

### 5-(1-Hydroxyethylidene)-2,2-dimethyl-1,3-dioxane-4,6-dione (IM-11) (JHS197)

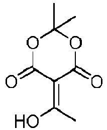

The title compound was prepared from acetyl chloride according to the general procedure A:
Yield: 83%, yellow solid. ¹H NMR (CDCl₃): δ = 15.12 (s, 1H), 2.68 (s, 3H), 1.74 (s, 6H) ppm. ¹³C NMR (CDCl₃): δ = 194.6, 170.2, 160.4, 104.9, 91.8, 26.8, 23.5 ppm.

### 6-Methyl-2,2-dimethyl-4H-1,3-dioxin-4-one (IM-12) (JHS198)

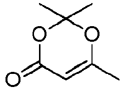

The title compound was prepared from IM-11 according to the general procedure B:
Yield: 85%, yellow oil. ¹H NMR (CDCl₃): δ = 5.19 (s, 1H), 1.94 (s, 3H), 1.64 (s, 6H) ppm. ¹³C NMR (CDCl₃): δ = 168.6, 161.1, 106.2, 93.7, 24.9, 19.8 ppm.

### Diethyl 1-(2,2-dimethyl-4-oxo-4H-1,3-dioxin-6-yl)methylphosphonate (IM-13) (JHS208)

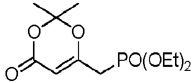

The title compound was prepared from IM-12 according to the general procedure C:
Yield: 62%, yellow resin. ¹H NMR (CDCl₃) δ = 5.39 (d, *J* = 3.6 Hz, 1H), 4.08-4.24 (m, 4H), 2.80 (d, *J* = 22.1 Hz, 2H), 1.71 (s, 6H), 1.35 (t, *J* = 7.1 Hz, 6H) ppm.¹³C NMR (75 MHz, CDCl₃): δ = 163.0 (d, *J* = 9.7 Hz), 160.5, 107.1, 96.2 (d, *J* = 8.2 Hz), 62.6 (d, *J* = 6.7 Hz), 32.4 (d, *J* = 137.1 Hz), 24.9, 16.3(d, *J* = 6.0 Hz) ppm.

### 6-((1E,3E)-Hepta-1,3-dien-1-yl)-2,2-dimethyl-4H-1,3-dioxin-4-one (IM-14) (JHS209)

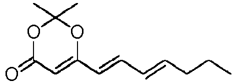

The title compound was prepared from IM-13 according to the general procedure D:
Yield: 42%. ¹H NMR (CDCl₃): δ = 6.92 (dd, *J* = 15.2, 9.9 Hz, 1H), 5.98-6.14 (m, 2H), 5.90 (d*, J* = 15.2 Hz, 1H), 5.21 (s, 1H), 2.16 (q, *J* = 7.2 Hz, 2H), 1.72 (s, 6H), 1.46 (sxt, *J* = 7.4 Hz, 2H), 0.95 (t, *J* = 7.4 Hz, 3H) ppm.¹³C NMR (CDCl₃): δ = 163.5, 161.7, 143.4, 138.5, 128.9, 120.6, 105.9, 93.6, 34.8, 24.8, 21.8, 13.4 ppm.

### 5-(1-Hydroxypropylidene)-2,2-dimethyl-1,3-dioxane-4,6-dione (IM-15) (JHS190D)

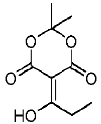

The title compound was prepared from propionyl chloride according to the general procedure A:
Yield: 93%, orange crystals. ¹H NMR (CDCl₃): δ = 15.35 (br. s, 1H), 3.10 (q, *J* = 7.4 Hz, 2H), 1.72 (s, 6H), 1.25 (t*, J* = 7.4 Hz, 3H) ppm.¹³C NMR (CDCl₃): δ = 198.9, 170.2, 160.2, 104.8, 90.9, 29.4, 26.7, 9.6 ppm.

### 6-Ethyl-2,2-dimethyl-4H-1,3-dioxin-4-one IM-16 (MAGR35)

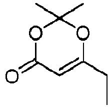

The title compound was prepared from IM-15 according to the general procedure B:
Yield: 80%, yellow oil. ¹H NMR (CDCl₃): δ = 5.22 (t, *J* = 1.0 Hz, 1H), 2.24 (dq, *J* = 7.6, 1.0 Hz, 2H), 1.67 (s, 6H), 1.12 (t, *J* = 7.6 Hz, 3H) ppm. ¹³C NMR (CDCl₃): δ = 173.1, 161.5, 106.2, 92.2, 26.7, 24.9, 9.9 ppm.

### Diethyl 1-(2,2-dimethyl-4-oxo-4H-1,3-dioxin-6-yl)ethylphosphonate (IM-17) (JHS 195)

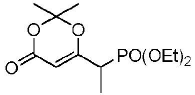

The title compound was prepared from IM-16 according to the general procedure C:
Yield: 75%. Light yellow oil. ¹H NMR (CDCl₃): δ = 5.37 (d, *J* = 3.5 Hz, 1H), 4.08-4.18 (m, 4H), 2.77 (dq, *J* = 23.9, 7.3 Hz, 1H), 1.69 (s, 6H), 1.30-1.45 (m, 9H) ppm.¹³C NMR (CDCl₃): δ = 168.1 (d, *J* = 8.2 Hz), 160.8 (d, *J* = 2.2 Hz), 106.8, 94.9 (d, *J* = 6.7 Hz), 62.1 (dd, *J* = 6.7, 4.5 Hz), 36.9 (d, *J* = 137.1 Hz), 24.8 (d, *J* = 70.8 Hz), 16.4 (d*, J* = 6.0 Hz), 12.1 (d, *J* = 6.0 Hz) ppm.

### (E)-5-(1-Hydroxy-2-methylhept-2-en-6-yn-1-ylidene)-2,2-dimethyl-1,3-dioxane-4,6-dione (IM-18) (JHS492)

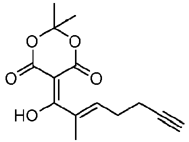

The title compound was prepared from IM-17 according to the general procedure D:
Yield: 53 %, colorless oil. ¹H NMR (CDCl₃) δ = 6.40 (dt, *J* = 7.2, 1.1 Hz, 1H), 5.43 (s, 1H), 2.39-2.50 (m, 2H), 2.29-2.37 (m, 2H), 1.95-2.00 (m, 1H), 1.81 (s, 3H), 1.70 (s, 6H) ppm. ¹³C NMR (CDCl₃) δ = 165.3, 162.2, 134.9, 128.5, 106.0, 91.8, 83.0, 69.1, 27.6, 24.9, 17.9, 12.3 ppm.

### 2,2-Dimethyl-6-((2E,4E)-octa-2,4-dien-2-yl)-4H-1,3-dioxin-4-one (IM-19) (MAGR38)

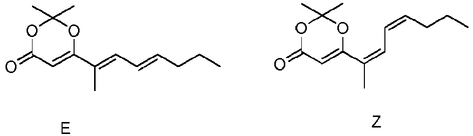

E/Z = 15 :1,
The title compound was prepared from IM-17 according to the general procedure D:
Yield: 42%. ¹H NMR (CDCl₃): δ = 6.85 (d, *J* = 11.3 Hz, 1H), 6.36 (dd, *J* = 14.8, 11.3 Hz, 1H), 6.07 (dt, *J* = 14.8, 7.5 Hz, 1H), 5.42 (s, 1H), 2.15-2.20 (m, 2H), 1.86 (s, 3H) 1.68 (s, 6H) 1.39-1.48 (m, 2H), 0.90 (t, *J* = 7.4 Hz, 1H) ppm.
¹³C NMR (CDCl₃): δ = 165.7, 162.2, 143.3, 134.3, 125.9, 124.4, 105.8, 91.6, 35.3, 24.9, 22.1, 13.6, 12.1 ppm.

### 5-((2E,4E)-1-Hydroxy-2,5-dimethylocta-2,4-dien-1-ylidene)-2,2-dimethyl-1,3-dioxane-4,6-dione (IM-20) (JHS485)

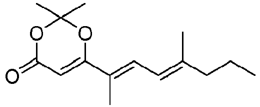

The title compound was prepared from IM-17 according to the general procedure D:
Yield: 60 %, yellow oil. ¹H NMR (CDCl₃) δ = 7.09 (d, *J* = 11.8 Hz, 1H), 6.14 (dd, *J* = 11.8, 1.2 Hz, 1H), 5.41 (s, 1H), 2.12 (t, *J* = 7.5 Hz, 2H), 1.85 (s, 3H), 1.83 (s, 3H), 1.68 (s, 6H), 1.47 (sxt, *J* = 7.4 Hz, 2H), 0.87 (t*, J* = 7.4 Hz, 3H) ppm. ¹³C NMR (CDCl₃) δ = 166.0, 162.3, 148.5, 130.0, 124.0, 120.6, 105.7, 91.2, 42.7, 24.8, 20.9, 17.0, 13.6, 11.9 ppm.

### 2,2-Dimethyl-6-((2E,4E)-nona-2,4-dien-2-yl)-4H-1,3-dioxin-4-one (IM-21) (MAGR40)

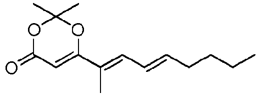

The title compound was prepared from IM-17 according to the general procedure D:
Yield: 19 %, yellow resin. 2*E*,4*E*/2*Z*,4*E* = 14:1, ¹H NMR (CDCl₃) 2*E*,4*E* only: δ = 6.83 (d, *J* = 11.1 Hz, 1*H*), 6.36 (dd, *J* = 14.8, 11.3 Hz, 1H), 6.10 (dt, *J* = 14.8, 7.4 Hz, 1H), 5.46 (s, 1H), 2.16-2.27 (m, 2H), 1.89 (s, 3H), 1.72 (s, 6H) 1.39-1.46 (m, 2H), 1.32-1.39 (m, 2H), 0.92 (t, *J* = 7.3 Hz, 1H) ppm. ¹³C NMR (CDCl₃): δ = 165.8, 162.4, 143.7, 134.3, 125.9, 124.6, 105.9, 91.8, 33.1, 31.1, 25.0, 22.2, 13.9, 12.2 ppm.

### (E)-2,2-Dimethyl-6-(5-phenylpent-2-en-2-yl)-4H-1,3-dioxin-4-one (IM-22) (MAGR165)

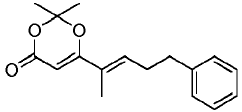

The title compound was prepared from IM-17 according to the general procedure D:
Yield: 13%, colorless resin. *E*-isomer only. ¹H NMR (CDCl₃): δ = 7.12-7.32 (m, 5H), 6.44 (t, *J* = 7.5 Hz, 1H), 5.40 (s, 1H), 2.76 (t, *J* = 7.6 Hz, 2H), 2.48-2.56 (m, 2H), 1.73 (s, 3H), 1.70 (s, 6H) ppm. ¹³C NMR (CDCl₃): δ = 165.6, 162.4, 141.0, 136.5, 128.4, 128.3, 127.8, 126.2, 106.0, 91.5, 35.0, 30.6, 24.9, 12.1 ppm.

### 2,2-Dimethyl-6-((2E,4E)-5-phenylpenta-2,4-dien-2-yl)-4H-1,3-dioxin-4-one (IM-23) (JHS203)

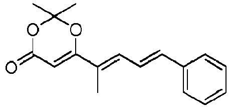

The title compound was prepared from IM-17 according to the general procedure D:
Yield: 50%, yellow oil. ¹H NMR (CDCl₃): δ = 7.48-7.51 (m, 2H), 7.30-7.40 (m, 4H), 7.14 (dd, *J* = 14.1, 11.3 Hz, 1H), 7.06 (d*, J* = 11.3 Hz, 1H), 6.90 (d, *J* = 14.1 Hz, 1H), 5.54 (s, 1H) 2.02 (s, 3H), 1.75 (s, 6H) ppm.¹³C NMR (CDCl₃): δ = 165.3, 162.2, 139.2, 136.6, 134.0, 128.8, 127.0, 127.0, 123.7, 106.0, 92.3, 25.0, 12.5 ppm.

### 6-((2E,4E)-5-(3-Bromophenyl)penta-2,4-dien-2-yl)-2,2-dimethyl-4H-1,3-dioxin-4-one (IM-24) (JHS488)

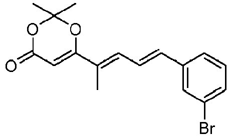

The title compound was prepared from IM-17 according to the general procedure D:
Yield: 50%, yellow solid. ¹H NMR (CDCl₃) δ = 7.61 (t, *J* = 1.6 Hz, 1H), 7.34-7.44 (m, 2H), 7.22 (t, *J* = 7.8 Hz, 1H), 7.10 (dd, *J* = 14.3, 11.4 Hz, 1H), 7.02 (d, *J* = 11.4 Hz, 1H), 6.78 (d, *J* = 14.3 Hz, 1H), 5.54 (s, 1H), 2.02 (s, 3H), 1.74 (s, 6H) ppm.¹³C NMR (CDCl₃): δ = 165.0, 162.0, 138.7, 137.2, 133.3, 131.4, 130.2, 129.5, 128.0, 125.7, 125.0, 122.9, 106.0, 92.6, 25.0, 12.5 ppm.

### 6-((2E,4E)-5-(3-((tert-Butyldimethylsilyl)oxy)phenyl)penta-2,4-dien-2-yl)-2,2-dimethyl-4H-1,3-dioxin-4-one (IM-25) (JHS223)

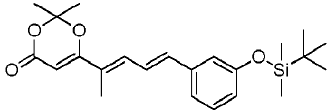

The title compound was prepared from IM-17 using IM-2 according to the general procedure D:
Yield: 16%, yellow oil. ¹H NMR (CDCl₃): δ = 7.22 (dd, *J* = 7.8, 7.8 Hz, 1H), 7.02-7.12 (m, 3 H), 6.93 (dd, *J* = 1.9, 1.8 Hz, 1H), 6.75-6.88 (m, 2H), 5.53 (s, 1H), 2.02 (s, 3H), 1.75 (s, 6H), 1.01 (s, 9H), 0.23 (s, 6H) ppm. ¹³C NMR (CDCl₃): δ = 165.3, 162.3, 156.1, 139.2, 138.1, 134.0, 129.7, 127.0, 123.9, 120.6, 120.2, 118.6, 106.0, 92.3, 25.7, 25.1, 18.2, 12.6, 4.4 ppm.

### (4E,6E)-(S)-2-Acetamidoethyl-7-(3-((tert-butyldimethylsilyl)oxy)phenyl)-4-methyl-3-oxohepta-4,6-dienethioate (IM-26) (JHS224)

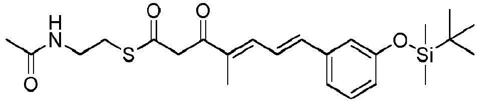

The title compound was prepared from IM-25 according to the general procedure E:
Yield: 53%, yellow oil. ¹H NMR (CDCl₃): δ = 7.11-7.30 (m, 2H), 6.96-7.11 (m, 2H), 6.70-6.96 (m, 3H), 6.11 (br. s, 1H), 4.01 (s, 2H), 3.35-3.51 (m, 2H), 3.08 (q, *J* = 6.1 Hz, 2H), 1.97 (s, 3H), 1.93 (s, 3H), 0.96 (s, 9H), 0.18 (s, 6H) ppm. ¹³C NMR (CDCl₃): δ = 193.1, 193.0, 170.3, 156.0, 141.3, 137.5, 135.5, 129.8, 128.1, 124.0, 121.1, 120.5, 118.8, 52.8, 39.2, 29.2, 25.6, 23.1, 18.2, 11.8, -4.4 ppm.

### 6-((2E,4E)-8-((tert-Butyldimethylsilyl)oxy)octa-2,4-dien-2-yl)-2,2-dimethyl-4H-1,3-dioxin-4-one (IM-27) (JHS240)

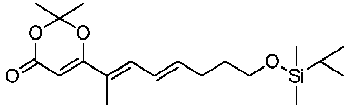

The title compound was prepared from IM-17 using IM-8 according to the general procedure D:
Yield: 29%, yellow oil. ¹H NMR (CDCl₃): δ = 6.83 (d, *J* = 11.2 Hz, 1H), 6.39 (ddd, *J* = 14.9, 11.2, 1.0 Hz, 1H), 6.09 (td, *J* = 14.9, 7.2 Hz, 1H), 5.44 (s, 1H), 3.61 (t, *J* = 6.2 Hz, 2H), 2.26 (q, *J* = 7.2 Hz, 2H), 1.87 (s, 3H), 1.71 (s, 6H), 1.58-1.67 (m, 2H), 0.89 (s, 9H), 0.04 (s, 6H). ¹³C NMR (CDCl₃): δ = 165.4, 162.3, 143.0, 137.8, 134.2, 126.1, 124.7, 105.9, 91.8, 62.4, 32.0, 29.3, 25.9, 25.0, 18.3, 12.2, -5.3ppm.

### (E)-2,2-Dimethyl-6-(oct-2-en-2-yl)-4H-1,3-dioxin-4-one (IM-28) (JHS288)

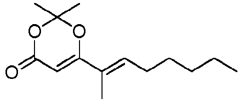

The title compound was prepared from IM-17 according to the general procedure D:
Yield: 37%, colorless oil. ¹H NMR (CDCl₃): δ = 6.41 (t*, J* = 7.4 Hz, 1H), 5.40 (s, 1H), 2.20 (q, *J* = 7.4 Hz, 2H), 1.79 (s, 3H), 1.70 (s, 6H), 1.40-1.49 (m, 2H), 1.27-1.36 (m, 4H), 0.90 (t, *J* = 6.9 Hz. 3H) ppm. ¹³C NMR (CDCl₃): δ = 165.8, 162.5, 138.1, 126.9, 105.9, 91.2, 31.6, 28.7, 28.5, 24.9, 22.4, 13.9, 12.1 ppm.

### 5-(1-Hydroxybutylidene)-2,2-dimethyl-1,3-dioxane-4,6-dione (IM-29) (JHS192)

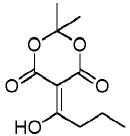

The title compound was prepared from butyryl chloride according to the general procedure A: Yield: 92%, yellow oil. ¹H NMR (CDCl₃): δ = 15.30 (s, 1H), 3.05 (t, *J* = 7.5 Hz, 2H), 1.73 (s, 6H), 1.72 (sxt, *J* = 7.5 Hz, 2H), 1.03 (t, *J* = 7.5 Hz, 3H) ppm. ¹³C NMR (CDCl₃): δ = 198.0, 170.5, 160.2, 104.7, 91.3, 37.4, 26.8, 19.6, 13.8 ppm.

### 6-Propyl-2,2-dimethyl-4H-1,3-dioxin-4-one (IM-30) (JHS194)

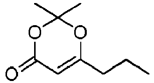

The title compound was prepared from IM-29 according to the general procedure B:
Yield: 82%, yellow oil. ¹H NMR (CDCl₃): δ = 5.22 (s, 1H), 2.18 (t*, J* = 7.5 Hz, 2H), 1.66 (s, 6H), 1.57 (sxt, *J* = 7.5 Hz, 2H), 0.95 (t, *J* = 7.5 Hz, 3H) ppm. ¹³C NMR (CDCl₃): δ = 171.9, 161.4, 106.2, 93.1, 35.4, 24.9, 19.1, 13.4 ppm.

### Diethyl 1-(2,2-dimethyl-4-oxo-4H-1,3-dioxin-6-yl)propylphosphonate (IM-31) (JHS 199)

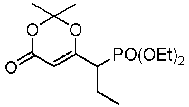

The title compound was prepared from IM-30 according to the general procedure C:
Yield: 45%, light yellow oil. ¹H NMR (acetone-d₆): δ = 5.46 (d, *J* = 3.4 Hz, 1H), 4.08-4.23 (m, 4H), 2.83 (ddd, *J* = 22.9, 10.8, 4.3 Hz, 1H), 1.77-2.01 (m, 2H), 1.69 (s, 6H), 1.26-1.34 (m, 6H), 1.00 (t, *J* = 7.4 Hz, 3H) ppm.¹³C NMR (acetone-d₆): δ = 167.5 (d*, J* = 8.2 Hz), 160.6 (d, *J* = 2.2 Hz), 107.6, 97.1 (d*, J* = 8.2 Hz), 63.7 (d, *J* = 6.7 Hz), 45.7 (d, *J* = 136.4 Hz), 25.3 (d, *J* = 7.5 Hz), 21.0 (d, *J* = 3.7 Hz), 16.8 (d*, J* = 6.0 Hz), 12.7 (d*, J* = 15.7 Hz) ppm.

### 2,2-Dimethyl-6-((3E,5E)-6-phenylhexa-3,5-dien-3-yl)-4H-1,3-dioxin-4-one (IM-32) (JHS241)

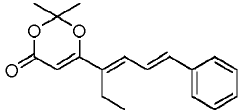

The title compound was prepared from IM-31 according to the general procedure D:
Yield: 25%, yellow solid. ¹H NMR (CDCl₃) 3*E*,5*E*-isomer only: δ = 7.41 (m, 2H), 7.19-7.32 (m, 3H), 7.03 (dd, *J* = 14.8, 11.3 Hz, 1H), 6.92 (d*, J* = 11.3 Hz, 1H), 6.82 (d*, J* = 14.8 Hz, 1H), 5.50 (s, 1H), 2.41 (q, *J* = 7.6 Hz, 2H), 1.67 (s, 6H), 1.06 (t, *J* = 7.6 Hz, 3H) ppm. ¹³C NMR (CDCl₃): δ=164.6, 162.4, 139.5, 136.6, 133.7, 133.5, 128.8, 127.0, 123.4, 106.0, 92.0, 25.0, 20.1, 14.4 ppm.

### 6-(Methoxycarbonylamino)hexanoic acid (IM-33) (JHS271)

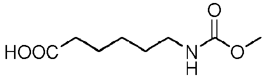

The title compound was prepared according to the following procedure:
To a stirred solution of γ-amino butyric acid (5.79 g, 56.1 mmol) and KOH (3.21 g, 57.2 mmol) in H₂O (20 mL) were added portionwise, alternately methylchlorofomate (4.82 mL, 62.3 mmol) and a solution of KOH (57.6 mmol) in H₂O (5 mL). After 2 h the reaction mixture was basified by adding a solution of KOH (4.50 g, 80.2 mmol) in H₂O (10 mL). The solution was washed with CH₂Cl₂ (2 x 75 mL). The aqueous layer was acidified with 30% H₂SO₄ causing the precipitation of a white solid. The mixture was extracted with CH₂Cl₂ (4 x 200 mL) and dried (MgSO₄). The solvent was evaporated *in vacuo.*
Yield: 88%, colorless oil. ¹H NMR (CDCl₃): δ = 10.33 (br. s, 1H), 5.88 (br. s, 0.30H, NH), 4.81 (br. s, 0.70H, NH), 3.66 (s, 3H), 3.08-3.20 (m, 2H), 2.35 (t, *J* = 7.4 Hz, 2H), 1.60-1.70 (m, 2H), 1.47-1.56 (m, 2H), 1.31-1.41 (m, 2H) ppm. ¹³C NMR (CDCl₃): δ = 178.9, 157.1, 52.0, 40.8, 33.8, 29.6, 26.0, 24.2 ppm.

### Methyl 6-(2,2-dimethyl-4,6-dioxo-1,3-dioxan-5-ylidene)-6-hydroxyhexylcarbamate (IM-34) (JHS273)

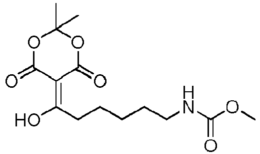

The title compound was prepared from IM-33 according to the general procedure F:
Yield: 99 %, orange oil. ¹H NMR (CDCl₃): δ = 15.31 (br. s, 1H), 4.70 (br. s, 1H), 3.66 (s, 3H), 3.12-3.25 (m, 2H), 3.07 (t, *J* = 7.6 Hz, 2H), 1.30-1.75 (m, 6H), 1.73 (s, 6H) ppm. ¹³C NMR (CDCl₃): δ = 197.8, 171.1, 157.1, 104.8, 91.3, 60.3, 52.0, 40.7, 29.6, 26.8, 26.3, 14.2 ppm.

### 5-(1-Hydroxy-2-methylpent-4-enylidene)-2,2-dimethyl-1,3-dioxane-4,6-dione (IM-35) (JHS286)

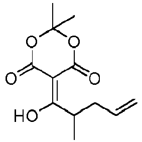

The title compound was prepared from 2-methyl-4-pentenoic acid according to the general procedure F:
Yield: 48 %, orange oil. Keto/enol = 1:6 ¹H NMR (CDCl₃): δ = 15.49 (br. s, 1H), 5.77 (m, 1H), 5.01-5.14 (m, 2H), 4.11-4.23 (m, 1H), 2.43-2.53 (m, 1H), 2.22-2.31 (m, 1H), 1.74 (s, 6H), 1.24 (d, *J* = 6.8 Hz, 3H) ppm. ¹³C NMR (CDCl₃): δ = 200.9, 170.7, 160.1, 135.0, 117.3, 104.7, 91.2, 38.1, 37.6, 26.6, 17.0 ppm.

### Ethyl 2-methyloctanoate (IM-36) (JHS278)

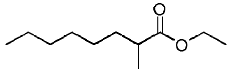

The title compound was prepared according to the following procedure:
The unsaturated ester IM-39 (1.20 g, 6.60 mmol) was dissolved in EtOH (50 mL). After addition of Pd/C (5%, 6 mg) the mixture was hydrogenated in a Parr apparatus at 3 bar H₂ for 24 h. The catalyst was filtered off and the filter cake was washed with EtOAc (30 mL). The combined filtrates were concentrated *in vacuo.* The residue was dissolved in CH₂Cl₂ (50 mL) and filtered over a short pad of SiO₂ eluting with CH₂Cl₂. The solvent was removed *in vacuo.*
Yield: 73%, colorless liquid. ¹H NMR (CDCl₃): δ = 4.13 (q, *J* = 7.1 Hz, 2H), 2.42 (tq, *J* = 6.9, 6.5 Hz, 1H), 1.59-1.71 (m, 1H), 1.33-1.46 (m, 1H), 1.22-1.33 (m, 8H), 1.26 (t, *J* = 7.1 Hz, 3H), 1.14 (d, *J* = 6.9 Hz, 3H), 0.88 (t*, J* = 6.7 Hz, 3H) ppm. ¹³C NMR (CDCl₃): δ = 177.0, 60.0, 39.6, 33.8, 31.7, 29.2, 27.2, 22.6, 17.1, 14.3, 14.0 ppm.

### 2-Methyloctanoic acid (IM-37) (JHS281)

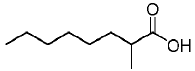

The title compound was prepared according to the following procedure:
To a solution of the ester IM-36 (0.90 g, 4.80 mmol) in MeOH (25 mL) was added a solution of NaOH (0.97 g, 24.2 mmol) in H₂O (8 mL). The resulting mixture was heated at reflux for 16 h. After cooling to room temperature the MeOH was evaporated *in vacuo.* The aqueous layer was cooled to 0 °C and acidified to pH 2 by addition of HC1 (2M). The mixture was extracted with EtOAc (3 x 40 mL). The combined organic layers were dried (MgSO₄) and concentrated under reduced pressure
Yield: 92%, white solid. ¹H NMR (CDCl₃): δ = 10.97 (br. s, 1H), 2.45 (tq, *J* = 7.1, 6.9 Hz, 1H), 1.64-1.75 (m, 1H), 1.69 (quin, *J* = 7.1, 1H), 1.25-1.37 (m, 8H), 1.18 (d*, J* = 6.9 Hz, 3H), 0.89 (t, *J* = 6.7 Hz, 3H) ppm. ¹³C NMR (CDCl₃): δ = 183.5, 39.4, 33.5, 31.7, 29.2, 27.1, 22.6, 16.8, 14.0 ppm.

### 5-(1-Hydroxy-2-methyloetylidene)-2,2-dimethyl-1,3-dioxane-4,6-dione (IM-38) (JHS284)

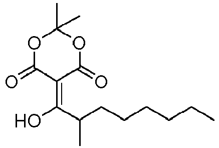

The title compound was prepared from IM-37 according to the general procedure F:
Yield: 64 %, orange oil. Keto/enol = 1:3. ¹H NMR (CDCl₃): δ = 15.46 (br. s, 1H), 4.03 (sxt, *J* = 6.8 Hz, 1H), 1.72 (s, 6H), 1.17-1.50 (m, 10H), 1.20 (d, *J* = 6.8 Hz, 3H), 0.85 (t, *J* = 6.8 Hz, 3H) ppm. ¹³C NMR (CDCl₃): δ = 202.1, 170.8, 160.1, 104.6, 91.0, 37.9, 34.0, 31.6, 29.2, 27.2, 26.8, 22.5, 17.4, 14.0 ppm.

### (2E,4E)-Ethyl 2-methylocta-2,4-dienoate (IM-39) (JHS276)

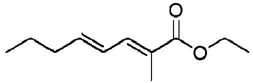

The title compound was prepared according to the following procedure:
(1-Ethoxycarbonylmethyl)triphenylphosphonium bromide (8.86 g, 20.0 mmol) and K₂CO₃ (4.00 g, 28.9 mmoL) were dissolved in a mixture of CH₂Cl₂ (60 mL) and H₂O (50 mL). After the dropwise addition of *E*-2-hexen-1-al (1.50 g, 15.0 mmol) the reaction mixture was heated at reflux overnight. The organic layer was washed with H₂O (2 x 50 mL), dried (MgSO₄) and concentrated *in vacuo.* The residue was purified by flash chromatography (SiO₂, *n-*hexane/EtOAc = 10:1).
Yield: 87%, light yellow oil. 2*E*,4*E*/2*Z*,4*E* = 20:1. ¹H NMR (CDCl₃) 2*E*,4*E*-isomer only: δ = 7.17 (d*, J* = 11.2 Hz, 1H), 6.35 (tq, *J* = 15.0, 11.2, 1.4 Hz, 1H), 6.07 (dt, *J* = 15.0, 7.5 Hz, 1H), 4.21 (q, *J* = 7.1 Hz, 2H), 2.17 (q, *J* = 7.3 Hz, 2H), 1.93 (s, 3H), 1.47 (sxt, *J* = 7.3 Hz, 2H), 1.31 (t*, J* = 7.1 Hz, 3H), 0.93 (t, *J* = 7.3 Hz, 3H) ppm. ¹³C NMR (CDCl₃): δ = 168.7, 142.9, 138.6, 126.1, 125.1, 60.4, 35.3, 22.2, 14.3, 13.7, 12.5 ppm.

### (2E,4E)-2-Methylocta-2,4-dienoic acid (IM-40) (JHS279)

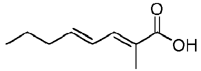

The title compound was prepared from IM-39 according to the following procedure:
To a solution of the ester IM-39 (1.30 g, 7.10 mmol) in MeOH (30 mL) was added a solution ofNaOH (1.32 g, 33.1 mmol) in H₂O (10 mL). The resulting mixture was heated at reflux for 16 h. After cooling to room temperature the MeOH was evaporated *in vacuo.* The aqueous layer was cooled to 0 °C and acidified to pH 2 by addition of HCl (2M). The mixture was extracted with EtOAc (3 x 50 mL). The combined organic layers were dried (MgSO₄) and concentrated under reduced pressure.
Yield. 91%; yellow solid. 2*E*,4*E*/2*Z*,4*E* = 20:1. ¹H NMR (CDCl₃) 2*E*,4*E*-isomer only: δ = 11.36 (br. s, 1H), 7.30 (d, *J* = 11.3 Hz, 1H), 6.38 (dd*, J* = 15.1, 11.3 Hz, 1H), 6.15 (dt, *J* = 15.1, 7.5 Hz, 1H), 2.12-2.28 (m, 2H), 1.94 (s, 3H), 1.41-1.52 (m, 2H), 0.94 (t*, J* = 7.4 Hz, 3H) ppm. ¹³C NMR (CDCl₃): δ = 174.3, 144.5, 141.1, 126.1, 124.1, 35.4, 22.1, 13.7, 12.1 ppm.

### (2E,4E)-Ethyl 2-methyl-5-phenylpenta-2,4-dienoate (IM-41) (JHS277)

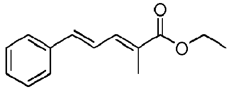

The title compound was prepared according to the following procedure:
(1-Ethoxycarbonylmethyl)triphenylphosphonium bromide (8.86 g, 20.0 mmol) and K₂CO₃ (4.00 g, 28.9 mmoL) were dissolved in a mixture of CH₂Cl₂ (60 mL) and H₂O (50 mL). After the dropwise addition of *E*-cinnamaldehyde (1.98 g, 15.0 mmol) the reaction mixture was heated at reflux overnight. The organic layer was washed with H₂O (2 x 50 mL), dried (MgSO₄) and concentrated *in vacuo.* The residue was purified by flash chromatography (SiO₂, *n*-hexane/EtOAc = 10:1).
Yield: 93%, light yellow oil. 2*E*,4*E*/2*Z*,4*E* = 11:1. ¹H NMR (CDCl₃) 2*E*,4*E*-isomer only: δ = 7.41 (m, 2H), 7.20-7.32 (m, 3H), 7.20 (d*, J* = 11.3 Hz, 1H), □□□□ (dd, *J* = 15.5, 11.3 Hz, 1H), 6.80 (d, *J* = 15.5 Hz, 1H), 4.15 (q, *J* = 7.1 Hz, 2H), 1.98 (s, 3H), 1.26 (t, *J* = 7.1 Hz, 3H) ppm. ¹³C NMR (CDCl₃): δ = 168.4, 138.9, 138.1, 136.6, 128.7, 128.6, 127.5, 127.0, 124.0, 60.6, 14.3, 12.9 ppm.

### (2E,4E)-2-Methyl-5-phenylpenta-2,4-dienoic acid (IM-42) (JHS280)

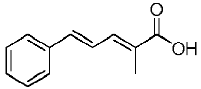

The title compound was prepared from IM-41 according to the following procedure:
To a solution of the ester IM-41 (3.00 g, 14.8 mmol) in MeOH (80 mL) was added a solution ofNaOH (2.77 g, 69.3 mmol) in H₂O (20 mL). The resulting mixture was heated at reflux for 16 h. After cooling to room temperature the MeOH was evaporated *in vacuo.* The aqueous layer was cooled to 0 °C and acidified to pH 2 by addition of HC1 (2M). The mixture was extracted with EtOAc (3 x 100 mL). The combined organic layers were dried (MgSO₄) and concentrated under reduced pressure.
Yield: 97%, white solid. 2*E*,4*E*/2*Z,*4*E* = 11:1. ¹H NMR (CDCl₃) 2*E*,4*E*-isomer only: δ = 11.27 (br. s, 1H), 7.40-7.44 (m, 3H), 7.23-7.32 (m, 2H), 7.22 (d, *J* = 11.2 Hz, 1H), □□□□ (dd, *J* = 15.4, 11.2 Hz, 1H), 6.85 (d, *J* = 15.4 Hz, 1H), 1.99 (s, 3H) ppm. ¹³C NMR (CDCl₃): δ = 173.9, 140.6, 140.2, 136.4, 128.9, 128.8, 127.2, 126.4, 123.7, 12.5 ppm.

### Ethyl (E)-3-methylhex-2-enoate (IM-43) (JHS406)

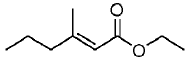

The title compound was prepared according to the following procedure:
To a solution ofNaH (1.46 g, 60.9 mmol) in THF (250 mL) was added dropwise a solution of triethyl phosphonoacetate (13.0 g, 58.1 mmol) in THF (50 mL) at 0 °C. The resulting mixture was allowed to warm to room temperature and stirred for 30 minutes. A solution of 2-pentanone (5.00 g, 58.1 mmol) in THF (50 mL) was slowly added and the reaction was heated to 80 °C overnight. The reaction was quenched by addition of a saturated NH₄Cl solution (200 mL). The resulting mixture was extracted with Et₂O (3 x 100 mL). The combined organic layers were washed with H₂O (100 mL) and brine (100 mL) and dried over MgSO₄. The solvents were evaporated *in vacuo* to afford a yellow liquid. The crude material was purified by flash column chromatography (SiO₂, *n*-hexane/EtOAc = 20:1) to afford the title compound as a colorless liquid.
Yield: 88%, colorless liquid. *E*/*Z* = 3.2:1, ¹H NMR (CDCl₃) *E*-isomer only: δ = 5.66 (q, *J* = 1.2 Hz, 1H), 4.14 (q, *J* = 7.1 Hz, 2H), 2.15 (d, *J* = 1.3 Hz, 3H), 2.08-2.14 (m, 2H), 1.51 (sxt, *J* = 7.4, 2H), 1.28 (t, *J* = 7.1 Hz, 3H), 0.91 (t*, J* = 7.4 Hz, 3H) ppm. ¹³C NMR (CDCl₃): δ = 166.9, 159.9, 115.6, 59.4, 42.9, 20.5, 18.6, 14.3, 13.6 ppm.

### (E)-3-Methylhex-2-enoic acid (IM-44) (JHS408)

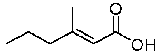

The title compound was prepared from IM-43 according to the following procedure:
To a solution of the ester IM-43 (2.60 g, 16.6 mmol) in MeOH (80 mL) was added a solution ofNaOH (3.30 g, 83.2 mmol) in H₂O (20 mL). The resulting mixture was heated at reflux for 2 h. After cooling to room temperature the MeOH was evaporated *in vacuo.* The aqueous layer was cooled to 0 °C and acidified to pH 2 by addition of HCl (2M). The mixture was extracted with EtOAc (3 x 100 mL). The combined organic layers were dried (MgSO₄) and concentrated under reduced.
Yield: 97%, white solid. ¹H NMR (CDCl₃): δ = 11.75 (br. s, 1H), 5.70 (s, 1H), 2.17 (d, *J* = 1.3 Hz, 3H), 2.12-2.20 (m, 2H), 1.53 (sxt, *J* = 7.4 Hz, 2H), 0.89-0.96 (m, 3H), 0.92 (t, *J* = 7.4 Hz, 3H) ppm. ¹³C NMR (CDCl₃): δ = 172.1, 163.5, 115.0, 43.2, 20.5, 19.0, 13.6 ppm.

### (E)-3-Methylhex-2-en-1-ol (IM-45) (JHS411)

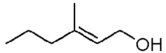

The title compound was prepared from IM-43 according to the following procedure:
A suspension of LiAlH₄ in anhydrous Et₂O was cooled to 0 °C. AlCl₃ was added and the resulting mixture was stirred for 15 minutes. A solution of IM-43 (5.00 g, 32.0 mmol) in Et₂O (15 mL) was added and stirring was continued for 1.5 h at 0 °C. Quenching with an aqueous solution of NaOH (5% m/m, 20 mL) lead to precipitation of aluminium hydroxides. The reaction mixture was filtered over a short bed of MgSO₄ which was rinsed with Et₂O (50 mL). Removal of the solvent *in vacuo* afforded the title compound as a colorless liquid.
Yield: 44%, colorless oil. *E*/*Z* = 4:1, ¹H NMR (CDCl₃) *E*-isomer only: δ = 5.41 (tq, *J* = 6.9, 1.3 Hz, 1H), 4.07-4.20 (m, 2H), 2.00 (t*, J* = 7.5 Hz, 2H), 1.66 (d, *J* = 1.3 Hz, 3H) 1.39-1.51 (m, 2H) 0.89 (t, *J* = 7.4 Hz, 3H) ppm. ¹³C NMR (CDCl₃): δ = 139.9, 123.3, 59.4, 41.6, 20.7, 16.1, 13.7 ppm.

### (E)-3-Methylhex-2-enal (IM-46) (JHS412)

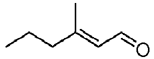

The title compound was prepared from IM-45 according to the following procedure:
To a stirred suspension of PCC (4.28 g, 19.9 mmol) and NaOAc (200 mg, 3.98 mmol) in CH₂Cl₂ (100 mL) a solution of the alcohol IM-45 (1.60 g, 14.0 mmol) in CH₂Cl₂ (100 mL) was added. After 7 h the reaction mixture was diluted with Et₂O (100 mL) and the mixture was filtered over short pad of celite. The solvents were evaporated *in vacuo* and the residue was purified by flash chromatography (SiO₂, *n*-hexane/EtOAc = 20:1 → 10:1).
Yield: 46%, colorless oil. *E*/*Z* = 2:1, ¹H NMR (CDCl₃) *E*-isomer only: δ = 9.99 (d, *J* = 8.0 Hz, 1H), 5.87 (dq, *J* = 8.0, 1.2 Hz, 1H), 2.19 (t, *J* = 7.4 Hz, 2H), 2.15 (d, *J* = 1.2 Hz, 3H), 1.54 (q, *J* = 7.4 Hz, 2H), 0.93 (t, *J* = 7.4 Hz, 3H) ppm. ¹³C NMR (CDCl₃): δ = 191.3, 164.1, 127.4, 42.6, 20.3, 17.4, 13.6 ppm.

### Ethyl (2E,4E)-2,5-dimethylocta-2,4-dienoate (IM-47) (JHS413)

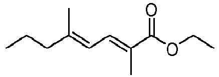

The title compound was prepared from IM-46 according to the following procedure:
To a suspension ofNaH (0.16 g, 6.55 mmol) in THF (30 mL) was added dropwise a solution of triethyl phosphonoacetate (1.49 g, 6.24 mmol) in THF (5 mL) at 0 °C. The resulting mixture was allowed to warm to room temperature and stirred for 30 minutes. IM-46 (0.70 g, 6.24 mmol), dissolved in THF (5 mL) was slowly added and the reaction was stirred at room temperature over night. The reaction was quenched by addition of a saturated NH₄Cl solution (50 mL). The resulting mixture was extracted with Et₂O (3 x 30 mL). The combined organic layers were washed once with H₂O (30 mL) and brine (30 mL) and dried over MgSO₄. The solvents were evaporated *in vacuo* to afford a yellow liquid. The residue was purified by flash column chromatography (SiO₂, *n*-hexane/EtOAc = 20:1) to afford the title compound as a colorless oil.
Yield: 57%, colorless oil. Mixture of regioisomers, approx: 2*E*,4*E* = 53%, 2*E*,4*Z* = 26%, 2*Z*,4*E* = 13%, 2*Z*,4*Z* = 7%; ¹H NMR (CDCl₃): 2*E*,4*E* and 2*E*,4*Z* isomers only: δ = □□58-□□67 (m, 1H), 6.17-6.21 (m, 0.35H), 6.16 (dq, *J* = 11.8, 1.3 Hz, 0.65H), 4.22 (q, *J* = 7.1 Hz, 2H), 2.28 (t*, J* = 7.5 Hz, 0.70H), 2.14 (t, *J* = 7.5 Hz, 1.30H), 1.85-1.95 (m, 6H), 1.43-1.57 (m, 2H), 1.31 (t, *J* = 7.1 Hz, 3H), 0.86-0.98 (m, 3H) ppm. ¹³C NMR (CDCl₃) mixture of regioisomers: δ = 169.1, 169.0, 148.5, 148.3, 148.1, 134.4, 134.1, 124.6, 124.4, 121.5, 120.7, 60.4, 42.8, 39.6, 34.6, 33.1, 31.9, 30.3, 29.7, 24.7, 21.5, 21.0, 20.8, 20.6, 20.5, 17.1, 14.3, 14.2, 14.1, 14.1, 13.9, 13.7, 12.4, 12.3 ppm.

### (2E,4E)-2,5-Dimethylocta-2,4-dienoic acid (IM-48) (JHS414)

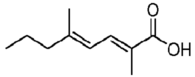

The title compound was prepared from IM-47 according to the following procedure:
To a solution of the ester IM-47 (0.70 g, 3.57 mmol) in MeOH (15 mL) was added a solution of NaOH (0.70 g, 17.5 mmol) in H₂O (5 mL). The resulting mixture was heated at reflux for 2 h. After cooling to room temperature the MeOH was evaporated *in vacuo.* The aqueous layer was cooled to 0 °C and acidified to pH 2 by addition of HCl* (2M). The mixture was extracted with EtOAc (3 x 100 mL). The combined organic layers were dried (MgSO₄) and concentrated under reduced pressure.
Yield: 83 %, yellow oil. Mixture of regioisomers, approx: 2*E*,4*E* = 53%, 2*E*,4*Z* = 26%, 2*Z*,4*E* = 13%, 2*Z*,4*Z* = 7%; ¹H NMR (CDCl₃) 2*E*,4*E* and 2*E*,4*Z* isomers only: δ = □□44-□□51 (m, 1H), 6.12-6.18 (m, 0.35H), 6.12 (dq, *J* = 11.9, 1.2 Hz, 0.65H), 4.22 (q*, J* = 7.1 Hz, 2H), 2.30 (t*, J* = 7.5 Hz, 0.70H), 2.17 (t*, J* = 7.5 Hz, 1.30H), 1.85-1.95 (m, 6H), 1.44-1.59 (m, 2H), 0.86-1.03 (m, 3H) ppm. ¹³C NMR (CDCl₃) mixture of regioisomers: δ = 174.6, 174.5, 173.8, 152.3, 150.2, 149.9, 136.8, 136.5, 124.7, 123.5, 123.4, 121.6, 120.8, 42.8, 39.6, 34.7, 33.2, 24.8, 21.6, 21.0, 20.6, 20.5, 20.5, 17.2, 13.9, 13.7, 12.0, 11.9 ppm.

### Synthesis and spectroscopic data of precursor compounds

### (S)-2-Acetamidoethyl-3-oxobutanethioate (1) (MAGR169)

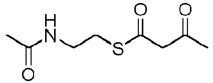

The title compound was prepared from IM-11 according to the general procedure G:
Yield 43 %. Keto/enol = 3:1 ¹H NMR (CDCl₃): δ = 12.56 (s, 0.25H, enol C=COH), 6.12 (s, 1H, NH), 5.45 (s, 0.25H, enol CO=CH), 3.70 (s, 1.50H, keto COCH₂CO), 3.41-3.47 (m, 2H, CH₂N), 3.04-3.10 (m, 2H, CH₂S), 2.25 (s, 2.25H, keto COCH₃), 1.93-1.96 (m, 3.75H, enol COHCH₃ + NHCOCH₃) ppm.¹³C NMR (CDCl₃): δ = 199.8, 194.2, 192.2, 174.0, 170.5, 170.3, 99.8, 58.0, 39.8, 39.1, 30.4, 29.1, 27.7, 23.1, 23.1, 21.0 ppm.

### (S)-2-Acetamidoethyl-3-oxohexanethioate (2) (MAGR170 / JHS 201)

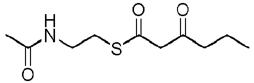

The title compound was prepared from IM-29 according to the general procedure G:
Yield 41 %. Keto/enol = 3:1 ¹H NMR (CDCl₃): δ = 12.60 (s, 0.25H, enol C=COH), 5.94 (s, 1H, NH), 5.47 (s, 0.25H, enol CO=CH), 3.70 (s, 1.50H, keto COCH₂CO), 3.44-3.52 (m, 2H, CH₂N), 3.07-3.13 (m, 2H, CH₂S), 2.53 (t*, J* = 7.2 Hz, 1.50H, keto COCH₂), 2.17 (t, *J* = 7.5 Hz, 0.50H, enol COHCH₂), 1.99 (s, 3H, COCH₃), 1.58-1.70 (m, 2H, CH₂CH₃), 0.92-0.99 (m, 3H, CH₂CH₃) ppm. ¹³C NMR (CDCl₃): δ = 202.1, 194.3, 192.4, 177.4, 170.4, 170.3, 99.2, 57.2, 45.3, 39.9, 39.2, 36.8, 35.8, 29.2, 27.8, 23.2, 23.1, 19.6, 19.5, 16.9, 13.6, 13.5 ppm.

### (S)-2-Acetamidoethyl-(E)-4-methyl-3-oxonon-4-en-8-ynethioate (3) (JHS493)

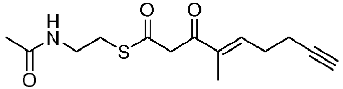

The title compound was prepared from IM-18 according to the general procedure G:
Yield 50 %. Keto/enol = 3:1. ¹H NMR (CDCl₃) keto only: δ = 6.62-6.72 (m, 1H), 6.08 (br. s, 1H), 3.98 (s, 2H), 3.47 (t, *J* = 6.2 Hz, 2H), 3.10 (q, *J* = 6.2 Hz, 2H), 2.28-2.57 (m, 4H), 2.01 (t, *J* = 2.6 Hz, 1H), 1.97 (s, 3H), 1.82 (s, 3H) ppm. ¹³C NMR (CDCl₃): δ = 193.3, 193.1, 170.5, 143.0, 138.1, 82.6, 69.5, 52.6, 39.2, 29.2, 28.1, 23.2, 17.6, 11.5 ppm.

### (4E,6E)-(S)-2-Acetamidoethyl-3-oxodeca-4,6-dienethioate (4) (JHS 210)

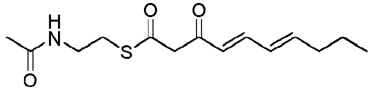

The title compound was prepared from IM-14 according to the general procedure E:
Yield 50 %. Orange powder. Keto/enol = 1:2 ¹H NMR (acetone-d₆) 4*E*,6*E*-enol only: δ = 12.46 (s, 1H), 7.36 (br. s, 1H), 7.13 (dd, *J* = 15.2, 10.7, 1H), 6.27-6.35 (m, 1H), 6.11-6.20 (m, 1H), 5.94 (d, *J* = 15.2 Hz, 1H), 5.65 (s, 1H), 3.37 (q, *J* = 6.6 Hz, 2H), 3.08 (t, *J* = 6.6 Hz, 2H), 2.12-2.22 (m, 2H), 1.87 (s, 3H), 1.41-1.51 (m, 2H), 0.92 (t*, J* = 7.5 Hz, 3H) ppm. ¹³C NMR (acetone-d₆): δ = 195.1, 192.3, 168.5, 143.8, 140.5, 130.5, 123.6, 101.3, 39.9, 35.8, 28.8, 22.9, 22.8, 14.0 ppm.

(4*E*,6*E*)-(*S*)-2-Acetamidoethyl-4-methyl-3-oxodeca-4,6-dienethioate (5) (MAGR41 / MAGR168)

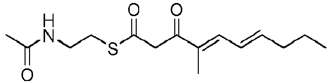

The title compound was from IM-19 prepared according to the general procedure E:
Yield 25 %. Keto/enol = 4:1 4*E*,6*E*/4*Z*,6*E* = 8:1. ¹H NMR (DMSO-d₆) 4*E*,6*E*-isomer keto only: δ = 8.03 (br. s, 1H), 7.21 (d, *J* = 11.0 Hz, 1H), 6.48-6.54 (m, 1H), 6.30 (dt, *J* = 15.1, 7.5 Hz, 1H), 4.15 (s, 2H), 3.17 (m, 2H), 2.92 (t, *J* = 6.9 Hz, 2H), 2.14-2.22 (m, 2H), 1.77-1.79 (m, 6H), 1.40-1.48 (m, 2H), 0.90 (t, *J* = 7.5 Hz, 3H) ppm. ¹³C NMR (DMSO-d₆): δ = 193.8, 193.0, 169.4, 145.6, 141.9, 132.9, 126.8, 52.4, 38.1, 34.8, 28.5, 22.5, 21.6, 13.6, 11.3 ppm.

### (S)-2-Acetamidoethyl-(4E,6E)-4,7-dimethyl-3-oxodeca-4,6-dienethioate (6) (JHS486)

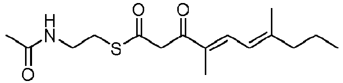

The title compound was from IM-20 prepared according to the general procedure E:
Yield 65 %. Keto/enol = 4:1, 4*E*,6*E*/4*Z*,6*E* = 2:1. ¹H NMR (acetone-d₆) 4*E*,6*E*-isomer keto only: δ = 7.49 (dd, *J* = 11.5, 1.2 Hz, 1H), 7.29 (br. s, 1H), 6.27-6.37 (m, 1H), 4.12 (s, 2H), 3.32 (q, *J* = 6.8 Hz, 2H), 3.02 (t*, J* = 6.8 Hz, 2H), 2.15-2.25 (m, 2H), 1.95 (s, 3H), 1.85 (s, 3H), 1.84 (s, 3H), 1.53 (sxt, *J* = 7.4 Hz, 2H), 0.91 (t*, J* = 7.4 Hz, 3H) ppm. ¹³C NMR (acetone-d₆) δ 194.0, 193.5, 170.2, 151.7, 138.2, 133.9, 122.1, 53.9, 43.5, 39.6, 29.8, 22.9, 21.7, 17.5, 14.1, 11.4 ppm.

### (4E,6E)-(S)-2-Acetamidoethyl-4-methyl-3-oxoundeca-4,6-dienethioate (7) (MAGR42)

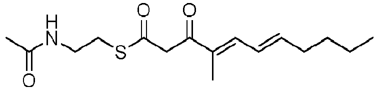

The title compound was prepared from IM-21 according to the general procedure E:
Yield 27 %. Keto/enol = 5.6:1, 4*E*,6*E*/4*Z*,6*E* = 15:1, ¹H NMR (DMSO-d₆) 4*E*,6*E*-isomer only: δ = 12.80 (s, 0.15H, enol C=COH), 8.07 (br. s, 0.15H enol NH), 8.03 (br. s, 0.85H keto NH), 7.22(d, *J* = 11.0 Hz, 0.85H, keto CH=CMe), 7.05 (d, *J* = 11.9 Hz, 0.15H, enol CH=CMe), 6.48-6.54 (m, 1H, CH=CH), 6.30 (dt, *J* = 15.1, 7.6 Hz, 0.85H, keto CHCH₂), 6.16 (dt, *J* = 14.8, 7.5 Hz, 0.15H, enol CHCH₂), 5.87 (s, 0.15H, enol CO=CH), 4.15 (s, 1.70H, keto COCH₂), 3.19-3.25 (m, 0.30H, enol CH₂N), 3.11-3.19 (m, 1.70H, keto CH₂N), 3.00 (t, *J* = 7.0 Hz, 0.30H, enol CH₂S), 2.92 (t, *J* = 6.9 Hz, 1.70H, keto CH₂S), 2.18-2.25 (m, 2H, C=CCH₂), 1.78-1.79 (m, 6H, C=CCH₃ and COCH₃), 1.28-1.44 (m, 4H, CH₂CH₂), 0.90 (t, *J* = 7.6 Hz, 3H, CH₂CH₃) ppm. ¹³C NMR (DMSO-d₆): 4*E*,6*E*-isomer keto only: δ = 193.7, 192.9, 169.2, 145.7, 141.8, 132.8, 126.6, 52.4, 38.1, 32.4, 30.4, 28.4, 22.5, 21.7, 13.7, 11.2 ppm.

### (E)-(S)-2-Acetamidoethyl-4-methyl-3-oxo-7-phenylhept-4-enethioate (8) (MAGR171)

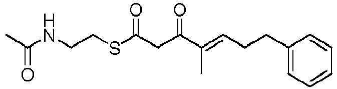

The title compound was prepared from IM-22 according to the general procedure E:
Yield 62 %. Keto/enol = 3:1, ¹H NMR (acetone-d₆): δ = 12.84 (s, 0.25H, enol C=COH), 7.47 (br. s, 1H, NH), 7.21-7.32 (m, 4H, Ar-H), 7.16-7.21 (m, 1H, Ar-H), 6.85 (t, *J* = 7.2 Hz, 0.75H, keto CMe=CH), 6.67 (t, *J* = 7.4 Hz, 0.25H, enol CMe=CH), 5.73 (s, 0.25H, CO=CH), 4.04 (s, 1.50H, keto COCH₂), 3.31-3.42 (m, 2H, CH₂N), 3.08 (t, *J* = 6.8 Hz, 0.50H, keto CH₂S), 3.02 (t, *J* = 6.9 Hz, 1.50H, enol CH₂S), 2.75-2.84 (m, 2 H, CH₂Ph), 2.53-2.65 (m, 2H, CH₂CH=C), 1.90 (s, 0.75H, enol COCH₃), 1.90 (s, 2.25H, keto COCH₃), 1.73 (s, 0.75H, enol C=CCH₃), 1.67 (s, 2.25H, keto C=CCH₃) ppm. ¹³C NMR (acetone-d₆): keto only: δ = 194.1, 193.4, 171.2, 145.7, 145.6, 142.2, 138.0, 129.4, 129.3, 127.0, 53.4, 39.7, 35.1, 32.0, 22.9, 11.3 ppm.

### (4E,6E)-(S)-2-Acetamidoethyl-4-methyl-3-oxo-7-phenylhepta-4,6-dienethioate (9) (JHS206)

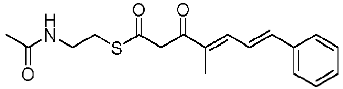

The title compound was prepared from IM-23 according to the general procedure E:
Yield 60 %, yellow solid. Keto/enol = 2:1, ¹H NMR (acetone-d₆): δ = 12.82 (s, 0.35H, enol C=COH), 7.99 (br. s, 1H, NH), 7.60-7.66 (m, 2H), 7.26-7.52 (m, 5H), 7.13 (d, *J* = 14.7 Hz, 0.65H, keto CMe=CH), 6.99 (d, *J* = 13.9 Hz, 0.35H, enol CMe=CH), 5.88 (s, 0.35H, enol CO=CH), 4.19 (s, 1.30H, keto COCH₂), 3.37-3.47 (m, 2H, CH₂N), 3.13 (t*, J* = 6.8 Hz, 0.70H, keto CH₂S), 3.06 (t, *J* = 6.7 Hz, 1.30H, enol CH₂S), 1.98-1.99 (m, 6H, COCH₃, C=CCH₃) ppm.¹³C NMR (acetone-d₆): δ = 194.4, 194.2, 173.3, 173.2, 142.6, 142.1, 139.8, 137.9, 137.5, 136.3, 135.8, 130.0, 129.8, 129.7, 129.5, 128.4, 128.1, 125.3, 125.3, 98.4, 53.3, 40.4, 39.9, 29.3, 28.3, 22.7, 20.9, 12.6, 11.9 ppm.

### (S)-2-Acetamidoethyl-(4E,6E)-7-(3-bromophenyl)-4-methyl-3-oxohepta-4,6-dienethioate (10) (JHS489)

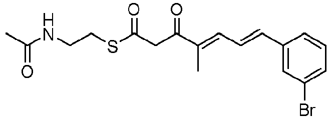

The title compound was prepared from IM-24 according to the general procedure E:
Yield 40 %, yellow solid. Keto/enol = 2:1, ¹H NMR (CDCl₃) keto only: δ = 7.22 (t, *J* = 7.9 Hz, 1H), 7.07-7.15 (m, 1H), 6.89 (d*, J* = 14.8 Hz, 1H), 6.07 (br. s, 1H), 4.05 (s, 1H), 3.48 (quin, *J* = 6.6 Hz, 2H), 3.11 (t, *J* = 6.6 Hz, 2H), 2.02 (d, *J* = 1.0 Hz, 3H), 1.97 (s, 3H) ppm.¹³C NMR (CDCl₃): δ = 193.1, 193.0, 170.4, 140.5, 139.4, 138.9, 136.4, 130.2, 129.9, 126.0, 125.7, 125.2, 123.0, 52.9, 39.2, 29.2, 23.1, 11.9 ppm.

### (4E,6E)-(S)-2-Acetamidoethyl-7-(3-hydroxyphenyl)-4-methyl-3-oxohepta-4,6-dienethioate (11) (JHS226)

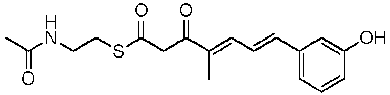

The title compound was prepared from IM-26 according to the following procedure:
To a solution of IM-26 (0.08 g, 0.17 mmol) in THF (1.50 mL) was added a solution of *n*Bu₄NF (1.0 M THF solution, 0.21 mL, 0.21 mmol) at 0 °C. After stirring for 6 h at room temperature, the reaction was stopped by adding saturated aqueous NH₄Cl (10 mL). The crude products were extracted with EtOAc (3 x 10 mL), and the combined organic layers were washed with brine, dried (MgSO₄), and concentrated *in vacuo.* The residue was purified by flash column chromatography (SiO₂, *n*-hexane/EtOAc = 1:1 → EtOAc) to afford the title compound.
Yield 92%, yellow solid. ¹H NMR (DMSO-d₆) keto only: δ = 9.51 (s, 1H), 8.03 (t, *J* = 5.30 Hz, 1H), 7.13-7.44 (m, 3H), 6.86-7.13 (m, 3H), 6.68-6.80 (m, 1H), 4.20 (s, 2H), 3.18 (q, *J* = 6.70 Hz, 2H), 2.94 (t, *J* = 6.70 Hz, 2H), 1.92 (s, 3H), 1.79 (s, 3H) ppm.¹³C NMR (DMSO-d₆): δ = 193.5, 192.9, 169.3, 157.7, 141.6, 141.2, 137.4, 134.8, 129.8, 124.4, 118.5, 116.4, 113.9, 52.5, 38.1, 28.5, 22.5, 11.5 ppm.

### (4E,6E)-(S)-2-Acetamidoethyl-10-((tert-butyldimethylsilyl)oxy)-4-methyl-3-oxodeca-4,6-dienethioate (12) (JHS247B)

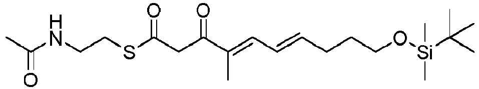

The title compound was prepared from IM-27 according to the general procedure E:
Yield 99 %. Keto/enol = 3:1 ¹H NMR (acetone-d₆): δ = 12.86 (s, 0.25H, enol C=COH), 7.40-7.60 (m, 1H, NH), 7.22 (d, *J* = 10.7 Hz, 0.75H, keto CH=CMe), 7.11 (d, *J* = 11.3 Hz, 0.25H, enol CH=CMe), 6.84 (m, 0.25H), 6.48-6.61 (m, 0.75H, CH=CH), 6.32 (dt, *J* = 15.0, 7.3 Hz, 0.75H, keto CHCH₂), 6.13 (dt, *J* = 14.8, 7.2 Hz, 0.25H, enol CHCH₂), 5.81 (s, 0.25H, enol CO=CH), 4.09 (s, 1.50H, keto COCH₂), 3.66-3.71 (m, 2H, CH₂O), 3.32-3.43 (m, 2H, CH₂N), 3.00-3.12 (m, 2H, CH₂S), 1.91-1.60 (m, 8H), 0.90 (s, 9H), 0.06 (s, 6H) ppm. ¹³C NMR (acetone-d₆): δ = 194.2, 193.6, 171.3, 171.0, 146.0, 142.5, 134.4, 127.7, 62.9, 60.6, 53.4, 39.7, 32.9, 32.8, 32.4, 26.4, 22.8, 20.9, 18.9, 14.6, 11.6, -5.1 ppm.

### (4E,6E)-(S)-2-Acetamidoethyl-10-hydroxy-4-methyl-3-oxodeca-4,6-dienethioate (13) (JHS266)

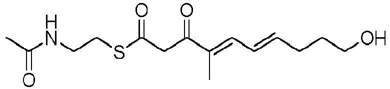

The title compound was prepared from 12 according to the following procedure:
To a solution of 12 (0.17 g, 0.40 mmol) in THF (4.0 mL) was added a solution of *n*Bu₄NF (1.0M THF solution, 1.0 mL, 1.00 mmol) at 0 °C. After stirring for 6 h at room temperature, the reaction was stopped by adding saturated aqueous NH₄Cl (30 mL). The crude products were extracted with EtOAc (3 x 30 mL), and the combined organic layers were washed with brine, dried (MgSO₄), and concentrated *in vacuo.* The residue was purified by flash column chromatography (SiO₂, *n*-hexane/EtOAc = 1:1 → EtOAc) to afford the title compound.
Yield 96 %, yellow oil. Keto/enol = 3:1.¹H NMR (acetone-d₆): δ = 12.75 (s, 0.25H, enol C=COH), 7.92-8.01 (m, 1H, NH), 7.22 (d, *J* = 10.7 Hz, 0.75H, keto CH=CMe), 7.05 (d, *J*= 11.3 Hz, 0.25H, enol CH=CMe), 6.46-6.60 (m, 1H, CH=CH), 6.32 (dt, *J* = 15.0, 7.3 Hz, 0.75H, keto CHCH₂), 6.13 (dt, *J* = 14.8, 7.2 Hz, 0.25H, enol CHCH₂), 5.77 (s, 0.25H, enol CO=CH), 4.09 (s, 1.50H, keto COCH₂), 3.57 (t, *J* = 6.5 Hz, 2H, CH₂OH), 3.30-3.42 (m, 2H, CH₂N), 3.08 (t, *J* = 6.8 Hz, 0.50H, enol CH₂S), 3.01 (t, *J* = 6.7 Hz, 1.50H, ketoCH₂S), 2.21-2.31 (m, 2H, C=CCH₂), 1.95 (s, 0.75H, enol COCH₃), 1.94 (s, 2.25H, keto COCH₃), 1.84 (s, 0.75H, enol C=CCH₃), 1.79 (s, 2.25H, keto C=CCH₃), 1.60-1.69 (m, 2H, CH₂CH₂OH) ppm. ¹³C NMR (acetone-d₆): δ = 194.7, 194.4, 173.5, 173.4, 146.6, 143.7, 143.1, 136.1, 134.2, 127.5, 127.3, 62.1, 53.1, 40.4, 40.3, 39.9, 38.6, 32.8, 32.5, 30.7, 28.1, 22.7, 12.3, 11.6 ppm.

### (E)-(S)-2-Acetamidoethyl-4-methyl-3-oxodec-4-enethioate (14) (JHS 291)

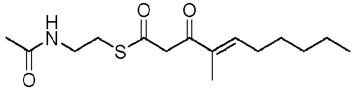

The title compound was prepared from IM-28 according to the general procedure E:
Yield 66%, yellow oil. Keto/enol = 3:1 ¹H NMR (acetone-d₆) 4*E*,6*E*-isomer keto only: δ = 7.35 (br. s, 1H, NH), 6.81 (t, *J* = 7.3 Hz, 1H), 4.06 (s, 2H), 3.30-3.41 (m, 2H), 3.01 (t, *J* = 6.7 Hz, 2H), 2.21-2.34 (m, 2H), 2.53-2.65 (m, 2H), 1.87 (s, 3H), 1.75 (s, 3H), 1.42-1.55 (m, 2H), 1.30-1.37 (m, 4H), 0.89 (t, *J* = 7.0 Hz. 3H) ppm. ¹H NMR (acetone-d₆): δ = 194.1, 193.4, 170.6, 146.9, 137.5, 53.5, 39.6, 32.3, 29.8, 29.6, 28.9, 23.2, 22.9, 14.3, 11.4 ppm.

### (4E,6E)-(S)-2-Acetamidoethyl-4-ethyl-3-oxo-7-phenythepta-4,6-dienethioate (15) (JHS246)

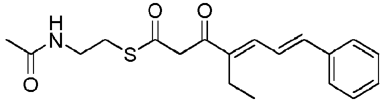

The title compound was prepared from IM-32 according to the general procedure E:
Yield 88 %, yellow resin. Keto/enol = 2:1, 4*E,*6*E*/4*Z,*6*E* = 10:1. ¹H NMR (acetone-d₆) 4*E*,6*E-i*somer only: δ = 12.99 (br. s, 0.35H, enol C=COH), 7.58-7.69 (m, 2H), 7.24-7.57 (m, 6H), 7.08-7.18 (m, 0.65H). 7.00 (d, *J* = 14.5 Hz, 0.35H), 5.95 (s, 0.35H, enol CO=CH), 4.19 (s, 1.30H, keto COCH₂), 3.33-3.45 (m, 2H, CH₂N), 3.12 (t, *J* = 6.8 Hz, 0.70H, enol CH₂S), 3.05 (t, *J* = 6.7 Hz, 1.30H, keto CH₂S), 2.56 (q, *J* = 7.5 Hz, 0.70H, enol CH₂CH₃), 2.55 (q, *J* = 7.5 Hz, 1.30H, keto CH₂CH₃), 1.92 (s, 1.05H, enol COCH₃), 1.90 (s, 1.95H, keto COCH₃), 1.11 (s, 1.05H, enol CH₂CH₃), 1.00 (s, 1.95H, keto CH₂CH₃) ppm.¹³C NMR (acetone-d₆): δ = 193.6, 193.5, 171.0, 142.7, 142.1, 142.0, 140.1, 137.9, 135.4, 130.0, 129.8, 129.7, 129.6, 128.4, 128.1, 125.0, 124.9, 98.2, 53.6, 40.0, 39.7, 29.6, 28.8, 22.9, 20.3, 19.8, 15.2, 14.7 ppm.

### (S)-2-Acetamidoethyl-8-(methoxycarbonylamino)-3-oxooctanethioate (16) (JHS 274)

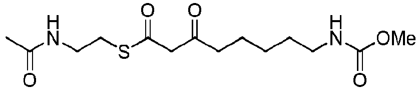

The title compound was prepared from IM-34 according to the general procedure G:
Yield 56 %, white solid. Keto/enol = 5:1 ¹H NMR (CDCl₃) keto only: δ = 6.25 (br. s, 1H), 4.90 (br. s, 1H, NHCOOCH₃), 3.62 (s, 2H), 3.58 (s, 3H), 3.37 (q, *J* = 6.2 Hz, 2H), 3.09 (q, *J* = 6.4 Hz, 2H), 3.02 (t, *J* = 6.4 Hz, 2H), 2.48 (t, *J* = 7.2 Hz, 2H), 1.91 (s, 3H), 1.38-1.59 (m, 4H), 1.19-1.30 (m, 2H) ppm. ¹³C NMR (CDCl₃): δ = 202.0, 192.2, 170.6, 157.2, 99.3, 57.3, 52.0, 43.1, 39.1, 29.7, 29.2, 25.9, 23.1, 22.8 ppm.

### (S)-2-Acetamidoethyl-4-methyl-3-oxohept-6-enethioate (17) (JHS287)

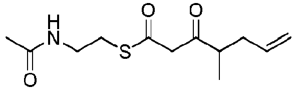

The title compound was prepared from IM-35 according to the general procedure G:
Yield 49%, yellow oil. Keto/enol = 2.3:1, ¹H NMR (acetone-d₆) keto only: δ = 7.63 (br. s, 1H), 5.69-5.83 (m, 1H), 5.00-5.10 (m, 2H), 3.89 (s, 2H), 3.32-3.41 (m, 2H), 3.04 (t, *J* = 6.7 Hz, 2H), 2.78 (sxt, *J* = 7.0, 1H), 2.31-2.42 (m, 1H), 2.05-2.22 (m, 1H), 1.91 (s, 3H), 1.08 (d, *J= 7.0* Hz, 3H) ppm. ¹³C NMR (acetone-d₆): δ = 205.7, 192.8, 171.3, 136.5, 117.3, 56.4, 46.8, 39.7, 37.3, 29.5, 22.9, 15.8 ppm.

### (S)-2-Acetamidoethyl-4-methyl-3-oxodecanethioate (18) (JHS 285)

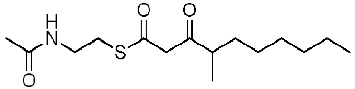

The title compound was prepared from IM-38 according to the general procedure G:
Yield 75%, yellow resin. Keto/enol = 3:1 ¹H NMR (acetone-d₆) keto only: δ = 7.60 (br. s, 1H), 3.87 (s, 2H), 3.35 (quin, *J* = 6.8 Hz, 2H), 3.05 (q, *J* = 6.8 Hz, 2H), 2.70 (quin, *J* = 6.8 Hz, 1H), 1.91 (s, 3H), 1.25-1.39 (m, 10H), 1.07 (d, *J* = 6.8 Hz, 3H), 0.87 (t, *J* = 6.8 Hz, 3H)ppm. ¹³C NMR (acetone-d₆): δ = 206.0, 193.0, 172.3, 56.2, 47.3, 39.8, 33.2, 32.4, 30.0, 29.3, 27.7, 23.3, 22.8, 16.1, 14.4 ppm.

### (2E,4E)-(S)-2-Acetamidoethyl-2-methylocta-2,4-dienethioate (19) (JHS282)

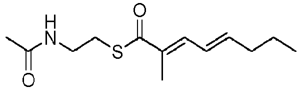

The title compound was prepared from IM-40 according to the general procedure H:
Yield 30%, yellow oil. ¹H NMR (acetone-d₆): δ = 7.69 (br. s, 1H), 7.16 (d, *J* = 10.9 Hz, 1H), 6.46 (d, *J* = 15.1, 10.9 Hz, 1H), 6.24 (d, *J* = 15.1, 7.2 Hz, 1H), 3.31-3.39 (m, 2H), 3.05 (t, *J* = 6.8 Hz, 2H), 2.18-2.24 (m, 2H), 1.94 (s, 3H), 1.93 (s, 3H), 1.44-1.52 (m, 2H), 0.92 (t, *J* = 7.4 Hz, 3H) ppm.¹³C NMR (acetone-d₆): δ = 193.0, 171.3, 145.6, 138.4, 133.5, 126.7, 40.0, 36.0, 28.9, 22.9, 22.8, 14.0, 12.7 ppm.

### (2E,4E)-(S)-2-Acetamidoethyl-2-methyl-5-phenylpenta-2,4-dienethioate (20) (JHS283)

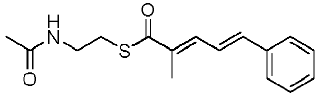

The title compound was prepared from IM-42 according to the general procedure H:
Yield 43%, yellow oil. 2*E*,4*E*/2*Z*,4*E* = 14:1, ¹H NMR (acetone-d₆) 4*E*,6*E*-isomer only: δ = 7.74 (t, *J* = 5.1 Hz, 1H), 7.60-7.63 (m, 2H), 7.28-7.40 (m, 4H), 7.28 (dd, *J* = 14.6, 11.0 Hz, 1H), 7.05 (d, *J* = 14.6 Hz, 1H), 3.38-3.44 (m, 2H), 3.11 (t, *J* = 6.7 Hz, 2H), 2.08 (s, 3H) ppm.¹³C NMR (acetone-d₆): δ =192.9, 170.9, 141.5, 138.2, 137.6, 135.7, 129.9, 129.7, 128.3, 124.6, 39.9, 29.2, 22.9, 13.0 ppm.

### (S)-2-Acetamidoethyl butanethioate (21) (JHS401)

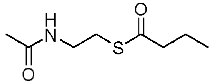

The title compound was prepared from butyryl chloride according to the general procedure H:
Yield 81%, yellow oil. ¹H NMR (acetone-d₆) δ = 7.36 (br. s, 1H), 3.28-3.34 (m, 2H), 2.98 (t, *J* = 6.9 Hz, 2H), 2.55 (t, *J* = 7.4 Hz, 2H), 1.87 (s, 3H), 1.59-1.70 (m, 2 H), 0.92 (t, *J* = 7.4 Hz, 3H) ppm. ¹³C NMR (acetone-d₆) δ = 199.1, 170.6, 46.4, 39.8, 29.0, 22.9, 19.8, 13.7 ppm.

### (S)-2-Acetamidoethyl-(E)-3-methylhex-2-enethioate (22) (JHS410)

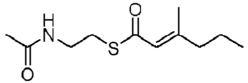

The title compound was prepared from IM-44 according to the general procedure H:
Yield 66%, light yellow oil. *E*/*Z* = 3:1, ¹H NMR (acetone-d₆): *E*-isomer only: δ = 7.29 (br. s, 1H), 6.01 (q, *J* = 1.2 Hz, 1H), 3.30-3.35 (m, 2H), 3.01 (t, *J* = 6.9 Hz, 2 H), 2.13-2.17 (m, 2H), 2.13 (d, *J* = 1.2 Hz, 3H), 1.87 (s, 3H), 1.48-1.57 (m, 2H), 0.91 (t, *J* = 7.6 Hz, 3H) ppm. ¹³C NMR (acetone-d₆) δ = 189.0, 170.3, 158.5, 123.4, 43.2, 40.0, 29.1, 22.9, 21.4, 19.7, 14.0 ppm.

### (S)-2-Acetamidoethyl-(2E,4E)-2,5-dimethylocta-2,4-dienethioate (23) (JHS415)

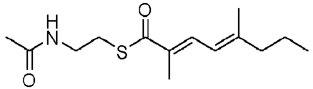

The title compound was prepared from IM-48 according to the general procedure H:
Yield 43%, yellow oil. Mixture of regioisomers, approx: 2*E*,4*E* = 53%, 2*E*,4*Z* = 26%, 2*Z*,4*E* = 13%, 2*Z*,4*Z* = 7%; ¹H NMR (acetone-d₆) 2*E*,4*E* and 2*E*,4*Z*-isomers only: δ = □□58-□□67 (m, 2H, C=CH + NH), 6.25-6.29 (m, 0.35H, C=CH), 6.22 (dq, *J* = 11.5, 1.3 Hz, 0.65H, C=CH), 3.29-3.37 (m, 2H, CH₂), 3.01-3.06 (m, 2H), 2.30 (t, *J=* 7.5 Hz, 0.70H, CH₂C=C), 2.18 (t, *J* = 7.5 Hz, 1.30H, CH₂C=C), 1.85-1.95 (m, 9H, CH₃C=C + CH₃CO), 1.45-1.57 (m, 2H, CH₂CH₃), 0.84-0.94 (m, 3H, CH₂CH₃) ppm. ¹³C NMR (acetone-d₆) mixture of regioisomers: δ = 192.9, 192.9, 170.4, 151.1, 150.9, 147.5, 144.5, 134.0, 133.8, 133.2, 133.0, 122.1, 121.3, 43.3, 40.2, 39.9, 39.7, 35.3, 33.9, 29.1, 24.9, 22.9, 22.3, 21.7, 21.3, 21.2, 21.0, 20.9, 17.4, 14.5, 14.2, 14.0, 12.6, 12.5 ppm.

### (S)-2-Acetamidoethyl benzothioate (24) (JHS417)

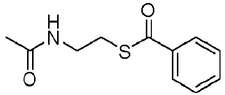

The title compound was prepared from benzoic acid according to the general procedure H:
Yield 75%, white resin. ¹H NMR (acetone-d₆) δ = 7.92-8.00 (m, 2H), 7.67 (tt, *J* = 7.4, 1.3 Hz, 1H), 7.54 (tt, *J* = 7.4, 1.3, 2H), 7.38 (br. s, 1H), 3.36-3.50 (m, 2H), 3.20 (t, *J* = 6.9 Hz, 2H), 1.88 (s, 3H) ppm. ¹³C NMR (acetone-d₆) δ = 191.8, 170.3, 138.0, 134.6, 129.8, 127.9, 39.7, 22.9 ppm.

### (S)-2-Acetamidoethyl-3-hydroxybenzothioate (25) (JHS427)

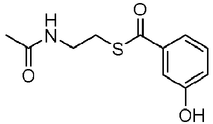

The title compound was prepared from 3-hydroxybezoic acid according to the general procedure H:
Yield 62%, white solid. ¹H NMR (acetone-d₆): δ = 9.15 (br. s, 1H), 7.54 (br. s, 1H), 7.40-7.46 (m, 2H), 7.29-7.39 (m, 1H), 7.07-7.17 (m, 1H), 3.38-3.50 (m, 2H), 3.19 (t, *J* = 6.7 Hz, 2H), 1.92 (s, 3H) ppm. ¹³C NMR (acetone-d₆) δ = 191.6, 171.0, 158.9, 139.3, 130.9, 121.7, 119.1, 114.4, 39.8, 29.4, 22.9 ppm.

### (4E,6E)-(S)-Phenyl-4-methyl-3-oxo-7-phenylhepta-4,6-dienethioate (26) (JHS289)

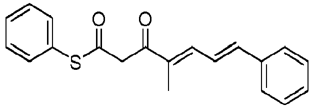

The title compound was prepared from IM-23 according to the general procedure I:
Yield 84%, yellow solid. Keto/enol = 1.2:1, ¹H NMR (acetone-d₆): δ = 12.78 (s, 0.45H, enol C=COH), 7.67-7.61 (m, 2H), 7.61-7.67 (m, 10H), 7.12 (d, *J* = 14.1 Hz, 0.55H, keto CMe=CH), 7.02 (d, *J* = 13.6 Hz, 0.45H, enol CMe=CH), 5.94 (s, 0.45H, enol CO=CH), 4.26 (s, 1.10H, keto COCH₂), 2.04 (s, 1.65H, keto C=CCH₃), 2.01 (s, 1.35H, enol C=CCH₃) ppm. ¹³C NMR (acetone-d₆): δ = 194.0, 193.8, 191.7, 171.6, 142.2, 141.8, 140.2, 137.9, 137.5, 136.4, 136.3, 135.9, 135.4, 130.5, 130.2, 130.2, 130.0, 129.8, 129.7, 129.6, 129.5, 128.8, 128.4, 128.2, 125.4, 125.2, 97.4, 53.0, 12.6, 11.9 ppm

## Claims

1. Process for producing a compound having a first chain (Western chain) and a second chain (Eastern chain) which are condensed forming a pyrone ring by providing in a microorganism synthetic pathway enzymes catalysing the synthesis of the compound, wherein the microorganism is genetically manipulated to comprise genes encoding synthetic pathway enzymes comprising the enzymes MxnA (SEQ ID NO: 1), MxnB (SEQ ID NO: 2), MxnC (SEQ ID NO: 3), MxnD (SEQ ID NO: 4), MxnE (SEQ ID NO: 5), MxnF (SEQ ID NO: 6), MxnG (SEQ ID NO: 7), MxnH (SEQ ID NO: 8), MxnI (SEQ ID NO: 9), MxnJ (SEQ ID NO: 10) and MxnK (SEQ ID NO: 11), incubating the microorganism and separating the compound from the microorganism,
**characterized in that**
at least one catalytic domain of an enzyme is absent or inactivated which domain is selected from the group consisting of
a) the ketosynthase (KS) of module 1E of MxnI comprising amino acids No. 694-885 of SEQ ID NO: 9,
b) the ketosynthase (KS) of module 2E of MxnI comprising amino acids No. 1945-2135 of SEQ ID NO: 9,
c) the ketosynthase (KS) of module 3E of MxnI comprising amino acids No. 3442-3633 of SEQ ID NO: 10,
d) the ketosynthase (KS) of module 4E of MxnJ comprising amino acids No. 605-795 of SEQ ID NO: 10,
e) the ketosynthase (KS) of module 5E of MxnJ comprising amino acids No. 2476-2660 of SEQ ID NO: 10,
f) the ketosynthase (KS) of module 6E of MxnJ comprising amino acids No. 3208-3399 of SEQ ID NO: 10,
g) the ketosynthase (KS) of module 1W of MxnK comprising amino acids No. 180-364 of SEQ ID NO: 11,
h) the ketosynthase (KS) of module 2W of MxnK comprising amino acids No. 1926-2110 of SEQ ID NO: 11,
i) the ketosynthase (KS)of module 3W of MxnK comprising amino acids No. 2684-2875 of SEQ ID NO: 11,
j) the ketosynthase (KS) of module 4W of MxnK comprising amino acids No. 3504-3688 of SEQ ID NO: 11,
k) the ketosynthase (KS) module 5W of MxnK comprising amino acids No. 5409-5593 of SEQ ID NO: 11,
l) the ketoreductase (KR) of module 2E of MxnI comprising amino acids No. 2903-3050 of SEQ ID NO: 9,
m) the ketoreductase (KR) of module 4E of MxnJ comprising amino acids No. 1568-1715 of SEQ ID NO: 10,
n) the ketoreductase (KR) of module 1W of MxnK comprising amino acids No. 1418-1560 of SEQ ID NO: 11,
o) the ketoreductase (KR) of module 4W of MxnK comprising amino acids No. 4470-4617 of SEQ ID NO: 11,
p) the carrier protein (CP) of module L of MxnI comprising amino acids No. 424-433 of SEQ ID NO:9,
q) the carrier protein (CP) of module 1E of MxnI comprising amino acids No. 1720-1726 of SEQ ID NO: 9,
r) the carrier protein (CP) of module 2E of MxnI comprising amino acids No. 3192-3198 of SEQ ID NO: 9,
s) the carrier protein (CP) of module 3E of MxnJ comprising amino acids No. 366-372 of SEQ ID NO: 9,
t) the carrier protein (CP) of module 4E of MxnJ comprising amino acids No. 2238-2244 of SEQ ID NO: 10,
u) the carrier protein (CP) of module 5E of MxnJ comprising amino acids No. 2967-2973 of SEQ ID NO: 10,
v) the carrier protein (CP) of module 6E of MxnJ comprising amino acids No. 3810-3816 of SEQ ID NO: 10,
w) the carrier protein (CP*) of module 6E of MxnJ comprising amino acids No. 3566-3572 of SEQ ID NO: 12,
x) the carrier protein (CP) of module 1W of MxnK, comprising amino acids No. 1683-1689 of SEQ ID NO: 11
y) the carrier protein (CP) of module 2W of MxnK comprising amino acids No. 2429-2435 of SEQ ID NO: 11,
z) the carrier protein (CP) of module 3W of MxnK comprising amino acids No. 3234-3240 of SEQ ID NO: 11,
aa) the carrier protein (CP) of module 4W of MxnK comprising amino acids No. 5165-5171 of SEQ ID NO: 11,
bb) the carrier protein (CP*) of module 5W of MxnK comprising amino acids No. 5894-5900 of SEQ ID NO: 11,
cc) the carrier protein (CP) of module 5W of MxnK comprising amino acids No. 5984-5990 of SEQ ID NO: 11,
dd) the dehydratase (DH) of module 2E of MxnI comprising amino acids No. 2402-2414 of SEQ ID NO: 9,
ee) the dehydratase (DH) of module 3E of MxnJ comprising amino acids No. 53-65 of SEQ ID NO: 9,
ff) the dehydratase (DH) of module 4E of MxnJ comprising amino acids No. 1091-1103 of SEQ ID NO: 10,
gg) the dehydratase (DH) of module 1W of MxnK comprising amino acids No. 955-967 of SEQ ID NO: 11,
hh) the dehydratase (DH) of module 4W of MxnK comprising amino acids No. 3980-3992 of SEQ ID NO: 11,
ii) the methyltransferase (MT) of module 4E of MxnJ comprising amino acids No. 1982-1987 of SEQ ID NO: 10,
jj) the methyltransferase (MT) of module 4W of MxnK comprising amino acids No. 4914-4919 of SEQ ID NO: 11,
wherein in embodiments in which at least one catalytic domain is inactivated in the synthetic pathway enzymes MxnK and/or MxnI-MxnJ, wherein the inactivated catalytic domain is a catalytic domain the inactivation of which in the absence of precursor results in a stop of synthesis by the enzyme when expressed in a microorganism, a Western chain precursor, and/or an Eastern chain precursor is added to a microorganism expressing the synthetic pathway enzymes, or wherein in embodiments in which the inactivation is by exchange of at least one catalytic center, domain, module or enzyme, the exchange is by insertion of at least one catalytic center, domain, module or enzyme selected from the enzymes CorJ (SEQ ID NO: 25), CorK (SEQ ID NO: 26), CorL (SEQ ID NO: 27), Orfl (SEQ ID NO: 27), CorM (SEQ ID NO: 28), CorM (SEQ ID NO: 28), CorN (SEQ ID NO: 29) and CorO (SEQ ID NO: 30) and their catalytic centers, domains and modules, or enzyme MxnI (SEQ ID NO: 9) is replaced by enzyme CorI (SEQ ID NO: 24).

2. Process according to claim 1, **characterized in that** at least one of enzyme MxnA (SEQ ID NO: 1) is replaced by enzyme CorA (SEQ ID NO: 16), enzyme MxnB (SEQ ID NO: 2) is replaced by enzyme CorB (SEQ ID NO: 17), enzyme MxnC (SEQ ID NO: 3) is replaced by enzyme CorC (SEQ ID NO: 18), enzyme MxnD (SEQ ID NO: 4) is replaced by enzyme CorD (SEQ ID NO: 19), enzyme MxnE (SEQ ID NO: 5) is replaced by enzyme CorE (SEQ ID NO: 20), enzyme MxnF (SEQ ID NO: 6) is replaced by enzyme CorF (SEQ ID NO: 21), enzyme MxnG (SEQ ID NO: 7) is replaced by enzyme CorG (SEQ ID NO: 22), enzyme MxnH (SEQ ID NO: 8) is replaced by enzyme CorH (SEQ ID NO: 23), enzyme MxnJ (SEQ ID NO: 10) is replaced by enzyme CorJ (SEQ ID NO: 25), enzyme MxnK (SEQ ID NO: 11) is replaced by enzymes CorK (SEQ ID NO: 26) and CorL (SEQ ID NO: 27).

3. Process according to claim 2, **characterized in that** at least one catalytic domain of an enzyme is absent or inactivated, which domain is selected from the group consisting of
a) the ketosynthase (KS) of module 1E of CorI comprising amino acids No. 688-880 of SEQ ID NO: 23,
b) the ketosynthase (KS) of module 2E of CorI comprising amino acids No. 1925-2115 of SEQ ID NO: 23,
c) the ketosynthase (KS) of module 3E of CorI comprising amino acids No. 3416-3607 of SEQ ID NO: 23,
d) the ketosynthase (KS) of module 4E of CorJ comprising amino acids No. 586-776 of SEQ ID NO: 24,
e) the ketosynthase (KS) of module 5E of CorJ comprising amino acids No. 2448-2632 of SEQ ID NO: 24,
f) the ketosynthase (KS) of module 6E of CorJ comprising amino acids No. 3172-3363 of SEQ ID NO: 24,
g) the ketosynthase (KS) of module 1W of CorK comprising amino acids No. 171-355 of SEQ ID NO: 25,
h) the ketosynthase (KS) of module 2W of CorK comprising amino acids No. 1898-2082 of SEQ ID NO: 25,
i) the ketosynthase (KS) of module 3W of CorK comprising amino acids No. 3362-3550 of SEQ ID NO: 25,
j) the ketosynthase (KS) of module 4W of CorL comprising amino acids No. 449-633 of SEQ ID NO: 26,
k) the ketosynthase (KS) of module 5W of CorL comprising amino acids No. 1678-1869 of SEQ ID NO: 26,
l) the ketosynthase (KS) of module 6W of CorL comprising amino acids No. 2484-2668 of SEQ ID NO: 26,
m) the ketosynthase (KS) of module 7W of CorL comprising amino acids No. 4355-4539 of SEQ ID NO: 26,
n) the ketoreductase (KR) of module 2E of CorI comprising amino acids No. 2874-3021 of SEQ ID NO: 23,
o) the ketoreductase (KR) of module 4E of CorJ comprising amino acids No. 1548-1695 of SEQ ID NO: 24,
p) the ketoreductase (KR) of module 1W of CorK comprising amino acids No. 1401-1543 of SEQ ID NO: 25,
q) the ketoreductase (KR) of module 2W of CorK, comprising amino acids No. 2826-2972 of SEQ ID NO: 25,
r) the ketoreductase (KR) of module 4W of CorL comprising amino acids No. 1139-1285 of SEQ ID NO: 26,
s) the ketoreductase (KR) of module 6W of CorL comprising amino acids No. 3427-3574 of SEQ ID NO: 26,
t) the carrier protein (CP) of module L of CorI comprising amino acids No. 416-425 of SEQ ID NO: 23,
u) the carrier protein (CP) of module 1E of CorI comprising amino acids No. 1706-1712 of SEQ ID NO: 23,
v) the carrier protein (CP) of module 2E of CorI comprising amino acids No. 3166-3172 of SEQ ID NO: 23,
w) the carrier protein (CP) of module 3E of CorJ comprising amino acids No. 350-356 of SEQ ID NO: 24,
x) the carrier protein (CP) of module 4E of CorJ comprising amino acids No. 2206-2212 of SEQ ID NO: 24,
y) the carrier protein (CP) of module 5E of CorJ comprising amino acids No. 2935-2941 of SEQ ID NO: 24,
z) the carrier protein (CP) of module 6E* of CorJ comprising amino acids No. 3530-3536 of SEQ ID NO: 24,
aa) the carrier protein (CP) of module 6E of CorJ comprising amino acids No. 3765-3771 of SEQ ID NO: 24,
bb) the carrier protein (CP) of module 1W of CorK comprising amino acids No. 1663-1669 of SEQ ID NO: 25,
cc) the carrier protein (CP) of module 2W of CorK comprising amino acids No. 3109-3115 of SEQ ID NO: 25,
dd) the carrier protein (CP) of module 3W* of CorK comprising amino acids No. 3900-3906 of SEQ ID NO: 25,
ee) the carrier protein (CP) of module 3W of CorK comprising amino acids No. 3994-4090 of SEQ ID NO: 25,
ff) the carrier protein (CP) of module 4W of CorL comprising amino acids No. 1443-1449 of SEQ ID NO: 25,
gg) the carrier protein (CP) of module 5W of CorL comprising amino acids No. 2208-2214 of SEQ ID NO: 26,
hh) the carrier protein (CP) of module 6W of CorL comprising amino acids No. 4116-4122 of SEQ ID NO: 26,
ii) the carrier protein (CP) of module 7W* of CorL comprising amino acids No. 4826-4832 of SEQ ID NO: 26,
jj) the carrier protein (CP) of module 7W of CorL comprising amino acids No. 4920-4926 of SEQ ID NO:26,
kk) the dehydratase (DH) of module 2E of CorI comprising amino acids No. 2380-2392 of SEQ ID NO: 23,
ll) the dehydratase (DH) of module 3E of CorJ comprising amino acids No. 44-56 of SEQ ID NO: 24,
mm) the dehydratase (DH) of module 4E of CorJ comprising amino acids No. 1073-1085 of SEQ ID NO: 24,
nn) the dehydratase (DH) of module 1W of CorK comprising amino acids No. 942-954 of SEQ ID NO: 25,
oo) the dehydratase (DH) of module 2W of CorK comprising amino acids No. 2319-2331 of SEQ ID NO: 25,
pp) the dehydratase (DH) of module 4W of CorL comprising amino acids No. 19-31 of SEQ ID NO: 26,
qq) the dehydratase (DH) of module 6W of CorL comprising amino acids No. 2949-2961 of SEQ ID NO: 26,
rr) the methyltransferase (MT) of module 4E of CorJ comprising amino acids No. 1962-1967 of SEQ ID NO: 24,
ss) the methyltransferase (MT) of module 6W of CorL comprising amino acids No. 3865-3870 of SEQ ID NO: 26.

4. Process according to one of the preceding claims, **characterized in that** in enzyme MxnK (SEQ ID NO: 11) the module 1W is inactivated and by adding a Western chain precursor having the structure or 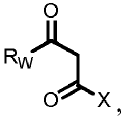 wherein R_{W} is a C1-C12 alkyl, aryl or aromatic group, and wherein X is an auxiliary group which is bound by a sulfur atom.

5. Process according to one of the preceding claims, **characterized in that** in enzyme MxnK (SEQ ID NO: 11) the module 2W is inactivated and by adding a Western chain precursor having the structure or 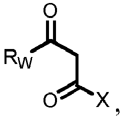 wherein R_{W} is a C1-C12 alkyl, aryl or aromatic group, and wherein X is an auxiliary group which is bound by a sulfur atom.

6. Process according to one of the preceding claims, **characterized in that** in enzyme MxnK (SEQ ID NO: 11) the module 3W is inactivated and by adding a Western chain precursor having the structure or 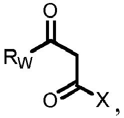 wherein R_{W} is a C1-C12 alkyl, aryl or aromatic group, and wherein X is an auxiliary group which is bound by a sulfur atom.

7. Process according to one of the preceding claims, **characterized in that** in enzyme MxnK (SEQ ID NO: 11) the module 4W is inactivated and by adding a Western chain precursor having the structure or 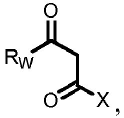 wherein R_{W} is a C1-C12 alkyl, aryl or aromatic group, and wherein X is an auxiliary group which is bound by a sulfur atom.

8. Process according to one of claims 5 to 7, **characterized by** the Western chain precursor having a structure selected from R_{W}-CH₂-CH₂-CH₂-C(O)-X, R_{W}-CH₂-CH₂-C(CH₃)=CH₂-C(O)-X, R_{W}-CH₂-CH₂-C(CH₃)=CH-CH=C(CH₃)-C(O)-X.

9. Process according to one of the preceding claims, **characterized in that** in enzyme MxnI (SEQ ID NO: 9) the load loading module (LM) comprising amino acids 1 to 466 of SEQ ID NO: 9 is inactivated and by adding an Eastern chain precursor having the structure 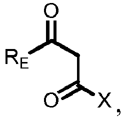 wherein X is an auxiliary group bound by a sulfur atom.

10. Process according to one of the preceding claims, **characterized in that** in enzyme MxnI (SEQ ID NO: 9) the module 1E is inactivated and by adding an Eastern chain precursor having the structure 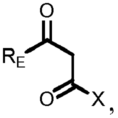 wherein X is an auxiliary group bound by a sulfur atom.

11. Process according to one of the preceding claims, **characterized in that** in enzyme MxnI (SEQ ID NO: 9) the loading module and module 3E comprising amino acids 1 to 1759 of SEQ ID NO: 9 are inactivated and by adding an Eastern chain precursor having the structure 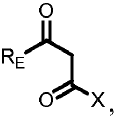 wherein X is an auxiliary group bound by a sulfur atom.

12. Process according to one of the preceding claims, **characterized in that** the enzyme MxnI (SEQ ID NO: 9) and the module 3E, comprising amino acids No. 1 to 405 of SEQ ID NO: 10 are functionally absent and by adding an Eastern chain precursor having the structure 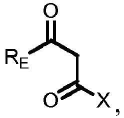 wherein X is an auxiliary group bound by a sulfur atom.

13. Process according to one of claims 5 to 12, wherein the precursor is selected from

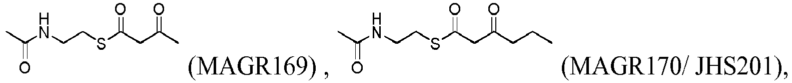

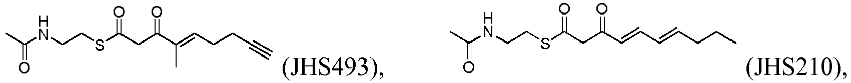

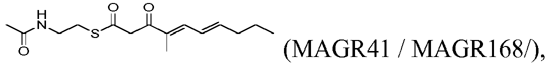

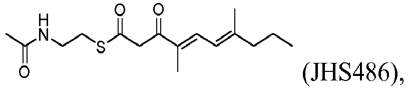

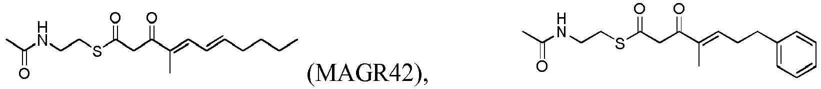

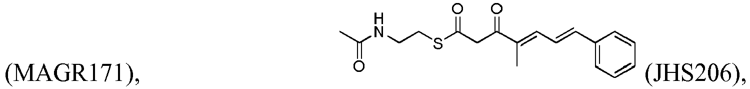

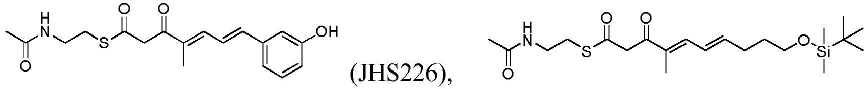

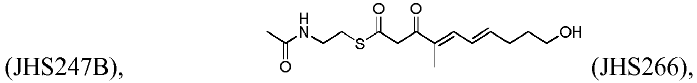

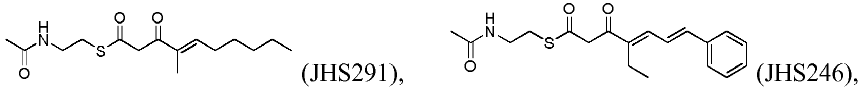

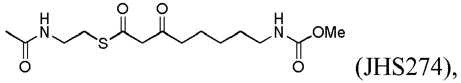

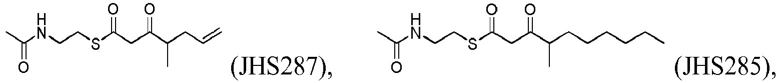

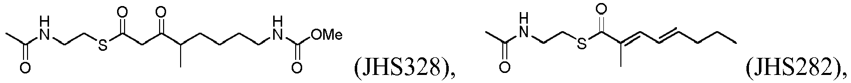

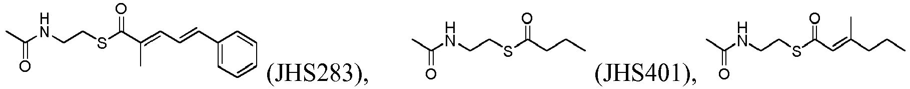

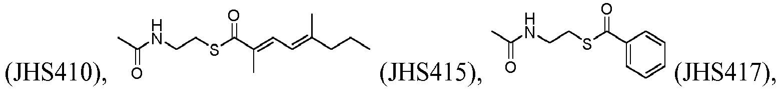

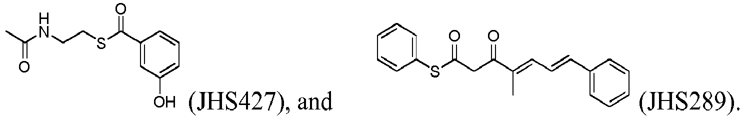

14. Process according to one of the preceding claims, **characterized in that** the enzyme MxnI the module 4E comprising amino acids No. 1 to 3052 of SEQ ID NO: 9 is inactivated and by adding an Eastern chain precursor having the structure 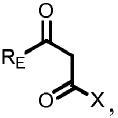 wherein X is an auxiliary group bound by a sulfur atom.

15. Process according to one of the preceding claims, **characterized in that** the enzyme MxnI (SEQ ID NO: 9) is functionally absent and that the enzyme MxnJ consists of module 6E consisting of amino acids No. 3007 to 3849 of SEQ ID NO: 10.

16. Process according to one of the preceding claims, **characterized in that** the enzymes, which at least comprise MxnB are separated from the microoganism and the compound is produced by the enzymes in a cell-free reaction.

## Patentansprüche

1. Verfahren zum Herstellen einer Verbindung, die eine erste Kette (Westkette) und eine zweite Kette (Ostkette) aufweist, die kondensiert sind und einen Pyronring bilden, durch Ausstatten eines Mikroorganismus mit synthetischen Stoffwechselenzymen, die die Synthese der Verbindung katalysieren, wobei der Mikroorganismus genetisch manipuliert ist, um Gene zu enthalten, die die synthetischen Stoffwechselenzyme kodieren, die die Enzyme MxnA (SEQ ID NO: 1), MxnB (SEQ ID NO: 2), MxnC (SEQ ID NO: 3), MxnD (SEQ ID NO: 4), MxnE (SEQ ID NO: 5), MxnF (SEQ ID NO: 6), MxnG (SEQ ID NO: 7), MxnH (SEQ ID NO: 8), MxnI (SEQ ID NO: 9), MxnJ (SEQ ID NO: 10) und MxnK (SEQ ID NO: 11) umfassen, Inkubieren des Mikroorganismus und Abtrennen der Verbindung von dem Mikroorganismus, **dadurch gekennzeichnet, dass** wenigstens eine katalytische Domäne eines Enzyms abwesend oder inaktiviert ist, wobei die Domäne aus der Gruppe ausgewählt ist, die aus
a) der Ketosynthase (KS) des Moduls 1E von MxnI, das die Aminosäuren Nr. 694-885 von SEQ ID NO: 9 umfasst,
b) der Ketosynthase (KS) des Moduls 2E von MxnI, das die Aminosäuren Nr. 1945-2135 von SEQ ID NO: 9 umfasst,
c) der Ketosynthase (KS) des Moduls 3E von MxnI, das die Aminosäuren Nr. 3442-3633 von SEQ ID NO: 10 umfasst,
d) der Ketosynthase (KS) des Moduls 4E von MxnJ, das die Aminosäuren Nr. 605-795 von SEQ ID NO: 10 umfasst,
e) der Ketosynthase (KS) des Moduls 5E von MxnJ, das die Aminosäuren Nr. 2476-2660 von SEQ ID NO: 10 umfasst,
f) der Ketosynthase (KS) des Moduls 6E von MxnJ, das die Aminosäuren Nr. 3208-3399 von SEQ ID NO: 10 umfasst,
g) der Ketosynthase (KS) des Moduls 1W von MxnK, das die Aminosäuren Nr. 180-364 von SEQ ID NO: 11 umfasst,
h) der Ketosynthase (KS) des Moduls 2W von MxnK, das die Aminosäuren Nr. 1926-2110 von SEQ ID NO: 11 umfasst,
i) der Ketosynthase (KS) des Moduls 3Wvon MxnK, das die Aminosäuren Nr. 2684-2875 von SEQ ID NO: 11 umfasst,
j) der Ketosynthase (KS) des Moduls 4W von MxnK, das die Aminosäuren Nr. 3504-3688 von SEQ ID NO: 11 umfasst,
k) der Ketosynthase (KS) des Moduls 5W von MxnK, dass die Aminosäuren Nr. 5409-5593 von SEQ ID NO: 11 umfasst,
l) der Ketoreduktase (KR) des Moduls 2E von MxnI, dass die Aminosäuren Nr. 2903-3050 von SEQ ID NO: 9 umfasst,
m) der Ketoreduktase (KR) des Moduls 4E von MxnJ, dass die Aminosäuren Nr. 1568-1715 von SEQ ID NO: 10 umfasst,
n) der Ketoreduktase (KR) des Moduls 1W von MxnK, dass die Aminosäuren Nr. 1418-1560 von SEQ ID NO: 11 umfasst,
o) der Ketoreduktase (KR) des Moduls 4W von MxnK, dass die Aminosäuren Nr. 4470-4617 von SEQ ID NO: 11 umfasst,
p) dem Trägerprotein (CP) des Moduls L von MxnI, dass die Aminosäuren Nr. 424-433 von SEQ ID NO: 9 umfasst,
q) dem Trägerprotein (CP) des Moduls 1E von MxnI, dass die Aminosäuren Nr. 1720-1726 von SEQ ID NO: 9 umfasst,
r) dem Trägerprotein (CP) des Moduls 2E von MxnI, dass die Aminosäuren Nr. 3192-3198 von SEQ ID NO: 9 umfasst,
s) dem Trägerprotein (CP) des Moduls 3E von MxnJ, dass die Aminosäuren Nr. 366-372 von SEQ ID NO: 9 umfasst,
t) dem Trägerprotein (CP) des Moduls 4E von MxnJ, dass die Aminosäuren Nr. 2238-2244 von SEQ ID NO: 10 umfasst,
u) dem Trägerprotein (CP) des Moduls 5E von MxnJ, dass die Aminosäuren Nr. 2967-2973 von SEQ ID NO: 10 umfasst,
v) dem Trägerprotein (CP) des Moduls 6E von MxnJ, dass die Aminosäuren Nr. 3810-3816 von SEQ ID NO: 10 umfasst,
w) dem Trägerprotein (CP*) des Moduls 6E von MxnJ, dass die Aminosäuren Nr. 3566-3572 von SEQ ID NO: 12 umfasst,
x) dem Trägerprotein (CP) des Moduls 1W von MxnK, dass die Aminosäuren Nr. 1683-1689 von SEQ ID NO: 11 umfasst,
y) dem Trägerprotein (CP) des Moduls 2W von MxnK, dass die Aminosäuren Nr. 2429-2435 von SEQ ID NO: 11 umfasst,
z) dem Trägerprotein (CP) des Moduls 3W von MxnK, dass die Aminosäuren Nr. 3234-3240 von SEQ ID NO: 11 umfasst,
aa) dem Trägerprotein (CP) des Moduls 4W von MxnK, dass die Aminosäuren Nr. 5165-5171 von SEQ ID NO: 11 umfasst,
bb) dem Trägerprotein (CP*) des Moduls 5W von MxnK, dass die Aminosäuren Nr. 5894-5900 von SEQ ID NO: 11 umfasst,
cc) dem Trägerprotein (CP) des Moduls 5W von MxnK, dass die Aminosäuren Nr. 5984-5990 von SEQ ID NO: 11 umfasst,
dd) der Dehydratase (DH) des Moduls 2E von MxnI, dass die Aminosäuren Nr. 2402-2414 von SEQ ID NO: 9 umfasst,
ee) der Dehydratase (DH) des Moduls 3E von MxnJ, dass die Aminosäuren Nr. 53-65 von SEQ ID NO: 9 umfasst,
ff) der Dehydratase (DH) des Moduls 4E von MxnJ, dass die Aminosäuren Nr. 1091-1103 von SEQ ID NO: 10 umfasst,
gg) der Dehydratase (DH) des Moduls 1W von MxnK, dass die Aminosäuren Nr. 955-967 von SEQ ID NO: 11 umfasst,
hh) der Dehydratase (DH) des Moduls 4W von MxnK, dass die Aminosäuren Nr. 3980-3992 von SEQ ID NO: 11 umfasst,
ii) der Methyltransferase (MT) des Moduls 4E von MxnJ, dass die Aminosäuren Nr. 1982-1987 von SEQ ID NO: 10 umfasst,
jj) der Methyltransferase (MT) des Moduls 4W von MxnK, dass die Aminosäuren Nr. 4914-4919 von SEQ ID NO: 11 umfasst,
besteht, wobei in den Ausführungsformen, in denen wenigstens eine katalytische Domäne in den synthetischen Stoffwechselenzymen MxnK und/oder MxnI-MxnJ inaktiviert ist, wobei die inaktivierte katalytische Domäne eine katalytische Domäne ist, deren Inaktivierung in Abwesenheit von Vorläufer zum Stop der Synthese durch das Enzym führt, wenn es in einem Mikroorganismus exprimiert wird, ein Vorläufer der Westkette und/oder ein Vorläufer der Ostkette dem Mikroorganismus zugegeben wird, der die syntetischen Stoffwechselenzyme exprimiert, oder wobei in Ausführungsformen, in denen die Inaktivierung durch Austausch von wenigstens einem katalytischen Zentrum, Domain, Module oder Enzym erfolgt, der Austausch durch Insertion wenigstens eines katalytischen Zentrums, Domäne, Moduls oder Enzym folgt, ausgewählt aus den Enzymen CorJ (SEQ ID NO: 25), CorK (SEQ ID NO: 26), CorL (SEQ ID NO: 27), Orfl (SEQ ID NO: 27), CorM (SEQ ID NO: 28), CorM (SEQ ID NO: 28), CorN (SEQ ID NO: 29) und CorO (SEQ ID NO: 30) und deren katalytischen Zentren, Domänen oder Modulen, oder Enzym MxnI (SEQ ID NO: 9) durch das Enzym CorI (SEQ ID NO: 24) ersetzt ist.

2. Verfahren nach Anspruch 1, **dadurch gekennzeichnet, dass** wenigstens eines von Enzymen MxnA (SEQ ID NO: 1) durch das Enzym CorA (SEQ ID NO: 16) ersetzt ist, Enzym MxnB (SEQ ID NO: 2) durch das Enzym CorB (SEQ ID NO: 17) ersetzt ist, Enzym MxnC (SEQ ID NO: 3) durch das Enzym CorC (SEQ ID NO: 18) ersetzt ist, Enzym MxnD (SEQ ID NO: 4) durch das Enzym CorD (SEQ ID NO: 19) ersetzt ist, Enzym MxnE (SEQ ID NO: 5) durch das Enzym CorE (SEQ ID NO: 20) ersetzt ist, Enzym MxnF (SEQ ID NO: 6) durch das Enzym CorF (SEQ ID NO: 21) ersetzt ist, Enzym MxnG (SEQ ID NO: 7) durch das Enzym CorG (SEQ ID NO: 22) ersetzt ist, Enzym MxnH (SEQ ID NO: 8) durch das Enzym CorH (SEQ ID NO: 23) ersetzt ist, Enzym MxnJ (SEQ ID NO: 10) durch das Enzym CorJ (SEQ ID NO: 25) ersetzt ist, Enzym MxnK (SEQ ID NO: 11) durch das Enzym CorK (SEQ ID NO: 26) und CorL (SEQ ID NO: 27) ersetzt ist.

3. Verfahren nach Anspruch 2, **dadurch gekennzeichnet, dass** wenigstens eine katalytische Domäne eines Enzyms abwesend oder inaktiviert ist, wobei die Domäne aus der Gruppe ausgewählt ist, die aus
a) der Ketosynthase (KS) des Moduls 1E von Corl, das die Aminosäuren Nr. 688-880 von SEQ ID NO: 23 umfasst,
b) der Ketosynthase (KS) des Moduls 2E von Corl, das die Aminosäuren Nr. 1925-2115 von SEQ ID NO: 23 umfasst,
c) der Ketosynthase (KS) des Moduls 3E von Corl, das die Aminosäuren Nr. 3416-3607 von SEQ ID NO: 23 umfasst,
d) der Ketosynthase (KS) des Moduls 4E von CorJ, das die Aminosäuren Nr. 586-776 von SEQ ID NO: 24 umfasst,
e) der Ketosynthase (KS) des Moduls 5E von CorJ, das die Aminosäuren Nr. 2448-2632 von SEQ ID NO: 24 umfasst,
f) der Ketosynthase (KS) des Moduls 6E von CorJ, das die Aminosäuren Nr. 3172-3363 von SEQ ID NO: 24 umfasst,
g) der Ketosynthase (KS) des Moduls 1W von CorK, das die Aminosäuren Nr. 171-355 von SEQ ID NO: 25 umfasst,
h) der Ketosynthase (KS) des Moduls 2W von CorK, das die Aminosäuren Nr. 1898-2082 von SEQ ID NO: 25 umfasst,
i) der Ketosynthase (KS) des Moduls 3W von CorK, das die Aminosäuren Nr. 3362-3550 von SEQ ID NO: 25 umfasst,
j) der Ketosynthase (KS) des Moduls 4W von CorL, das die Aminosäuren Nr. 449-633 von SEQ ID NO: 26 umfasst,
k) der Ketosynthase (KS) des Moduls 5W von CorL, das die Aminosäuren Nr. 1678-1869 von SEQ ID NO: 26 umfasst,
l) der Ketosynthase (KS) des Moduls 6W von CorL, das die Aminosäuren Nr. 2484-2668 von SEQ ID NO: 26 umfasst,
m) der Ketosynthase (KS) des Moduls 7W von CorL, das die Aminosäuren Nr. 4355-4539 von SEQ ID NO: 26 umfasst,
n) der Ketoreduktase (KR) des Moduls 2E von Corl, das die Aminosäuren Nr. 2874-3021 von SEQ ID NO: 23 umfasst,
o) der Ketoreduktase (KR) des Moduls 4E von CorJ, das die Aminosäuren Nr. 1548-1695 von SEQ ID NO: 24 umfasst,
p) der Ketoreduktase (KR) des Moduls 1W von CorK, das die Aminosäuren Nr. 1401-1543 von SEQ ID NO: 25 umfasst,
q) der Ketoreduktase (KR) des Moduls 2W von CorK, das die Aminosäuren Nr. 2826-2972 von SEQ ID NO: 25 umfasst,
r) der Ketoreduktase (KR) des Moduls 4W von CorL, das die Aminosäuren Nr. 1139-1285 von SEQ ID NO: 26 umfasst,
s) der Ketoreduktase (KR) des Moduls 6W von CorL, das die Aminosäuren Nr. 3427-3574 von SEQ ID NO: 26 umfasst,
t) dem Trägerprotein (CP) des Moduls L von Corl, das die Aminosäuren Nr. 416-425 von SEQ ID NO: 23 umfasst,
u) dem Trägerprotein (CP) des Moduls 1E von Corl, das die Aminosäuren Nr. 1706-1712 von SEQ ID NO: 23 umfasst,
v) dem Trägerprotein (CP) des Moduls 2E von Corl, das die Aminosäuren Nr. 3166-3172 von SEQ ID NO: 23 umfasst,
w) dem Trägerprotein (CP) des Moduls 3E von CorJ, das die Aminosäuren Nr. 350-356 von SEQ ID NO: 24 umfasst,
x) dem Trägerprotein (CP) des Moduls 4E von CorJ, das die Aminosäuren Nr. 2206-2212 von SEQ ID NO: 24 umfasst,
y) dem Trägerprotein (CP) des Moduls 5E von CorJ, das die Aminosäuren Nr. 2935-2941 von SEQ ID NO: 24 umfasst,
z) dem Trägerprotein (CP) des Moduls 6E* von CorJ, das die Aminosäuren Nr. 3530-3536 von SEQ ID NO: 24 umfasst,
aa) dem Trägerprotein (CP) des Moduls 6E von CorJ, das die Aminosäuren Nr. 3765-3771 von SEQ ID NO: 24 umfasst,
bb) dem Trägerprotein (CP) des Moduls 1W von CorK, das die Aminosäuren Nr. 1663-1669 von SEQ ID NO: 25 umfasst,
cc) dem Trägerprotein (CP) des Moduls 2W von CorK, das die Aminosäuren Nr. 3109-3115 von SEQ ID NO: 25 umfasst,
dd) dem Trägerprotein (CP) des Moduls 3W* von CorK, das die Aminosäuren Nr. 3900-3906 von SEQ ID NO: 25 umfasst,
ee) dem Trägerprotein (CP) des Moduls 3W von CorK, das die Aminosäuren Nr. 3994-4090 von SEQ ID NO: 25 umfasst,
ff) dem Trägerprotein (CP) des Moduls 4W von CorL, das die Aminosäuren Nr. 1443-1449 von SEQ ID NO: 25 umfasst,
gg) dem Trägerprotein (CP) des Moduls 5W von CorL, das die Aminosäuren Nr. 2208-2214 von SEQ ID NO: 26 umfasst,
hh) dem Trägerprotein (CP) des Moduls 6W von CorL, das die Aminosäuren Nr. 4116-4122 von SEQ ID NO: 26 umfasst,
ii) dem Trägerprotein (CP) des Moduls 7W* von CorL, das die Aminosäuren Nr. 4826-4832 von SEQ ID NO: 26 umfasst,
jj) dem Trägerprotein (CP) des Moduls 7W von CorL, das die Aminosäuren Nr. 4920-4926 von SEQ ID NO: 26 umfasst,
kk) der Dehydratase (DH) des Moduls 2E von Corl, das die Aminosäuren Nr. 2380-2392 von SEQ ID NO: 23 umfasst,
ll) der Dehydratase (DH) des Moduls 3E von CorJ, das die Aminosäuren Nr. 44-56 von SEQ ID NO: 24 umfasst,
mm) der Dehydratase (DH) des Moduls 4E von CorJ, das die Aminosäuren Nr. 1073-1085 von SEQ ID NO: 24 umfasst,
nn) der Dehydratase (DH) des Moduls 1W von CorK, das die Aminosäuren Nr. 942-954 von SEQ ID NO: 25 umfasst,
oo) der Dehydratase (DH) des Moduls 2W von CorK, das die Aminosäuren Nr. 2319-2331 von SEQ ID NO: 25 umfasst,
pp) der Dehydratase (DH) des Moduls 4W von CorL, das die Aminosäuren Nr. 19-31 von SEQ ID NO: 26 umfasst,
qq) der Dehydratase (DH) des Moduls 6W von CorL, das die Aminosäuren Nr. 2949-2961 von SEQ ID NO: 26 umfasst,
rr) der Methyltransferase (MT) des Moduls 4E von CorJ, das die Aminosäuren Nr. 1962-1967 von SEQ ID NO: 24 umfasst,
ss) der Methyltransferase (MT) des Moduls 6W von CorL, das die Aminosäuren Nr. 3865-3870 von SEQ ID NO: 26 umfasst,
besteht.

4. Verfahren nach einem der voranstehenden Ansprüche, **dadurch gekennzeichnet, dass** in dem Enzym MxnK (SEQ ID NO: 11) das Modul 1W inaktiviert ist und durch Zugeben eines Vorläufers der Westkette mit der Struktur 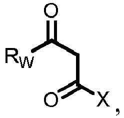 wobei R_{W} eine C1-C12 Alkyl-, Aryl- oder aromatische Gruppe ist, und wobei X eine Nebengruppe ist, die durch ein Schwefelatom gebunden ist.

5. Verfahren nach einem der voranstehenden Ansprüche, **dadurch gekennzeichnet, dass** in dem Enzym MxnK (SEQ ID NO: 11) das Modul 2W inaktiviert ist, und durch Zugeben eines Vorläufers der Westkette mit der Struktur 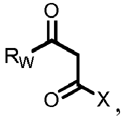 wobei R_{W} eine C1-C12 Alkyl-, Aryl- oder aromatische Gruppe ist, und wobei X eine Nebengruppe ist, die durch ein Schwefelatom gebunden ist.

6. Verfahren nach einem der voranstehenden Ansprüche, **dadurch gekennzeichnet, dass** in dem Enzym MxnK (SEQ ID NO: 11) das Modul 3W inaktiviert ist, und durch Zugeben eines Vorläufers der Westkette mit der Struktur 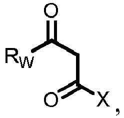 wobei R_{W} eine C1-C12 Alkyl-, Aryl- oder aromatische Gruppe ist, und wobei X eine Nebengruppe ist, die durch ein Schwefelatom gebunden ist.

7. Verfahren nach einem der voranstehenden Ansprüche, **dadurch gekennzeichnet, dass** in dem Enzym MxnK (SEQ ID NO: 11) das Modul 4W inaktiviert ist, und durch Zugeben eines Vorläufers der Westkette mit der Struktur 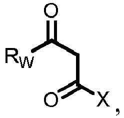 wobei R_{W} eine C1-C12 Alkyl-, Aryl- oder aromatische Gruppe ist, und wobei X eine Nebengruppe ist, die durch ein Schwefelatom gebunden ist.

8. Verfahren nach einem der Ansprüche 5 bis 7, **dadurch gekennzeichnet, dass** der Vorläufer der Westkette eine Struktur aufweist, die ausgewählt ist unter R_{W}-CH₂-CH₂-CH₂-C(O)-X, R_{W}-CH₂-CH₂-C(CH₃)=CH₂-C(O)-X, R_{W}-CH₂-CH₂-C(CH₃)=CH-CH=C(CH₃)-C(O)-X.

9. Verfahren nach einem der voranstehenden Ansprüche, **dadurch gekennzeichnet, dass** in dem Enzym MxnI (SEQ ID NO: 9) das Lademodul (LM), das die Aminosäuren 1 bis 466 der SEQ ID NO: 9 umfasst, inaktiviert ist und durch Zugeben eines Vorläufers der Ostkette mit der Struktur 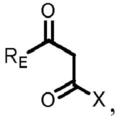 wobei X eine Nebengruppe ist, die durch ein Schwefelatom gebunden ist.

10. Verfahren nach einem der voranstehenden Ansprüche, **dadurch gekennzeichnet, dass** in dem Enzym MxnI (SEQ ID NO: 9) das Modul 1E inaktiviert ist und durch Zugeben eines Vorläufers der Ostkette mit der Struktur 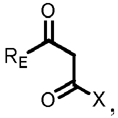 wobei X eine Nebengruppe ist, die durch ein Schwefelatom gebunden ist.

11. Verfahren nach einem der voranstehenden Ansprüche, **dadurch gekennzeichnet, dass** in dem Enzym MxnI (SEQ ID NO: 9) das Lademodul und das Modul 3E, das die Aminosäuren 1 bis 1759 der SEQ ID NO: 9 umfasst, inaktiviert ist und durch Zugeben eines Vorläufers der Ostkette mit der Struktur 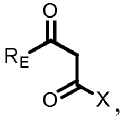 wobei X eine Nebengruppe ist, die durch ein Schwefelatom gebunden ist.

12. Verfahren nach einem der voranstehenden Ansprüche, **dadurch gekennzeichnet, dass** das Enzym MxnI (SEQ ID NO: 9) und das Modul 3E, das die Aminosäuren 1 bis 405 der SEQ ID NO: 10 umfasst, funktional abwesend sind und durch Zugeben eines Vorläufers der Ostkette mit der Struktur 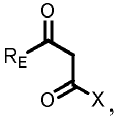 wobei X eine Nebengruppe ist, die durch ein Schwefelatom gebunden ist.

13. Verfahren nach einem der Ansprüche 5 bis 12, bei dem der Vorläufer ausgewählt ist unter

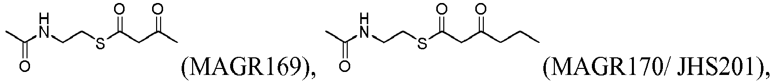

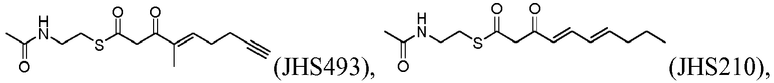

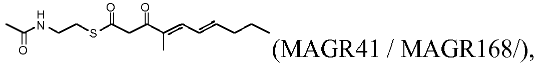

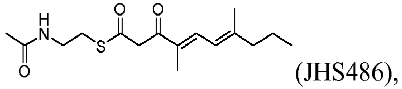

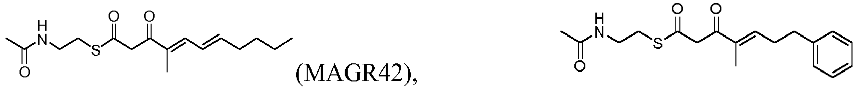

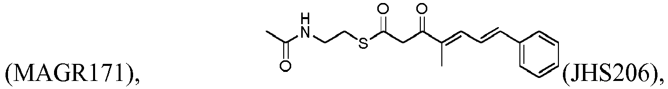

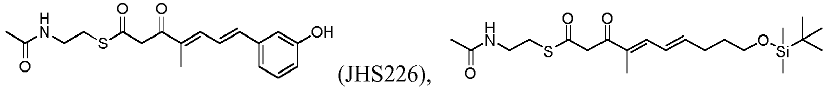

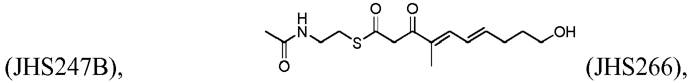

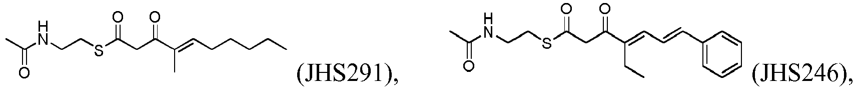

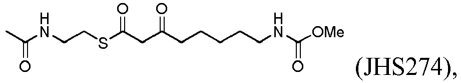

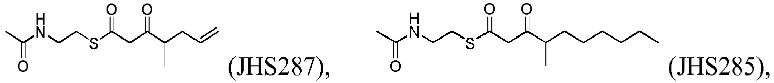

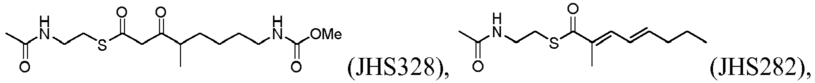

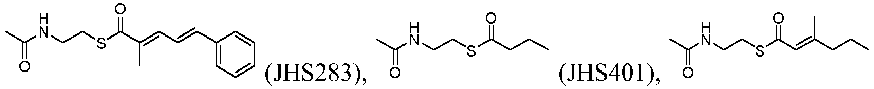

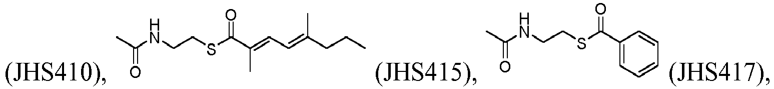

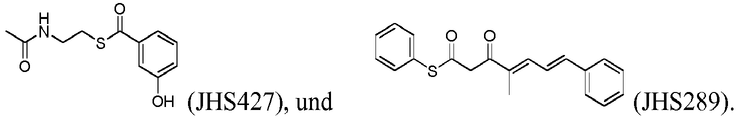

14. Verfahren nach einem der voranstehenden Ansprüche, **dadurch gekennzeichnet, dass** in dem Enzym MxnI das Modul 4E, das die Aminosäuren 1 bis 3052 von SEQ ID NO: 9 umfasst, inaktiviert ist, und durch Zugeben eines Vorläufers der Ostkette mit der Struktur 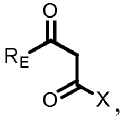 wobei X eine Nebengruppe ist, die durch ein Schwefelatom gebunden ist.

15. Verfahren nach einem der voranstehenden Ansprüche, **dadurch gekennzeichnet, dass** das Enzym MxnI (SEQ ID NO: 9) funktional abwesend ist und dass das Enzym MxnJ aus Modul 6E besteht, das aus den Aminosäuren Nr. 3007 bis 3849 von SEQ ID NO: 10 besteht.

16. Verfahren nach einem der voranstehenden Ansprüche, **dadurch gekennzeichnet, dass** die Enzyme, die zumindest MxnB umfassen, von dem Mikroorganismus getrennt werden und die Verbindung in einer zellfreien Reaktion von den Enzymen hergestellt wird.

## Revendications

1. Procédé de production d'un composé ayant une première chaîne (chaîne occidentale) et une seconde chaîne (chaîne orientale) qui sont condensées en formant un cycle pyrone en constituant dans un microorganisme une voie de synthèse d'enzymes catalysant la synthèse du composé, le microorganisme étant génétiquement manipulé pour comprendre des gènes codant pour des enzymes de la voie de synthèse comprenant les enzymes MxnA (SEQ ID NO: 1), MxnB (SEQ ID NO: 2), MxnC (SEQ ID NO: 3), MxnD (SEQ ID NO: 4), MxnE (SEQ ID NO: 5), MxnF (SEQ ID NO: 6), MxnG (SEQ ID NO: 7), MxnH (SEQ ID NO: 8), MxnI (SEQ ID NO: 9), MxnJ (SEQ ID NO: 10) et MxnK (SEQ ID NO: 11), en incubant le microorganisme et en séparant le composé du microorganisme,
**caractérisé en ce qu'**au moins un domaine catalytique d'une enzyme est absent ou inactivé,
domaine choisi dans le groupe constitué des éléments suivants
a) la cétosynthase (KS) du module IE de MxnI comprenant les acides aminés No. 694-885 de SEQ ID NO: 9,
b) la cétosynthase (KS) du module 2E de MxnI comprenant les acides aminés No. 1945-2135 de SEQ ID NO: 9,
c) la cétosynthase (KS) du module 3E de MxnI comprenant les acides aminés No. 3442-3633 de SEQ ID NO: 10,
d) la cétosynthase (KS) du module 4E de MxnJ comprenant les acides aminés No. 605-795 de SEQ ID NO: 10,
e) la cétosynthase (KS) du module 5E de MxnJ comprenant les acides aminés No. 2476-2660 de SEQ ID NO: 10,
f) la cétosynthase (KS) du module 6E de MxnJ comprenant les acides aminés No. 3208-3399 de SEQ ID NO: 10,
g) la cétosynthase (KS) du module 1W de MxnK comprenant les acides aminés No. 180-364 de SEQ ID NO: 11,
h) la cétosynthase (KS) du module 2W de MxnK comprenant les acides aminés No. 1926-2110 de SEQ ID NO. 11,
i) la cétosynthase (KS) du module 3W de MxnK comprenant les acides aminés No. 2684-2875 de SEQ ID NO. 11,
j) la cétosynthase (KS) du module 4W de MxnK comprenant les acides aminés No. 3504-3688 de SEQ ID NO. 11,
k) la cétosynthase (KS) du module 5W de MxnK comprenant les acides aminés No. 5409-5593 de SEQ ID NO: 11,
l) la cétoréductase (KR) du module 2E de MxnI comprenant les acides aminés No. 2903-3050 de SEQ ID NO: 9,
m) la cétoréductase (KR) du module 4E de MxnJ comprenant les acides aminés No. 1568-1715 de SEQ ID NO: 10,
n) la cétoréductase (KR) du module 1W de MxnK comprenant les acides aminés No. 1418-1560 de SEQ ID NO: 11,
o) la cétoréductase (KR) du module 4W de MxnK comprenant les acides aminés No. 4470-4617 de SEQ ID NO: 11,
p) la protéine porteuse (CP) du module L de MxnI comprenant les acides aminés No. 424-433 de SEQ ID NO: 9,
q) la protéine porteuse (CP) du module IE de MxnI comprenant les acides aminés No. 1720-1726 de SEQ ID NO: 9,
r) la protéine porteuse (CP) du module 2E de MxnI comprenant les acides aminés No. 3192-3198 de SEQ ID NO: 9,
s) la protéine porteuse (CP) du module 3E de MxnJ comprenant les acides aminés No. 366-372 de SEQ ID NO: 9,
t) la protéine porteuse (CP) du module 4E de MxnJ comprenant les acides aminés No. 2238-2244 de SEQ ID NO: 10,
u) la protéine porteuse (CP) du module 5E de MxnJ comprenant les acides aminés No. 2967-2973 de SEQ ID NO: 10,
v) la protéine porteuse (CP) du module 6E de MxnJ comprenant les acides aminés No. 3810-3816 de SEQ ID NO: 10,
w) la protéine porteuse (CP*) du module 6E de MxnJ comprenant les acides aminés No. 3566-3572 de SEQ ID NO: 12,
x) la protéine porteuse (CP) du module 1W de MxnK comprenant les acides aminés No. 1683-1689 de SEQ ID NO: 11,
y) la protéine porteuse (CP) du module 2W de MxnK comprenant les acides aminés No. 2429-2435 de SEQ ID NO: 11,
z) la protéine porteuse (CP) du module 3W de MxnK comprenant les acides aminés No. 3234-3240 de SEQ ID NO: 11,
aa) la protéine porteuse (CP) du module 4W de MxnK comprenant les acides aminés No. 5165-5171 de SEQ IDNO: 11,
bb) la protéine porteuse (CP*) du module 5W de MxnK comprenant les acides aminés No. 5894-5900 de SEQ ID NO: 11,
cc) la protéine porteuse (CP) du module 5W de MxnK comprenant les acides aminés No. 5984-5990 de SEQ ID NO: 11,
dd) la déshydratase (DH) du module 2E de MxnI comprenant les acides aminés No. 2402-2414 de SEQ ID NO:9,
ee) la déshydratase (DH) du module 3E de MxnJ comprenant les acides aminés No. 53-65 de SEQ ID NO: 9,
ff) la déshydratase (DH) du module 4E de MxnJ comprenant les acides aminés No. 1091-1103 de SEQ ID NO: 10,
gg) la déshydratase (DH) du module 1W de MxnK comprenant les acides aminés No. 955-967 de SEQ ID NO: 11,
hh) la déshydratase (DH) du module 4W de MxnK comprenant les acides aminés No. 3980-3992 de SEQ ID NO: 11,
ii) la méthyltransférase (MT) du module 4E de MxnJ comprenant les acides aminés No. 1982-1987 de SEQ ID NO: 10,
jj) la méthyltransférase (MT) du module 4W de MxnK comprenant les acides aminés No. 4914-4919 de SEQ ID NO: 11,
dans lequel, dans des modes de réalisation dans lesquels au moins un domaine catalytique est inactivé dans les enzymes de la voie de synthèse MxnK et/ou MxnI-MxnJ, dans lequel le domaine catalytique inactivé est un domaine catalytique dont l'inactivation en l'absence de précurseur entraîne un arrêt de la synthèse de l'enzyme, lorsqu'elle est exprimée dans un microorganisme, un précurseur de chaîne occidentale et/ou un précurseur de chaîne orientale est ajoutée à un microorganisme exprimant les enzymes de la voie de synthèse, ou dans des modes de réalisation dans lesquels l'inactivation se fait par échange d'au moins un centre catalytique. module ou enzyme, l'échange se fait par insertion d'au moins un centre catalytique , domaine, module ou enzyme choisi parmi les enzymes CorJ (SEQ ID NO: 25), CorK (SEQ ID NO: 26), CorL (SEQ ID NO: 27), Orfl (SEQ ID NO: 27), CorM (SEQ ID NO: 28), CorM (SEQ ID NO: 28), CorN (SEQ ID NO: 29) et CorO (SEQ ID NO: 30) et leur centres, domaines et modules catalytiques, ou l'enzyme MxnI (SEQ ID NO: 9) est remplacé par l'enzyme CorI (SEQ ID NO: 24).

2. Procédé selon la revendication 1, **caractérisé en ce qu'**au moins une des enzymes MxnA (SEQ ID NO: 1) est remplacée par l'enzyme CorA (SEQ ID NO: 16), l'enzyme MxnB (SEQ ID NO: 2) est remplacée par l'enzyme CorB (SEQ ID NO: 17), l'enzyme MxnC (SEQ ID NO: 3) est remplacée par l'enzyme CorC (SEQ ID NO: 18), l'enzyme MxnD (SEQ ID NO: 4) est remplacée par l'enzyme CorD (SEQ ID NO: 19), l'enzyme MxnE (SEQ ID NO: 5) est remplacée par l'enzyme CorE (SEQ ID NO: 20), l'enzyme MxnF (SEQ ID NO: 6) est remplacée par l'enzyme CorF (SEQ ID NO: 21), l'enzyme MxnG (SEQ ID NO: 7) est remplacée par l'enzyme CorG (SEQ ID NO: 22), l'enzyme MxnH (SEQ ID NO: 8) est remplacée par l'enzyme CorH (SEQ ID NO: 23), l'enzyme MxnJ (SEQ ID NO: 10) est remplacée par l'enzyme CorJ (SEQ ID NO: 25), l'enzyme MxnK (SEQ ID NO: 11) est remplacée par l'enzyme CorK (SEQ ID NO: 26) et CorL (SEQ ID NO: 27).

3. Procédé selon la revendication 2, **caractérisé en ce qu'**au moins un domaine catalytique d'une enzyme est absent ou inactivé, lequel domaine est choisi dans le groupe constitué des éléments suivants:
a) la cétosynthase (KS) du module IE de CorI comprenant les acides aminés No. 688-880 de SEQ ID NO: 23,
b) la cétosynthase (KS) du module 2E de CorI comprenant les acides aminés No. 1925-2115 de SEQ ID NO: 23,
c) la cétosynthase (KS) du module 3E de CorI comprenant les acides aminés No. 3416-3607 de SEQ ID NO: 23,
d) la cétosynthase (KS) du module 4E de CorJ comprenant les acides aminés No. 586-776 de SEQ ID NO: 24,
e) la cétosynthase (KS) du module 5E de CorJ comprenant les acides aminés No. 2448-2632 de SEQ ID NO: 24,
f) la cétosynthase (KS) du module 6E de CorJ comprenant les acides aminés No. 3172-3363 de SEQ ID NO: 24,
g) la cétosynthase (KS) du module 1W de CorK comprenant les acides aminés No. 171-355 de SEQ ID NO: 25,
h) la cétosynthase (KS) du module 2W de CorK comprenant les acides aminés No. 1898-2082 de SEQ ID NO: 25,
i) la cétosynthase (KS) du module 3W de CorK comprenant les acides aminés No. 3362-3550 de SEQ ID NO: 25,
j) la cétosynthase (KS) du module 4W de CorL comprenant les acides aminés No. 449-633 de SEQ ID NO: 26,
k) la cétosynthase (KS) du module 5W de CorL comprenant les acides aminés No. 1678-1869 de SEQ ID NO: 26,
l) la cétosynthase (KS) du module 6W de CorL comprenant les acides aminés No. 2484-2668 de SEQ ID NO: 26,
m) la cétosynthase (KS) du module 7W de CorL comprenant les acides aminés No. 4355-4539 de SEQ ID NO: 26,
n) la cétoréductase (KR) du module 2E de CorI comprenant les acides aminés No. 2874-3021 de SEQ ID NO: 23,
o) la cétoréductase (KR) du module 4E de CorJ comprenant les acides aminés No. 1548-1695 de SEQ ID NO: 24,
p) la cétoréductase (KR) du module 1W de CorK comprenant les acides aminés No. 1401-1543 de SEQ ID NO: 25,
q) la cétoréductase (KR) du module 2W de CorK comprenant les acides aminés No. 2826-2972 de SEQ ID NO: 25,
r) la cétoréductase (KR) du module 4W de CorL comprenant les acides aminés No. 1139-1285 de SEQ ID NO: 26,
s) la cétoréductase (KR) du module 6W de CorL comprenant les acides aminés No. 3427-3574 de SEQ ID NO: 26,
t) la protéine porteuse (CP) du module L de CorI comprenant les acides aminés No. 416-425 de SEQ ID NO: 23,
u) la protéine porteuse (CP) du module IE de CorI comprenant les acides aminés No. 1706-1712 de SEQ ID NO: 23,
v) la protéine porteuse (CP) du module 2E de CorI comprenant les acides aminés No. 3166-3172 de SEQ ID NO: 23,
w) la protéine porteuse (CP) du module 3E de CorJ comprenant les acides aminés No. 350-356 de SEQ ID NO: 24,
x) la protéine porteuse (CP) du module 4E de CorJ comprenant les acides aminés No. 2206-2212 de SEQ ID NO: 24,
y) la protéine porteuse (CP) du module 5E de CorJ comprenant les acides aminés No. 2935-2941 de SEQ ID NO: 24,
z) la protéine porteuse (CP) du module 6E* de CorJ comprenant les acides aminés No. 3530-3536 de SEQ ID NO: 24,
aa) la protéine porteuse (CP) du module 6E de CorJ comprenant les acides aminés No. 3765-3771 de SEQ ID NO: 24,
bb) la protéine porteuse (CP) du module 1W de CorK comprenant les acides aminés No. 1663-1669 de SEQ ID NO: 25,
cc) la protéine porteuse (CP) du module 2W de CorK comprenant les acides aminés No. 3109-3115 de SEQ ID NO: 25,
dd) la protéine porteuse (CP) du module 3W* de CorK comprenant les acides aminés No. 3900-3906 de SEQ ID NO: 25,
ee) la protéine porteuse (CP) du module 3W de CorK comprenant les acides aminés No. 3994-4090 de SEQ ID NO: 25,
ff) la protéine porteuse (CP) du module 4W de CorL comprenant les acides aminés No. 1443-1449 de SEQ ID NO: 25,
gg) la protéine porteuse (CP) du module 5W de CorL comprenant les acides aminés No. 2208-2214 de SEQ ID NO: 26,
hh) la protéine porteuse (CP) du module 6W de CorL comprenant les acides aminés No. 4116-4122 de SEQ ID NO: 26,
ii) la protéine porteuse (CP) du module 7W* de CorL comprenant les acides aminés No. 4826-4832 de SEQ ID NO: 26,
jj) la protéine porteuse (CP) du module 7W de CorL comprenant les acides aminés No. 4920-4926 de SEQ ID NO: 26,
kk) la déshydratase (DH) du module 2E de CorI comprenant les acides aminés No. 2380-2392 de SEQ ID NO: 23,
ll) la déshydratase (DH) du module 3E de CorJ comprenant les acides aminés No. 44-56 de SEQ ID NO: 24,
mm) la déshydratase (DH) du module 4E de CorJ comprenant les acides aminés No. 1073-1085 de SEQ ID NO: 24,
nn) la déshydratase (DH) du module 1W de CorK comprenant les acides aminés No. 942-954 de SEQ ID NO: 25,
oo) la déshydratase (DH) du module 2W de CorK comprenant les acides aminés No. 2319-2331 de SEQ ID NO: 25,
pp) la déshydratase (DH) du module 4W de CorL comprenant les acides aminés No. 19-31 de SEQ ID NO: 26,
qq) la déshydratase (DH) du module 6W de CorL comprenant les acides aminés No. 2949-2961 de SEQ ID NO: 26,
rr) la méthyltransférase (MT) du module 4E de CorJ comprenant les acides aminés No. 1962-1967 de SEQ ID NO: 24,
ss) la méthyltransférase (MT) du module 6W de CorL comprenant les acides aminés No. 3865-3870 de SEQ ID NO: 26.

4. Procédé selon la revendication 1, **caractérisé en ce que** dans l'enzyme MxnK (SEQ ID NO: 11) le module 1W est inactivé et en ajoutant un précurseur de chaîne occidentale ayant la structure 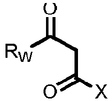 dans laquelle R_{W} est un groupe alkyle, aryle ou aromatique en C1-C12, et dans lequel X est un groupe auxiliaire qui est lié par un atome de soufre.

5. Procédé selon l'une des revendications précédentes, **caractérisé en ce que** dans l'enzyme MxnK (SEQ ID NO: 11) le module 2W est inactivé et en ajoutant un précurseur de chaîne occidentale ayant la structure 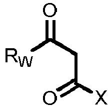 dans laquelle R_{W} est un groupe alkyle, aryle ou aromatique en C1-C12, et dans lequel X est un groupe auxiliaire qui est lié par un atome de soufre.

6. Procédé selon l'une des revendications précédentes, **caractérisé en ce que** dans l'enzyme MxnK (SEQ ID NO: 11) le module 3W est inactivé et en ajoutant un précurseur de chaîne occidentale ayant la structure 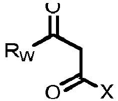 dans laquelle R_{W} est un groupe alkyle, aryle ou aromatique en C1-C12, et dans lequel X est un groupe auxiliaire qui est lié par un atome de soufre.

7. Procédé selon l'une des revendications précédentes, **caractérisé en ce que** dans l'enzyme MxnK (SEQ ID NO: 11) le module 4W est inactivé et en ajoutant un précurseur de chaîne occidentale ayant la structure 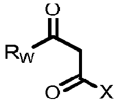 dans laquelle R_{W} est un groupe alkyle, aryle ou aromatique en C1-C12, et dans lequel X est un groupe auxiliaire qui est lié par un atome de soufre.

8. Procédé selon l'une des revendications 5 à 7, **caractérisé par** le précurseur de chaîne occidentale ayant une structure choisie parmi R_{W}-CH₂-CH₂-CH₂-C(O)-X, R_{W}-CH₂-CH₂-C(CH₃)=CH₂-C(O)-X, R_{W}-CH₂-CH₂-C(CH₃)=CH-CH=C(CH₃)-C(O)-X.

9. Procédé selon l'une des revendications précédentes, **caractérisé en ce que** dans l'enzyme MxnI (SEQ ID NO: 9) le module de chargement (LM) comprenant les acides aminés 1 à 466 de SEQ ID NO: 9) est inactivé et en ajoutant un précurseur de chaîne orientale ayant la structure 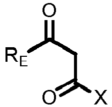 dans laquelle X est un groupe auxiliaire lié par un atome de soufre.

10. Procédé selon l'une des revendications précédentes, **caractérisé en ce que** dans l'enzyme MxnI (SEQ ID NO: 9) le module 1E est inactivé et en ajoutant un précurseur de chaîne orientale ayant la structure 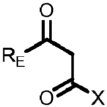 dans laquelle X est un groupe auxiliaire lié par un atome de soufre.

11. Procédé selon l'une des revendications précédentes, **caractérisé en ce que** dans l'enzyme MxnI (SEQ ID NO: 9) le module de chargement et le module 3E comprenant les acides aminés 1 à 1759 de SEQ ID NO: 9 est inactivé et en ajoutant un précurseur de chaîne orientale ayant la structure 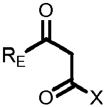 dans laquelle X est un groupe auxiliaire lié par un atome de soufre.

12. Procédé selon l'une des revendications précédentes, **caractérisé en ce que** dans l'enzyme MxnI (SEQ ID NO: 9) le module 3E, comprenant les acides aminés No. 1 à 405 de SEQ ID NO: 10 sont fonctionnellement absents et en ajoutant un précurseur de chaîne orientale ayant la structure 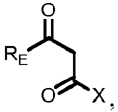 dans laquelle X est un groupe auxiliaire lié par un atome de soufre.

13. Procédé selon l'une des revendications 5 à 12, dans lequel le précurseur est choisi parmi

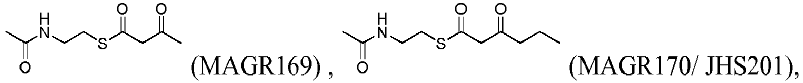

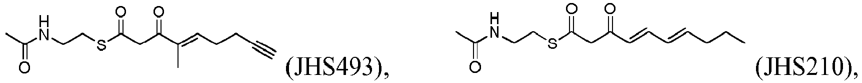

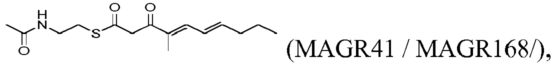

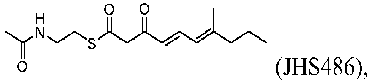

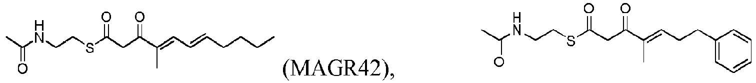

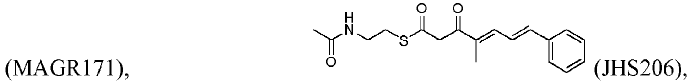

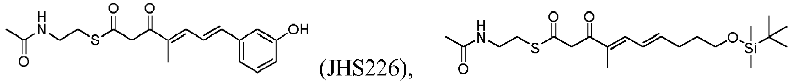

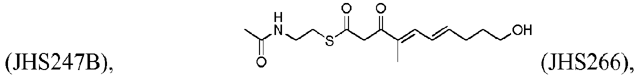

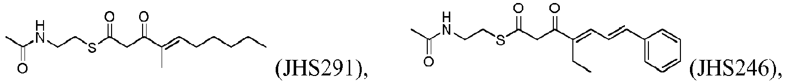

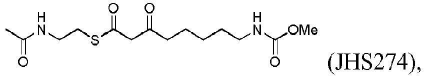

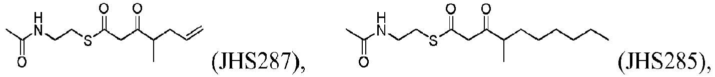

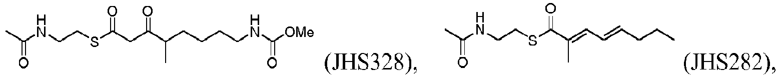

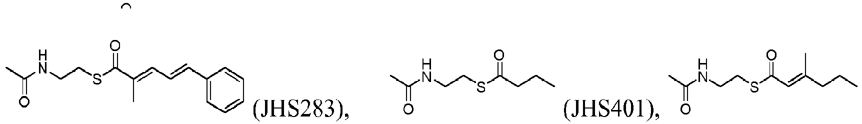

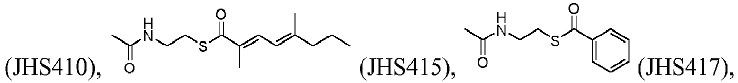

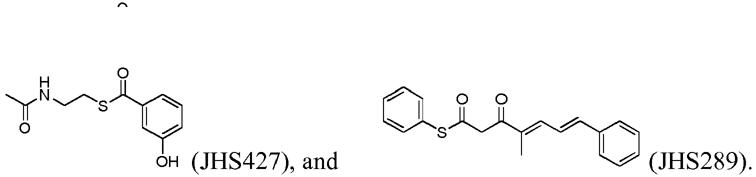

14. Procédé selon l'une des revendications précédentes, **caractérisé en ce que** dans l'enzyme MxnI, le module 4E comprenant les acides aminés No. 1 à 3052 de SEQ ID NO: 9 est inactivé et en ajoutant un précurseur de chaîne orientale ayant la structure 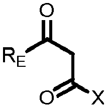 dans laquelle X est un groupe auxiliaire lié par un atome de soufre.

15. Procédé selon l'une des revendications précédentes, **caractérisé en ce que** dans l'enzyme MxnI (SEQ ID NO: 9) est fonctionnellement absent et que l'enzyme MxnJ est constituée du module 6E consistant en les acides aminés No. 3007 à 3849 de SEQ ID NO: 10.

16. Procédé selon l'une des revendications précédentes, **caractérisé en ce que** les enzymes, qui comprennent au moins MxnB sont séparés du microoganisme et le composé est produit par les enzymes dans une réaction sans cellules.
